# EUROPEAN PATENT APPLICATION

(11) **EP 4 837 923 A2**
(43) Date of publication of application: **11.11.2026**
(21) Application number: 26188168.4
(22) Date of filing: 08.05.2020
(51) Int. Cl.: C07K 16/40

(54) **ANTIBODY DRUG CONJUGATES**

(30) Priority: 10.05.2019 US 201962846494 P; 31.05.2019 US 201962855367 P; 23.12.2019 US 201962952768 P; 28.04.2020 US 202063016682 P
(62) Document number(s) of the earlier application(s) in accordance with Art. 76 EPC: 20726551.3 / 3 965 891
(71) Applicant: Takeda Pharmaceutical Company Limited, Osaka-shi, Osaka 541-0045 (JP)
(72) Inventor: ENGLAND, Dylan Bradley, Cambridge, Massachusetts 02139 (US); LANGSTON, Steve P., Cambridge, Massachusetts 02139 (US); LEE, Hong Myung, Cambridge, Massachusetts 02139 (US); MA, Liting, Cambridge, Massachusetts 02139 (US); SHI, Zhan, Cambridge, Massachusetts 02139 (US); VYSKOCIL, Stepan, Cambridge, Massachusetts 02139 (US); WANG, Jianing, Cambridge, Massachusetts 02139 (US); XU, He, Cambridge, Massachusetts 02139 (US); NISHIMOTO, Yutaka, Osaka-shi, Osaka 541-0045 (JP); ISHII, Yumiko, Osaka-shi, Osaka 541-0045 (JP)
(74) Representative: Harris, Jennifer Lucy

(57) **Abstract**

The present disclosure provides antibody drug conjugates comprising STING modulators. Also provided are compositions comprising the antibody drug conjugates. The compounds and compositions are useful for stimulating an immune response in a subject in need thereof.

## Description

### REFERENCE TO SEQUENCE LISTING SUBMITTED ELECTRONICALLY

The content of the electronically submitted sequence listing (Name: 3817_053PC03_Seqlisting_ST25; Size: 7,104 bytes and Date of Creation: April 22, 2020) is incorporated herein by reference in its entirety.

### FIELD

The present disclosure provides antibody drug conjugates comprising STING modulators. Also provided are compositions comprising the antibody drug conjugates. The compounds and compositions are useful for stimulating an immune response in a subject in need thereof.

### BACKGROUND

Antibody drug conjugates (ADCs), a rapidly growing class of targeted therapeutics, represent a promising new approach toward improving the selectivity and cytotoxic activity of drugs. These therapeutic agents are comprised of an antibody (or antibody fragment) that can be linked to a payload drug to form an immunoconjugate. The antibody directs the ADC to bind to the targeted cell. The ADC can then be internalized and release its payload which provides treatment for the cell. As the ADC is directed to its targeted cell, the side effects of conjugated drugs may be lower than those encountered when systematically administering the same agent.

The adaptor protein STING (Stimulator of Interferon Genes) has been shown to play a role in the innate immune system. Activation of the STING pathway triggers an immune response that results in generation of specific killer T-cells that shrink tumors and can provide long-lasting immunity so the tumors do not recur. The activated STING pathway also contributes to the antiviral response by producing antiviral and pro-inflammatory cytokines that fight the virus and mobilize both the innate and adaptive immune systems, ultimately resulting in long-lasting immunity against the pathogenic virus. The potential therapeutic benefits of enhancing both innate and adaptive immune responses make STING an attractive target for drug discovery. Cyclic dinucleotides may function as STING agonists and are being tested in clinical trials. However, their anionic properties make them poorly membrane permeable, which may limit their ability to engage STING inside the cell, often resulting in unwanted distribution of these compounds within the bloodstream.

There is still a need for new STING agonists as well as improved methods for delivering them to the targeted cell.

### SUMMARY

In a first aspect, the present disclosure provides a compound of formula (I): 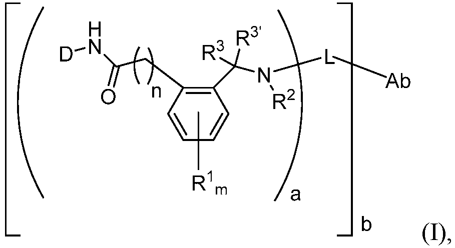 or a pharmaceutically acceptable salt thereof, wherein:
a is an integer from 1 to 20;
b is an integer from 1 to 20;
m is 0, 1, 2, 3, or 4;
n is 0 or 1;
D-NH- is a portion of an amino-substituted compound, wherein the amino-substituted compound has the formula D-NH₂;
each R¹ is independently selected from C₁-C₄alkyl, O-C₁-C₄alkyl, and halogen;
R² is selected from C₁-C₄alkyl and -(CH₂CH₂O)ₛ-CH₃; wherein s is an integer from 1 to 10;
R³ and R^{3'} are each independently selected from hydrogen and C₁-C₃alkyl;
L is a cleavable linker; and
Ab is an antibody, antibody fragment or an antigen-binding fragment.

In a first embodiment of the first aspect, a is an integer from 1 to 4; b is an integer from 1 to 10; and m is 0.

In a second embodiment of the first aspect, a is an integer from 1 to 4; m is 0; n is 0; and R³ and R^{3'} are each hydrogen.

In a third embodiment of the first aspect, L is 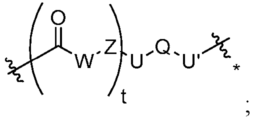 wherein:
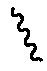 is the point of attachment to the nitrogen atom;
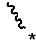 is the point of attachment to Ab;
t is an integer from 1 and 10;
W is absent or a self-immolative group;
Z is absent or a peptide of 2 to 5 amino acids;
U and U' are independently absent or a spacer; and
Q is a heterobifunctional group;
provided that W and Z are not both absent.

In a fourth embodiment of the first aspect, W is a self-immolative group selected from 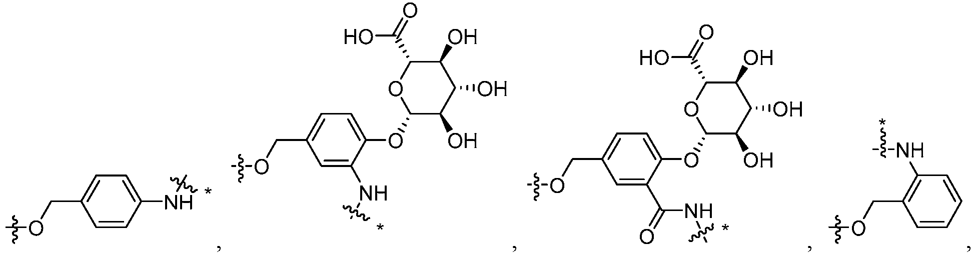 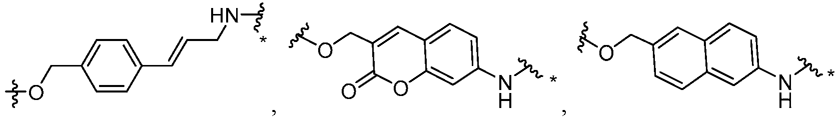 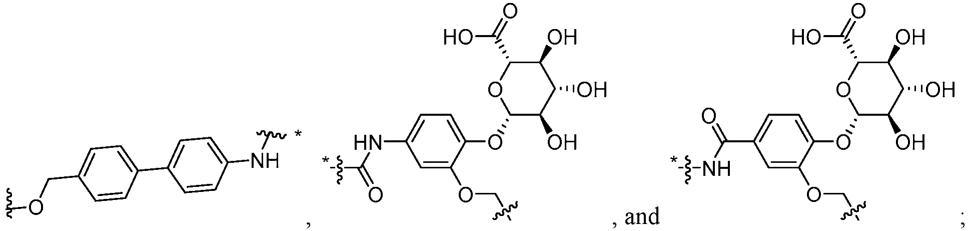 wherein:
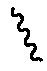 is the point of attachment to the carbonyl group; and
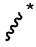 is the point of attachment to Z.

In a fifth embodiment of the first aspect, W is selected from

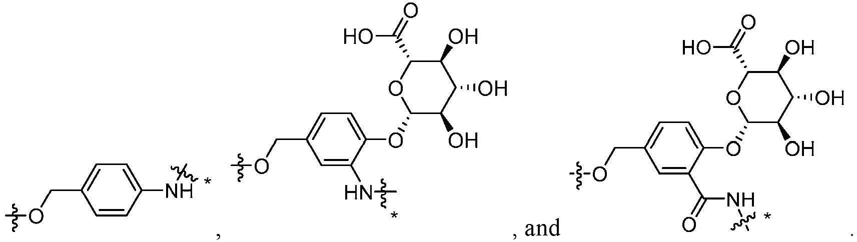

In a sixth embodiment of the first aspect, W is

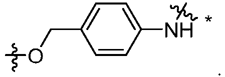

In a seventh embodiment of the first aspect, Z is a peptide capable of being enzymatically cleaved. In an eighth embodiment, Z is cathepsin cleavable. In a ninth embodiment, Z is a two-amino acid peptide selected from Val-Cit, Cit-Val, Val-Ala, Ala-Val, Phe-Lys, and Lys-Phe. In a tenth embodiment of the first aspect, Z is Val-Ala or Ala-Val.

In an eleventh embodiment of the first aspect, U and U' are independently absent or selected from 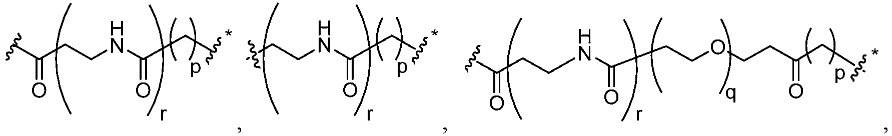 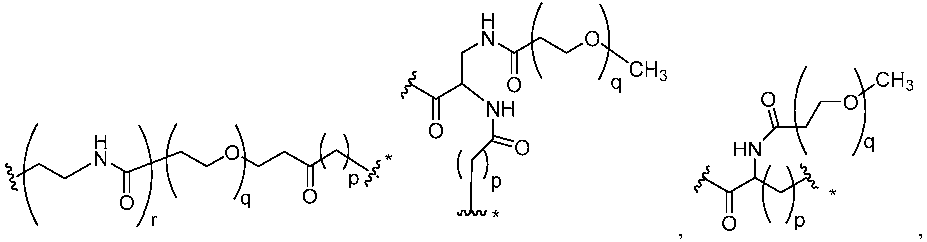 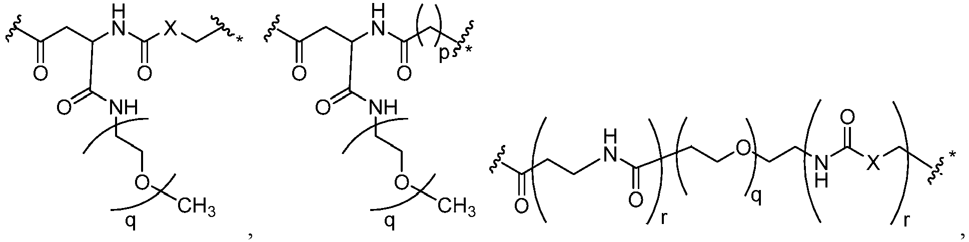 and 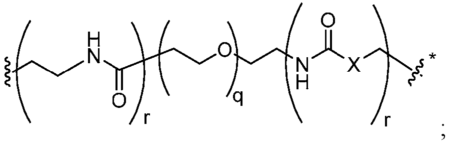 wherein:
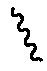 is the point of attachment to Z;
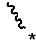 is the point of attachment to Q;
p is an integer from 1 to 6;
q is an integer from 1 to 20;
X is O or -CH₂-; and
each r is independently 0 or 1.

In a twelfth embodiment of the first aspect, U' is absent and U is

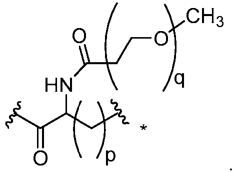

In a thirteenth embodiment of the first aspect, Q is a heterobifunctional group which is capable of being attached to Ab through chemical or enzyme-mediated conjugation. In aeleventh fourteenth embodiment, Q is selected from 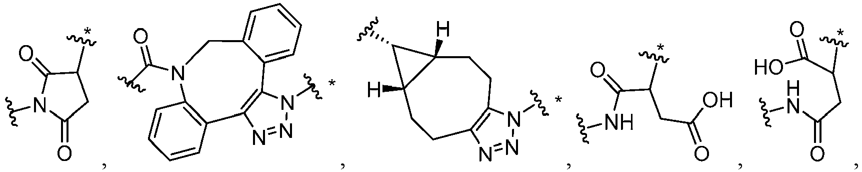 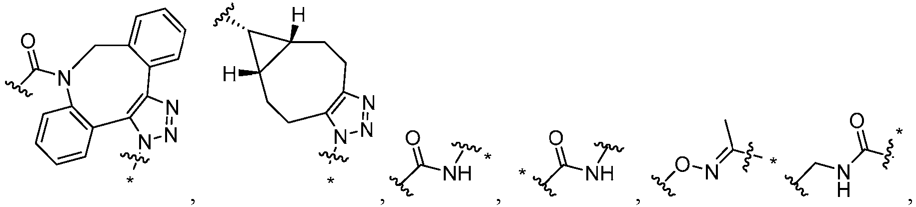 and 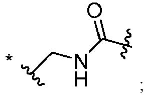 wherein
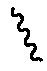 is the point of attachment to U or, when U is absent, the point of attachment to Z; and
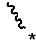 is the point of attachment to U', or, when U' is absent, the point of attachment to Ab.

In a fifteenth embodiment of the first aspect, the present disclosure provides a compound of formula (I), or a pharmaceutically acceptable salt thereof, wherein L is: 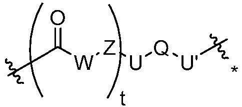 and wherein t is 1; W is absent or a self-immolative group; and Z is absent or a peptide of 2 amino acids.

In a sixteenth embodiment of the first aspect, the present disclosure provides a compound of formula (I), or a pharmaceutically acceptable salt thereof, wherein R² is -CH₃, or - (CH₂CH₂O)ₛ-CH₃ and s is an integer from 1 to 10.

In a seventeenthh embodiment of the first aspect, the present disclosure provides a compound of formula (I), or a pharmaceutically acceptable salt thereof, wherein D-NH₂ is an amino-substituted compound that modulates STING activity. In an eighteenth embodiment of the first aspect, the amino-substituted compound that modulates STING activity comprises a guanine or adenine derivative.

In a nineteenth embodiment the amino-substituted compound that modulates STING activity is a compound of formula (II): 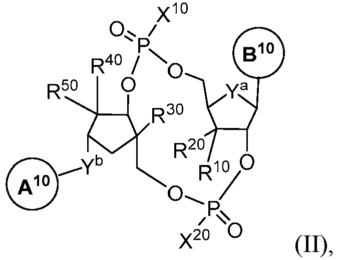 or a pharmaceutically acceptable salt thereof, wherein:
X¹⁰ is SH or OH;
X²⁰ is SH or OH;
Y^{a} is O, S, or CH₂;
Y^{b} is O, S, NH, or NR^{a}, wherein R^{a} is C₁-C₄alkyl;
R¹⁰ is hydrogen, fluoro, OH, NH₂, OR^{b}, or NHR^{b};
R²⁰ is hydrogen or fluoro;
R³⁰ is hydrogen; R⁴⁰ is hydrogen, fluoro, OH, NH₂, OR^{b}, or NHR^{b}; or R³⁰ and R⁴⁰ are taken together to form CH₂O;
R⁵⁰ is hydrogen or fluoro;
R^{b} is C₁-C₆alkyl, halo(C₁-C₆)alkyl, or C₃-C₆cycloalkyl;
Ring A¹⁰ is an optionally substituted 5 or 6-membered monocyclic heteroaryl ring containing 1-4 heteroatoms selected from N, O, or S, or an optionally substituted 9 or 10 membered bicyclic heteroaryl ring containing 1-5 heteroatoms selected from N, O, or S; wherein ring A¹⁰ comprises at least one N atom in the ring, and wherein Y^{b} is attached to a carbon atom of ring A¹⁰; and
Ring B¹⁰ is an optionally substituted 9 or 10-membered bicyclic heteroaryl ring containing from 2 to 5 heteroatoms selected from N, O, or S; wherein ring B¹⁰ comprises at least two N atoms in the ring;
provided that either ring A¹⁰ or ring B¹⁰ is attached to the carbonyl group of formula (I) through an -NH- group.

In a twentieth embodiment, the amino-substituted compound that modulates STING activity is 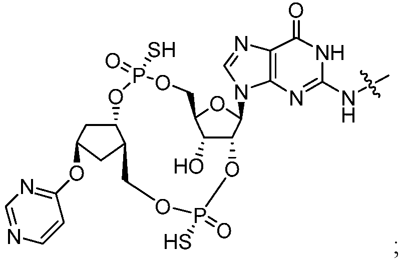 or a pharmaceutically acceptable salt thereof, wherein 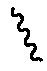 is the point of attachment to the carbonyl group of formula (I).

In twenty-first embodiment of the first aspect, the amino-substituted compound that modulates STING activity is a compound of formula (III): 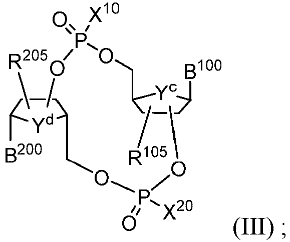 or a pharmaceutically acceptable salt thereof; wherein
X¹⁰ is SH or OH;
X²⁰ is SH or OH;
Y^{c} is O, S, or CH₂;
Y^{d} is O, S, or CH₂;
B¹⁰⁰ is a group represented by formula (**B¹-A**) or formula (**B¹-B**): 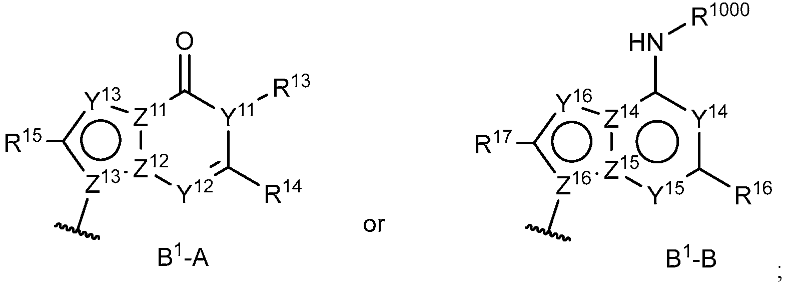
R¹³, R¹⁴, R¹⁵, R¹⁶ and R¹⁷ are each independently a hydrogen atom or a substituent;
R¹⁰⁰⁰ is hydrogen or a bond to the carbonyl group of formula (I);
Y¹¹, Y¹², Y¹³, Y¹⁴, Y¹⁵ and Y¹⁶ are each independently N or CR^{1a}, wherein R^{1a} is hydrogen or a substituent;
Z¹¹, Z¹², Z¹³, Z¹⁴, Z¹⁵ and Z¹⁶ are each independently N or C;
R¹⁰⁵ is a hydrogen atom or a substituent;
B²⁰⁰ is a group represented by formula (**B²-A**) or formula (**B²-B**): 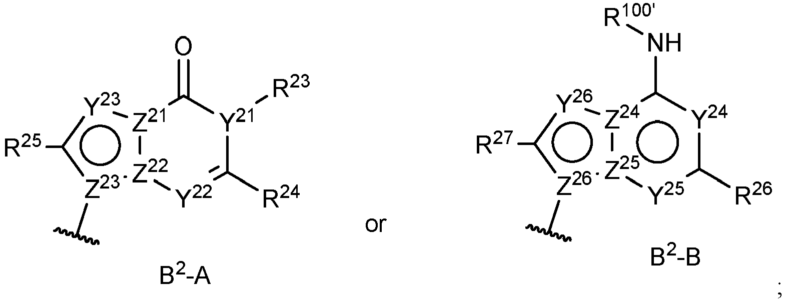
R²³, R²⁴, R²⁵, R²⁶ and R²⁷ are each independently a hydrogen atom or a substituent;
R^{100'} is hydrogen or a bond to the carbonyl group of formula (I);
Y²¹, Y²², Y²³, Y²⁴, Y²⁵ and Y²⁶ are each independently N or CR^{2a}, wherein R^{2a} is hydrogen or a substituent;
Z²¹, Z²², Z²¹, Z²⁴, Z²⁵ and Z²⁶ are each independently N or C; and
R²⁰⁵ is a hydrogen atom or a substituent; wherein R¹⁰⁵ and R²⁰⁵ are each independently attached to 2- or 3-position of the 5-membered ring they are attached to respectively;
provided that:
   one of B¹⁰⁰ or B²⁰⁰ is attached to the carbonyl group of formula (I) through an -NH-group.

In a twenty-second embodiment of the first aspect, the amino-substituted compound that modulates STING activity is a compound of formula (IIIa), 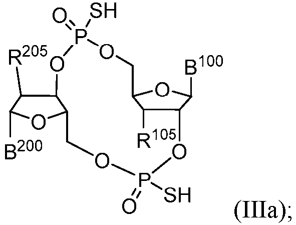 or a pharmaceutically acceptable salt thereof; wherein
B¹⁰⁰ is a group represented by formula (**B¹-A**) or formula (**B¹-B**): 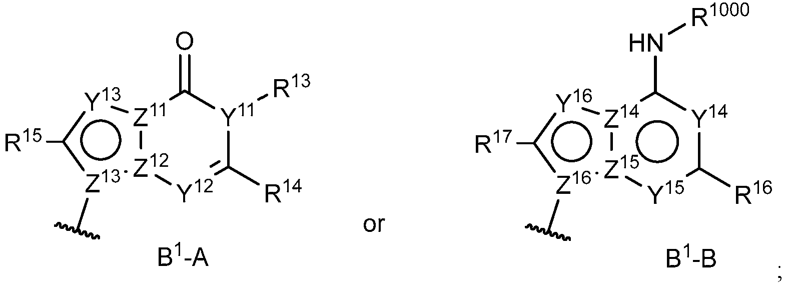
R¹³, R¹⁴, R¹⁵, R¹⁶ and R¹⁷ are each independently a hydrogen atom or a substituent;
R¹⁰⁰⁰ is hydrogen or a bond to the carbonyl group of formula (I);
Y¹¹, Y¹², Y¹³, Y¹⁴, Y¹⁵ and Y¹⁶ are each independently N or CR^{1a}, wherein R^{1a} is hydrogen or a substituent;
Z¹¹, Z¹², Z¹³, Z¹⁴, Z¹⁵ and Z¹⁶ are each independently N or C;
R¹⁰⁵ is a hydrogen atom or a substituent;
B²⁰⁰ is a group represented by formula (**B²-A**) or formula (**B²-B**): 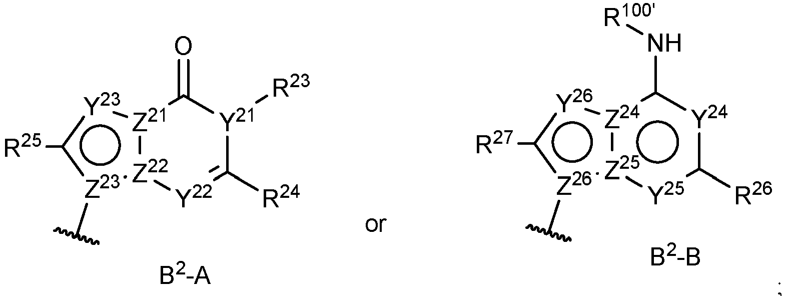
R²³, R²⁴, R²⁵, R²⁶ and R²⁷ are each independently a hydrogen atom or a substituent;
R^{100'} is hydrogen or a bond to the carbonyl group of formula (I);
Y²¹, Y²², Y²³, Y²⁴, Y²⁵ and Y²⁶ are each independently N or CR^{2a}, wherein R^{2a} is hydrogen or a substituent;
Z²¹, Z²², Z²¹, Z²⁴, Z²⁵ and Z²⁶ are each independently N or C; and
R²⁰⁵ is a hydrogen atom or a substituent; wherein R¹⁰⁵ and R²⁰⁵ are each independently attached to 2- or 3-position of the 5-membered ring they are attached to respectively;
provided that:
   one of B¹⁰⁰ or B²⁰⁰ is: 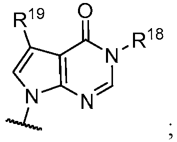
wherein:
   R¹⁸ is hydrogen or C₁₋₆ alkyl; and
   R¹⁹ is a halogen atom;
   and the other is attached to the carbonyl group of formula (I) through an -NH- group.

In a twenty-third embodiment of the first aspect, the amino-substituted compound that modulates STING activity is: 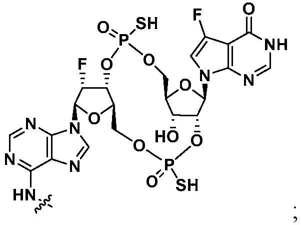 or a pharmaceutically acceptable salt thereof, wherein 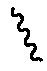 is the point of attachment to the carbonyl group of formula (I).

In a second aspect, the present disclosure provides a compound of formula (IV): 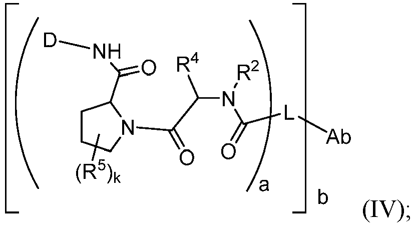 or a pharmaceutically acceptable salt thereof, wherein:
a is an integer from 1 to 20;
b is an integer from 1 to 20;
k is 0, 1, 2, or 3;
m is 0, 1, 2, 3, or 4;
D-NH- is a portion of an amino-substituted compound, where the amino-substituted compound has the formula D-NH₂;
R² is selected from H, C₁-C₄alkyl and -(CH₂CH₂O)ₛ-CH₃; wherein s is an integer from 1 and 10;
R⁴ is selected from hydrogen and any naturally occurring amino acid side chain;
R⁵ is selected from C₁-C₄alkyl, and O-C₁-C₄alkyl;
L is a cleavable linker; and
Ab is an antibody, antibody fragment or an antigen-binding fragment.

In a first embodiment of the second aspect, L is 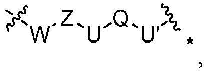 wherein:
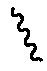 is the point of attachment to the carbonyl group;
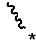 is the point of attachment to Ab;
W is a self-immolative group;
Z is absent or a peptide of 2 to 5 amino acids; and
U and U' are independently absent or a spacer; and
Q is a heterobifunctional group.

In a third aspect, the present disclosure provides a compound of formula (V): 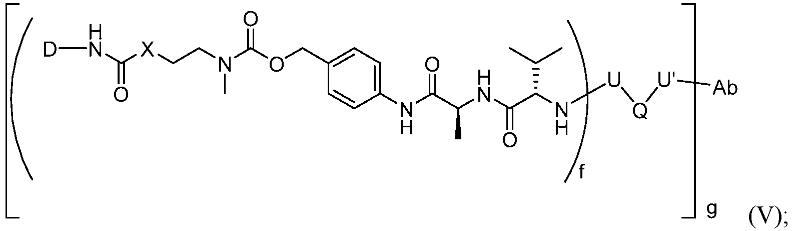 or a pharmaceutically acceptable salt thereof, wherein:
D-NH- is 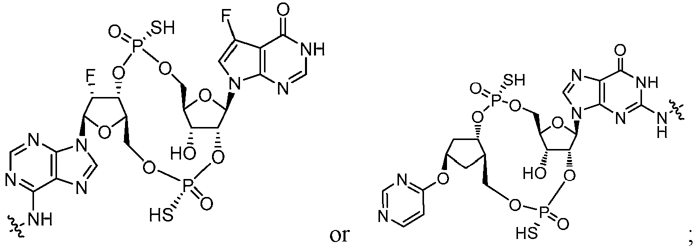
X is O or CH₂;
f is an integer from 1 to 10;
g is an integer from 1 to 20;
U and U' are independently absent or a spacer;
Q is a heterobifunctional group; and
Ab is an antibody Ab is an antibody, antibody fragment or an antigen-binding fragment.

In a first embodiment of the third aspect, X is O.

In a fourth aspect, the present disclosure provides a compound of formula (VI): 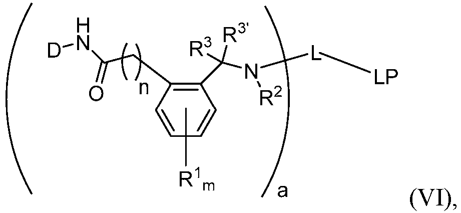 or a pharmaceutically acceptable salt thereof, wherein:
a is an integer from 1 to 20;
m is 0, 1, 2, 3, or 4;
n is 0 or 1;
D-NH- is a portion of an amino-substituted compound, wherein the amino-substituted compound has the formula D-NH₂;
each R¹ is independently selected from C₁-C₄alkyl, O-C₁-C₄alkyl, and halogen;
R² is selected from C₁-C₄alkyl and -(CH₂CH₂O)ₛ-CH₃; wherein s is an integer from 1 to 10;
R³ and R^{3'} are each independently selected from hydrogen and C₁-C₃alkyl;
L is a cleavable linker; and
LP is a lipid.

In a first embodiment of the fourth aspect, the lipid is cholesterol or a phospholipid. In a second embodiment of the fourth aspect, the lipid is a phospholipid. In a third embodiment of the fourth aspect, the phospholipid is selected from a phosphatidylcholine, a phosphatidylgycerol, a phosphatidic acid, a phosphatidylethanolamine, a phosphatidylserine, a sphingomyelin, a soybean phospholipid, and an egg yolk phospholipid. In a fifth embodiment of the fourth aspect, the phospholipid is a phosphatidylethanolamine, wherein the phosphadtidylethanolamine is 1,2-distearoyl-sn-glycero-3-phosphorylethanolamine.

In a sixth embodiment of the fourth aspect, the present disclosure provides a lipid complex comprising a compound of formula (VI), or a pharmaceutically acceptable salt thereof. In a seventh embodiment, the lipid complex is in the form of a liposome.

In an eighth embodiment of the fourth aspect, the lipid complex further comprises one or more phospholipids. In a ninth embodiment, the one or more phospholipids are selected from a phosphatidylcholine, a phosphatidylgycerol, a phosphatidic acid, a phosphatidylethanolamine, a phosphatidylserine, a sphingomyelin, a soybean phospholipid, and an egg yolk phospholipid. In a tenth embodiment, the phospholipid is a phosphatidylcholine, wherein the phosphatidylcholine is 1,2-distearoyl-*sn*-glycero-3-phosphocholine.

In an eleventh embodiment of the fourth aspect, the lipid complex further comprises a fatty alcohol and/or cholesterol.

In a twelfth embodiment of the fourth aspect, the lipid complex further comprises at least one PEGylated lipid. In a thirteenth embodiment, the PEGylated lipid is selected from a PEGylated phospholipid and PEGylated diacylglycerol. In a fourteenth embodiment, the PEGylated lipid is N-(carbonyl-methoxypolyethyleneglycol 2000)-1,2-distearoyl-*sn*-glycero-3-phosphoethanolamine.

In a fifth aspect, the present disclosure provides a pharmaceutical composition comprising a compound or complex described herein, or a pharmaceutically acceptable salt thereof, and one or more pharmaceutically acceptable carriers.

In a sixth aspect, the present disclosure provides a method of treating cancer in a subject in need thereof, the method comprising administering to the subject a pharmaceutically acceptable amount of a compound or complex described herein.

In a seventh aspect, the present disclosure provides a method for stimulating an immune response in a subject in need thereof, the method comprising administering to the subject a pharmaceutically acceptable amount of a compound or complex described herein.

### BRIEF DESCRIPTION OF THE FIGURES

Figure 1 depicts the preparation of Ab-STING agonist conjugates via stochastic cysteine conjugation.
Figure 2 depicts the preparation of Ab-STING agonist conjugates via transglutaminase conjugation.
Figure 3 depicts the preparation of Ab-STING agonist conjugates via transglutaminase conjugation.
Figure 4 depicts the preparation of Ab-STING agonist conjugates via sortase conjugation. (SEQ ID3 and SEQ ID 4)
Figure 5 depicts the payload release of ADC-1 in the tritosomal assay.
Figure 6 depicts the payload release of ADC-3 in the tritosomal assay.
Figure 7 depicts the payload release of ADC-9 in the tritosomal assay.
Figure 8 depicts plasma PK of ADC-1 in GCC expressing CT26 bearing Balb/C mice (0.1 mg/kg payload dose).
Figure 9 depicts plasma PK of ADC-8 in GCC expressing CT26 bearing Balb/C mice (0.055 mg/kg payload dose, released CDN BLQ).
Figure 10 depicts plasma PK of ADC-9 in GCC expressing CT26 bearing Balb/C mice (0.05 mg/kg payload dose, released CDN BLQ).
Figure 11 depicts efficacy of ADC-1 in GCC expressing CT26 bearing Balb/C mice (payload doses are shown).
Figure 12 depicts efficacy of ADC-5 in GCC expressing CT26 bearing Balb/C mice (payload doses are shown).
Figure 13 depicts efficacy of ADC-8 in GCC expressing CT26 bearing Balb/C mice (payload doses are shown).
Figure 14 depicts efficacy of ADC-9 in GCC expressing CT26 bearing Balb/C mice (payload doses are shown).

### DETAILED DESCRIPTION

Unless defined otherwise, all technical and scientific terms used herein have the same meaning as is commonly understood by one of skill in the art to which this disclosure belongs. All patents and publications referred to herein are incorporated by reference in their entireties.

The singular forms "a," "an," and "the" include plural referents unless the context dictates otherwise.

As used herein, the term "or" is a logical disjunction (i.e., and/or) and does not indicate an exclusive disjunction unless expressly indicated such as with the terms "either," "unless," "alternatively," and words of similar effect.

As used herein, the term "about" refers to ± 10%.

### Antibody Drug Conjugates

In some embodiments, the present disclosure provides a compound of formula (I), 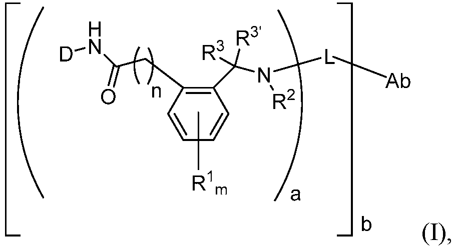 or a pharmaceutically acceptable salt thereof, wherein:
a is an integer from 1 to 20;
b is an integer from 1 to 20;
m is 0, 1, 2, 3, or 4;
n is 0 or 1;
D-NH- is a portion of an amino-substituted compound, wherein the amino-substituted compound has the formula D-NH₂;
each R¹ is independently selected from C₁-C₄alkyl, O-C₁-C₄alkyl, and halogen;
R² is selected from C₁-C₄alkyl and -(CH₂CH₂O)ₛ-CH₃; wherein s is an integer from 1 to 10;
R³ and R^{3'} are each independently selected from hydrogen and C₁-C₃alkyl;
L is a cleavable linker; and
Ab is an antibody, antibody fragment, or an antigen-binding fragment.

In some embodiments, the present disclosure provides a compound of formula (IV), 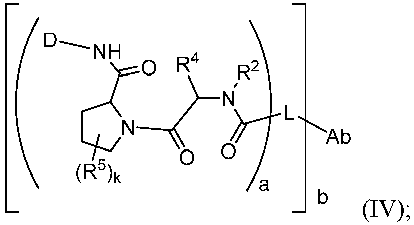 or a pharmaceutically acceptable salt thereof, wherein:
a is an integer from 1 to 20;
b is an integer from 1 to 20;
k is 0, 1, 2, or 3;
m is 0, 1, 2, 3, or 4;
D-NH- is a portion of an amino-substituted compound, where the amino-substituted compound has the formula D-NH₂;
R² is selected from H, C₁-C₄alkyl and -(CH₂CH₂O)ₛ-CH₃; wherein s is an integer from 1 and 10;
R⁴ is selected from hydrogen and any naturally occurring amino acid side chain;
R⁵ is selected from C₁-C₄alkyl, and O-C₁-C₄alkyl;
L is a cleavable linker; and
Ab is an antibody, antibody fragment or an antigen-binding fragment.

In some embodiments, the present disclosure provides a compound of formula (V): 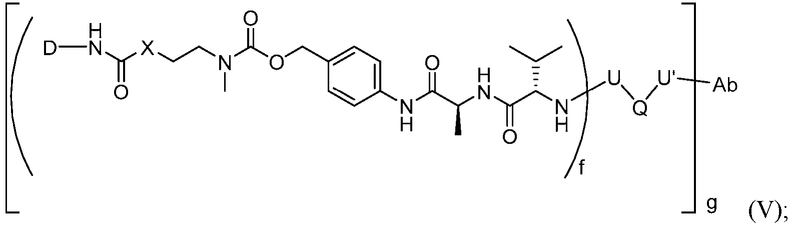 or a pharmaceutically acceptable salt thereof, wherein:
D-NH is 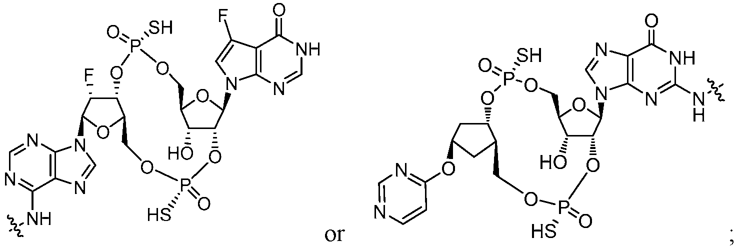
X is O or CH₂;
f is an integer from 1 to 10;
g is an integer from 1 to 20;
U and U' are independently absent or a spacer;
Q is a heterobifunctional group; and
Ab is an antibody, antibody fragment or an antigen-binding fragment.

In compounds of formula (I) and (IV), the amino substituted compound D-NH₂ can be any amino-containing drug. In certain embodiments, D-NH₂ is an immunomodulator. As used herein, the term "immunomodulator," refers to a compound that produces an immune response in a subject in need thereof. Examples of immunomodulators include, but are not limited to, IDO inhibitors, MEK inhibitors, STING modulators, CCR4 antagonists, PD-1/PD-L1 inhibitors, TLR7/8 agonists, and the like. In some embodiments, D-NH₂ is a STING modulator. In some embodiments, D-NH₂ comprises a guanine or adenine derivative. In some embodiments, the STING modulator is a cyclic dinucleotide, or a cyclic dinucleotide-like compound (each, a CDN) of the formula:

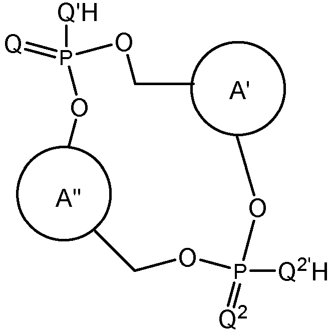

wherein A' and A" are each independently nucleosides or synthetic analogs thereof; and each of Q, Q', Q², and Q^{2'} are independently oxygen or sulfur. In some embodiments, Q^{'} and Q^{2'} are sulfur. Examples of CDN's, including cyclic dinucleotides and cyclic dinucleotide-like compounds that are within the scope of the present disclosure include, but are not limited to, those disclosed within US 9,724,408, US 10,106,574, US 9,549,944, US 9,695,212, US 9,718,848, US 9,994,607, US 10,047,115, US20180230178, US20180064745, US2018028132, US20180002369, US20180186828, US20190016750, US20180162899, US20180369268, US2018273578, US20180092937, WO2017/027646, and WO2018/100558; as well as compounds such as c-di-GMP, 2'3'-cGAMP and SB 11285 (Spring Bank Pharmaceuticals).

In certain embodiments the STING modulator is a compound of Formula (II): 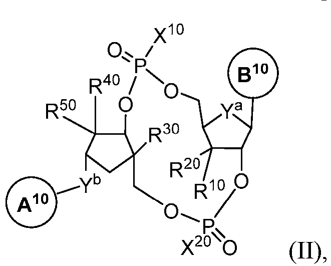 or a pharmaceutically acceptable salt thereof, wherein:
X¹⁰ is -SH or -OH;
X²⁰ is -SH or -OH;
Y^{a} is -O-, -S-, or -CH₂-;
Y^{b} is -O-, -S-, -NH-, or -NR^{a}-, wherein R^{a} is C₁-C₄alkyl;
R¹⁰ is hydrogen, fluoro, -OH, -NH₂, -OR^{b}, or -NHR^{b};
R²⁰ is hydrogen or fluoro;
R³⁰ is hydrogen; R⁴⁰ is hydrogen, fluoro, -OH, -NH₂, -OR^{b}, or -NHR^{b}; or R³⁰ and R⁴⁰ are taken together to form -CH₂O-;
R⁵⁰ is hydrogen or fluoro;
R^{b} is C₁-C₆alkyl, halo(C₁-C₆)alkyl, or C₃-C₆cycloalkyl;
Ring A¹⁰ is an optionally substituted 5- or 6-membered monocyclic heteroaryl ring containing 1-4 heteroatoms selected from N, O, or S, or an optionally substituted 9- or 10-membered bicyclic heteroaryl ring containing 1-5 heteroatoms selected from N, O, or S; wherein ring A¹⁰ comprises at least one N atom in the ring, and wherein Y^{b} is attached to a carbon atom of ring A¹⁰; and
Ring B¹⁰ is an optionally substituted 9- or 10-membered bicyclic heteroaryl ring containing 2-5 heteroatoms selected from N, O, or S; wherein ring B¹⁰ comprises at least two N atoms in the ring.

As described herein, ring A¹⁰ and ring B¹⁰ can contain one or more substituents and thus can be optionally substituted. Suitable substituents on the unsaturated carbon atom of a heteroaryl group include, and are generally selected from, -halo, -NO₂, -CN, -R⁺, -C(R⁺)=C(R⁺)₂, -C≡C-R⁺, -OR⁺, -SR°, -S(O)R°, -SO₂R°, -SO₃R⁺, -SO₂N(R⁺)₂, -N(R⁺)₂, -NR⁺C(O)R⁺, -NR⁺C(S)R⁺, -NR⁺C(O)N(R⁺)₂, -NR⁺C(S)N(R⁺)₂, -N(R⁺)C(=NR⁺)-N(R⁺)₂, -N(R⁺)C(=NR⁺)-R°, -NR⁺CO₂R⁺, -NR⁺SO₂R°, -NR⁺SO₂N(R⁺)₂, -O-C(O)R⁺, -O-CO₂R⁺, -OC(O)N(R⁺)₂, -C(O)R⁺, -C(S)R°, -CO₂R⁺, -C(O)-C(O)R⁺, -C(O)N(R⁺)₂, -C(S)N(R⁺)₂, -C(O)N(R⁺)-OR⁺, -C(O)N(R⁺)C(=NR⁺)-N(R⁺)₂, -N(R⁺)C(=NR⁺)-N(R⁺)-C(O)R⁺, -C(=NR⁺)-N(R⁺)₂, -C(=NR⁺)-OR⁺, -N(R⁺)-N(R⁺)₂, -C(=NR⁺)-N(R⁺)-OR⁺, -C(R°)=N-OR⁺, -P(O)(R⁺)₂, -P(O)(OR⁺)₂, -O-P(O)-OR⁺, and -P(O)(NR⁺)-N(R⁺)₂, wherein R⁺, independently, is hydrogen or an optionally substituted aliphatic, aryl, heteroaryl, cycloaliphatic, or heterocyclyl group, or two independent occurrences of R⁺ are taken together with their intervening atom(s) to form an optionally substituted 5-7-membered aryl, heteroaryl, cycloaliphatic, or heterocyclyl. In some embodiments, R⁺, independently, is hydrogen , C₁₋₆ aliphatic, or C₃₋₆ cycloaliphatic. Each R° is, independently, an optionally substituted aliphatic, aryl, heteroaryl, cycloaliphatic, or heterocyclyl group.

As detailed above, in some embodiments, two independent occurrences of R⁺ (or any other variable similarly defined in the specification and claims herein), are taken together with their intervening atom(s) to form a monocyclic or bicyclic ring selected from 3-13-membered cycloaliphatic, 3-12-membered heterocyclyl having 1-5 heteroatoms independently selected from nitrogen, oxygen, or sulfur, 6-10-membered aryl, or 5-10-membered heteroaryl having 1-5 heteroatoms independently selected from nitrogen, oxygen, or sulfur.

In some embodiments the STING modulator is a compound of formula (IIA): 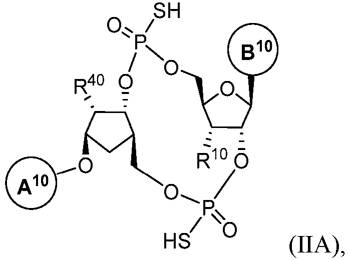 or a pharmaceutically acceptable salt thereof, wherein R¹⁰ and R⁴⁰ are each independently hydrogen, fluoro, -OH, or -OCH₂CF₃ and rings A¹⁰ and B¹⁰ are as defined for the compound of formula (II), provided that either ring A¹⁰ or ring B¹⁰ is attached to the carbonyl group of compound (I), (IV) or (V) parent molecular moiety through an -NH- group.

In some embodiments, ring A¹⁰ is an optionally substituted 6-membered monocyclic heteroaryl ring containing 1, 2, or 3 nitrogen atoms.

In some embodiments, ring B¹⁰ is: 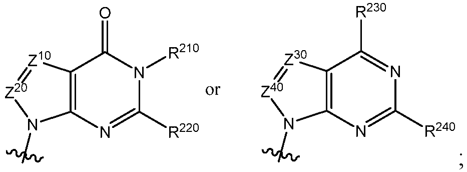 wherein:
Z¹⁰, Z²⁰, Z³⁰, and Z⁴⁰ are each independently N or CR²⁰⁰;
R²¹⁰ is hydrogen or C₁-C₆alkyl, halo(C₁-C₆)alkyl, or C₃-C₆cycloalkyl;
R²³⁰ is hydrogen or -NH₂; and
R²⁰⁰, R²²⁰, and R²⁴⁰ are each independently hydrogen, halogen, -OH, -NH₂, -CN, C₁-C₆alkyl, halo(C₁-C₆)alkyl, or C₃-C₆cycloalkyl.

In some embodiments the STING modulator is: 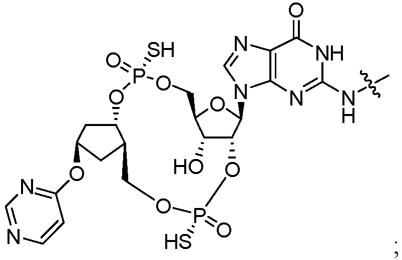 or a pharmaceutically acceptable salt thereof, wherein 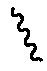 is the point of attachment to the carbonyl group of the parent molecular moiety.

In certain embodiments the STING modulator is a cyclic dinucleotide of formula (X): 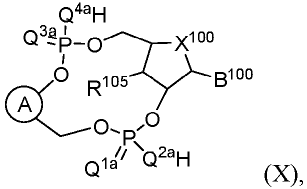 or a pharmaceutically acceptable salt thereof, wherein:

the partial structure represented by formula (A-1): 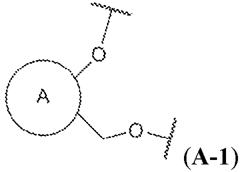 is a partial structure represented by formula (Z-A), or formula (Z-B): 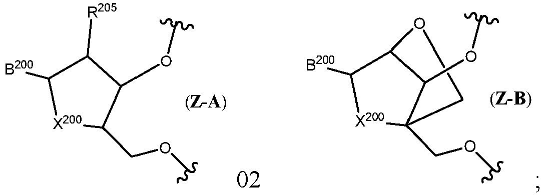
R¹⁰⁵ and R²⁰⁵ are each independently a hydroxy group or a halogen atom;
B¹⁰⁰ is a group represented by formula (**B¹-A**) or formula (**B¹-B**): 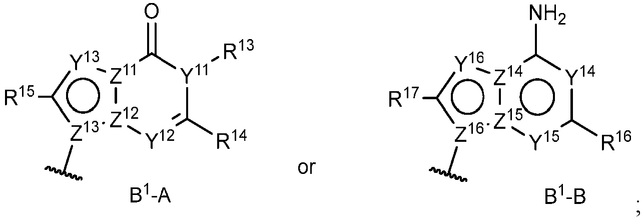
R¹³, R¹⁴, R¹⁵, R¹⁶ and R¹⁷ are each independently a hydrogen atom or a substituent;
Y¹¹, Y¹², Y¹³, Y¹⁴, Y¹⁵ and Y¹⁶ are each independently N or CR^{1a};
Z¹¹, Z¹², Z¹³, Z¹⁴, Z¹⁵ and Z¹⁶ are each independently N or C;
R^{1a} is a hydrogen atom or a substituent;
B²⁰⁰ is a group represented by formula (**B²-A**) or formula (**B²-B**): 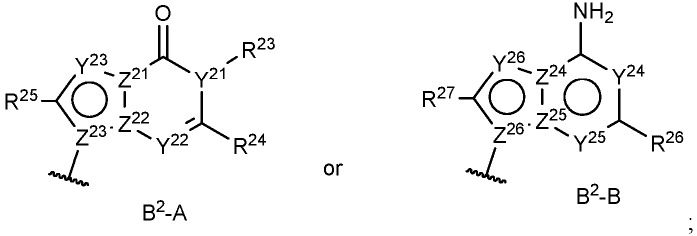
R²³, R²⁴, R²⁵, R²⁶ and R²⁷ are each independently a hydrogen atom or a substituent;
Y²¹, Y²², Y²³, Y²⁴, Y²⁵ and Y²⁶ are each independently N or CR^{2a};
Z²¹, Z²², Z²¹, Z²⁴, Z²⁵ and Z²⁶ are each independently N or C;
R^{2a} is a hydrogen atom or a substituent;
X¹ and X² are each independently an oxygen atom or a sulfur atom; and
Q^{1a}, Q^{2a}, Q3a and Q^{4a} are each independently an oxygen atom or a sulfur atom; and
one of B¹⁰⁰ or B²⁰⁰ is attached to the 5-membered ring of the parent structure through an -NH- group.

In another embodiment, one of B¹⁰⁰ or B²⁰⁰ is: 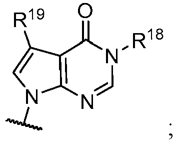 wherein:
R¹⁸ is hydrogen or C₁₋₆ alkyl; and the other is attached to the 5-membered ring of the parent structure through an -NH- group; and
R¹⁹ a halogen atom; and the other is attached to the carbonyl group of formula (I) through an -NH- group.

In another aspect, the present disclosure provides an antibody drug conjugate as described herein.

As described herein, certain R groups are selected from hydrogen and a substituent. Examples of the substituent include a halogen atom, a cyano group, a nitro group, an optionally substituted hydrocarbon group, an optionally substituted heterocyclic group, an acyl group, an optionally substituted amino group, an optionally substituted carbamoyl group, an optionally substituted thiocarbamoyl group, an optionally substituted sulfamoyl group, an optionally substituted hydroxy group, an optionally substituted sulfanyl (SH) group and an optionally substituted silyl group.

As used herein, the In the present specification, examples of the "hydrocarbon group" (including "hydrocarbon group" of "optionally substituted hydrocarbon group") include a C₁₋₆ alkyl group, a C₂₋₆ alkenyl group, a C₂₋₆ alkynyl group, a C₃₋₁₀ cycloalkyl group, a C₃₋₁₀ cycloalkenyl group, a C₆₋₁₄ aryl group and a C₇₋₁₆ aralkyl group.

In the present specification, examples of the "optionally substituted hydrocarbon group" include a hydrocarbon group optionally having substituent(s) selected from the following Substituent Group A, which includes:
(1) a halogen atom,
(2) a nitro group,
(3) a cyano group,
(4) an oxo group,
(5) a hydroxy group,
(6) an optionally halogenated C₁₋₆ alkoxy group,
(7) a C₆₋₁₄ aryloxy group (e.g., phenoxy, naphthoxy),
(8) a C₇₋₁₆ aralkyloxy group (e.g., benzyloxy),
(9) a 5- to 14-membered aromatic heterocyclyloxy group (e.g., pyridyloxy),
(10) a 3- to 14-membered non-aromatic heterocyclyloxy group (e.g., morpholinyloxy, piperidinyloxy),
(11) a C₁₋₆ alkyl-carbonyloxy group (e.g., acetoxy, propanoyloxy),
(12) a C₆₋₁₄ aryl-carbonyloxy group (e.g., benzoyloxy, 1-naphthoyloxy, 2-naphthoyloxy),
(13) a C₁₋₆ alkoxy-carbonyloxy group (e.g., methoxycarbonyloxy, ethoxycarbonyloxy, propoxycarbonyloxy, butoxycarbonyloxy),
(14) a mono- or di-C₁₋₆ alkyl-carbamoyloxy group (e.g., methylcarbamoyloxy, ethylcarbamoyloxy, dimethylcarbamoyloxy, diethylcarbamoyloxy),
(15) a C₆₋₁₄ aryl-carbamoyloxy group (e.g., phenylcarbamoyloxy, naphthylcarbamoyloxy),
(16) a 5- to 14-membered aromatic heterocyclylcarbonyloxy group (e.g., nicotinoyloxy),
(17) a 3- to 14-membered non-aromatic heterocyclylcarbonyloxy group (e.g., morpholinylcarbonyloxy, piperidinylcarbonyloxy),
(18) an optionally halogenated C₁₋₆ alkylsulfonyloxy group (e.g., methylsulfonyloxy, trifluoromethylsulfonyloxy),
(19) a C₆₋₁₄ arylsulfonyloxy group optionally substituted by a C₁₋₆ alkyl group (e.g., phenylsulfonyloxy, toluenesulfonyloxy),
(20) an optionally halogenated C₁₋₆ alkylthio group,
(21) a 5- to 14-membered aromatic heterocyclic group,
(22) a 3- to 14-membered non-aromatic heterocyclic group,
(23) a formyl group,
(24) a carboxy group,
(25) an optionally halogenated C₁₋₆ alkyl-carbonyl group,
(26) a C₆₋₁₄ aryl-carbonyl group,
(27) a 5- to 14-membered aromatic heterocyclylcarbonyl group,
(28) a 3- to 14-membered non-aromatic heterocyclylcarbonyl group,
(29) a C₁₋₆ alkoxy-carbonyl group,
(30) a C₆₋₁₄ aryloxy-carbonyl group (e.g., phenyloxycarbonyl, 1-naphthyloxycarbonyl, 2-naphthyloxycarbonyl),
(31) a C₇₋₁₆ aralkyloxy-carbonyl group (e.g., benzyloxycarbonyl, phenethyloxycarbonyl),
(32) a carbamoyl group,
(33) a thiocarbamoyl group,
(34) a mono- or di-C₁₋₆ alkyl-carbamoyl group,
(35) a C₆₋₁₄ aryl-carbamoyl group (e.g., phenylcarbamoyl),
(36) a 5- to 14-membered aromatic heterocyclylcarbamoyl group (e.g., pyridylcarbamoyl, thienylcarbamoyl),
(37) a 3- to 14-membered non-aromatic heterocyclylcarbamoyl group (e.g., morpholinylcarbamoyl, piperidinylcarbamoyl),
(38) an optionally halogenated C₁₋₆ alkylsulfonyl group,
(39) a C₆₋₁₄ arylsulfonyl group,
(40) a 5- to 14-membered aromatic heterocyclylsulfonyl group (e.g., pyridylsulfonyl, thienylsulfonyl),
(41) an optionally halogenated C₁₋₆ alkylsulfinyl group,
(42) a C₆₋₁₄ arylsulfinyl group (e.g., phenylsulfinyl, 1-naphthylsulfinyl, 2-naphthylsulfinyl),
(43) a 5- to 14-membered aromatic heterocyclylsulfinyl group (e.g., pyridylsulfinyl, thienylsulfinyl),
(44) an amino group,
(45) a mono- or di-C₁₋₆ alkylamino group (e.g., methylamino, ethylamino, propylamino, isopropylamino, butylamino, dimethylamino, diethylamino, dipropylamino, dibutylamino, N-ethyl-N-methylamino),
(46) a mono- or di-C₆₋₁₄ arylamino group (e.g., phenylamino),
(47) a 5- to 14-membered aromatic heterocyclylamino group (e.g., pyridylamino),
(48) a C₇₋₁₆ aralkylamino group (e.g., benzylamino),
(49) a formylamino group,
(50) a C₁₋₆ alkyl-carbonylamino group (e.g., acetylamino, propanoylamino, butanoylamino),
(51) a (C₁₋₆ alkyl)(C₁₋₆ alkyl-carbonyl) amino group (e.g., N-acetyl-N-methylamino),
(52) a C₆₋₁₄ aryl-carbonylamino group (e.g., phenylcarbonylamino, naphthylcarbonylamino),
(53) a C₁₋₆ alkoxy-carbonylamino group (e.g., methoxycarbonylamino, ethoxycarbonylamino, propoxycarbonylamino, butoxycarbonylamino, tert-butoxycarbonylamino),
(54) a C₇₋₁₆ aralkyloxy-carbonylamino group (e.g., benzyloxycarbonylamino),
(55) a C₁₋₆ alkylsulfonylamino group (e.g., methylsulfonylamino, ethylsulfonylamino),
(56) a C₆₋₁₄ arylsulfonylamino group optionally substituted by a C₁₋₆ alkyl group (e.g., phenylsulfonylamino, toluenesulfonylamino),
(57) an optionally halogenated C₁₋₆ alkyl group,
(58) a C₂₋₆ alkenyl group,
(59) a C₂₋₆ alkynyl group,
(60) a C₃₋₁₀ cycloalkyl group,
(61) a C₃₋₁₀ cycloalkenyl group, and
(62) a C₆₋₁₄ aryl group.

The number of the above-mentioned substituents in the "optionally substituted hydrocarbon group" is, for example, 1 to 5, typically1 to 3. When the number of the substituents is two or more, the respective substituents may be the same or different.

In the present specification, examples of the "heterocyclic group" (including "heterocyclic group" of "optionally substituted heterocyclic group") include (i) an aromatic heterocyclic group, (ii) a non-aromatic heterocyclic group and (iii) a 7- to 10-membered bridged heterocyclic group, each containing, as a ring-constituting atom besides carbon atom, 1 to 4 heteroatoms selected from a nitrogen atom, a sulfur atom and an oxygen atom.

In the present specification, examples of the "aromatic heterocyclic group" (including "5- to 14-membered aromatic heterocyclic group") include a 5- to 14-membered (typically 5- to 10-membered) aromatic heterocyclic group containing, as a ring-constituting atom besides carbon atom, 1 to 4 heteroatoms selected from a nitrogen atom, a sulfur atom and an oxygen atom.

Suitable examples of the "aromatic heterocyclic group" include 5- or 6-membered monocyclic aromatic heterocyclic groups such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl and the like; and
8- to 14-membered fused polycyclic (typically bi- or tricyclic) aromatic heterocyclic groups such as benzothiophenyl, benzofuranyl, benzimidazolyl, benzoxazolyl, benzisoxazolyl, benzothiazolyl, benzisothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acridinyl, phenazinyl, phenothiazinyl, phenoxazinyl and the like.

In the present specification, examples of the "non-aromatic heterocyclic group" (including "3- to 14-membered non-aromatic heterocyclic group") include a 3- to 14-membered (typically 4- to 10-membered) non-aromatic heterocyclic group containing, as a ring-constituting atom besides carbon atom, 1 to 4 heteroatoms selected from a nitrogen atom, a sulfur atom and an oxygen atom.

Suitable examples of the "non-aromatic heterocyclic group" include 3- to 8-membered monocyclic non-aromatic heterocyclic groups such as aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisooxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl and the like; and

9- to 14-membered fused polycyclic (typically bi- or tricyclic) non-aromatic heterocyclic groups such as dihydrobenzofuranyl, dihydrobenzimidazolyl, dihydrobenzoxazolyl, dihydrobenzothiazolyl, dihydrobenzisothiazolyl, dihydronaphtho[2,3-b]thienyl, tetrahydroisoquinolyl, tetrahydroquinolyl, 4H-quinolizinyl, indolinyl, isoindolinyl, tetrahydrothieno[2,3-c]pyridinyl, tetrahydrobenzazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolinyl, tetrahydroacrydinyl, tetrahydrophenazinyl, tetrahydrothioxanthenyl, octahydroisoquinolyl and the like.

In the present specification, suitable examples of the "7- to 10-membered bridged heterocyclic group" include quinuclidinyl and 7-azabicyclo[2.2.1]heptanyl.

In the present specification, examples of the "nitrogen-containing heterocyclic group" include a "heterocyclic group" containing at least one nitrogen atom as a ring-constituting atom.

In the present specification, examples of the "optionally substituted heterocyclic group" include a heterocyclic group optionally having substituent(s) selected from the above-mentioned Substituent Group A.

The number of the substituents in the "optionally substituted heterocyclic group" is, for example, 1 to 3. When the number of the substituents is two or more, the respective substituents may be the same or different.

In the present specification, examples of the "acyl group" include a formyl group, a carboxy group, a carbamoyl group, a thiocarbamoyl group, a sulfino group, a sulfo group, a sulfamoyl group and a phosphono group, each optionally having 1 or 2 substituents selected from a C₁₋₆ alkyl group, a C₂₋₆ alkenyl group, a C₃₋₁₀ cycloalkyl group, a C₃₋₁₀ cycloalkenyl group, a C₆₋₁₄ aryl group, a C₇₋₁₆ aralkyl group, a 5- to 14-membered aromatic heterocyclic group and a 3-to 14-membered non-aromatic heterocyclic group, each of which optionally has 1 to 3 substituents selected from a halogen atom, an optionally halogenated C₁₋₆ alkoxy group, a hydroxy group, a nitro group, a cyano group, an amino group and a carbamoyl group.

Examples of the "acyl group" also include a hydrocarbon-sulfonyl group, a heterocyclylsulfonyl group, a hydrocarbon-sulfinyl group and a heterocyclylsulfinyl group.

Here, the hydrocarbon-sulfonyl group means a hydrocarbon group-bonded sulfonyl group, the heterocyclylsulfonyl group means a heterocyclic group-bonded sulfonyl group, the hydrocarbon-sulfinyl group means a hydrocarbon group-bonded sulfinyl group and the heterocyclylsulfinyl group means a heterocyclic group-bonded sulfinyl group.

Suitable examples of the "acyl group" include a formyl group, a carboxy group, a C₁₋₆ alkyl-carbonyl group, a C₂₋₆ alkenyl-carbonyl group (e.g., crotonoyl), a C₃₋₁₀ cycloalkyl-carbonyl group (e.g., cyclobutanecarbonyl, cyclopentanecarbonyl, cyclohexanecarbonyl, cycloheptanecarbonyl), a C₃₋₁₀ cycloalkenyl-carbonyl group (e.g., 2-cyclohexenecarbonyl), a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a C₆₋₁₄ aryloxy-carbonyl group (e.g., phenyloxycarbonyl, naphthyloxycarbonyl), a C₇₋₁₆ aralkyloxy-carbonyl group (e.g., benzyloxycarbonyl, phenethyloxycarbonyl), a carbamoyl group, a mono- or di-C₁₋₆ alkyl-carbamoyl group, a mono- or di-C₂₋₆ alkenyl-carbamoyl group (e.g., diallylcarbamoyl), a mono- or di-C₃₋₁₀ cycloalkyl-carbamoyl group (e.g., cyclopropylcarbamoyl), a mono- or di-C₆₋₁₄ aryl-carbamoyl group (e.g., phenylcarbamoyl), a mono- or di-C₇₋₁₆ aralkyl-carbamoyl group, a 5- to 14-membered aromatic heterocyclylcarbamoyl group (e.g., pyridylcarbamoyl), a thiocarbamoyl group, a mono- or di-C₁₋₆ alkyl-thiocarbamoyl group (e.g., methylthiocarbamoyl, N-ethyl-N-methylthiocarbamoyl), a mono- or di-C₂₋₆ alkenyl-thiocarbamoyl group (e.g., diallylthiocarbamoyl), a mono- or di-C₃₋₁₀ cycloalkyl-thiocarbamoyl group (e.g., cyclopropylthiocarbamoyl, cyclohexylthiocarbamoyl), a mono- or di-C₆₋₁₄ aryl-thiocarbamoyl group (e.g., phenylthiocarbamoyl), a mono- or di-C₇₋₁₆ aralkyl-thiocarbamoyl group (e.g., benzylthiocarbamoyl, phenethylthiocarbamoyl), a 5- to 14-membered aromatic heterocyclylthiocarbamoyl group (e.g., pyridylthiocarbamoyl), a sulfino group, a C₁₋₆ alkylsulfinyl group (e.g., methylsulfinyl, ethylsulfinyl), a sulfo group, a C₁₋₆ alkylsulfonyl group, a C₆₋₁₄ arylsulfonyl group, a phosphono group and a mono- or di-C₁₋₆ alkylphosphono group (e.g., dimethylphosphono, diethylphosphono, diisopropylphosphono, dibutylphosphono).

In the present specification, examples of the "optionally substituted amino group" include an amino group optionally having 1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group, a mono- or di-C₇₋₁₆ aralkyl-carbamoyl group, a C₁₋₆ alkylsulfonyl group and a C₆₋₁₄ arylsulfonyl group, each of which optionally has 1 to 3 substituents selected from Substituent Group A.

Suitable examples of the optionally substituted amino group include an amino group, a mono- or di-(optionally halogenated C₁₋₆ alkyl) amino group (e.g., methylamino, trifluoromethylamino, dimethylamino, ethylamino, diethylamino, propylamino, dibutylamino), a mono- or di-C₂₋₆ alkenylamino group (e.g., diallylamino), a mono- or di-C₃₋₁₀ cycloalkylamino group (e.g., cyclopropylamino, cyclohexylamino), a mono- or di-C₆₋₁₄ arylamino group (e.g., phenylamino), a mono- or di-C₇₋₁₆ aralkylamino group (e.g., benzylamino, dibenzylamino), a mono- or di-(optionally halogenated C₁₋₆ alkyl)-carbonylamino group (e.g., acetylamino, propionylamino), a mono- or di-C₆₋₁₄ aryl-carbonylamino group (e.g., benzoylamino), a mono- or di-C₇₋₁₆ aralkyl-carbonylamino group (e.g., benzylcarbonylamino), a mono- or di-5- to 14-membered aromatic heterocyclylcarbonylamino group (e.g., nicotinoylamino, isonicotinoylamino), a mono- or di-3- to 14-membered non-aromatic heterocyclylcarbonylamino group (e.g., piperidinylcarbonylamino), a mono- or di-C₁₋₆ alkoxy-carbonylamino group (e.g., tert-butoxycarbonylamino), a 5- to 14-membered aromatic heterocyclylamino group (e.g., pyridylamino), a carbamoylamino group, a (mono- or di-C₁₋₆ alkyl-carbamoyl) amino group (e.g., methylcarbamoylamino), a (mono- or di-C₇₋₁₆ aralkyl-carbamoyl) amino group (e.g., benzylcarbamoylamino), a C₁₋₆ alkylsulfonylamino group (e.g., methylsulfonylamino, ethylsulfonylamino), a C₆₋₁₄ arylsulfonylamino group (e.g., phenylsulfonylamino), a (C₁₋₆ alkyl)(C₁₋₆ alkyl-carbonyl) amino group (e.g., N-acetyl-N-methylamino) and a (C₁₋₆ alkyl)(C₆₋₁₄ aryl-carbonyl) amino group (e.g., N-benzoyl-N-methylamino).

In the present specification, examples of the "optionally substituted carbamoyl group" include a carbamoyl group optionally having 1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from Substituent Group A.

Suitable examples of the optionally substituted carbamoyl group include a carbamoyl group, a mono- or di-C₁₋₆ alkyl-carbamoyl group, a mono- or di-C₂₋₆ alkenyl-carbamoyl group (e.g., diallylcarbamoyl), a mono- or di-C₃₋₁₀ cycloalkyl-carbamoyl group (e.g., cyclopropylcarbamoyl, cyclohexylcarbamoyl), a mono- or di-C₆₋₁₄ aryl-carbamoyl group (e.g., phenylcarbamoyl), a mono- or di-C₇₋₁₆ aralkyl-carbamoyl group, a mono- or di-C₁₋₆ alkyl-carbonyl-carbamoyl group (e.g., acetylcarbamoyl, propionylcarbamoyl), a mono- or di-C₆₋₁₄ aryl-carbonyl-carbamoyl group (e.g., benzoylcarbamoyl) and a 5- to 14-membered aromatic heterocyclylcarbamoyl group (e.g., pyridylcarbamoyl).

In the present specification, examples of the "optionally substituted thiocarbamoyl group" include a thiocarbamoyl group optionally having 1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from Substituent Group A.

Suitable examples of the optionally substituted thiocarbamoyl group include a thiocarbamoyl group, a mono- or di-C₁₋₆ alkyl-thiocarbamoyl group (e.g., methylthiocarbamoyl, ethylthiocarbamoyl, dimethylthiocarbamoyl, diethylthiocarbamoyl, N-ethyl-N-methylthiocarbamoyl), a mono- or di-C₂₋₆ alkenyl-thiocarbamoyl group (e.g., diallylthiocarbamoyl), a mono- or di-C₃₋₁₀ cycloalkyl-thiocarbamoyl group (e.g., cyclopropylthiocarbamoyl, cyclohexylthiocarbamoyl), a mono- or di-C₆₋₁₄ aryl-thiocarbamoyl group (e.g., phenylthiocarbamoyl), a mono- or di-C₇₋₁₆ aralkyl-thiocarbamoyl group (e.g., benzylthiocarbamoyl, phenethylthiocarbamoyl), a mono- or di-C₁₋₆ alkyl-carbonyl-thiocarbamoyl group (e.g., acetylthiocarbamoyl, propionylthiocarbamoyl), a mono- or di-C₆₋₁₄ aryl-carbonyl-thiocarbamoyl group (e.g., benzoylthiocarbamoyl) and a 5- to 14-membered aromatic heterocyclylthiocarbamoyl group (e.g., pyridylthiocarbamoyl).

In the present specification, examples of the "optionally substituted sulfamoyl group" include a sulfamoyl group optionally having 1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from Substituent Group A.

Suitable examples of the optionally substituted sulfamoyl group include a sulfamoyl group, a mono- or di-C₁₋₆ alkyl-sulfamoyl group (e.g., methylsulfamoyl, ethylsulfamoyl, dimethylsulfamoyl, diethylsulfamoyl, N-ethyl-N-methylsulfamoyl), a mono- or di-C₂₋₆ alkenyl-sulfamoyl group (e.g., diallylsulfamoyl), a mono- or di-C₃₋₁₀ cycloalkyl-sulfamoyl group (e.g., cyclopropylsulfamoyl, cyclohexylsulfamoyl), a mono- or di-C₆₋₁₄ aryl-sulfamoyl group (e.g., phenylsulfamoyl), a mono- or di-C₇₋₁₆ aralkyl-sulfamoyl group (e.g., benzylsulfamoyl, phenethylsulfamoyl), a mono- or di-C₁₋₆ alkyl-carbonyl-sulfamoyl group (e.g., acetylsulfamoyl, propionylsulfamoyl), a mono- or di-C₆₋₁₄ aryl-carbonyl-sulfamoyl group (e.g., benzoylsulfamoyl) and a 5- to 14-membered aromatic heterocyclylsulfamoyl group (e.g., pyridylsulfamoyl).

In the present specification, examples of the "optionally substituted hydroxy group" include a hydroxy group optionally having a substituent selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group, a mono- or di-C₇₋₁₆ aralkyl-carbamoyl group, a C₁₋₆ alkylsulfonyl group and a C₆₋₁₄ arylsulfonyl group, each of which optionally has 1 to 3 substituents selected from Substituent Group A.

Suitable examples of the optionally substituted hydroxy group include a hydroxy group, a C₁₋₆ alkoxy group, a C₂₋₆ alkenyloxy group (e.g., allyloxy, 2-butenyloxy, 2-pentenyloxy, 3-hexenyloxy), a C₃₋₁₀ cycloalkyloxy group (e.g., cyclohexyloxy), a C₆₋₁₄ aryloxy group (e.g., phenoxy, naphthyloxy), a C₇₋₁₆ aralkyloxy group (e.g., benzyloxy, phenethyloxy), a C₁₋₆ alkyl-carbonyloxy group (e.g., acetyloxy, propionyloxy, butyryloxy, isobutyryloxy, pivaloyloxy), a C₆₋₁₄ aryl-carbonyloxy group (e.g., benzoyloxy), a C₇₋₁₆ aralkyl-carbonyloxy group (e.g., benzylcarbonyloxy), a 5- to 14-membered aromatic heterocyclylcarbonyloxy group (e.g., nicotinoyloxy), a 3- to 14-membered non-aromatic heterocyclylcarbonyloxy group (e.g., piperidinylcarbonyloxy), a C₁₋₆ alkoxy-carbonyloxy group (e.g., tert-butoxycarbonyloxy), a 5- to 14-membered aromatic heterocyclyloxy group (e.g., pyridyloxy), a carbamoyloxy group, a C₁₋₆ alkyl-carbamoyloxy group (e.g., methylcarbamoyloxy), a C₇₋₁₆ aralkyl-carbamoyloxy group (e.g., benzylcarbamoyloxy), a C₁₋₆ alkylsulfonyloxy group (e.g., methylsulfonyloxy, ethylsulfonyloxy) and a C₆₋₁₄ arylsulfonyloxy group (e.g., phenylsulfonyloxy).

In the present specification, examples of the "optionally substituted sulfanyl group" include a sulfanyl group optionally having a substituent selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group and a 5- to 14-membered aromatic heterocyclic group, each of which optionally has 1 to 3 substituents selected from Substituent Group A and a halogenated sulfanyl group.

Suitable examples of the optionally substituted sulfanyl group include a sulfanyl (-SH) group, a C₁₋₆ alkylthio group, a C₂₋₆ alkenylthio group (e.g., allylthio, 2-butenylthio, 2-pentenylthio, 3-hexenylthio), a C₃₋₁₀ cycloalkylthio group (e.g., cyclohexylthio), a C₆₋₁₄ arylthio group (e.g., phenylthio, naphthylthio), a C₇₋₁₆ aralkylthio group (e.g., benzylthio, phenethylthio), a C₁₋₆ alkyl-carbonylthio group (e.g., acetylthio, propionylthio, butyrylthio, isobutyrylthio, pivaloylthio), a C₆₋₁₄ aryl-carbonylthio group (e.g., benzoylthio), a 5- to 14-membered aromatic heterocyclylthio group (e.g., pyridylthio) and a halogenated thio group (e.g., pentafluorothio).

In the present specification, examples of the "optionally substituted silyl group" include a silyl group optionally having 1 to 3 substituents selected from a C₁₋₆ alkyl group, a C₂₋₆ alkenyl group, a C₃₋₁₀ cycloalkyl group, a C₆₋₁₄ aryl group and a C₇₋₁₆ aralkyl group, each of which optionally has 1 to 3 substituents selected from Substituent Group A. Examples of the optionally substituted silyl group include a tri-C₁₋₆ alkylsilyl group (e.g., trimethylsilyl, tert-butyl(dimethyl)silyl).

In some embodiments, the STING modulator is a compound of formula (III): 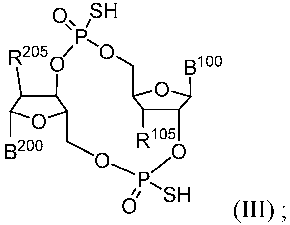 or a pharmaceutically acceptable salt thereof, wherein:
R¹⁰⁵ and R²⁰⁵ are each independently a hydroxyl group or a halogen atom;
B¹⁰⁰ is a group represented by formula (**B¹-A**) or formula (**B¹-B**): 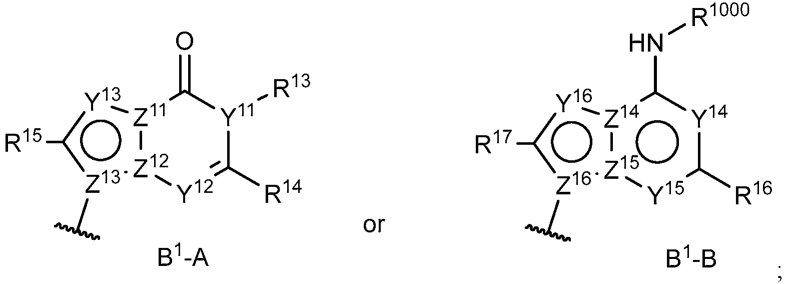
R¹³, R¹⁴, R¹⁵, R¹⁶ and R¹⁷ are each independently a hydrogen atom or a substituent;
R¹⁰⁰⁰ is hydrogen or a bond to the carbonyl group of formula (I);
Y¹¹, Y¹², Y¹³, Y¹⁴, Y¹⁵ and Y¹⁶ are each independently N or CR^{1a};
Z¹¹, Z¹², Z¹³, Z¹⁴, Z¹⁵ and Z¹⁶ are each independently N or C;
R¹⁰⁵ and R²⁰⁵ are each independently a hydroxyl group or a halogen atom;
R^{1a} is a hydrogen atom or a substituent;
B²⁰⁰ is a group represented by formula (**B²-A**) or formula (**B²-B**): 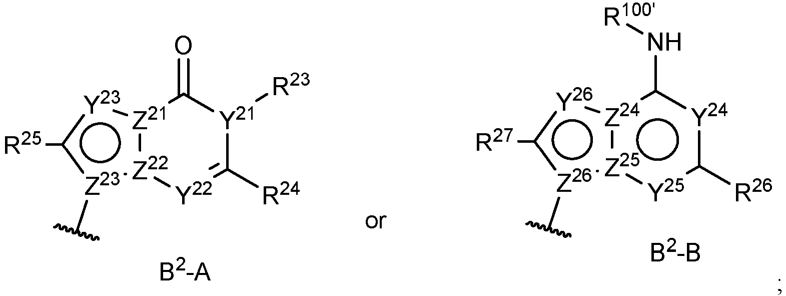
R²³, R²⁴, R²⁵, R²⁶ and R²⁷ are each independently a hydrogen atom or a substituent;
R^{100'} is hydrogen or a bond to the carbonyl group of formula (I);
Y²¹, Y²², Y²³, Y²⁴, Y²⁵ and Y²⁶ are each independently N or CR^{2a};
Z²¹, Z²², Z²¹, Z²⁴, Z²⁵ and Z²⁶ are each independently N or C;
R^{2a} is a hydrogen atom or a substituent;
X¹ and X² are each independently an oxygen atom or a sulfur atom; and
Q¹, Q², Q3 and Q⁴ are each independently an oxygen atom or a sulfur atom.
provided that:
   one of B¹⁰⁰ or B²⁰⁰ is: 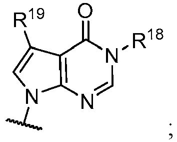 wherein:
   R¹⁸ is hydrogen or C₁₋₆ alkyl; and
   R¹⁹ a halogen atom, and the other one of B¹⁰⁰ or B²⁰⁰ is attached to the 5-membered ring of the parent structure through an -NH- group.

In some embodiments the STING modulator is a compound of formula (III), or a pharmaceutically acceptable salt thereof, wherein R²⁰⁵ is F and B¹⁰⁰ is

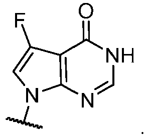

In some embodiments the cyclic dinucleotide is: 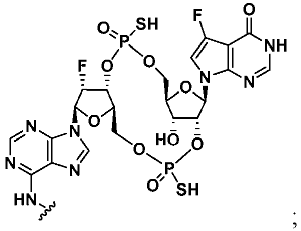 or a pharmaceutically acceptable salt thereof, wherein 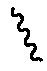 is the point of attachment to the carbonyl group of the parent molecular moiety.

The group "L" is a cleavable linker. As used herein, the term "linker" refers to any chemical moiety capable of connecting the antibody, antibody fragment, or antigen-binding fragment (Ab) to the drug-containing moiety within the compounds of formula (I) and (IV). The linker can be branched, and can be substituted with from 1 to 20 drug-containing moieties. In some embodiments, the linker can be substituted with from 1 to 10 drug-containing moieties. In some embodiments, the linker can be substituted with from 1 to 5 drug-containing moieties. In some embodiments, the linker can be substituted with one or two drug-containing moieties. In some embodiments, the linker can be substituted with one drug-containing moiety.

Linker "L" is cleavable. In certain embodiments the linker can be susceptible to acid-induced cleavage, photo-induced cleavage, enzymatic cleavage, or the like, at conditions under which the drug and/or antibody can remain active. In some embodiments, the cleavable linker can be cleaved enzymatically. In some embodiments, the cleavable linker can be cleaved by a protease, peptidase, esterase, glycosidase, phosphodiesterase, phosphatase, or lipase. In some embodiments, the cleavable linker can be cleaved by a protease. Examples of proteases include, but are not limited to, cathepsin B, VAGP tetrapeptide, and the like. In certain embodiments the linker can be any of those disclosed in PCT publications WO 2018/200812, WO 2018/100558, which are incorporated by reference in their entireties.

In certain embodiments, "L" has the formula: 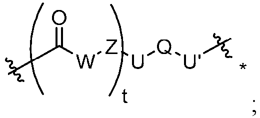 wherein:
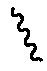 is the point of attachment to the nitrogen atom; and
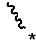 is the point of attachment to Ab.

In some embodiments, "L" has the formula: 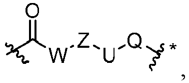 wherein:
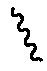 is the point of attachment to the nitrogen atom;
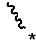 is the point of attachment to the antibody;

The group "W" is absent or a self-immolative group. As used herein, the term "self-immolative," refers to a group that undergoes an electronic cascade which results in the release of the group to which it is attached. In some embodiments, the self-immolative group comprises one or more groups which can undergo 1,4-elimination, 1,6-elimination, 1,8-elimination, 1,6-cyclization elimination, 1,5-cyclization elimination, 1,3-cyclization elimination, intramolecular 5-exo-trig cyclization, and/or 6-exo-trig cyclization. In certain embodiments the self-immolative group can be any of those disclosed in PCT publications WO 2018/200812, WO 2018/100558, which are incorporated by reference in their entireties.

The group "Z" is absent or a peptide of 2 to 5 amino acids. In certain embodiments, the peptide is the site of cleavage of the linker, thereby facilitating release of the drug upon exposure to intracellular proteases, such as lysosomal enzymes (Doronina et al. (2003) Nat. Biotechnol. 21:778-784). Examples of peptides having two amino acids include, but are not limited to, alanine-alanine (ala-ala), valine-citrulline (vc or val-cit), alanine-phenylalanine (af or ala-phe); phenylalanine-lysine (fk or phe-lys); phenylalanine-homolysine (phe-homolys); and N-methyl-valine-citrulline (Me-val-cit). Examples of peptides having three amino acids include, but are not limited to, glycine-valine-citrulline (gly-val-cit) and glycine-glycine-glycine (gly-gly-gly). The amino acid combinations above can also be present in the reverse order (i.e., cit-val).

The peptides of the present disclosure may comprise naturally-occurring and/or non-natural amino acid residues. The term "naturally-occurring amino acid" refer to Ala, Asp, Cys, Glu, Phe, Gly, His, He, Lys, Leu, Met, Asn, Pro, Gin, Arg, Ser, Thr, Val, Trp, and Tyr. "Non-natural amino acids" (i.e., amino acids do not occur naturally) include, by way of non-limiting example, homoserine, homoarginine, citrulline, phenylglycine, taurine, iodotyrosine, seleno-cysteine, norleucine ("Nle"), norvaline ("Nva"), beta-alanine, L- or D-naphthalanine, ornithine ("Om"), and the like. Peptides can be designed and optimized for enzymatic cleavage by a particular enzyme, for example, a tumor-associated protease, cathepsin B, C and D, or a plasmin protease.

Amino acids also include the D-forms of natural and non-natural amino acids. "D-" designates an amino acid having the "D" (dextrorotary) configuration, as opposed to the configuration in the naturally occurring ("L-") amino acids. Natural and non-natural amino acids can be purchased commercially (Sigma Chemical Co., Advanced Chemtech) or synthesized using methods known in the art.

The groups "U" and "U'" are independently absent or a spacer. As used herein, the term "spacer," refers to chemical moiety that serves as a connector. In the present disclosure the spacer can connect the antibody, antibody fragment, or antigen fragment to the heterobifunctional group and/or connect the heterobifunctional group to peptide "Z," or, when "Z" is absent, to group "W". Non-limiting exemplary spacers include -NH-, -S-, -O-, -NHC(=O)CH₂CH₂-, - S(=O)₂-CH₂CH₂-, - C(=O)NHNH-, -C(=O)O-, -C(=O)NH-, -CH₂-, -CH₂CH₂-, -CH₂CH₂CH₂-, -CH₂=CH₂-, -C≡C-, -CH=N-O-, polyethylene glycol (PEG), 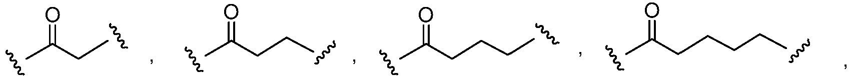 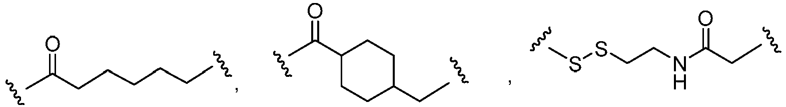 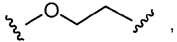 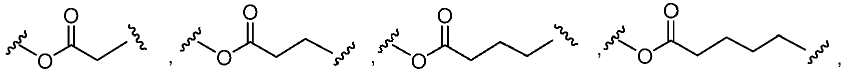 and

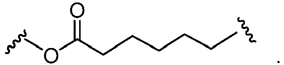

In the compounds of the present disclosure, when "U" is present, it can be a branched group substituted by from 1 to 10 "-C(O)-W-Z-" groups. In some embodiments, "U" is substituted by from 1 to 5 "-C(O)-W-Z-" groups. In some embodiments, "U" is substituted with 1 or 2 "-C(O)-W-Z-" groups. In some embodiments, "U" is substituted with 1 "-C(O)-W-Z-" group. In certain embodiments the spacer can be any of those disclosed in PCT publications WO 2018/200812, WO 2018/100558, which are incorporated by reference in their entireties.

Group "Q" is a heterobifunctional group. In the present disclosure, the term "heterobifunctional group" refers to a chemical moiety that connects the linker of which it is a part to the antibody, antibody fragment, or antigen-binding fragment. *See, e.g.,* WO 2017/191579. Heterobifunctional groups are characterized as having different reactive groups at either end of the chemical moiety. The heterobifunctional group may be attached directly to "Ab," or alternatively, may connect through linker "U'". Attachment to "Ab," can be accomplished through chemical or enzymatic conjugation, or a combination of both. Chemical conjugation involves the controlled reaction of accessible amino acid residues on the surface of the antibody with a reaction handle on "Q" or "U'". Examples of chemical conjugation include, but are not limited to, lysine amide coupling, cysteine coupling, and coupling via a non-natural amino acid incorporated by genetic engineering, wherein non-natural amino acid residues with a desired reaction handle are installed onto "Ab". In enzymatic conjugation, an enzyme mediates the coupling of the linker with an accessible amino residue on the antibody, antibody fragment, or antigen-binding fragment. Examples of enzymatic conjugation include, but are not limited to, transpeptidation using sortase, transpeptidation using microbial transglutaminase, and N-glycan engineering. Chemical conjugation and enzymatic conjugation may also be used sequentially. For example, enzymatic conjugation can also be used for installing unique reaction handles on "Ab" to be utilized in subsequent chemical conjugation. In certain embodiments the heterobifunctional group can be any of those disclosed in PCT publications WO 2018/200812, WO 2018/100558, which are incorporated by reference in their entireties.

In some embodiments, "Q" is selected from 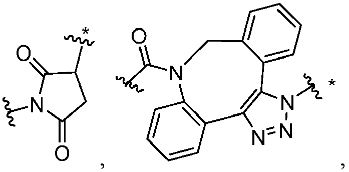 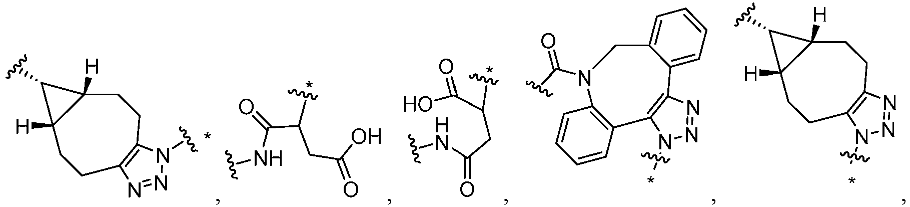 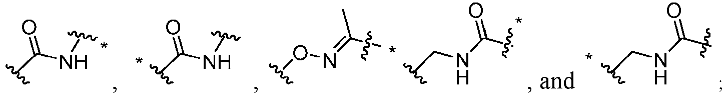 wherein
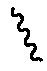 is the point of attachment to U, or, when U is absent, the point of attachment to Z; and
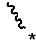 is the point of attachment to U', or, when U' is absent, the point of attachment to Ab.

In certain embodiments, the present disclosure provides a compound of formula (XX): 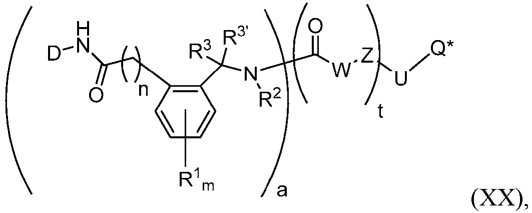 or a pharmaceutically acceptable salt thereof, wherein n, m, a, t, D-NH-, R¹, R², R³, R^{3'}, W, Z, and U are as described herein and wherein Q* is reactive functional group capable of conjugating to an antibody, antibody fragment, or antigen-binding fragment. Examples of suitable Q* groups include, but are not limited to, activated carboxylic acid groups, such as acid chloride -C(O)-Cl and acid anhydrides, haloacetamide, maleimide, alkyne, cycloalkyne, such as a cyclooctyne, oxanoboradiene, norbornene, azide, diaryl tetrazine, monoaryl tetrazine, aldehyde, ketone, hydroxylamine, vinylsulfone, and aziridine. In certain embodiments the reactive functional group can be any of those disclosed in PCT publications WO 2018/200812, WO 2018/100558, which are incorporated by reference in their entireties.

Group "Ab" is an antibody, antibody fragment or an antigen-binding fragment. An antibody is a protein generated by the immune system that is capable of recognizing and binding to a specific antigen. A target antigen generally has numerous binding sites, also called epitopes, recognized by CDRs on multiple antibodies. Each antibody that specifically binds to a different epitope has a different structure. Thus, one antigen may have more than one corresponding antibody. The term "antibody" herein is used in the broadest sense and specifically covers monoclonal antibodies, single domain antibodies, polyclonal antibodies, multispecific antibodies (e.g., bispecific antibodies), and antibody fragments , so long as they exhibit the desired biological activity. Antibodies may be murine, human, humanized, chimeric, or derived from other species. (Janeway, C., Travers, P., Walport, M., Shlomchik (2001) Immuno Biology, 5th Ed., Garland Publishing, New York).

The term "antibody," as used herein, also refers to a full-length immunoglobulin molecule or an immunologically active portion of a full-length immunoglobulin molecule, i.e., a molecule that contains an antigen binding site that immunospecifically binds an antigen of a target of interest or part thereof, such targets including but not limited to, cancer cell or cells that produce autoimmune antibodies associated with an autoimmune disease. The immunoglobulin disclosed herein can be of any type (e.g., IgG, IgE, IgM, IgD, and IgA), class (e.g., IgG1, IgG2, IgG3, IgG4, IgA1 and IgA2) or subclass of immunoglobulin molecule. The immunoglobulins can be derived from any species. In one aspect, however, the immunoglobulin is of human, murine, or rabbit origin.

The term "single domain antibody," also known as a nanobody, is an antibody fragment consisting of a single monomeric variable antibody domain with a molecular weight of from about 12 kDa to about 15kDa. Single body antibodies can be based on heavy chain variable domains or light chains. Examples of single domain antibodies include, but are not limited to, V_{H}H fragments and V_{NAR} fragments. See, for example, Harmsen M. M. et al. Applied Microbiology and Biotechnology 77 (1): 13-22.

"Antibody fragments" comprise a portion of an intact antibody, generally the antigen binding or variable region thereof. Examples of antibody fragments include Fab, Fab', F(ab').sub.2, and Fv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

An "intact antibody" is one which comprises an antigen-binding variable region as well as a light chain constant domain (CL) and heavy chain constant domains, CH1, CH2 and CH3. The constant domains may be native sequence constant domains (e.g., human native sequence constant domains) or amino acid sequence variant thereof.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method first described by Kohler et al (1975) Nature 256:495, or may be made by recombinant DNA methods (see, U.S. Pat. No. 4,816,567). The "monoclonal antibodies" may also be isolated from phage antibody libraries using the techniques described in Clackson et al (1991) Nature, 352:624-628; Marks et al (1991) J. Mol. Biol., 222:581-597; for example.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al (1984) Proc. Natl. Acad. Sci. USA, 81:6851-6855). Chimeric antibodies of interest herein include "primatized" antibodies comprising variable domain antigen-binding sequences derived from a non-human primate (e.g., Old World Monkey, Ape etc.) and human constant region sequences.

Various methods have been employed to produce monoclonal antibodies (MAbs). Hybridoma technology, which refers to a cloned cell line that produces a single type of antibody, uses the cells of various species, including mice (murine), hamsters, rats, and humans. Another method to prepare MAbs uses genetic engineering including recombinant DNA techniques. Monoclonal antibodies made from these techniques include, among others, chimeric antibodies and humanized antibodies. A chimeric antibody combines DNA encoding regions from more than one type of species. For example, a chimeric antibody may derive the variable region from a mouse and the constant region from a human. A humanized antibody comes predominantly from a human, even though it contains nonhuman portions. Like a chimeric antibody, a humanized antibody may contain a completely human constant region. But unlike a chimeric antibody, the variable region may be partially derived from a human. The nonhuman, synthetic portions of a humanized antibody often come from CDRs in murine antibodies. In any event, these regions are crucial to allow the antibody to recognize and bind to a specific antigen. While useful for diagnostics and short-term therapies, murine antibodies cannot be administered to people long-term without increasing the risk of a deleterious immunogenic response. This response, called Human Anti-Mouse Antibody (HAMA), occurs when a human immune system recognizes the murine antibody as foreign and attacks it. A HAMA response can cause toxic shock or even death.

Chimeric and humanized antibodies reduce the likelihood of a HAMA response by minimizing the nonhuman portions of administered antibodies. Furthermore, chimeric and humanized antibodies can have the additional benefit of activating secondary human immune responses, such as antibody dependent cellular cytotoxicity.

The intact antibody may have one or more "effector functions" which refer to those biological activities attributable to the Fc region (a native sequence Fc region or amino acid sequence variant Fc region) of an antibody. Examples of antibody effector functions include C1q binding; complement dependent cytotoxicity; Fc receptor binding; antibody-dependent cell-mediated cytotoxicity (ADCC); phagocytosis; down regulation of cell surface receptors (e.g., B cell receptor; BCR), etc.

Depending on the amino acid sequence of the constant domain of their heavy chains, intact antibodies can be assigned to different "classes". There are five major classes of intact antibodies: IgA, IgD, IgE, IgG, and IgM, and several of these may be further divided into "subclasses" (isotypes), e.g., IgG1, IgG2, IgG3, IgG4, IgA, and IgA2. The heavy-chain constant domains that correspond to the different classes of antibodies are called alpha., delta., epsilon., .gamma., and .mu., respectively. The subunit structures and three-dimensional configurations of different classes of immunoglobulins are well known.

Useful non-immunoreactive protein, polypeptide, or peptide antibodies include, but are not limited to, transferrin, epidermal growth factors ("EGF"), bombesin, gastrin, gastrin-releasing peptide, platelet-derived growth factor, IL-2, IL-6, transforming growth factors ("TGF"), such as TGF-.alpha. and TGF-.beta., vaccinia growth factor ("VGF"), insulin and insulin-like growth factors I and II, lectins and apoprotein from low density lipoprotein.

Useful polyclonal antibodies are heterogeneous populations of antibody molecules derived from the sera of immunized animals. Various procedures well known in the art may be used for the production of polyclonal antibodies to an antigen-of-interest. For example, for the production of polyclonal antibodies, various host animals can be immunized by injection with an antigen of interest or derivative thereof, including but not limited to rabbits, mice, rats, and guinea pigs. Various adjuvants may be used to increase the immunological response, depending on the host species, and including but not limited to Freund's (complete and incomplete) adjuvant, mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvum. Such adjuvants are also well known in the art.

Useful monoclonal antibodies are homogeneous populations of antibodies to a particular antigenic determinant (e.g., a cancer cell antigen, a viral antigen, a microbial antigen, a protein, a peptide, a carbohydrate, a chemical, nucleic acid, or fragments thereof). A monoclonal antibody (mAb) to an antigen-of-interest can be prepared by using any technique known in the art which provides for the production of antibody molecules by continuous cell lines in culture. These include, but are not limited to, the hybridoma technique originally described by Kohler and Milstein (1975, Nature 256, 495-497), the human B cell hybridoma technique (Kozbor et al., 1983, Immunology Today 4:72), and the EBV-hybridoma technique (Cole et al., 1985, Monoclonal Antibodies and Cancer Therapy, Alan R. Liss, Inc., pp. 77-96). Such antibodies may be of any immunoglobulin class including IgG, IgM, IgE, IgA, and IgD and any subclass thereof. The hybridoma producing the mAbs used in this disclosure may be cultivated in vitro or in vivo.

Useful monoclonal antibodies include, but are not limited to, human monoclonal antibodies, humanized monoclonal antibodies, antibody fragments, or chimeric human-mouse (or other species) monoclonal antibodies. Human monoclonal antibodies may be made by any of numerous techniques known in the art (e.g., Teng et al., 1983, Proc. Natl. Acad. Sci. U.S.A. 80, 7308-7312; Kozbor et al., 1983, Immunology Today 4, 72-79; and Olsson et al., 1982, Meth. Enzymol. 92, 3-16).

The antibody can also be a bispecific antibody. Methods for making bispecific antibodies are known in the art. Traditional production of full-length bispecific antibodies is based on the coexpression of two immunoglobulin heavy chain-light chain pairs, where the two chains have different specificities (Milstein et al., 1983, Nature 305:537-539). Because of the random assortment of immunoglobulin heavy and light chains, these hybridomas (quadromas) produce a potential mixture of 10 different antibody molecules, of which only one has the correct bispecific structure. Purification of the correct molecule, which is usually performed using affinity chromatography steps, is rather cumbersome, and the product yields are low. Similar procedures are disclosed in WO 93/08829, and in Traunecker et al., EMBO J. 10:3655-3659 (1991).

According to a different approach, antibody variable domains with the desired binding specificities (antibody-antigen combining sites) are fused to immunoglobulin constant domain sequences. The fusion may be with an immunoglobulin heavy chain constant domain, comprising at least part of the hinge, C.sub.H2, and C.sub.H3 regions. The first heavy-chain constant region (C.sub.H1) may contain the site necessary for light chain binding, present in at least one of the fusions. Nucleic acids with sequences encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

Bispecific antibodies may have a hybrid immunoglobulin heavy chain with a first binding specificity in one arm, and a hybrid immunoglobulin heavy chain-light chain pair (providing a second binding specificity) in the other arm. This asymmetric structure facilitates the separation of the desired bispecific compound from unwanted immunoglobulin chain combinations, as the presence of an immunoglobulin light chain in only one half of the bispecific molecule provides for a facile way of separation (WO 94/04690; Suresh et al., Methods in Enzymology, 1986, 121:210; Rodrigues et al., 1993, J. of Immunology 151:6954-6961; Carter et al., 1992, Bio/Technology 10: 163-167; Carter et al., 1995, J. of Hematotherapy 4:463-470; Merchant et al., 1998, Nature Biotechnology 16:677-681. Using such techniques, bispecific antibodies can be prepared for conjugation as ADC in the treatment or prevention of disease as defined herein.

Hybrid or bifunctional antibodies can be derived either biologically, i.e., by cell fusion techniques, or chemically, especially with cross-linking agents or disulfide-bridge forming reagents, and may comprise whole antibodies or fragments thereof (EP 105360; WO 83/03679; EP 217577).

The antibody can be a functionally active fragment, derivative or analog of an antibody that immunospecifically binds to cancer cell antigens, viral antigens, or microbial antigens or other antibodies bound to tumor cells or matrix. In this regard, "functionally active" means that the fragment, derivative or analog is able to elicit anti-anti-idiotype antibodies that recognize the same antigen that the antibody from which the fragment, derivative or analog is derived recognized. Specifically, in an exemplary embodiment the antigenicity of the idiotype of the immunoglobulin molecule can be enhanced by deletion of framework and CDR sequences that are C-terminal to the CDR sequence that specifically recognizes the antigen. To determine which CDR sequences bind the antigen, synthetic peptides containing the CDR sequences can be used in binding assays with the antigen by any binding assay method known in the art (e.g., the BIA core assay) (See, for e.g., Kabat et al., 1991, Sequences of Proteins of Immunological Interest, Fifth Edition, National Institute of Health, Bethesda, Md.; Kabat E et al., 1980, J. of Immunology 125 (3):961-969).

Other useful antibodies include fragments of antibodies such as, but not limited to, F(ab')2 fragments, which contain the variable region, the light chain constant region and the CH1 domain of the heavy chain can be produced by pepsin digestion of the antibody molecule, and Fab fragments, which can be generated by reducing the disulfide bridges of the F(ab')2 fragments. Other useful antibodies are heavy chain and light chain dimers of antibodies, or any minimal fragment thereof such as Fvs or single chain antibodies (SCAs) (e.g., as described in U.S. Pat. No. 4,946,778; Bird, 1988, Science 242:423-42; Huston et al., 1988, Proc. Natl. Acad. Sci. USA 85:5879-5883; and Ward et al., (1989) Nature 334:544-54), or any other molecule with the same specificity as the antibody.

Additionally, recombinant antibodies, such as chimeric and humanized monoclonal antibodies, comprising both human and non-human portions, which can be made using standard recombinant DNA techniques, are useful antibodies. A chimeric antibody is a molecule in which different portions are derived from different animal species, such as those having a variable region derived from a murine monoclonal and human immunoglobulin constant regions. (See, e.g., Cabilly et al., U.S. Pat. No. 4,816,567; and Boss et al., U.S. Pat. No. 4,816,397). Humanized antibodies are antibody molecules from non-human species having one or more complementarity determining regions (CDRs) from the non-human species and a framework region from a human immunoglobulin molecule. (See, e.g., Queen, U.S. Pat. No. 5,585,089) Such chimeric and humanized monoclonal antibodies can be produced by recombinant DNA techniques known in the art, for example using methods described in WO 87/02671; EP 184,187; EP 171496; EP 173494; WO 86/01533; U.S. Pat. No. 4,816,567; EP 12023; Berter et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Cancer. Res. 47:999-1005; Wood et al., 1985, Nature 314:446-449; and Shaw et al., 1988, J. Natl. Cancer Inst. 80:1553-1559; Morrison, 1985, Science 229:1202-1207; Oi et al., 1986, BioTechniques 4: 214; U.S. Pat. No. 5,225,539; Jones et al., 1986, Nature 321 :552-525; Verhoeyan et al. (1988) Science 239:1534; and Beidler et al., 1988, J. Immunol. 141:4053-4060.

Completely human antibodies can be produced using transgenic mice that are incapable of expressing endogenous immunoglobulin heavy and light chains genes, but which can express human heavy and light chain genes. The transgenic mice are immunized in the normal fashion with a selected antigen, e.g., all or a portion of a polypeptide of the disclosure. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies. See, e.g., U.S. Pat. Nos. 5,625,126; 5,633,425; 5,569,825; 5,661,016; 5,545,806. Other human antibodies can be obtained commercially from, for example, Abgenix, Inc. (Freemont, Calif.) and Genpharm (San Jose, Calif.).

Completely human antibodies that recognize a selected epitope can be generated using a technique referred to as "guided selection." In this approach a selected non-human monoclonal antibody, e.g., a mouse antibody, is used to guide the selection of a completely human antibody recognizing the same epitope. (Jespers et al. (1994) Biotechnology 12:899-903). Human antibodies can also be produced using various techniques known in the art, including phage display libraries (Hoogenboom and Winter, J. Mol. Biol., 227:381 (1991); Marks et al., J. Mol. Biol., 222:581 (1991)).

The antibody may be a fusion protein of an antibody, or a functionally active fragment thereof, for example in which the antibody is fused via a covalent bond (e.g., a peptide bond), at either the N-terminus or the C-terminus to an amino acid sequence of another protein (or portion thereof, such as at least 10, 20 or 50 amino acid portion of the protein) that is not the antibody. The antibody or fragment thereof may be covalently linked to the other protein at the N-terminus of the constant domain.

Antibodies include analogs and derivatives that are either modified, i.e., by the covalent attachment of any type of molecule as long as such covalent attachment permits the antibody to retain its antigen binding immunospecificity. For example, but not by way of limitation, the derivatives and analogs of the antibodies include those that have been further modified, e.g., by glycosylation, acetylation, pegylation, phosphorylation, amidation, derivatization by known protecting/blocking groups, proteolytic cleavage, linkage to a cellular antibody unit or other protein, etc. Any of numerous chemical modifications can be carried out by known techniques, including, but not limited to specific chemical cleavage, acetylation, formylation, metabolic synthesis in the presence of tunicamycin, etc. Additionally, the analog or derivative can contain one or more unnatural amino acids.

The antibodies in antibody drug conjugates include antibodies having modifications (e.g., substitutions, deletions or additions) in amino acid residues that interact with Fc receptors. In particular, antibodies include antibodies having modifications in amino acid residues identified as involved in the interaction between the anti-Fc domain and the FcRn receptor (see, e.g., WO 97/34631). Antibodies immunospecific for a cancer cell antigen can be obtained commercially, for example, from Genentech (San Francisco, Calif.) or produced by any method known to one of skill in the art such as, e.g., chemical synthesis or recombinant expression techniques. The nucleotide sequence encoding antibodies immunospecific for a cancer cell antigen can be obtained, e.g., from the GenBank database or a database like it, the literature publications, or by routine cloning and sequencing.

The antibody of the ADC may be a monoclonal antibody, e.g. a murine monoclonal antibody, a chimeric antibody, or a humanized antibody. The antibody may be an antibody fragment, e.g. a Fab fragment.

Known antibodies for the treatment or prevention of cancer can be conjugated as ADCs. Antibodies immunospecific for a cancer cell antigen can be obtained commercially or produced by any method known to one of skill in the art such as, e.g., recombinant expression techniques. The nucleotide sequence encoding antibodies immunospecific for a cancer cell antigen can be obtained, e.g., from the GenBank database or a database like it, the literature publications, or by routine cloning and sequencing. Examples of antibodies available for the treatment of cancer include, but are not limited to, humanized anti-HER2 monoclonal antibody for the treatment of patients with metastatic breast cancer; RITUXAN.RTM. (rituximab; Genentech) which is a chimeric anti-CD20 monoclonal antibody for the treatment of patients with non-Hodgkin's lymphoma; OvaRex (AltaRex Corporation, MA) which is a murine antibody for the treatment of ovarian cancer; Panorex (Glaxo Wellcome, NC) which is a murine IgG.sub.2a antibody for the treatment of colorectal cancer; Cetuximab Erbitux (Imclone Systems Inc., NY) which is an anti-EGFR IgG chimeric antibody for the treatment of epidermal growth factor positive cancers, such as head and neck cancer; Vitaxin (MedImmune, Inc., MD) which is a humanized antibody for the treatment of sarcoma; Campath I/H (Leukosite, MA) which is a humanized IgG.sub.1 antibody for the treatment of chronic lymphocytic leukemia (CLL); Smart MI95 (Protein Design Labs, Inc., CA) which is a humanized anti-CD33 IgG antibody for the treatment of acute myeloid leukemia (AML); LymphoCide (Immunomedics, Inc., NJ) which is a humanized anti-CD22 IgG antibody for the treatment of non-Hodgkin's lymphoma; Smart ID10 (Protein Design Labs, Inc., CA) which is a humanized anti-HLA-DR antibody for the treatment of non-Hodgkin's lymphoma; Oncolym (Techniclone, Inc., CA) which is a radiolabeled murine anti-HLA-Dr10 antibody for the treatment of non-Hodgkin's lymphoma; Allomune (BioTransplant, CA) which is a humanized anti-CD2 mAb for the treatment of Hodgkin's Disease or non-Hodgkin's lymphoma; Avastin (Genentech, Inc., CA) which is an anti-VEGF humanized antibody for the treatment of lung and colorectal cancers; Epratuzamab (Immunomedics, Inc., NJ and Amgen, CA) which is an anti-CD22 antibody for the treatment of non-Hodgkin's lymphoma; and CEAcide (Immunomedics, NJ) which is a humanized anti-CEA antibody for the treatment of colorectal cancer.

Other antibodies useful in the treatment of cancer include, but are not limited to, antibodies against the following antigens: CA125 (ovarian), CA15-3 (carcinomas), CA19-9 (carcinomas), L6 (carcinomas), Lewis Y (carcinomas), Lewis X (carcinomas), alpha fetoprotein (carcinomas), CA 242 (colorectal), placental alkaline phosphatase (carcinomas), prostate specific antigen (prostate), prostatic acid phosphatase (prostate), epidermal growth factor (carcinomas), MAGE-1 (carcinomas), MAGE-2 (carcinomas), MAGE-3 (carcinomas), MAGE-4 (carcinomas), anti-transferrin receptor (carcinomas), p97 (melanoma), MUC1-KLH (breast cancer), CEA (colorectal), gp100 (melanoma), MART1 (melanoma), PSA (prostate), IL-2 receptor (T-cell leukemia and lymphomas), CD20 (non-Hodgkin's lymphoma), CD52 (leukemia), CD33 (leukemia), CD22 (lymphoma), human chorionic gonadotropin (carcinoma), CD38 (multiple myeloma), CD40 (lymphoma), mucin (carcinomas), P21 (carcinomas), MPG (melanoma), and Neu oncogene product (carcinomas). Some specific, useful antibodies include, but are not limited to, BR96 mAb (Trail, P. A., et al Science (1993) 261, 212-215), BR64 (Trail, P A, et al Cancer Research (1997) 57, 100-105, mAbs against the CD40 antigen, such as S2C6 mAb (Francisco, J. A., et al Cancer Res. (2000) 60:3225-3231), mAbs against the CD70 antigen, such as 1F6 mAb, and mAbs against the CD30 antigen, such as AC10 (Bowen, M. A., et al (1993) J. Immunol., 151:5896-5906; Wahl et al., 2002 Cancer Res. 62 (13):3736-42). Many other internalizing antibodies that bind to tumor associated antigens can be used and have been reviewed (Franke, A. E., et al Cancer Biother Radiopharm. (2000) 15:459-76; Murray, J. L., (2000) Semin Oncol., 27:64-70; Breitling, F., and Dubel, S., Recombinant Antibodies, John Wiley, and Sons, New York, 1998).

Antibodies that bind to antigens associated with antigen presenting cells such as CD40, OX40L, Endoglin, DEC-205, 4-1BBL, CD36, CD36, CD204, MARCO, DC-SIGN, CLEC9A, CLEC5A, Dectin 2, CLEC10A, CD206, CD64, CD32A, CD1A, HVEM, CD32B, PD-L1, BDCA-2, XCR-1, and CCR2 can also be conjugated as ADCs.

Known antibodies for the treatment or prevention of an autoimmune disorders may be conjugated as ADCs. Autoimmune disorders include systemic lupus erythematosus (SLE), rheumatoid arthritis, Sjogren's syndrome, immune thromobocytopenia, and multiple sclerosis. Antibodies immunospecific for an antigen of a cell that is responsible for producing autoimmune antibodies can be obtained by any method known to one of skill in the art such as, e.g., chemical synthesis or recombinant expression techniques. SLE is marked by the overexpression of interferon-alpha (IFN-.alpha.) cytokine genes (Bennett et al (2003) Jour. Exp. Med. 197:711-723). Type-1 interferons (IFN-.alpha./.beta.) play a significant role in the pathogenesis of lupus (Santiago-Raber (2003) Jour. Exp. Med. 197:777-788). Knockout mice (-IFN-.alpha./.beta.) showed significantly reduced anti-erythrocyte autoantibodies, erythroblastosis, hemolytic anemia, anti-DNA autobodies, kidney disease, and mortality. These results suggest that Type-1 IFNs mediate murine lupus, and that reducing their activity in the human counterpart may be beneficial. Anti-IFN Ab conjugated to bis 1,8 naphthalimide drug moieties may be effective therapeutic agents against SLE and other autoimmune disorders.

In another embodiment, useful antibodies in ADC are immunospecific for the treatment of autoimmune diseases include, but are not limited to, Anti-Nuclear Antibody; Anti ds DNA; Anti ss DNA, Anti Cardiolipin Antibody IgM, IgG; Anti Phospholipid Antibody IgM, IgG; Anti SM Antibody; Anti Mitochondrial Antibody; Thyroid Antibody; Microsomal Antibody; Thyroglobulin Antibody; Anti SCL-70; Anti-Jo; Anti-U.sub. IRNP; Anti-La/SSB; Anti SSA; Anti SSB; Anti Perital Cells Antibody; Anti Histones; Anti-RNP; C-ANCA; P-ANCA; Anti centromere; Anti-Fibrillarin, and Anti-GBM Antibody.

Antibodies of an ADC can bind to both a receptor or a receptor complex expressed on an activated lymphocyte. The receptor or receptor complex can comprise an immunoglobulin gene superfamily member, a TNF receptor superfamily member, an integrin, a cytokine receptor, a chemokine receptor, a major histocompatibility protein, a lectin, or a complement control protein. Non-limiting examples of suitable immunoglobulin superfamily members are CD2, CD3, CD4, CD8, CD 19, CD22, CD28, CD79, CD90, CD 152/CTLA-4, PD-1, and ICOS. Non-limiting examples of suitable TNF receptor superfamily members are CD27, CD40, CD95/Fas, CD134/OX40, CD137/4-1BB, TNF-R1, TNFR-2, RANK, TACI, BCMA, osteoprotegerin, Apo2/TRAIL-R1, TRAIL-R2, TRAIL-R3, TRAIL-R4, and APO-3. Non-limiting examples of suitable integrins are CD11a, CD11b, CD11c, CD18, CD29, CD41, CD49a, CD49b, CD49c, CD49d, CD49e, CD49f, CD 103, and CD 104. Non-limiting examples of suitable lectins are C-type, S-type, and I-type lectin.

As used herein, the term "viral antigen" includes, but is not limited to, any viral peptide, polypeptide protein (e.g., HIV gp120, HIV nef, RSV F glycoprotein, influenza virus neuraminidase, influenza virus hemagglutinin, HTLV tax, herpes simplex virus glycoprotein (e.g., Gb, Gc, Gd, and Ge) and hepatitis B surface antigen) that is capable of eliciting an immune response. As used herein, the term "microbial antigen" includes, but is not limited to, any microbial peptide, polypeptide, protein, saccharide, polysaccharide, or lipid molecule (e.g., a bacterial, fungi, pathogenic protozoa, or yeast polypeptide including, e.g., LPS and capsular polysaccharide 5/8) that is capable of eliciting an immune response.

Antibodies immunospecific for a viral or microbial antigen can be obtained commercially, for example, from BD Biosciences (San Francisco, Calif.), Chemicon International, Inc. (Temecula, Calif.), or Vector Laboratories, Inc. (Burlingame, Calif.) or produced by any method known to one of skill in the art such as, e.g., chemical synthesis or recombinant expression techniques. The nucleotide sequence encoding antibodies that are immunospecific for a viral or microbial antigen can be obtained, e.g., from the GenBank database or a database like it, the literature publications, or by routine cloning and sequencing.

In some embodiments, useful antibodies in the present ADCs are those that treat or prevent viral or microbial infection in accordance with the methods disclosed herein. Examples of antibodies available useful for the treatment of viral infection or microbial infection include, but are not limited to, SYNAGIS (MedImmune, Inc., MD) which is a humanized anti-respiratory syncytial virus (RSV) monoclonal antibody useful for the treatment of patients with RSV infection; PRO542 (Progenics) which is a CD4 fusion antibody useful for the treatment of HIV infection; OSTAVIR (Protein Design Labs, Inc., CA) which is a human antibody useful for the treatment of hepatitis B virus; PROTOVIR (Protein Design Labs, Inc., CA) which is a humanized IgG, antibody useful for treating cytomegalovirus (CMV); and anti-LPS antibodies.

Other antibodies useful in ADCs for the treatment of infectious diseases include, but are not limited to, antibodies against the antigens from pathogenic strains of bacteria (Streptococcus pyogenes, Streptococcus pneumoniae, Neisseria gonorrheae, Neisseria meningitidis, Corynebacterium diphtheriae, Clostridium botulinum, Clostridium perfringens, Clostridium tetani, Hemophilus influenzae, Klebsiella pneumoniae, Klebsiella ozaenas, Klebsiella rhinoscleromotis, Staphylococcus aureus, Vibrio colerae, Escherichia coli, Pseudomonas aeruginosa, Campylobacter (Vibrio) fetus, Aeromonas hydrophila, Bacillus cereus, Edwardsiella tarda, Yersinia enterocolitica, Yersinia pestis, Yersinia pseudotuberculosis, Shigella dysenteriae, Shigella flexneri, Shigella sonnei, Salmonella typhimurium, Treponema pallidum, Treponema pertenue, Treponema carateneum, Borrelia vincentii, Borrelia burgdorferi, Leptospira icterohemorrhagiae, Mycobacterium tuberculosis, Pneumocystis carinii, Francisella tularensis, Brucella abortus, Brucella suis, Brucella melitensis, Mycoplasma spp., Rickettsia prowazeki, Rickettsia tsutsugumushi, Chlamydia spp.); pathogenic fungi (Coccidioides immitis, Aspergillus fumigatus, Candida albicans, Blastomyces dermatitidis, Cryptococcus neoformans, Histoplasma capsulatum); protozoa (Entomoeba histolytica, Toxoplasma gondii, Trichomonas tenas, Trichomonas hominis, Trichomonas vaginalis, Tryoanosoma gambiense, Trypanosoma rhodesiense, Trypanosoma cruzi, Leishmania donovani, Leishmania tropica, Leishmania braziliensis, Pneumocystis pneumonia, Plasmodium vivax, Plasmodium falciparum, Plasmodium malaria); or Helminiths (Enterobius vermicularis, Trichuris trichiura, Ascaris lumbricoides, Trichinella spiralis, Strongyloides stercoralis, Schistosoma japonicum, Schistosoma mansoni, Schistosoma haematobium, and hookworms).

Other antibodies useful in ADCs for treatment of viral disease include, but are not limited to, antibodies against antigens of pathogenic viruses, including as examples and not by limitation: Poxviridae, Herpesviridae, Herpes Simplex virus 1, Herpes Simplex virus 2, Adenoviridae, Papovaviridae, Enteroviridae, Picornaviridae, Parvoviridae, Reoviridae, Retroviridae, influenza viruses, parainfluenza viruses, mumps, measles, respiratory syncytial virus, rubella, Arboviridae, Rhabdoviridae, Arenaviridae, Hepatitis A virus, Hepatitis B virus, Hepatitis C virus, Hepatitis E virus, Non-A/Non-B Hepatitis virus, Rhinoviridae, Coronaviridae, Rotoviridae, and Human Immunodeficiency Virus.

The term "amino acid sequence variant" refers to polypeptides having amino acid sequences that differ to some extent from a native sequence polypeptide. Ordinarily, amino acid sequence variants will possess at least about 70% sequence identity with at least one receptor binding domain of a native antibody or with at least one ligand binding domain of a native receptor, and typically, they will be at least about 80%, more typically, at least about 90% homologous by sequence with such receptor or ligand binding domains. The amino acid sequence variants possess substitutions, deletions, and/or insertions at certain positions within the amino acid sequence of the native amino acid sequence. Amino acids are designated by the conventional names, one-letter and three-letter codes.

"Sequence identity" is defined as the percentage of residues in the amino acid sequence variant that are identical after aligning the sequences and introducing gaps, if necessary, to achieve the maximum percent sequence identity. Methods and computer programs for the alignment are well known in the art. One such computer program is "Align 2," authored by Genentech, Inc., which was filed with user documentation in the United States Copyright Office, Washington, D.C. 20559, on Dec. 10, 1991.

The terms "Fc receptor" or "FcR" are used to describe a receptor that binds to the Fc region of an antibody. An exemplary FcR is a native sequence human FcR. Moreover, a FcR may be one which binds an IgG antibody (a gamma receptor) and includes receptors of the Fc.gamma.RI, Fc.gamma.RII, and Fc.gamma. RIII subclasses, including allelic variants and alternatively spliced forms of these receptors. Fc.gamma.RII receptors include Fc.gamma.RIIA (an "activating receptor") and Fc.gamma.RIIB (an "inhibiting receptor"), which have similar amino acid sequences that differ primarily in the cytoplasmic domains thereof. Activating receptor Fc.gamma.RIIA contains an immunoreceptor tyrosine-based activation motif (ITAM) in its cytoplasmic domain. Inhibiting receptor Fc.gamma.RIIB contains an immunoreceptor tyrosine-based inhibition motif (ITIM) in its cytoplasmic domain. (See review M. in Daeron, Annu. Rev. Immunol., 15:203-234 (1997)). FcRs are reviewed in Ravetch and Kinet, Annu. Rev. Immunol., 9:457-92 (1991); Capel et al., Immunomethods, 4:25-34 (1994); and de Haas et al., J. Lab. Clin. Med., 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol., 117:587 (1976) and Kim et al., J. Immunol., 24:249 (1994)).

"Complement dependent cytotoxicity" or "CDC" refers to the ability of a molecule to lyse a target in the presence of complement. The complement activation pathway is initiated by the binding of the first component of the complement system (C1q) to a molecule (e.g., an antibody) complexed with a cognate antigen. To assess complement activation, a CDC assay, e.g., as described in Gazzano-Santoro et al., J. Immunol. Methods, 202:163 (1996), may be performed.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end. The constant domain of the light chain is aligned with the first constant domain of the heavy chain, and the light-chain variable domain is aligned with the variable domain of the heavy chain. Particular amino acid residues are believed to form an interface between the light chain and heavy chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a beta.-sheet configuration, connected by three hypervariable regions, which form loops connecting, and in some cases forming part of, the .beta.-sheet structure. The hypervariable regions in each chain are held together in close proximity by the FRs and, with the hypervariable regions from the other chain, contribute to the formation of the antigen-binding site of antibodies (see Kabat et al (1991) Sequences of Proteins of Immunological Interest, 5th Ed. Public Health Service, National Institutes of Health, Bethesda, Md.). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody which are responsible for antigen-binding. The hypervariable region generally comprises amino acid residues from a "complementarity determining region" or "CDR" (e.g., residues 24-34 (L1), 50-56 (L2) and 89-97 (L3) in the light chain variable domain and 31-35 (H1), 50-65 (H2) and 95-102 (H3) in the heavy chain variable domain; Kabat et al supra) and/or those residues from a "hypervariable loop" (e.g., residues 26-32 (L1), 50-52 (L2) and 91-96 (L3) in the light chain variable domain and 26-32 (H1), 53-55 (H2) and 96-101 (H3) in the heavy chain variable domain; Chothia and Lesk (1987) J. Mol. Biol., 196:901-917). "Framework Region" or "FR" residues are those variable domain residues other than the hypervariable region residues as herein defined.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the VH-VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')2 antibody fragments originally were produced as pairs of Fab' fragments which have hinge cysteines between them. Other chemical couplings of antibody fragments are also known.

The "light chains"of antibodies from any vertebrate species can be assigned to one of two clearly distinct types, called kappa and lambda, based on the amino acid sequences of their constant domains.

"Single-chain Fv" or "scFv" antibody fragments comprise the VH and VL domains of antibody, wherein these domains are present in a single polypeptide chain. The Fv polypeptide may further comprise a polypeptide linker between the VH and VL domains which enables the scFv to form the desired structure for antigen binding. For a review of scFv, see Pluckthun in The Pharmacology of Monoclonal Antibodies, vol. 113, Rosenburg and Moore eds., Springer-Verlag, New York, pp. 269-315 (1994). Anti-ErbB2 antibody scFv fragments are described in WO 93/16185; U.S. Pat. Nos. 5,571,894; and 5,587,458.

The term "diabodies" refers to small antibody fragments with two antigen-binding sites, which fragments comprise a variable heavy domain (VH) connected to a variable light domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al (1993) Proc. Natl. Acad. Sci. USA 90:6444-6448.

"Humanized" forms of non-human (e.g., rodent) antibodies are chimeric antibodies that contain minimal sequence derived from non-human immunoglobulin. Humanization is a method to transfer the murine antigen binding information to a non-immunogenic human antibody acceptor, and has resulted in many therapeutically useful drugs. The method of humanization generally begins by transferring all six murine complementarity determining regions (CDRs) onto a human antibody framework (Jones et al, (1986) Nature 321:522-525). These CDR-grafted antibodies generally do not retain their original affinity for antigen binding, and in fact, affinity is often severely impaired. Besides the CDRs, select non-human antibody framework residues must also be incorporated to maintain proper CDR conformation (Chothia et al (1989) Nature 342:877). The transfer of key mouse framework residues to the human acceptor in order to support the structural conformation of the grafted CDRs has been shown to restore antigen binding and affinity (Riechmann et al., (1992) J. Mol. Biol. 224, 487-499; Foote and Winter, (1992) J. Mol. Biol. 224:487-499; Presta et al., (1993) J. Immunol. 151, 2623-2632; Werther et al., (1996) J. Immunol. Methods 157:4986-4995; and Presta et al (2001) Thromb. Haemost. 85:379-389). For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see U.S. Pat. No. 6,407,213; Jones et al (1986) Nature, 321:522-525; Riechmann et al (1988) Nature 332:323-329; and Presta, (1992) Curr. Op. Struct. Biol., 2:593-596.

A "parent antibody" is an antibody comprising an amino acid sequence from which one or more amino acid residues are replaced by one or more cysteine residues. The parent antibody may comprise a native or wild type sequence. The parent antibody may have pre-existing amino acid sequence modifications (such as additions, deletions and/or substitutions) relative to other native, wild type, or modified forms of an antibody. A parent antibody is directed against a target antigen of interest. Antibodies directed against nonpolypeptide antigens (such as tumor-associated glycolipid antigens; see U.S. Pat. No. 5,091,178) are also contemplated.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In certain embodiments, the antibody will be purified (1) to greater than 95% by weight of antibody as determined by the Lowry method, or more than 99% by weight, (2) to a degree sufficient to obtain at least 15 residues of N-terminal or internal amino acid sequence by use of a gas phase protein sequencer, or (3) to homogeneity by SDS-PAGE under reducing or nonreducing conditions using Coomassie blue or silver stain. Isolated antibody includes the antibody in situ within recombinant cells since at least one component of the antibody's natural environment will not be present. Ordinarily, however, isolated antibody will be prepared by at least one purification step.

An antibody "which binds" a molecular target or an antigen of interestis one capable of binding that antigen with sufficient affinity such that the antibody is useful in targeting a cell expressing the antigen.

The terms "treat" or "treatment" refer to both therapeutic treatment and prophylactic or preventative measures, wherein the object is to prevent or slow down (lessen) an undesired physiological change or disorder, such as the development or spread of cancer. For purposes of this disclosure, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

"Phage display" is a technique by which variant polypeptides are displayed as fusion proteins to a coat protein on the surface of phage, e.g., filamentous phage, particles. One utility of phage display lies in the fact that large libraries of randomized protein variants can be rapidly and efficiently sorted for those sequences that bind to a target molecule with high affinity. Display of peptide and protein libraries on phage has been used for screening millions of polypeptides for ones with specific binding properties. Polyvalent phage display methods have been used for displaying small random peptides and small proteins, typically through fusions to either PIII or PVIII of filamentous phage. Wells and Lowman, Curr. Opin. Struct. Biol., 3:355-362 (1992), and references cited therein. In monovalent phage display, a protein or peptide library is fused to a phage coat protein or a portion thereof, and expressed at low levels in the presence of wild type protein. Avidity effects are reduced relative to polyvalent phage so that sorting is on the basis of intrinsic ligand affinity, and phagemid vectors are used, which simplify DNA manipulations. Lowman and Wells, Methods: A companion to Methods in Enzymology, 3:205-0216 (1991). Phage display includes techniques for producing antibody-like molecules (Janeway, C., Travers, P., Walport, M., Shlomchik (2001) Immunobiology, 5th Ed., Garland Publishing, New York, p 627-628).

A "phagemid" is a plasmid vector having a bacterial origin of replication, e.g., Co1E1, and a copy of an intergenic region of a bacteriophage. The phagemid may be used on any known bacteriophage, including filamentous bacteriophage and lambdoid bacteriophage. The plasmid will also generally contain a selectable marker for antibiotic resistance. Segments of DNA cloned into these vectors can be propagated as plasmids. When cells harboring these vectors are provided with all genes necessary for the production of phage particles, the mode of replication of the plasmid changes to rolling circle replication to generate copies of one strand of the plasmid DNA and package phage particles. The phagemid may form infectious or non-infectious phage particles. This term includes phagemids which contain a phage coat protein gene or fragment thereof linked to a heterologous polypeptide gene as a gene fusion such that the heterologous polypeptide is displayed on the surface of the phage particle. The compounds described herein can be in the form of pharmaceutically or pharmaceutically acceptable salts. In some embodiments, such salts are derived from inorganic or organic acids or bases. For reviews of suitable salts, *see, e.g.,* Berge et al., J. Pharm. Sci., 1977, 66, 1-19 and Remington: The Science and Practice of Pharmacy, 20th Ed., A. Gennaro (ed.), Lippincott Williams & Wilkins (2000).

In the present disclosure, group "Ab" (i.e., the antibodies, antibody fragments, and/or antigen fragments) can be conjugated to more than one drug-containing moiety. In some embodiments, "Ab" can be conjugated to from 1 to 20 drug-containing moieties. In some embodiments, "Ab" can be conjugated to from 1 to 10 drug-containing moieties. In some embodiments, "Ab" can be conjugated to from 1 to 5 drug-containing moieties. In some embodiments, "Ab" can be conjugated to from 1 or 2 drug-containing moieties. In some embodiments, "Ab" can be conjugated to one drug-containing moiety.

The compounds described herein can be in the form of pharmaceutically or pharmaceutically acceptable salts. In some embodiments, such salts are derived from inorganic or organic acids or bases. For reviews of suitable salts, *see, e.g.,* Berge et al., J. Pharm. Sci., 1977, 66, 1-19 and Remington: The Science and Practice of Pharmacy, 20th Ed., A. Gennaro (ed.), Lippincott Williams & Wilkins (2000).

Examples of suitable acid addition salts include acetate, adipate, alginate, aspartate, benzoate, benzene sulfonate, bisulfate, butyrate, citrate, camphorate, camphor sulfonate, cyclopentanepropionate, digluconate, dodecylsulfate, ethanesulfonate, fumarate, lucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenyl-propionate, picrate, pivalate, propionate, succinate, tartrate, thiocyanate, tosylate and undecanoate.

Examples of suitable base addition salts include ammonium salts; alkali metal salts, such as sodium and potassium salts; alkaline earth metal salts, such as calcium and magnesium salts; salts with organic bases, such as dicyclohexylamine salts, *N*-methyl-D-glucamine; and salts with amino acids such as arginine, lysine, and the like.

For example, Berge lists the following FDA-approved commercially marketed salts: anions acetate, besylate (benzenesulfonate), benzoate, bicarbonate, bitartrate, bromide, calcium edetate (ethylenediaminetetraacetate), camsylate (camphorsulfonate), carbonate, chloride, citrate, dihydrochloride, edetate (ethylenediaminetetraacetate), edisylate (1,2-ethanedisulfonate), estolate (lauryl sulfate), esylate (ethanesulfonate), fumarate, gluceptate (glucoheptonate), gluconate, glutamate, glycollylarsanilate (glycollamidophenylarsonate), hexylresorcinate, hydrabamine (*N,N*'-di(dehydroabietyl)ethylenediamine), hydrobromide, hydrochloride, hydroxynaphthoate, iodide, isethionate (2-hydroxyethanesulfonate), lactate, lactobionate, malate, maleate, mandelate, mesylate (methanesulfonate), methylbromide, methylnitrate, methylsulfate, mucate, napsylate (2-naphthalenesulfonate), nitrate, pamoate (embonate), pantothenate, phosphate/diphosphate, polygalacturonate, salicylate, stearate, subacetate, succinate, sulfate, tannate, tartrate, teoclate (8-chlorotheophyllinate) and triethiodide; organic cations benzathine (*N,N*'-dibenzylethylenediamine), chloroprocaine, choline, diethanolamine, ethylenediamine, meglumine (*N*-methylglucamine) and procaine; and metallic cations aluminum, calcium, lithium, magnesium, potassium, sodium and zinc.

Berge additionally lists the following non-FDA-approved commercially marketed (outside the United States) salts: anions adipate, alginate, aminosalicylate, anhydromethylenecitrate, arecoline, aspartate, bisulfate, butylbromide, camphorate, digluconate, dihydrobromide, disuccinate, glycerophosphate, hemisulfate, hydrofluoride, hydroiodide, methylenebis(salicylate), napadisylate (1,5-naphthalenedisulfonate), oxalate, pectinate, persulfate, phenylethylbarbiturate, picrate, propionate, thiocyanate, tosylate and undecanoate; organic cations benethamine (*N*-benzylphenethylamine), clemizole (1-*p*-chlorobenzyl-2-pyrrolildine-1'-ylmethylbenzimidazole), diethylamine, piperazine and tromethamine (tris(hydroxymethyl)aminomethane); and metallic cations barium and bismuth.

The compounds described herein may also comprise suitable carriers, excipients, and auxiliaries that may differ depending on the mode of administration.

In some embodiments, the pharmaceutical compositions can be formulated as a suitable parenteral dosage form. Said formulations can be prepared by various methods known in the art. The pharmaceutical compositions can be administered directly into the bloodstream, into muscle, or directly into an organ. Suitable means for parenteral administration include intravenous, intraarterial, intraperitoneal, intrathecal, intraventricular, intraurethral, intrasternal, intracranial, intramuscular, and subcutaneous. Suitable devices for parenteral administration include needle injectors, needle-free injectors, and infusion techniques.

Parenteral compositions are typically aqueous solutions which may contain excipients such as salts, carbohydrates and buffering agents. However, the composition may also be formulated a sterile non-aqueous solution or as a dried form to be used in conjunction with a suitable vehicle such as sterile pyrogen-free water.

The preparation of parenteral compositions under sterile conditions, for example, by lyophilization, can be readily accomplished using standard techniques known well to those of skill in the art.

Compositions for parenteral administration can be formulated to be immediate and/or modified release. Modified release formulations include delayed-, sustained-, pulsed-, controlled-, targeted, and programmed release. Thus, the compositions can be formulated as a solid, semi-solid, or thixotropic liquid for administration as an implanted depot providing modified release of the active agent.

The parenteral formulations can be admixed with other suitable pharmaceutically acceptable excipients used in parenteral dosage forms such as, but not limited to, preservatives.

In another embodiment, the pharmaceutical compositions can be formulated as suitable oral dosage forms such as tablets, capsules, powders, pellets, suspensions, solutions, emulsions, and the like. Other suitable carriers can be present such as disintegrants, diluents, chelating agents, binders, glidants, lubricants, fillers, bulking agents, anti-adherants, and the like.

Oral dosage formulations may also contain other suitable pharmaceutical excipients such as sweeteners, vehicle/wetting agents, coloring agents, flavoring agents, preservatives, viscosity enhancing/thickening agents, and the like.

The dose of the pharmaceutical compositions of the present disclosure can be tailored to the individual patient.

### Lipid Conjugates

In certain embodiments, the present disclosure provides a compound of formula (VI), 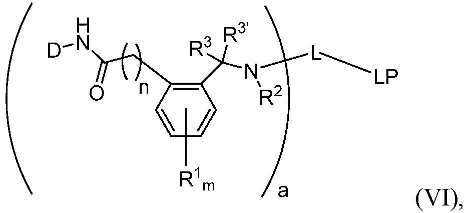 or a pharmaceutically acceptable salt thereof, wherein:
a is an integer from 1 to 20;
m is 0, 1, 2, 3, or 4;
n is 0 or 1;
D-NH- is a portion of an amino-substituted compound, wherein the amino-substituted compound has the formula D-NH₂;
each R¹ is independently selected from C₁-C₄alkyl, O-C₁-C₄alkyl, and halogen;
R² is selected from C₁-C₄alkyl and -(CH₂CH₂O)ₛ-CH_{3;} wherein s is an integer from 1 to 10;
R³ and R^{3'} are each independently selected from hydrogen and C₁-C₃alkyl;
L is a cleavable linker; and
LP is a lipid.

In some embodiments, D-NH₂ is a STING modulator. In some embodiments, D-NH2 is a cyclic dinucleotide (CDN) or a CDN-like compound. In some embodiments, D-NH₂ is one of the formulas described herein.

In certain embodiments, the present disclosure provides a compound of formula (VI), wherein L has a formula 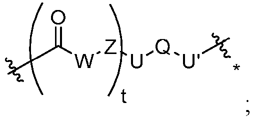 wherein W, Z, t, U, Q, and U' are as described herein.

In some embodiments, the compound of formula (VI) is associated with a lipid complex. The term "lipid complex" is an art-recognized term in the preparation of pharmaceutical compounds. Lipid complexes are characterized by non-covalent bonding between the lipid and the compound of formula (VI). Without being bound by a particular theory, compound of formula (VI) can be associated with the lipid complex through electrostatic interaction, hydrophilic interaction, hydrophobic interactions, or any combination thereof.

In some embodiments the lipid complexes described herein take the form of lipid particles. In one embodiment, the complexes are in the form of lipid nanoparticles. In one embodiment, the complexes are in the form of liposomes. In some embodiments, the liposomes of the present disclosure comprise a lipid monolayer or a lipid multi-layer. In some embodiments the liposome comprises a lipid bilayer and an aqueous core. In some embodiments, the average particle size of the liposome is from about 5 nm to about 1 µm. In some embodiments, the average particle size of the lipid nanoparticle or liposome is from about 5 nm to about 500 nm, from about 10 nm to about 150 nm, and from about 30 nm to about 100 nm. In some embodiments the compound of formula (V) resides in the aqueous core, the lipid bilayer, or a combination thereof.

In certain embodiments, LP can be cholesterol or a phospholipid. In some embodiments, LP is a phospholipid. In some embodiments the phospholipid is selected from phosphatidylcholine, phosphatidylgycerol, phosphatidic acid, phosphatidylethanolamine, phosphatidylserine, soybean phospholipid, and egg yolk phospholipid. In some embodiments, the phospholipid is a phosphatidylethanolamine. In some embodiments, the phospholipid is 1,2-distearoyl-*sn*-glycero-3-phosphorylethanolamine.

In some embodiments, In some embodiments, the present disclosure provides a lipid complex comprising a compound of formula (VI). In some embodiments, the lipid complex can be a liposome.

In some embodiments the lipid complex comprises from about 0.5 to about 40 mole percent of the compound of formula (VI), or a pharmaceutically acceptable salt thereof. In some embodiments the lipid complex comprises from about 1 to about 35 mole percent of the compound of formula (VI), or a pharmaceutically acceptable salt thereof. In some embodiments the lipid complex comprises from about 1 to about 30 mole percent of the compound of formula (VI), or a pharmaceutically acceptable salt thereof. In some embodiments the lipid complex comprises from about 1 to about 20 mole percent of the compound of formula (VI), or a pharmaceutically acceptable salt thereof. In some embodiments the lipid complex comprises from about 1 to about 15 mole percent of the compound of formula (VI), or a pharmaceutically acceptable salt thereof.

In some embodiments, the lipid complex further comprises one or more additional phospholipids. In some embodiments, the phospholipid is selected from phosphatidylcholine, phosphatidylgycerol, phosphatidic acid, phosphatidylethanolamine, phosphatidylserine, sphingomyelin, soybean phospholipid, and egg yolk phospholipid.

In some embodiments the lipid complex comprises from about 30 to about 90 mole percent of one or more phospholipids. In some embodiments the lipid complex comprises from about 40 to about 85 mole percent of one or more phospholipids. In some embodiments the lipid complex comprises from about 50 to about 80 mole percent of one or more phospholipids.

In some embodiments, the lipid complex further comprises a fatty alcohol or cholesterol. In some embodiments the lipid complex comprises from about 5 to about 35 mole percent of cholesterol. In some embodiments the lipid complex comprises from about 5 to about 30 mole percent of cholesterol. In some embodiments the lipid complex comprises from about 5 to about 25 mole percent of cholesterol.

In some embodiments the lipid complex further comprises at least one PEGylated phospholipid. In some embodiments, the at least one PEGylated lipid is a PEGylated phospholipid. In some embodiments, the PEGylated phospholipid is selected from N-(carbonyl-methoxypolyethyleneglycol 5000)-1,2-distearoyl-*sn*-glycero-3-phosphoethanolamine, N-(carbonyl-methoxypolyethyleneglycol 2000)-1,2-distearoyl-*sn*-glycero-3-phosphoethanolamine, N-(carbonyl-methoxypolyethyleneglycol 2000)-1,2-dipalmitoyl-*sn*-glycero-3-phosphoethanolamine, N-(carbonyl-methoxypolyethyleneglycol 5000)-1,2-dipalmitoyl-*sn-*glycero-3-phosphoethanolamine, N-(carbonyl-methoxypolyethyleneglycol 2000)-1,2-dimyristoyl-sn-glycero-3-phosphoethanolamine ,N-(carbonyl-methoxypolyethyleneglycol 5000)-1,2-dimyristoyl-*sn*-glycero-3-phosphoethanolamine, N-(carbonyl-methoxypolyethyleneglycol 2000)-distearoyl-*rac*-glycerol, and N-(carbonyl-methoxypolyethyleneglycol 5000)-distearoyl-*rac*-glycerol, apharmaceutically acceptable salt, or mixtures thereof. In some embodiments the PEGylated phospholipid is N-(carbonyl-methoxypolyethyleneglycol 2000)-1,2-distearoyl-*sn-*glycero-3-phosphoethanolamine.

In some embodiments the lipid complex comprises from about 0 to about 6 mole percent of one or more PEGylated phospholipids. In some embodiments the lipid complex comprises from about 0.5 and 5 mole percent of one or more PEGylated phospholipids. In some embodiments the lipid complex comprises from about 1 and 5 mole percent of one or more PEGylated phospholipids. In some embodiments the lipid complex comprises about 3 mole percent of one or more PEGylated phospholipids. In some embodiments the lipid complex comprises about 4 mole percent of one or more PEGylated phospholipids. In some embodiments the lipid complex comprises about 5 mole percent of one or more PEGylated phospholipids.

### Method of Use of Compounds and Compositions

Certain compounds described herein are STING agonists and thus are useful in stimulating an immune response in subjects thereof. The compositions can be used in the treatment of viruses.

Compounds of the present disclosure show STING modulating/agonistic activity. Certain compounds of the present disclosure can be superior in terms of efficacy expression, pharmacokinetics (e.g., absorption, distribution, metabolism, excretion), solubility (e.g., water solubility), interaction with other medicaments (e.g., drug-metabolizing enzyme inhibitory action), safety (e.g., acute toxicity, chronic toxicity, genetic toxicity, reproductive toxicity, cardiotoxicity, carcinogenicity, central toxicity) and/or stability (e.g., chemical stability, stability to an enzyme), and can be useful as a medicament.

A compound of the present disclosure can be used for increasing STING activity in a mammal (e.g., mouse, rat, hamster, rabbit, cat, dog, cow, sheep, monkey, human).

A compound of the present disclosure can be used as a medicament such as an agent for the prophylaxis or treatment of diseases that can be influenced by STING (in the present specification, sometimes to be abbreviated as "STING-related diseases"), for example, cancers - e.g., colorectal cancers (e.g., colorectal cancer, rectal cancer, anus cancer, familial colorectal cancer, hereditary nonpolyposis colorectal cancer, gastrointestinal stromal tumor), lung cancers (e.g., non-small-cell lung cancer, small-cell lung cancer, malignant mesothelioma), mesothelioma, pancreatic cancers (e.g., pancreatic ductal carcinoma, pancreatic endocrine tumor), pharynx cancer, larynx cancer, esophageal cancer, stomach cancers (e.g., papillary adenocarcinoma, mucinous adenocarcinoma, adenosquamous carcinoma), duodenal cancer, small intestinal cancer, breast cancers (e.g., invasive ductal carcinoma, non-invasive ductal carcinoma, inflammatory breast cancer), ovarian cancers (e.g., ovarian epithelial cancer, extragonadal germ cell tumor, ovarian germ cell tumor, ovarian low-malignant potential tumor), testis tumor, prostate cancers (e.g., hormone-dependent prostate cancer, non-hormone dependent prostate cancer, castration-resistant prostate cancer), liver cancers (e.g., hepatocellular cancer, primary liver cancer, extrahepatic bile duct cancer), thyroid cancers (e.g., medullary thyroid carcinoma), renal cancers (e.g., renal cell cancers (e.g., clear cell renal cell cancer), transitional cell cancer of renal pelvis and ureter), uterine cancers (e.g., cervical cancer, uterine body cancer, uterus sarcoma), gestational choriocarcinoma, brain tumors (e.g., medulloblastoma, glioma, pineal astrocytic tumors, pilocytic astrocytoma, diffuse astrocytoma, anaplastic astrocytoma, pituitary adenoma), retinoblastoma, skin cancers (e.g., basalioma, malignant melanoma), sarcomas (e.g., rhabdomyosarcoma, leiomyosarcoma, soft tissue sarcoma, spindle cell sarcoma), malignant bone tumor, bladder cancer, blood cancers (e.g., multiple myeloma, leukemias (e.g., acute myelogenous leukemia), malignant lymphoma, Hodgkin's disease, chronic myeloproliferative disease), cancer of unknown primary; a cancer growth inhibitor; a cancer metastasis inhibitor; an apoptosis promoter; an agent for the treatment of precancerous lesions (e.g., myelodysplastic syndromes); and the like.

In certain embodiments, a compound of the present disclosure can be used as a medicament for colorectal cancer, breast cancer, skin cancer, malignant lymphoma or lung cancer.

Furthermore, a compound of the present disclosure or the combination agent of the present disclosure can be used concurrently with a non-drug therapy. To be precise, a compound of the present disclosure or the combination agent of the present disclosure can be combined with a non-drug therapy such as (1) surgery, (2) hypertensive chemotherapy using angiotensin II etc., (3) gene therapy, (4) thermotherapy, (5) cryotherapy, (6) laser cauterization and (7) radiotherapy.

For example, by using a compound of the present disclosure before or after the above-mentioned surgery and the like, or before or after a combined treatment of two or three kinds thereof, effects such as prevention of emergence of resistance, prolongation of Disease-Free Survival, suppression of cancer metastasis or recurrence, prolongation of life and the like may be afforded.

In addition, it is possible to combine a treatment with a compound of the present disclosure or the combination agent of the present disclosure with a supportive therapy: (i) administration of antibiotic (e.g., β-lactam type such as pansporin and the like, macrolide type such as clarithromycin and the like) for the complication with various infectious diseases, (ii) administration of high-calorie transfusion, amino acid preparation or general vitamin preparation for the improvement of malnutrition, (iii) administration of morphine for pain mitigation, (iv) administration of a pharmaceutical agent for ameliorating side effects such as nausea, vomiting, anorexia, diarrhea, leucopenia, thrombocytopenia, decreased hemoglobin concentration, hair loss, hepatopathy, renopathy, DIC, fever and the like and (v) administration of a pharmaceutical agent for suppressing multiple drug resistance of cancer and the like.

### Examples

### General Synthetic Methods and Intermediates

The compounds of the present disclosure can be prepared by one of ordinary skill in the art in light of the present disclosure and knowledge in the art, and/or by reference to the schemes shown below and the synthetic examples. Exemplary synthetic routes are set forth in Schemes below and in Examples. It should be understood that the variables, for example "R" groups) appearing in the following schemes and examples are to be read independently from those appearing elsewhere in the application. One of ordinary skill in the art would readily understand how the schemes and examples shown below illustrate the preparation of the compounds described herein.

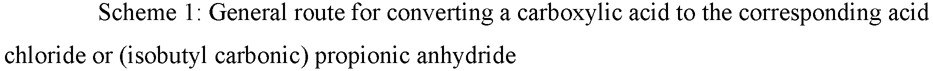

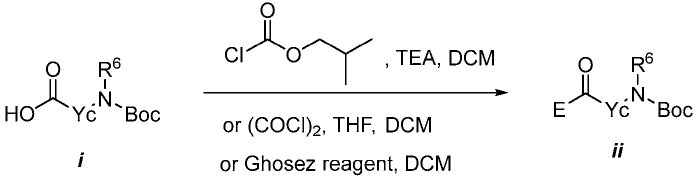

Scheme 1 shows a general route for activating the carboxylic acid i to the corresponding acid chloride or (isobutyl carbonic) propionic anhydride such as *ii,* where Yc is an alkyl or aromatic chain, R⁶ is methyl or a polyether-containing alkyl chain. Preparation of *ii* where E is OCO₂CH₂CH(CH₃)₂ can be achieved by treating i with isobutyl chloroformate in presence of a base. When E is Cl, formation of *ii* is achieved by treating *i* with oxalyl chloride or with Ghosez reagent (*See* α-Chloro enamines, reactive intermediates for synthesis: 1-Chloro-N,N,2-trimethylpropenylamine. Haveaux B., et al. Org. Synth. 1980, 59, 26.)

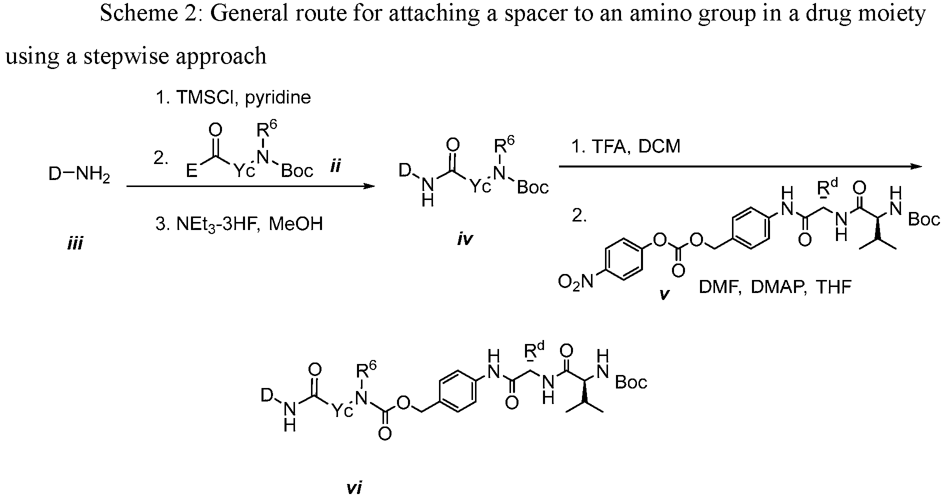

Scheme 2 shows a general method for attaching a spacer such as *ii* to an amino group in a drug moiety such as *iii.* After compound *iii* is subjected to a global silyl protection by treating with trimethyl silyl chloride and pyridine, it is reacted with activated spacer *ii.* Removal of the silyl groups with NEt₃-3HF gives *iv.* Deprotection of the amine of *iv* is accomplished by treatment with an acid such as TFA or HCl, and the resulting amine salt is treated with an activated dipeptide carbonate *v* to afford *vi.*

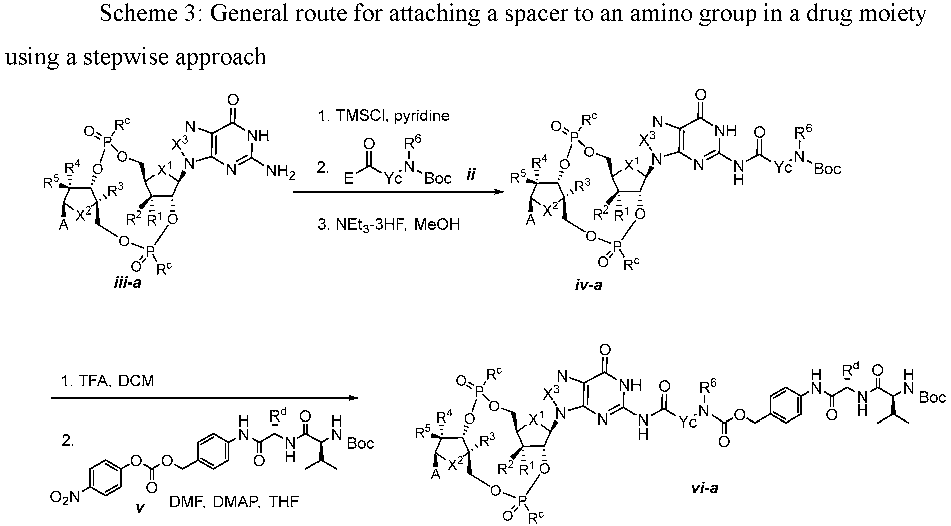

Scheme 3 shows a general method for attaching a spacer such as *ii* to an amino group in a drug moiety such as *iii-a* where X¹and X² are oxygen, sulfur or CH₂, X³ is nitrogen or CH, R³ is hydrogen or fluoro, R⁴ is an hydroxyl, hydrogen, fluoro or is taken together with R³ to form OCH₂, R^{c} is OH or SH, R^{d} is methyl or (CH₂)₃NHC(O)NH₂. After compound *iii-a* is subjected to a global silyl protection by treating with trimethyl silyl chloride and pyridine, it is reacted with activated spacer *ii.* Removal of the silyl groups with NEt₃-3HF gives *iv-a.* Deprotection of the amine of *iv-a* is accomplished by treatment with an acid such as TFA or HCl, and the resulting amine salt is treated with an activated dipeptide carbonate *v* to afford *vi-a.*

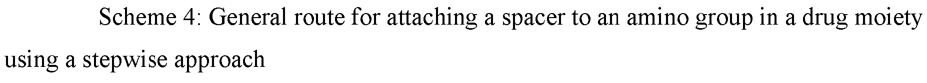

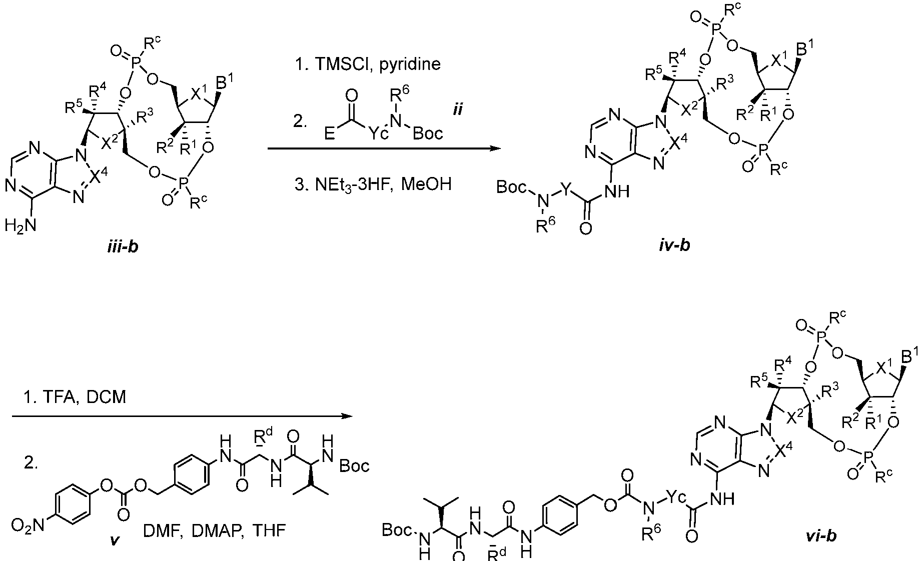

Scheme 4 shows a general method for attaching a spacer group such as *ii* to an amino group in a drug moiety such as *iii-b* following the general method described in Scheme 3.

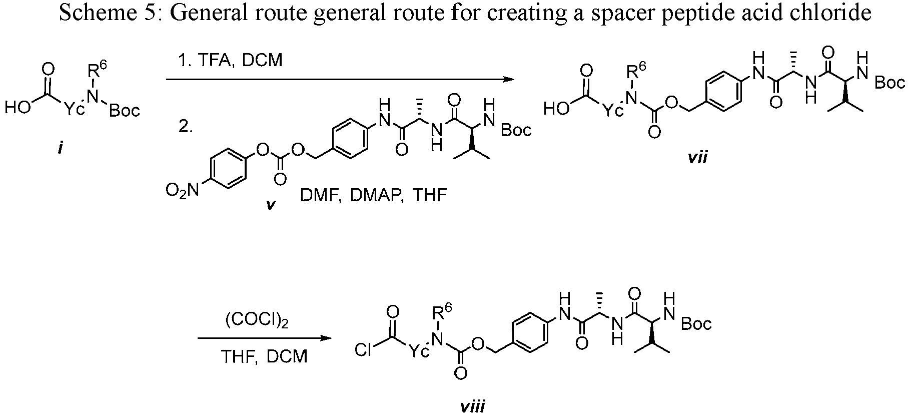

Scheme 5 shows a general method for the preparation of acid chloride *viii.* Deprotection of Boc-amine *i* using an acid such as TFA or HCl is followed by treatment with the activated carbonate *v* to give carboxylic acid *vii.* Conversion of *vii* to the acid chloride *viii* can be accomplished by treatment with oxalyl chloride or sulfonyl chloride.

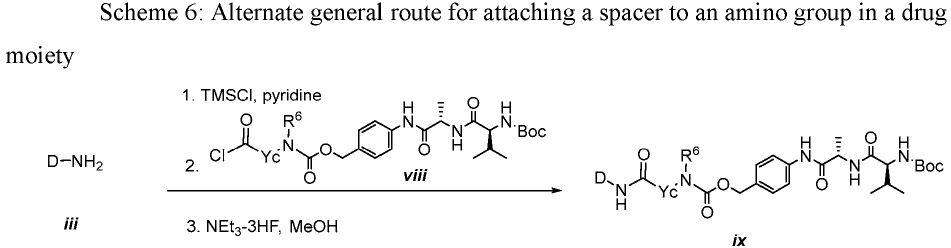

Scheme 6 shows an alternate general method for the direct conversion of payload *iii* to compound *ix.* After global silyl protection with trimethylsilyl chloride, *iii* is treated with acid chloride *viii.* Removal of silyl group gives *ix.*

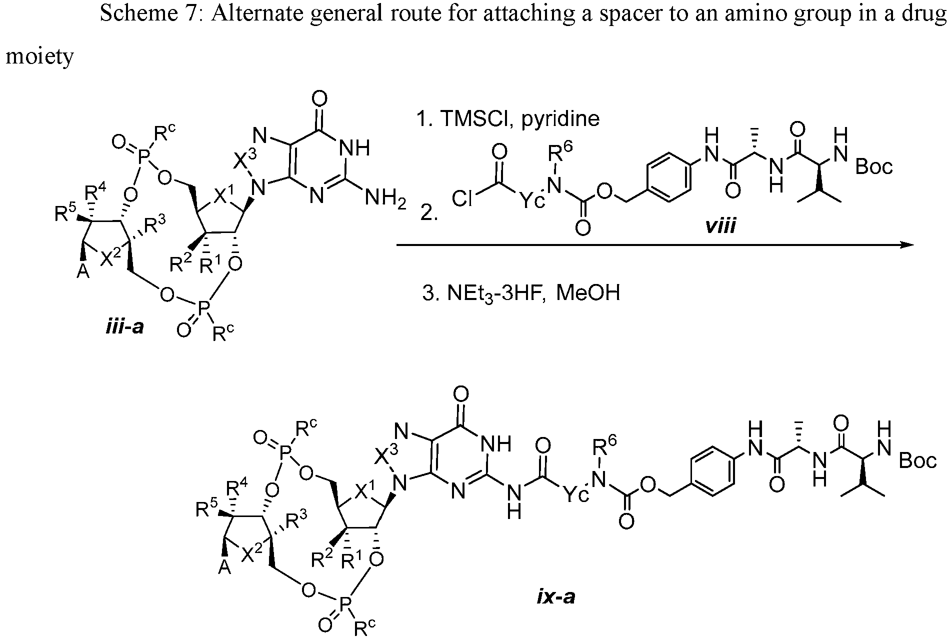

Scheme 7 shows an alternate general method for the direct conversion of payload *iii-a* to compound *ix-a* following the general method described in Scheme 6.

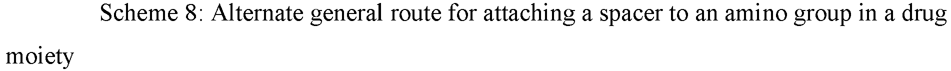

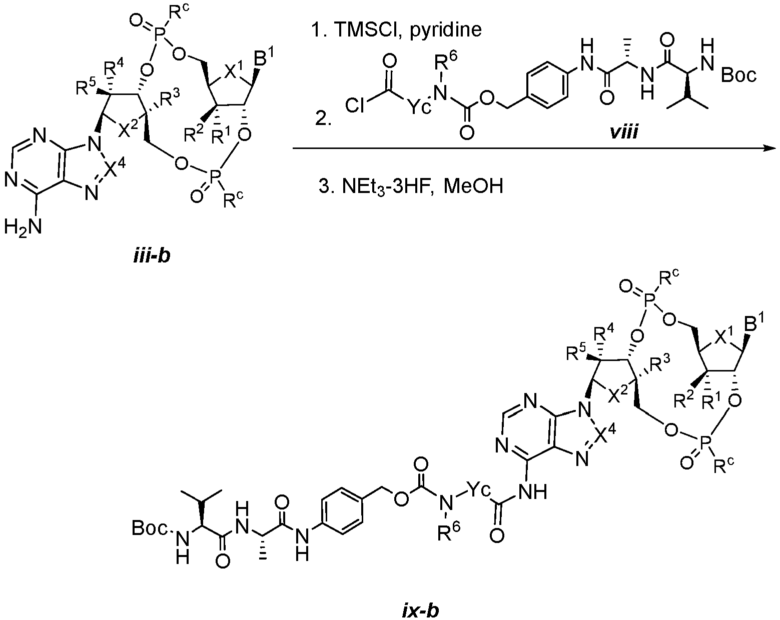

Scheme 8 shows an alternate general method for attaching a spacer to the amino group in a drug moiety such as *iii-b* following the general method described in Scheme 6.

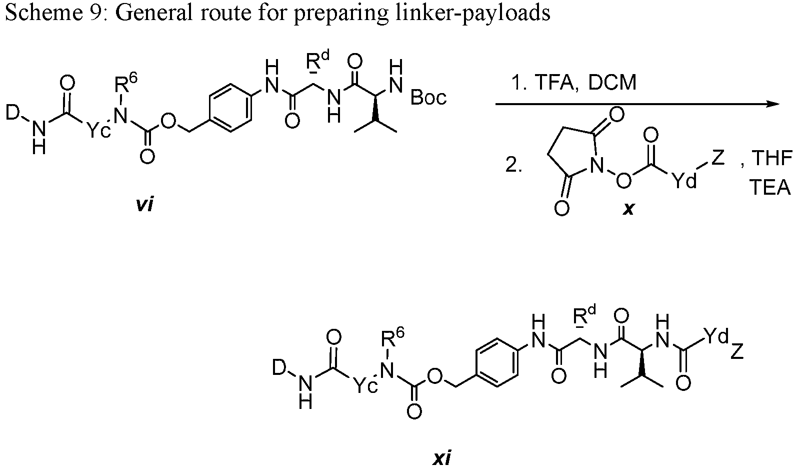

Scheme 9 shows a general method for preparing linker-payload *xi* from *vi* where Yd is a linear or branched alkyl chain or polyether chain, and Z is the conjugation handle such as maleimide, BCN, or DBCO. Deprotection of the amine of *vi* with an acid such as TFA or HCl is followed by reaction with activated ester x to give linker-payload *xi.*

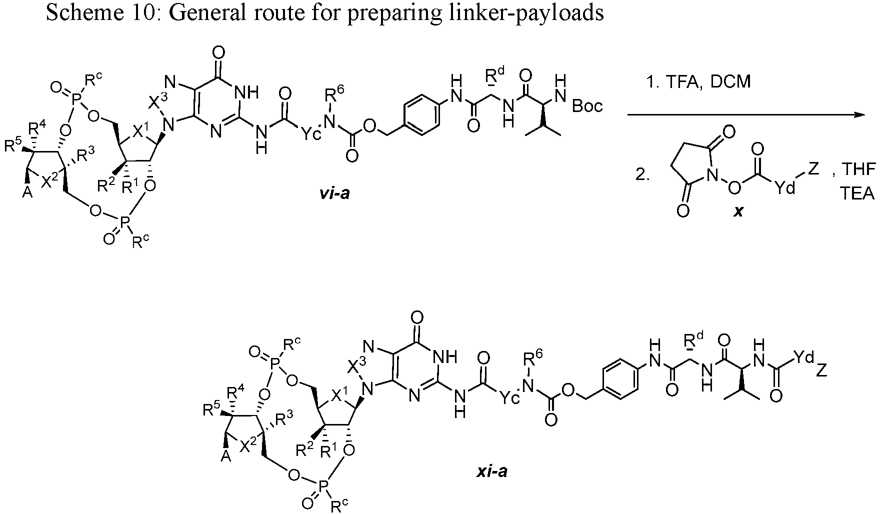

Scheme 10 shows a general method for preparing linker-payload *xi-a* from *vi-a* following the general method described in Scheme 9.

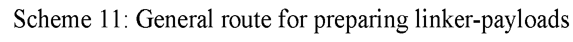

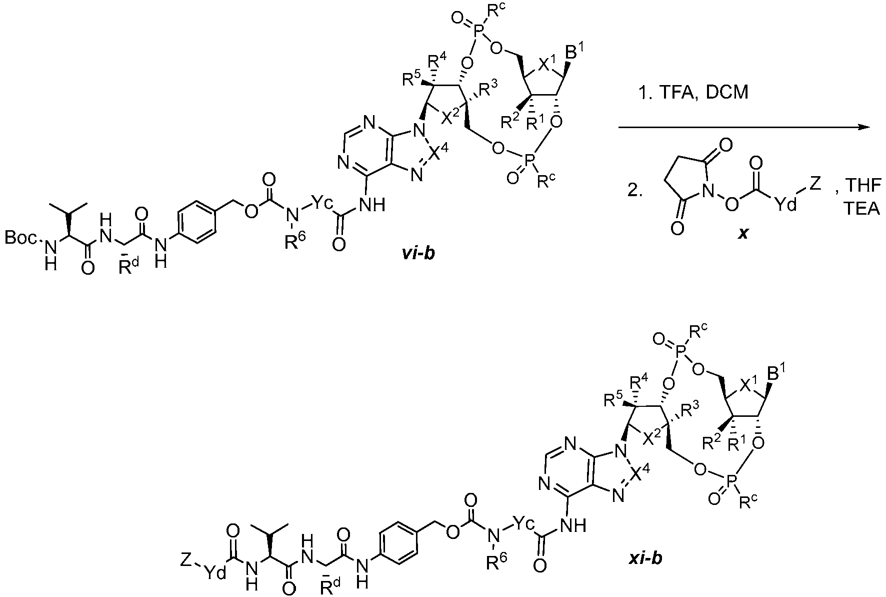

Scheme 11 shows a general method for attaching the conjugation handle to compounds such as *vi-b* following the general method described in Scheme 9.

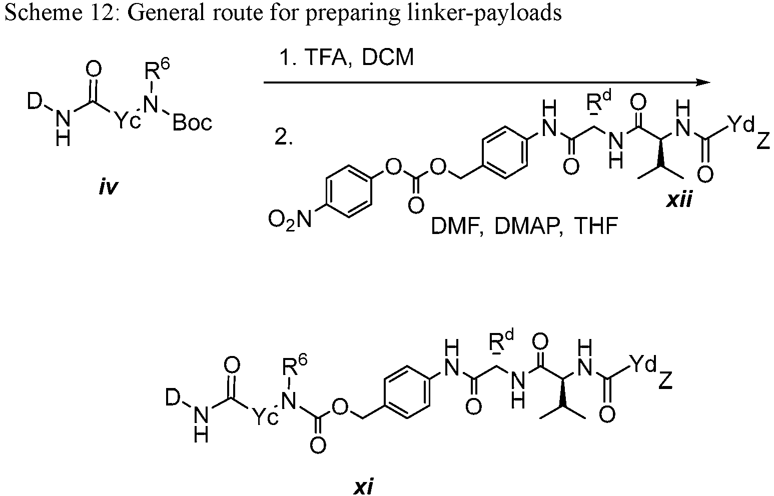

Scheme 12 shows an alternative method to prepare linker-payload *xi* from *iv.* Deprotection of the amine of *iv* with an acid such as TFA or HCl is followed by reaction *xii* to afford *xi.*

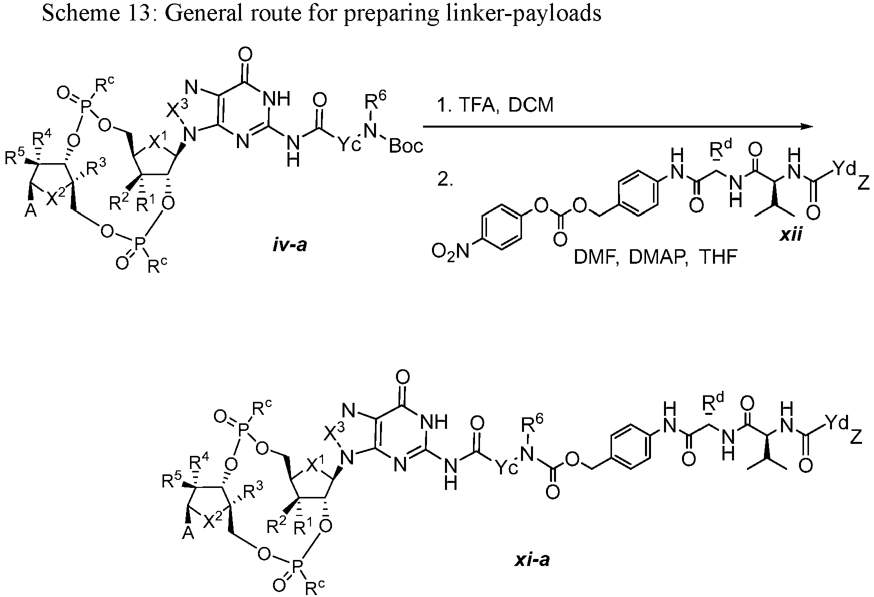

Scheme 13 shows an alternative method to prepare linker-payload *xi-a* from *iv-a* following the general method described in Scheme 12.

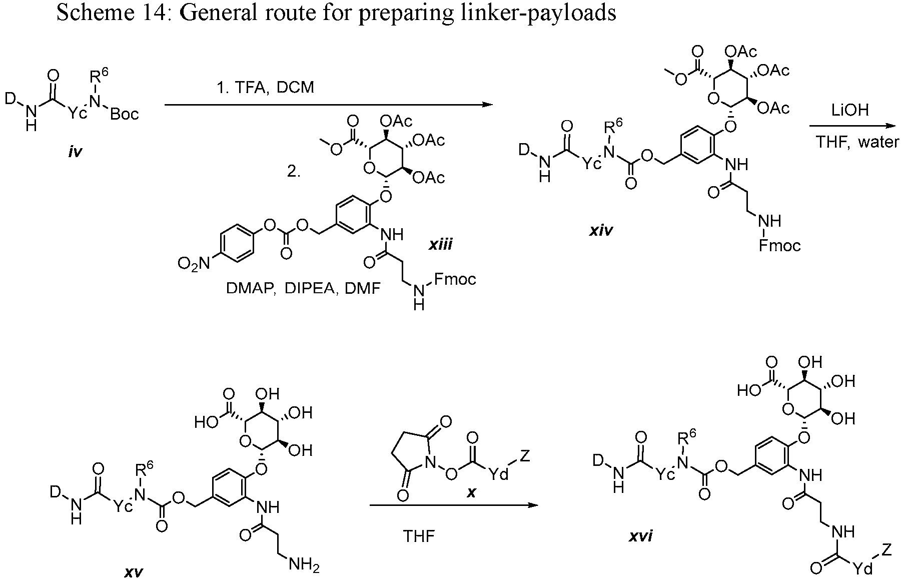

Scheme 14 shows a general method for preparing a glucuronidate-containing linker payload xvi. Deprotection of the amine of *iv* with an acid such as TFA or HCl is followed by reaction with activated carbonate *xiii* to give *xiv.* Global acetate hydrolysis and removal of FMOC group in *xiv* with a base such as lithium hydroxide give xv. Treating xv with the activated conjugation handle x affords the glucuronidate-containing linker payload *xvi.*

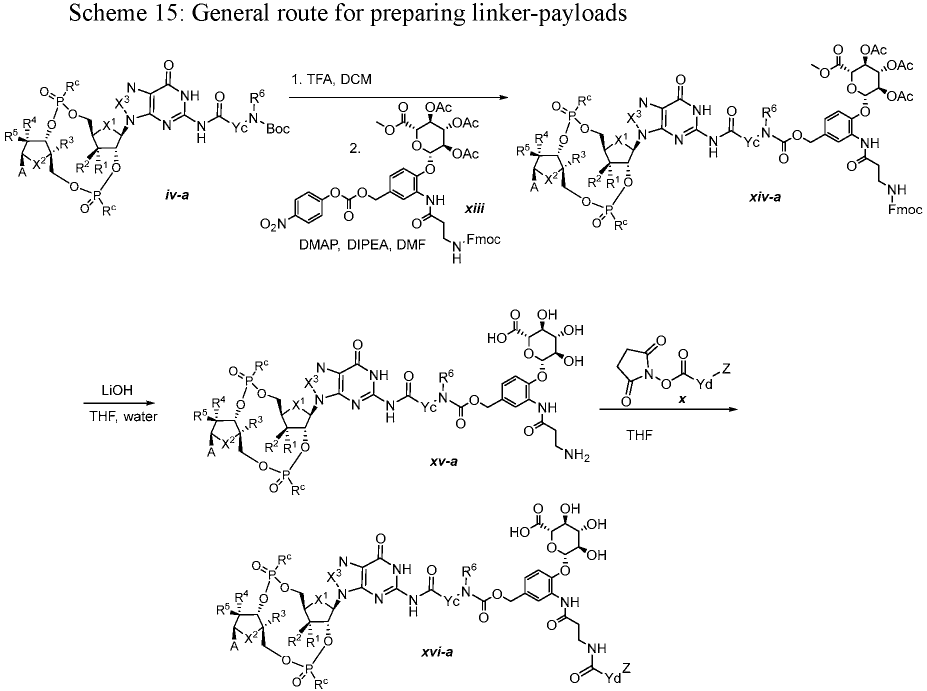

Scheme 15 shows a general method for preparing a glucuronidate-containing linker payload *xvi-a* following the general method described in Scheme 14.

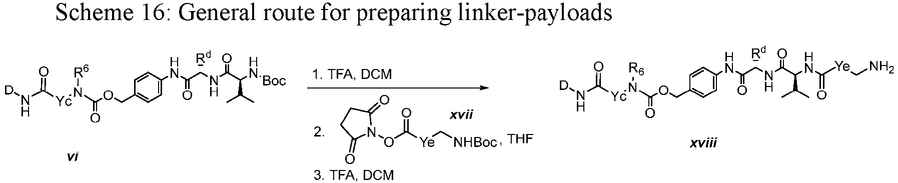

Scheme 16 shows a general method for preparing a terminal amine-containing linker payload *xviii,* where Ye is an linear alkyl chain or polyether-containing alkyl chain. Deprotection of the amine of vi with an acid such as TFA or HCl is followed by reaction with activated conjugation handle *xvii.* Removal of the terminal protecting group with acid such as TFA affords the terminal amine-containing linker payload *xviii.*

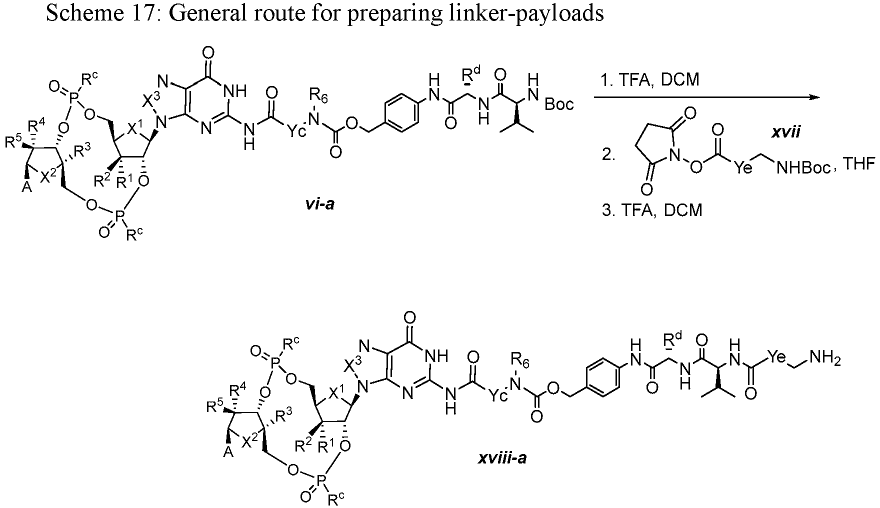

Scheme 17 shows a general method for preparing a terminal amine-containing linker payload *xviii-a* following the general method described in Scheme 16.

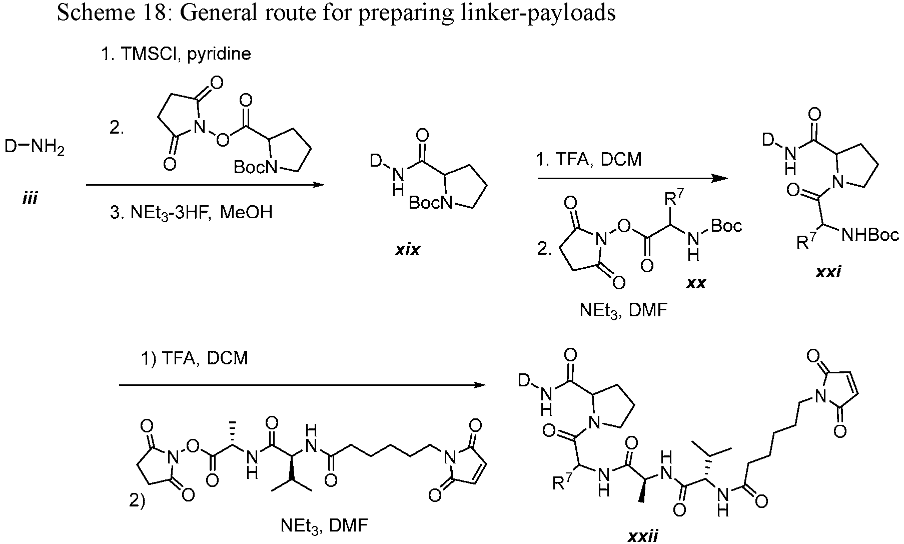

Scheme 18 shows a general method of preparing a linker payload such as *xxii* which incorporates a dipeptide spacer, where R⁷ is hydrogen, an alkyl or aromatic moiety. Compound *iii* is globally protected by treating with trimethyl silyl chloride and pyridine and then is treated with Boc-proline succinimide ester. Removal of silyl group with a fluoride source such as NEt₃-3HF gives xix. Deprotection of the amine of *xix* with an acid such as TFA or HCl is followed by reaction with a succinimide ester activated amino acid *xx* to give xxi. Deprotection of the amine of *xxi* with an acid such as TFA or HCl is followed by installation of a maleimide handle to afford linker payload *xxii.*

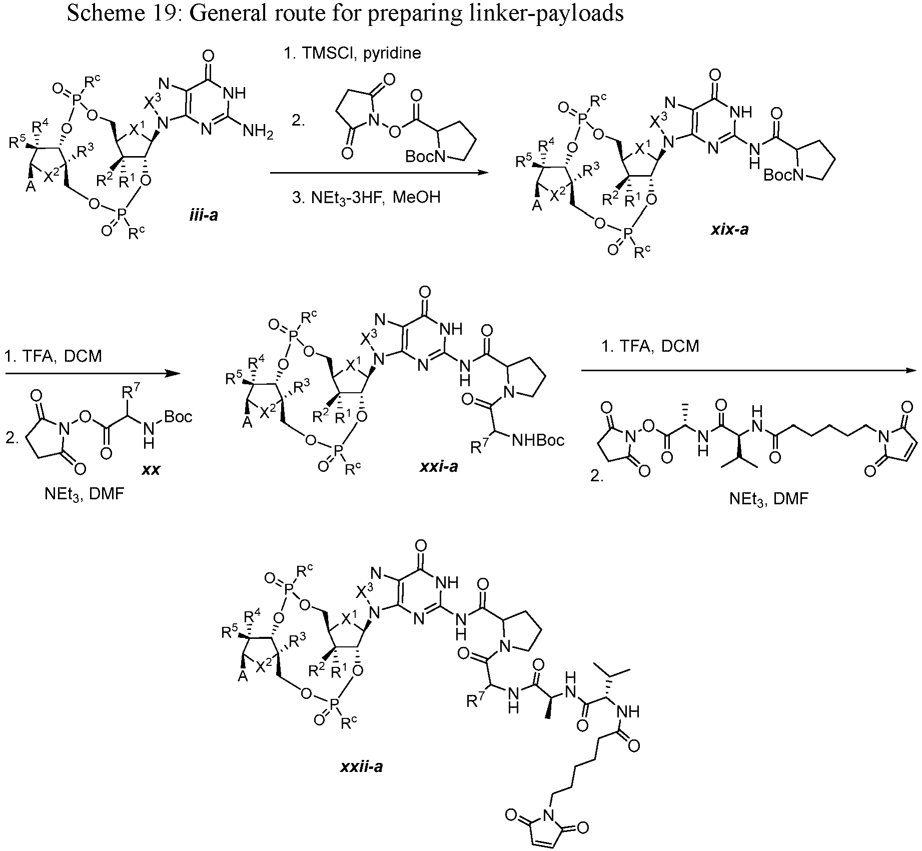

Scheme 19 shows a general method of preparing a linker payload such as *xxii-a* following the general method described in Scheme 18.

### Preparation of Exemplary ADC's and Intermediates

### Definitions

- AA: LCMS method using ammonium acetate
- Ac: acetate
- ACN: acetonitrile
- atm: atmosphere
- aq: aqueous
- BCN: bicyclo[6.1.0]nonyne
- BLQ: below limit of quantitation
- Bn: benzyl
- Boc: *tert*-butoxycarbonyl
- *t*Bu: *tert*-butyl
- Bz: benzoyl
- C: Celsius
- calcd: calculated
- Cbz: benzyloxycarbonyl
- DAR: drug antibody ratio
- DBCO: dibenzocyclooctyne
- DCC: *N,N*-dicyclohexylcarbodiimide
- DCM: dichloromethane
- DIPEA: *N,N*-diisopropylethylamine
- DMAP: 4-dimethylaminopyridine
- DMA: *N,N*-dimethylacetamide
- DMB: 2,4-dimethoxybenzyl
- DMF: *N,N*-dimethylformamide
- DMSO: dimethylsulfoxide
- DTT: dithiothreitol
- ε: extinction coefficient
- E 0.1%: 0.1% solution extinction coefficient
- EDC: 1-(3-dimethylaminopropyl)-3-ethylcarbodiimide
- EDTA: ethylenediaminetetraacetic acid
- Et: ethyl
- EtOH: ethanol
- EtOAc: ethyl acetate
- FA: LCMS method using formic acid
- Fmoc: fluorenylmethyloxycarbonyl
- h: hours
- HATU: 1-[bis(dimethylamino)methylene]-1*H*-1,2,3-triazolo[4,5-*b*]pyridinium 3-oxid hexafluorophosphate
- HIC: hydrophobic interaction chromatography
- HOAt: 1-hydroxy-7-azabenzotriazole
- HPLC: high pressure liquid chromatography
- HRMS: high resolution mass spectrometry
- IC₅₀: inhibitory concentration 50%
- LC: liquid chromatography
- LCMS: liquid chromatography mass spectrometry
- *m*/*z*: mass to charge
- MHz: mega hertz
- Me: methyl
- MeOH: methanol
- µM: micron
- min: minutes
- mL: milliliters
- MS: mass spectrum
- MWCO: molecular weight cut-off
- NMR: nuclear magnetic resonance
- PBS: phosphate buffered saline
- PE: petroleum ether
- Ph: phenyl
- psi: pounds per square inch
- psig: pounds per square inch gauge
- Pyr: pyridine
- QTOF: quadrupole time-of-flight
- rt: room temperature
- SEC: size exclusion chromatography
- SFC: supercritical fluid chromatography
- TBS: *tert*-butyldimethylsilyl
- TCEP: (tris(2-carboxyethyl)phosphine)
- TEA: triethylamine
- TFA: trifluoroacetic acid
- THF: tetrahydrofuran
- TIPS: triisopropylsilyl
- TMS: trimethylsilyl
- Tris: tris(hydroxymethyl)aminomethane
- UPLC: ultra performance liquid chromatography

### Analytical Methods

### NMR conditions:

¹H NMR spectra were acquired on a 400 MHz Bruker spectrometer unless otherwise stated. ³¹P NMR spectra were recorded at 162 MHz on a 400 MHz Bruker spectrometer and acquired with ¹H decoupling unless otherwise stated. ¹⁹F NMR spectra were recorded at 376 MHz on a 400 MHz Bruker spectrometer unless otherwise stated.

### HRMS conditions:

HRMS spectra were acquired using an Agilent 1260 Infinity II Bio-inert Multisampler, Bio-inert Column compartment, Bio-inert Quaternary Pump, DAD multi-wavelength detector, and an Agilent 6545 LC-QTOF mass spectrometry instruments, with an Osaka Soda Capcell PAK C1 UG120 (5 µm, 2.0 mm ID x 35 mm length) reverse-phase column. Solvent systems for LC consisted of 0.1% formic acid in water for mobile phase A, and 0.1% formic acid in acetonitrile for mobile phase B, or equivalent solvent system. Typically, LC elution commenced at a 95%/5% mobile phase A/B ratio. After an interval of isocratic flow, typically 0.5 min, the mobile phase was changed to 100%B over a 1.5 min linear gradient. Flow rate was 0.7 mL/min for a 5 min run.

Typically, 2 uL of a 1 mM solution in DMSO was injected into the mass spectrometer. QTOF settings: gas temperature 35 °C, drying gas flow rate = 10 L/min, nebulizer pressure 55 psig, sheath gas temperature and flow were 35 °C and 12 L/min, respectively. The following voltage settings were applied: capillary voltage 4500V, nozzle voltage 2000V, fragmentor voltage 75V.

Data was analyzed by using Agilent Mass Hunter Qualitative Analysis software, version 8.07.00 SP2. Mass error [ppm] was expressed as difference between the exact and observed mass divided by exact mass multiplied by 1x10⁶.

### LCMS conditions:

LCMS spectra were recorded on a Hewlett-Packard HP1100 or Agilent 1100 Series LC system connected to a Micromass mass spectrometer using reverse phase C18 columns. Various gradients and run times were selected in order to best characterize the compounds. Mobile phases were based on ACN/water or MeOH/water gradients and contained either 0.1% formic acid (methods indicated as FA) or 10 mM ammonium acetate (methods indicated as AA). One example of a solvent gradient that was used was 100% mobile phase A (mobile phase A = 99% water + 1% ACN + 0.1% formic acid) to 100% mobile phase B (mobile phase B = 95% ACN + 5% water + 0.1% formic acid) at a flow rate of 1 mL/min for a 16.5 min run.

In some cases, LCMS spectra were recorded on an Agilent 1290 Infinity UPLC system connected to an Agilent 6130 mass spectrometer, a Waters Acquity UPLC system connected to a Waters Acquity SQ mass spectrometer, or an Agilent 1100 Series HPLC system connected to a Waters Micromass ZQ mass spectrometer using reverse phase C18 columns. Various gradients and run times were selected in order to best characterize the compounds. Mobile phases were based on ACN/water or MeOH/water gradients and contained either 0.1% formic acid (methods indicated as FA) or 10 mM ammonium acetate (methods indicated as AA). One example of a solvent gradient that was used was 95% mobile phase A (mobile phase A = 99% water + 1% ACN + 0.1% formic acid) to 100% mobile phase B (mobile phase B = 95% ACN + 5% water + 0.1% formic acid) at a flow rate of 0.5 mL/min for a 5 min run.

### Preparative HPLC:

Preparative HPLC separations were conducted using 18x150 mm Sunfire C-18 columns eluting with water-ACN gradients using a Gilson instrument operated by 322 pumps with the UV/visible 155 detector triggered fraction collection set to between 200 nm and 400 nm. Mass gated fraction collection is conducted on an Agilent 1100 LC/MSD instrument.

One of ordinary skill in the art will recognize that modifications of the gradient, column length, and flow rate are possible and that some conditions may be more suitable for compound characterization than others, depending on the chemical species being analyzed.

### Preparative SFC:

Preparative SFC was conducted using 10, 20 or 30 mm x 250 mm ChiralPak columns (typically IA, IB, IC, ID, IE and IF), 10 or 20 mm x 250 mm Phenomenex Lux Cellulose-4 or 2-ethylpyridine columns eluting with appropriate percentages of supercritical carbon dioxide and alcohol containing either 0.3% diethylamine, 0.3% TEA, 0.3% formic acid or without any acid or base additives. Isocratic conditions with flow rates in the range of 10-100 mL/min and a column temperature of 40 °C are typical. Preparative SFC is conducted on a Jasco SFC prep purification system with UV/visible triggered fraction collection set to between 200 nm and 400 nm and back pressure regulation set to 10 MPa.

One of ordinary skill in the art will recognize that modifications of the gradient, column length, and flow rate are possible and that some conditions may be more suitable for compound characterization than others, depending on the chemical species being analyzed.

### Analytical SEC conditions:

SEC spectra were recorded on a Hewlett-Packard HP1100 or an Agilent 1100 Series LC system with Diode Array Detector using a SEC column (typically Tosoh Biosep TSK Gel, G3000SWxl; P/N 8541; 250A; 5um; 7.8mm x 300mm) at 280 nm. Mobile phase was 100 mM sodium phosphate, 300 mM sodium chloride, pH 6.8, 10% acetonitrile (v/v) or 1xPBS. A typical run is isocratic at a flow rate of 1 mL/min for 20 min.

### Analytical HIC conditions:

HIC spectra were recorded on a Hewlett-Packard HP1100 or Agilent 1100 Series LC system with Diode Array Detector using a HIC column (typically Tosoh Butyl-NPR, 4.6 x 35 mm, 2.5 um, P/N: 14947) at 280 nm. Mobile phase A was 25 mM sodium phosphate, 1.5 M ammonium sulfate, pH 7, and Mobile phase B was 75% 25 mM sodium phosphate, pH 7, 25% isopropanol. For a typical 20 min run, a 12 min linear gradient from 95%/5% A/B to 100%B would be used between initial and final intervals of isocratic flow.

### LC-QTOF conditions:

LCMS spectra were recorded on an Agilent 1260 Bioinert Series LC system connected to an Agilent 6545 QTOF mass spectrometer using a reverse phase column heated to 80 °C (typically Agilent, PLRP-S, 5 µm, 1000 Å, 2.1 mm x 50 mm). Various gradients and run times were selected in order to best characterize the compounds. Mobile phases were based on ACN/water gradients and contained 0.1% formic acid. One example of a solvent gradient that was used was 95% mobile phase A (mobile phase A = 99% water + 1% ACN + 0.1% formic acid) to 100% mobile phase B (mobile phase B = 95% ACN + 5% water + 0.1% formic acid) with conditions shown in Table 1.

**Table 1**

| Time (min) | Flow (mL/min) | %A | %B |
|---|---|---|---|
| 0 | 0.35 | 82 | 18 |
| 1 | 0.35 | 82 | 18 |
| 2 | 0.35 | 70 | 30 |
| 19 | 0.5 | 50 | 50 |
| 19.5 | 0.5 | 10 | 90 |
| 21 | 0.5 | 10 | 90 |
| 21.1 | 0.5 | 82 | 18 |
| 22 | 0.5 | 82 | 18 |

Samples were either intact or reduced (20 uL of 1~5 mg/mL ADC solution treated with 4 uL of 0.5M DTT solution at 37 °C for 30 min). Raw data was deconvoluted within appropriate mass range using Agilent BioConfirm software to obtain protein molecular weight(s), and the Agilent DAR Calculator was used to calculate DAR.

### LC/MS/MS conditions:

LC/MS/MS analysis was performed using Shimadzu UFLC LC-20AD XR binary pump and SIL-30AC MP autosampler system and AB SCIEX Triple Quad 4500 ESI Mass spectrometry.

Typically, 5 uL sample aliquots were injected into the LC/MS/MS after passing through a Waters Xselect C18 CSH 3.5u 2.1 mm ID x 30 mm column. Mobile phase A contained 0.1% formic acid in water, and mobile phase B contained 0.1% formic acid in 5% water with 95% acetonitrile. Total run time was 3 min at 1.5 mL/min with a linear gradient from 100% A to 100% B over 1.5 min flow rate. Initially, the instrument was running at 100% aqueous mobile phase solvent for 0.5 min, and then it was increased to 100% organic solvent in next 1.5 min.

### Preparative SEC:

Preparative SEC purification was conducted on a Gilson Preparative HPLC system with UV Detector using a SEC column (typically GE Superdex 200 Increase 10/300 GL). Mobile phase was 1xPBS (pH 7.4). A typical run is isocratic at a flow rate of 1 mL/min for 30 min. Fraction collection was triggered based on UV threshold (at 214 and 280 nm).

### ADC concentration:

ADC concentration was calculated from the UV absorbance at 280 nm measured by NanoDrop (2000c; Fisher Scientific) coefficient after subtraction of the UV absorbance from the corresponding linker-payload constructs.

### Example 1

### tert-Butyl N-[(2-chlorocarbonylphenyl)methyl]-N-methyl-carbamate (Intermediate 1)

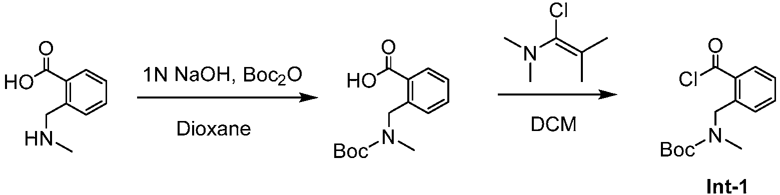

### Step 1: 2-(((tert-Butoxycarbonyl)(methyl)amino)methyl)benzoic acid

To a solution of 2-[(methylamino)methyl]benzoic acid (7.50 g, 44 mmol) in 1,4-dioxane (136 mL) and 1N sodium hydroxide in water (182 mL) was added di-*tert*-butyl dicarbonate (19.2 g, 88 mmol). The reaction mixture was allowed to stir at rt for 2 h. The reaction mixture was then concentrated to remove the organic solvent and the pH was adjusted to pH 3 using 1N HCl. The crude product was extracted with EtOAc (3x). The combined organic phases were washed with brine, dried (Na₂SO₄), filtered and evaporated. The crude compound was purified by silica gel chromatography (0-50% EtOAc/DCM) to provide 2-(((*tert-*butoxycarbonyl)(methyl)amino)methyl)benzoic acid (8.88 g, 75%). LCMS (AA): *m*/*z* = 264.1 (M-H).

### Step 2: tert-Butyl N-[(2-chlorocarbonylphenyl)methyl]-N-methyl-carbamate, Intermediate 1

1-Chloro-*N,N*,2-trimethylpropenylamine (0.52 mL, 4.0 mmol) was added slowly to a solution of 2-(((tert-butoxycarbonyl)(methyl)amino)methyl)benzoic acid (525 mg, 2.0 mmol) in DCM (16 mL) at 0 °C. The reaction mixture was then warmed to rt and stirred for 1 h. The reaction mixture was then concentrated to dryness to provide crude *tert*-butyl N-[(2-chlorocarbonylphenyl)methyl]-N-methyl-carbamate (Intermediate 1, 562 mg, 100%).

### Example 2

### tert-Butyl N-[[2-(2-chloro-2-oxo-ethyl)phenyl]methyl]-N-methyl-carbamate (Intermediate 2)

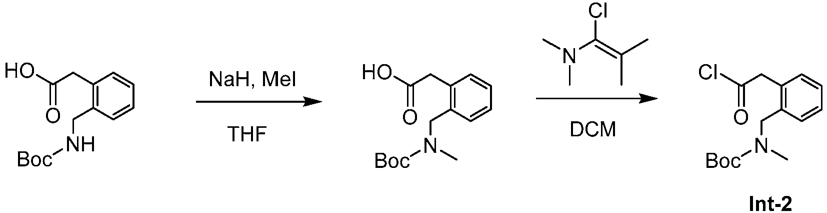

### Step 1: 2-[2-[[tert-Butoxycarbonyl('methyl)amino]methyl]phenyl]acetic acid

To a solution of 2-(2-(((*tert*-butoxycarbonyl)amino)methyl)phenyl)acetic acid (1.50 g, 5.7 mmol) in THF (20 mL) was added iodomethane (2.8 mL, 45.2 mmol) at rt. The reaction mixture was cooled to 0 °C and sodium hydride (60% dispersion in mineral oil, 905 mg, 22.6 mmol) was added in 3 portions. The reaction mixture was then allowed to stir at rt for 16 h. The reaction mixture was quenched with water and the organic solvent was removed by concentration. The aqueous phase was acidified to pH 3 using 1N HCl and the crude product was extracted with Et₂O (2x). The combined organic phases were washed with brine, dried (Na₂SO₄), filtered and evaporated to provide 2-[2-[[*tert-*butoxycarbonyl(methyl)amino]methyl]phenyl]acetic acid (1.36 g, 86%). LCMS (AA): *m*/*z* = 280.2 (M+H).

### Step 2: tert-Butyl N-[[2-(2-chloro-2-oxo-ethyl)phenyl]methyl]-N-methyl-carbamate, Intermediate 2

The title compound was prepared following the procedure described in Example 1, step 2, starting with 2-[2-[[*tert*-butoxycarbonyl(methyl)amino]methyl]phenyl]acetic acid (650 mg, 2.33 mmol) to provide crude Intermediate 2 (693 mg, 100%).

### Example 3

### Isobutoxycarbonyl 4-[tert-butoxycarbonyl(methyl)amino]butanoate (Intermediate 3)

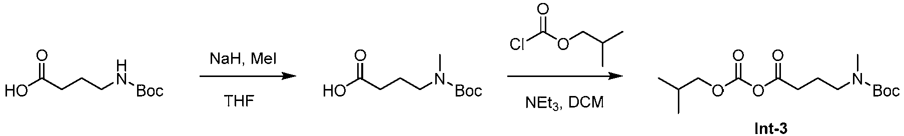

### Step 1: 4-((tert-Butoxycarbonyl)(methyl)amino)butanoic acid

The title compound was prepared following the procedure described in Example 2, step 1, using 4-(*tert*-butoxycarbonylamino)butanoic acid (2.92 g, 14.4 mmol) in place of 2-(2-(((*tert-*butoxycarbonyl)amino)methyl)phenyl)acetic acid to provide 4-((*tert-*butoxycarbonyl)(methyl)amino)butanoic acid (2.89 g, 93%). LCMS (AA): *m*/*z* = 216.1 (M-H).

### Step 2: Isobutoxycarbonyl 4-[tert-butoxycarbonyl(methyl)amino]butanoate, Intermediate 3

To a solution of 4-((*tert*-butoxycarbonyl)(methyl)amino)butanoic acid (360 mg, 1.57 mmol) and triethylamine (0.24 mL, 1.73 mmol) dissolved in DCM (7 mL) and cooled to 0 °C was added isobutyl chloroformate (0.22 mL, 1.73 mmol) dropwise. Upon complete addition, the reaction mixture was stirred at rt for 2 h. The mixture was then concentrated to dryness to provide crude isobutoxycarbonyl 4-[*tert*-butoxycarbonyl(methyl)amino]butanoate (Intermediate 3, 499 mg, 100%).

### Example 4

The compounds listed below were prepared as described in Example 3, substituting the starting material shown in Table 2 for 4-(*tert*-butoxycarbonylamino)butanoic acid.

**Table 2**

| Starting material | Product | Note |
|---|---|---|
| 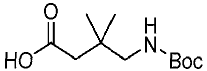 | 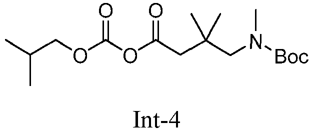 | |
| 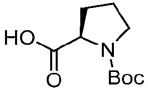 | 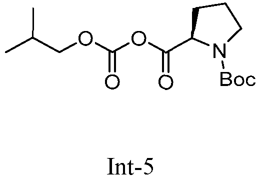 | Step 1 was skipped |

### Example 5

### N-{9-[(2R,5R,7R,8R,10R,12aR,14R,15aS,16R)-16-Hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}-2-[(methylamino)methyl]benzamide (Intermediate 6)

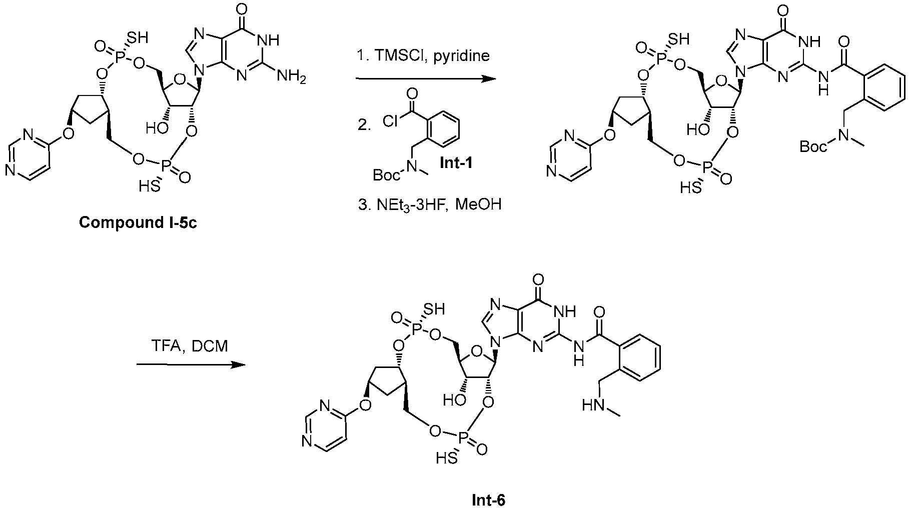

### Step 1: tert-Butyl [2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate

2-Amino-9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-1,9-dihydro-6H-purin-6-one as the *N,N-*diethylethanamine salt (Compound I-5c, see PCT/IB2018/058846, 160 mg, 0.19 mmol) was dissolved in dry pyridine and concentrated to dryness (3 x 2mL) and then placed under vacuum for 15 min. The residue was dissolved in pyridine (3 mL) under an argon atmosphere and chlorotrimethylsilane (0.15 mL, 1.13 mmol) was added. The reaction mixture was allowed to stir at rt for 30 min. Intermediate 1 (800 mg, 2.82 mmol) dissolved in pyridine (3 mL) was then added via syringe. The reaction mixture was stirred at rt under an argon atmosphere for 16 h. The reaction mixture was then concentrated to dryness and MeOH (10 mL) and ammonium hydroxide (28-30% solution in water, 10 mL) were added and allowed to stir for 30 min. The reaction mixture was concentrated to dryness and the residue was dissolved in MeOH (15 mL). Triethylamine trihydrofluoride (0.12 mL, 0.75 mmol) was added and the reaction mixture was stirred at rt for 30 min. The reaction mixture was concentrated to dryness and the crude residue was adsorbed onto Celite and purified by reverse phase flash column chromatography (0-50% ACN/ aqueous triethylammonium acetate (10 mM)) to provide *tert-*butyl [2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate as the *N,N-*diethylethanamine salt (120 mg, 58%). LCMS (AA): *m*/*z* = 897.3 (M+H). ¹H NMR (400 MHz, CD₃OD) δ 8.70 (s, 1H), 8.49 (s, 1H), 8.41 (d, *J =* 6.0 Hz, 1H), 7.70 (d, *J =* 7.5 Hz, 1H), 7.58 - 7.52 (m, 1H), 7.44 - 7.39 (m, 1H), 7.33 (d, *J =* 7.8 Hz, 1H), 6.85 (d, *J =* 6.0 Hz, 1H), 6.17 (d, J = 8.3 Hz, 1H), 5.64 - 5.58 (m, 1H), 5.49 - 5.44 (m, 1H), 5.08 - 5.02 (m, 1H), 4.88 - 4.78 (m, 1H), 4.76 - 4.67 (m, 2H), 4.37 - 4.21 (m, 3H), 4.06 - 4.00 (m, 1H), 3.83 - 3.74 (m, 1H), 2.82 (s, 3H), 2.60 - 2.32 (m, 4H), 1.54 - 1.46 (m, 1H), 1.42 (s, 9H). ³¹P NMR (162 MHz, CD₃OD) δ 55.19 (s, 1P), 53.20 (s, 1P).

### Step 2: N-{9-[(2R,5R,7R,8R,10R,12aR,14R,15aS,16R)-16-Hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}-2-[(methylamino)methyl]benzamide, Intermediate 6

*tert*-Butyl [2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate as the *N,N-*diethylethanamine salt (50 mg, 0.045 mmol) was added to a round bottom flask and cooled to 0 °C. A solution of trifluoroacetic acid (0.24 mL, 3.2 mmol) and DCM (0.58 mL) was then added via syringe and the reaction mixture was stirred at 0 °C for 30 min. The reaction mixture was then concentrated to dryness and placed under vacuum for 2 h to provide Intermediate 6 as the 2,2,2-trifluoroacetate salt (41 mg, 100%). LCMS (AA): *m*/*z* = 797.1 (M+H).

### Example 6

### 4-{[(2S)-2-{[(2S)-2-{[6-(2,5-Dioxo-2,5-dihydro-1H-pyrrol-1-yl)hexanoyl]amino}-3-methylbutanoyl]amino}propanoyl]amino}benzyl [2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate, Compound 1 (C-1)

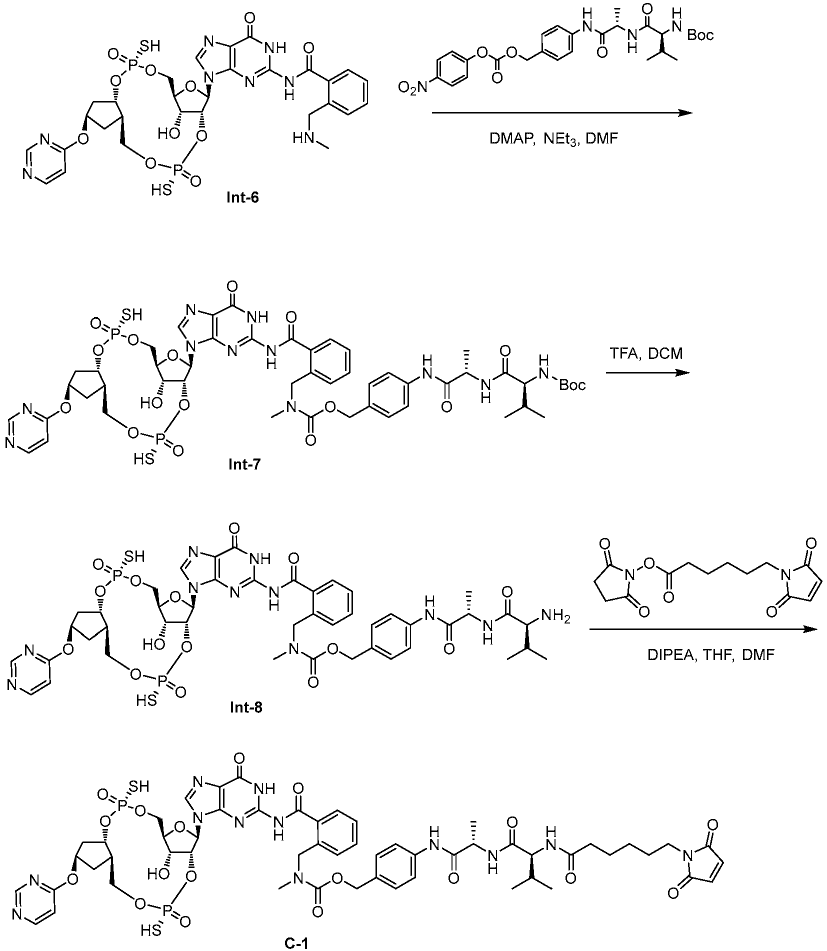

### Step 1: 4-{[(2S)-2-({(2S)-2-[(tert-Butoxycarbonyl)amino]-3-methylbutanoyl }amino)propanoyl]amino }benzyl [2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate, Intermediate 7

To a solution of *tert*-butyloxycarbonyl-valyl-alanyl-(4-aminobenzyl)-(4-nitrophenyl)carbonate (58 mg, 0.10 mmol) and 4-dimethylaminopyridine (12 mg, 0.10 mmol) in DMF (0.38 mL) and triethylamine (0.055 mL, 0.40 mmol) was added a solution of Intermediate 6 (45 mg, 0.05 mmol) in DMF (1.5 mL) at rt. The reaction mixture was allowed to stir at rt for 15 min. Celite was added and the mixture was concentrated to dryness. The crude residue absorbed onto Celite was purified by reverse phase flash column chromatography (0-40% ACN/aqueous triethylammonium acetate (10 mM)) to provide 4-{[(2*S*)-2-({(2*S*)-2-[(*tert-*butoxycarbonyl)amino]-3-methylbutanoyl}amino)propanoyl]amino}benzyl [2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate as the *N,N-*diethylethanamine salt (Intermediate 7, 10 mg, 16%). LCMS (AA): *m*/*z* = 1216.3 (M+H). ¹H NMR (400 MHz, CD₃OD) δ 8.70 (s, 1H), 8.43 (brs, 1H), 8.40 (d, *J =* 5.9 Hz, 1H), 7.70 (d, *J =* 7.5 Hz, 1H), 7.56 - 7.46 (m, 3H), 7.42 - 7.38 (m, 1H), 7.32 - 7.19 (m, 3H), 6.84 (d, *J =* 5.5 Hz, 1H), 6.15 (d, J = 7.3 Hz, 1H), 5.62 - 5.56 (m, 1H), 5.54 - 5.48 (m, 1H), 5.08 - 5.02 (m, 1H), 5.04 (s, 2H), 4.88 - 4.83 (m, 1H), 4.81 - 4.76 (m, 2H), 4.53 - 4.47 (m, 1H), 4.37 - 4.22 (m, 3H), 4.08 - 4.03 (m, 1H), 3.92 - 3.89 (m, 1H), 3.82 - 3.74 (m, 1H), 2.88 (s, 3H), 2.58 - 2.30 (m, 4H), 2.10 - 2.03 (m, 1H), 1.54 - 1.47 (m, 1H), 1.46 - 1.44 (m, 3H), 1.44 (s, 9H), 0.98 (d, *J =* 6.8 Hz, 3H), 0.93 (d, *J =* 6.8 Hz, 3H). ³¹P NMR (162 MHz, CD₃OD) δ 55.01 (s, 1P), 53.03 (s, 1P).

### Step 2: 4-{[(2S)-2-[[(2S)-2-Amino-3-methylbutanoyl]amino}propanoyl]amino}benzyl [2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate, Intermediate 8

The title compound was prepared following the procedure described in Example 5, step 2, using Intermediate 7 (10 mg, 0.008 mmol) in place of *tert*-butyl [2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate and stirring at rt for 10 min to provide 4-{[(2*S*)-2-{[(2*S*)-2-amino-3-methylbutanoyl]amino}propanoyl]amino}benzyl [2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate as the 2,2,2-trifluoroacetate salt (Intermediate 8, 9 mg, 100%). LCMS (AA): *m*/*z* = 1116.3 (M+H).

### Step 3: 4-{[(2S)-2-{[(2S)-2-{[6-(2,5-Dioxo-2,5-dihydro-1H-pyrrol-1-yl)hexanoyl]amino}-3-methylbutanoyl]amino}propanoyl]amino}benzyl [2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate, Compound C-1

To a solution of Intermediate 8 (8 mg, 0.007 mmol) and *N*-succinimidyl 6-maleimidohexanoate (3.1 mg, 0.010 mmol) in THF (0.20 mL) and DMF (0.10 mL) was added *N,N*-diisopropylethylamine (2.5 uL, 0.014 mmol) dropwise. The reaction mixture was allowed to stir at rt for 1 h. The reaction mixture was then concentrated to dryness and the crude residue was purified by reverse phase flash column chromatography (0-100% ACN/aqueous ammonium acetate (10 mM)) to provide 4-{[(2*S*)-2-{[(2*S*)-2-{[6-(2,5-dioxo-2,5-dihydro-1H-pyrrol-1-yl)hexanoyl]amino}-3-methylbutanoyl]amino}propanoyl]amino}benzyl [2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate as the ammonium salt (C-1, 4.2 mg, 45%). LCMS (AA): *m*/*z =* 1309.4 (M+H). HRMS (*m*/*z*): [M+H]⁺ calcd for C₅₅H₆₆N₁₂O₁₈P₂S₂ 1309.3607; found, 1309.3639.

### Example 7

### 2-[tert-Butoxycarbonyl(methyl)amino]ethyl carbonochloridate (Intermediate 9)

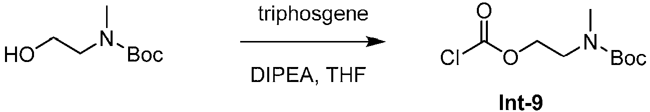

### Step 1: 2-[tert-Butoxycarbonyl(methyl)amino]ethyl carbonochloridate, Intermediate 9

To a solution of *tert*-butyl N-(2-hydroxyethyl)-N-methylcarbamate (296 mg, 1.69 mmol) in THF (4.2 mL) and *N,N-*diisopropylethylamine (1 mL, 5.9 mmol) cooled to 0 °C was added triphosgene (752 mg, 2.53 mmol) slowly. The reaction mixture was warmed to rt and stirred for 1 h. The reaction mixture was filtered, the solids were washed with THF and the filtrate was concentrated to dryness to provide crude 2-[*tert*-butoxycarbonyl(methyl)amino]ethyl carbonochloridate (Intermediate 9, 402 mg, 100%).

### Example 8

### [4-[[(2S)-2-[[(2S)-2-(tert-Butoxycarbonylamino)-3-methylbutanoyl]amino]propanoyl]amino]phenyl]methyl N-[(2-chlorocarbonylphenyl)methyl]-N-methyl-carbamate (Intermediate 10)

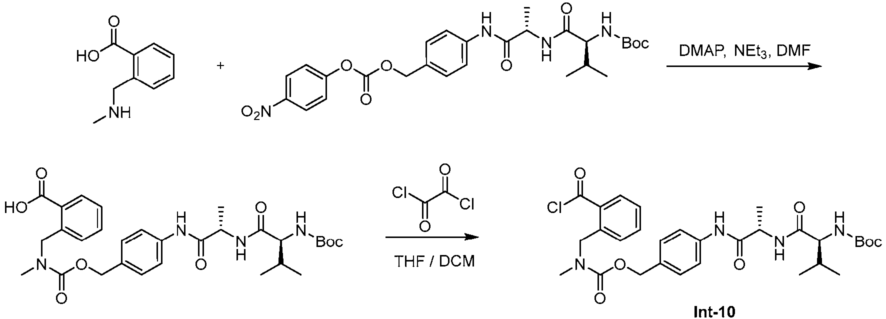

### Step 1: 2-[[[4-[[(2S)-2-[[(2S)-2-(tert-Butoxycarbonylamino)-3-methylbutanoyl]amino]propanoyl]amino]phenyl]methoxycarbonyl-methyl-amino]methyl]benzoic acid

The title compound was prepared following the procedure described in Example 6, step 1, starting with 2-[(methylamino)methyl]benzoic acid HCl (150 mg, 0.72 mmol) instead of Intermediate 6. Following purification by silica gel chromatography (0-25% MeOH/DCM) 2-[[[4-[[(2*S*)-2-[[(2*S*)-2-(*tert*-butoxycarbonylamino)-3-methylbutanoyl]amino]propanoyl]amino]phenyl]methoxycarbonyl-methyl-amino]methyl]benzoic acid (306 mg, 67%) was obtained. LCMS (AA): *m*/*z* = 583.4 (M-H).

### Step 2: [4-[[(2S)-2-[[(2S)-2-(tert-Butoxycarbonylamino)-3-methylbutanoyl]amino]propanoyl]amino]phenyl]methyl N-[(2-chlorocarbonylphenyl)methyl]-N-methyl-carbamate, Intermediate 10

To a solution of 2-[[[4-[[(2*S*)-2-[[(2*S*)-2-(*tert*-butoxycarbonylamino)-3-methylbutanoyl]amino]propanoyl]amino]phenyl]methoxycarbonyl-methyl-amino]methyl]benzoic acid (295 mg, 0.50 mmol) in THF (1.5 mL) cooled to 0 °C was added oxalyl chloride (2.0 M solution in DCM, 0.25 mL, 0.50 mmol) followed by 3 drops of DMF. The reaction mixture was allowed to stir at 0 °C for 45 min. The mixture was then concentrated to dryness to provide crude Intermediate 10 (304 mg, 100%).

### Example 8A

### 4-{[(2S)-2-({(2S)-2-[(tert-butoxycarbonyl)amino]-3-methylbutanoyl}amino)propanoyl]amino}benzyl (4-chloro-4-oxobutyl)methylcarbamate

### (Intermediate 34)

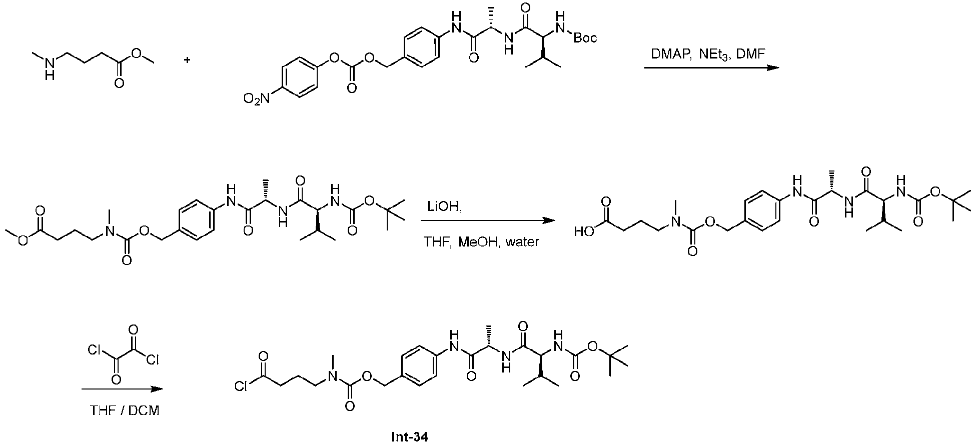

### Step 1: methyl 4-[{[(4-{[(2S)-2-({(2S)-2-[(tert-butoxycarbonyl)amino]-3-methylbutanoyl}amino)propanoyl]amino}benzyl)oxy]carbonyl}(methyl)amino]butanoate

The title compound was prepared following the procedure described in Example 6, step 1, starting with methyl 4-(methylamino)butanoate HCl (1.44 g, 8.16 mmol) instead of Intermediate 6. Following purification by silica gel chromatography (0-70% EtOAc/DCM) methyl 4-[{[(4-{[(2S)-2-({(2S)-2-[(tert-butoxycarbonyl)amino]-3-methylbutanoyl}amino)propanoyl]amino}benzyl)oxy]carbonyl}(methyl)amino]butanoate (3.45 g, 92%) was obtained. LCMS (AA): *m*/*z* = 549.3 (M-H).

### Step 2: 4-[{[(4-{[(2S)-2-({(2S)-2-[(tert-butoxycarbonyl)amino]-3-methylbutanoyl }amino)propanoyl]amino}benzyl)oxy]carbonyl } (methyl)amino]butanoic acid

To a solution of methyl 4-[{[(4-{[(2S)-2-({(2S)-2-[(tert-butoxycarbonyl)amino]-3-methylbutanoyl }amino)propanoyl]amino}benzyl)oxy]carbonyl}(methyl)amino]butanoate (3.45 g, 6.27 mmol) in THF (40.7 mL) and water (20.3 mL) was added lithium hydroxide monohydrate (657 mg, 15.7 mmol) at 0 °C. The homogeneous mixture was then warmed to rt and stirred for 2 h. This reaction mixture was cooled to 0 °C and hydrochloric acid (1.0 mol/L in water, 1.83 mL). Adjust this mixture to pH< 4 by adding additional 1*N* HCl. This solution was warmed to rt and diluted with EtOAc and water. The aqueous phase was further extracted with EtOAc. Combined organic phases were washed with brine and dried over magnesium sulfate. After filtration and removal of solvents, the residue was purified by silica gel chromatography (0-10% MeOH/EtOAc). Removal of solvents afforded 4-[{[(4-{[(2S)-2-({(2S)-2-[(tert-butoxycarbonyl)amino]-3-methylbutanoyl }amino)propanoyl]amino}benzyl)oxy]carbonyl } (methyl)amino]butanoic acid (3.17 g, 94%) as a white solid. LCMS (AA): *m*/*z* = 535.3 (M-H).

### Step 3: 4-{[(2S)-2-({(2S)-2-[(tert-butoxycarbonyl)amino]-3-methylbutanoyl }amino)propanoyl]amino}benzyl (4-chloro-4-oxobutyl)methylcarbamate

The title compound was prepared following the procedure described in Example 8, step 2, starting with 4-[{[(4-{[(2S)-2-({(2S)-2-[(tert-butoxycarbonyl)amino]-3-methylbutanoyl }amino)propanoyl]amino}benzyl)oxy]carbonyl } (methyl)amino]butanoic acid (387 mg, 0.721 mmol) instead of 2-[[[4-[[(2*S*)-2-[[(2*S*)-2-(*tert*-butoxycarbonylamino)-3-methylbutanoyl]amino]propanoyl]amino]phenyl] methoxycarbonyl-methyl-amino]methyl]benzoic acid to provide crude Intermediate 34 (400 mg, 100%) upon concentration of the reaction mixture.

### Example 9

### 4-{[(2S)-2-({(2S)-2-[(tert-Butoxycarbonyl)amino]-3-methylbutanoyl }amino)propanoyl]amino }benzyl [2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate, (Intermediate 7)

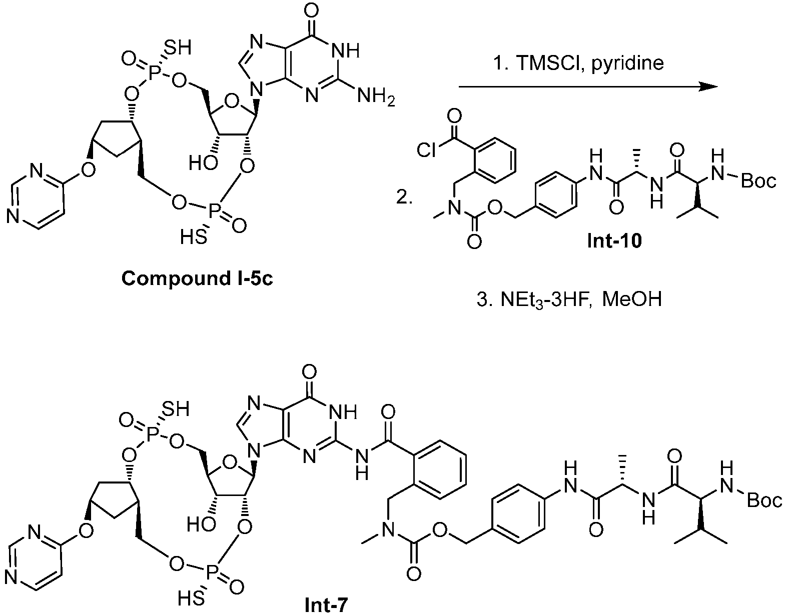

### 4-{(2S)-2-({(2S)-2-[(tert-Butoxycarbonyl)amino]-3-methylbutanoyl }amino)propanoyl]amino }benzyl [2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate, Intermediate7

The title compound was prepared following the procedure described in Example 5, step 1, substituting Intermediate 10 for tert-butyl N-[(2-chlorocarbonylphenyl)methyl]-N-methylcarbamate (Intermediate 1). The crude product was purified by reverse phase flash column chromatography (0-50% ACN/aqueous ammonium bicarbonate (5 mM)) to provide Intermediate 7 as the ammonium salt. This material was then dissolved in MeOH (2 mL) and triethylamine (5 mL) was added. The mixture was shaken for 1 min solvents removed and lyophilized overnight to provide 4-{[(2*S*)-2-({(2*S*)-2-[(*tert*-butoxycarbonyl)amino]-3-methylbutanoyl }amino)propanoyl]amino }benzyl [2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate as the *N,N-*diethylethanamine salt (147 mg, 48%). LCMS (AA): *m*/*z* = 1216.3 (M+H). ¹H NMR identical to Example 6, step 1.

### Example 10

### 4-{[(2S)-2-({(2S)-2-[(tert-Butoxycarbonyl)amino]-3-methylbutanoyl }amino)propanoyl]amino }benzyl [2-(chlorocarbonyl)benzyl]2,5,8,11-tetraoxatridecan-13-ylcarbamate (Intermediate 11)

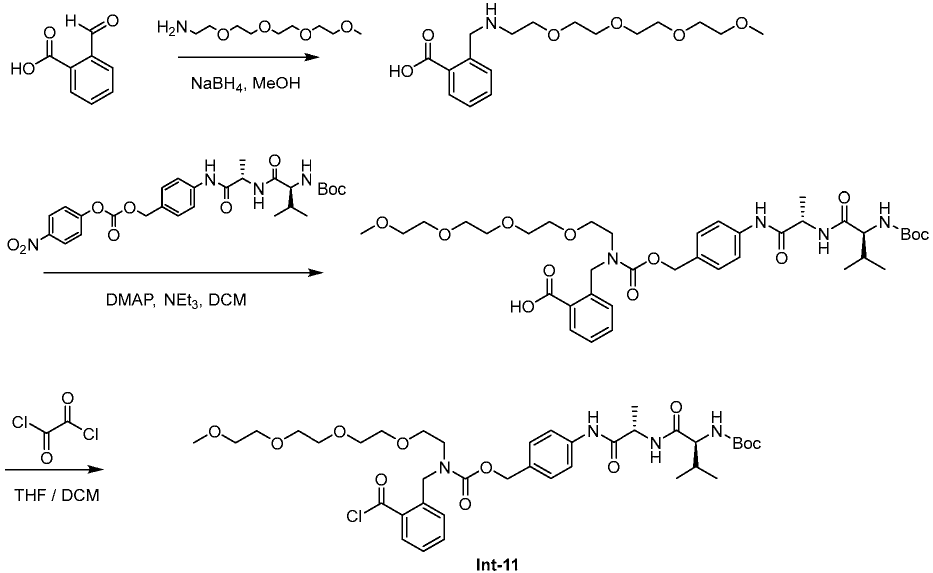

### Step 1: 2-(2,5,8,11-Tetraoxa-14-azapentadecan-15-yl)benzoic acid

To a solution of 2-carboxybenzaldehyde (1.10 g, 7.3 mmol) dissolved in MeOH (15 mL) was added 2,5,8,11-tetraoxatridecan-13-amine (1.82 g, 8.8 mmol) and the reaction mixture was stirred at rt for 1 h. Sodium borohydride (139 mg, 3.7 mmol) was then added slowly in portions. The reaction mixture was stirred at rt for 1 h. The reaction mixture was quenched by the addition of acetone (0.65 mL) and then concentrated to dryness. The crude residue was purified by silica gel chromatography (0-25% MeOH/DCM) to provide 2-(2,5,8,11-tetraoxa-14-azapentadecan-15-yl)benzoic acid (2.25 g, 87%). LCMS (AA): *m*/*z* = 342.3 (M+H).

### Step 2: 2-(14-{[(4-{[(2S)-2-({(2S)-2-[(tert-Butoxycarbonyl)amino]-3-methylbutanoyl}amino)propanoyl]amino}benzyl)oxy]carbonyl}-2,5,8,11-tetraoxa-14-azapentadecan-15-yl)benzoic acid

The title compound was prepared following the procedure described in Example 6, step 1, starting with 2-(2,5,8,11-tetraoxa-14-azapentadecan-15-yl)benzoic acid (764 mg, 2.24 mmol) instead of Intermediate 6 using DCM as the solvent and stirring for 16 h. Following purification by silica gel chromatography (0-25% MeOH/DCM) 2-(14-{[(4-{[(2*S*)-2-({(2*S*)-2-[(*tert-*butoxycarbonyl)amino]-3-methylbutanoyl}amino)propanoyl]amino}benzyl)oxy]carbonyl}-2,5,8,11-tetraoxa-14-azapentadecan-15-yl)benzoic acid (855 mg, 47%) was obtained. LCMS (AA): *m*/*z* = 759.4 (M-H).

### Step 3: 4-{[(2S)-2-({(2S)-2-[(tert-Butoxycarbonyl)amino]-3-methylbutanoyl }amino)propanoyl]amino }benzyl [2-(chlorocarbonyl)benzyl]2,5,8,11-tetraoxatridecan-13-ylcarbamate, Intermediate 11

The title compound was prepared following the procedure described in Example 8, step 2, starting with 2-(14-{[(4-{[(2*S*)-2-({(2*S*)-2-[(*tert*-butoxycarbonyl)amino]-3-methylbutanoyl}amino)propanoyl]amino}benzyl)oxy]carbonyl}-2,5,8,11-tetraoxa-14-azapentadecan-15-yl)benzoic acid (591 mg, 0.777 mmol) instead of 2-[[[4-[[(2*S*)-2-[[(2*S*)-2-(tert-butoxycarbonylamino)-3-methyl-butanoyl]amino]propanoyl]amino]phenyl]methoxycarbonylmethyl-amino]methyl]benzoic acid to provide crude Intermediate 11 (605 mg, 100%) upon concentration of the reaction mixture.

### Example 11

The compounds listed below in Table 3 were prepared as described in Example 5, substituting the starting material shown in the table for *tert*-butyl N-[(2-chlorocarbonylphenyl)methyl]-N-methyl-carbamate (Intermediate 1).

**Table 3**

| Starting material | Product | Analytical data |
|---|---|---|
| 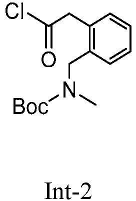 10 equiv. used in Step 1 | 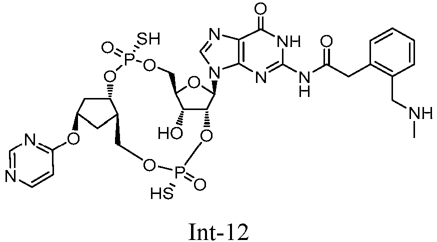 | LCMS (AA): *m*/*z =* 811.2 (M+H) |
| 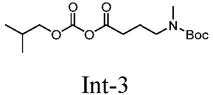 6 equiv. used in Step 1 | 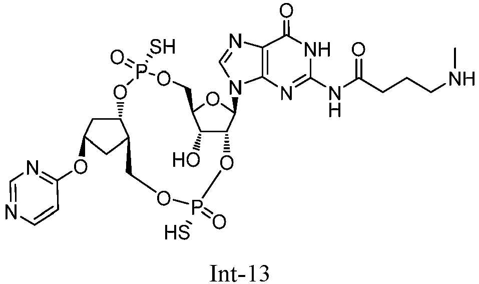 | LCMS (AA): *m*/*z =* 749.2 (M+H) |
| 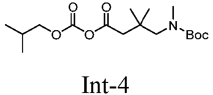 6 equiv. used in Step 1 | 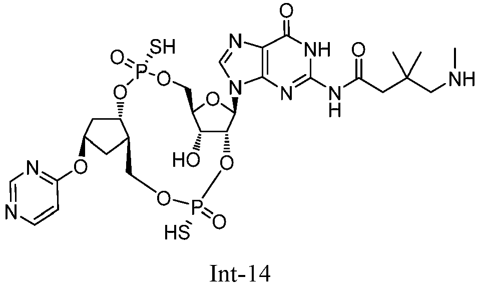 | LCMS (AA): *m*/*z =* 777.2 (M+H) |
| 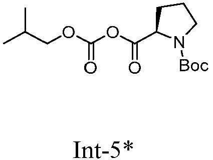 8 equiv. used in Step 1 | 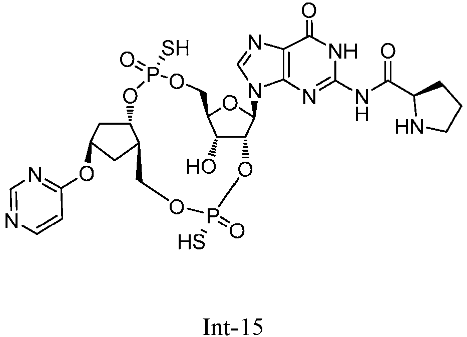 | LCMS (AA): *m*/*z =* 747.0 (M+H) |
| 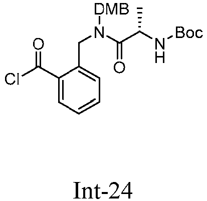 6 equiv. used in Step 1 | 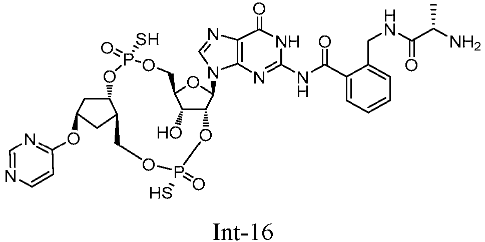 | LCMS (AA): *m*/*z =* 854.1 (M+H) |

| | | |
|---|---|---|
| * In step 2, 4M HCl in 1,4-dioxane with methanol as the solvent was used for the deprotection step. | | |

### Example 12

### N-{9-[(2R,5R,7R,8R,10R,12aR,14R,15R,15aR, 6R)-15-Fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-1][1,3,6,9,11,2,10]pentaoxadiphosphacyclotetradecin-14-yl]-9H-purin-6-yl}-4-(methylamino)butanamide (Intermediate 17)

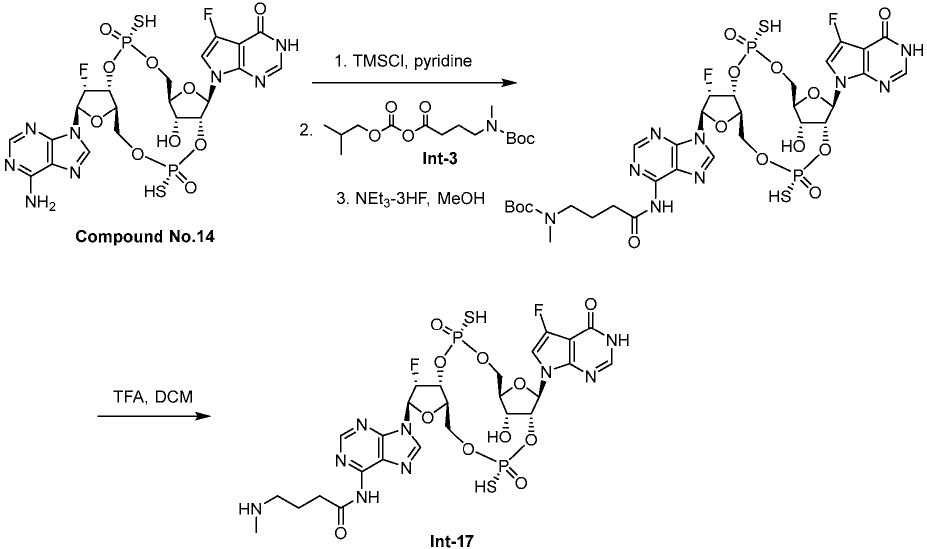

### Step 1: tert-Butyl [4-({9-[(2R,5R,7R,8R,10R,12aR,14R,15R,15aR,16R)-15-fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-l][1,3,6,9,11,2,10]pentaoxadiphosphacyclotetradecin-14-yl]-9H-purin-6-yl}amino)-4-oxobutyl]methylcarbamate

The title compound was prepared following the procedure described in Example 5, step 1, using 7-[(2*R*,5*R*,7*R*,8*R*,10*R*,12a*R*,14*R*,15*R*,15a*R*,16*R*)-14-(6-amino-9H-purin-9-yl)-15-fluoro-16-hydroxy-2,10-dioxido-2,10-disulfanyloctahydro-12H-5,8-methanofuro[3,2-l][1,3,6,9,11,2,10]pentaoxadiphosphacyclotetradecin-7-yl]-5-fluoro-3,7-dihydro-4H-pyrrolo[2,3-d]pyrimidin-4-one as the sodium salt (Compound No. 14, see WO2018100558A2, 200 mg, 0.265 mmol) and Intermediate 3 (503 mg, 1.59 mmol) in place of 2-amino-9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-1,9-dihydro-6H-purin-6-one and Intermediate 1 respectively. Purification by reverse phase flash column chromatography (0-50% ACN/aqueous ammonium bicarbonate (5 mM)) gave *tert*-butyl [4-({9-[(2*R*,5*R*,7*R*,8*R*,10*R*,12a*R*,14*R*,15*R*,15a*R*,16*R*)-15-fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-l][1,3,6,9,11,2,10]pentaoxadiphosphacyclotetradecin-14-yl]-9H-purin-6-yl}amino)-4-oxobutyl]methylcarbamate as the ammonium salt (148 mg, 59%). LCMS (AA): *m*/*z* = 910.5 (M+H). ¹H NMR (400 MHz, D₂O) δ 8.70 (s, 1H), 8.34 (s, 1H), 8.01 (s, 1H), 7.28 (s, 1H), 6.56 (d, *J* = 16.1 Hz, 1H), 6.43 (d, *J* = 8.4 Hz, 1H), 5.67 (dd, J = 51.0, 3.9 Hz, 1H), 5.18 - 4.99 (m, 2H), 4.82 - 4.77 (m, 1H), 4.63 - 4.58 (m, 1H), 4.49 - 4.38 (m, 3H), 4.31 - 4.25 (m, 1H), 4.12 - 4.06 (m, 1H), 3.43 - 3.36 (m, 2H), 2.88 (s, 3H), 2.73 - 2.68 (m, 2H), 2.04 - 1.97 (m, 2H), 1.34 (s, 9H). ³¹P NMR (162 MHz, D₂O) δ 55.47 (s, 1P), 52.07 (s, 1P). ¹⁹F NMR (376 MHz, CD₃OD) δ -165.50 (m, 1F), -203.45 (m, 1F).

### Step 2: N-{9-[(2R,5R,7R,8R,10R,12aR,14R,15R,15aR,16R)-15-Fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-l][1,3,6,9,11,2,10]pentaoxadiphosphacyclotetradecin-14-yl]-9H-purin-6-yl}-4-(methylamino)butanamide, Intermediate 17

The title compound was prepared following the procedure described in Example 5, step 2, using *tert*-butyl [4-({9-[(2*R*,5*R*,7*R*,8*R*,10*R*,12a*R*,14*R*,15*R*,15a*R*,16*R*)-15-fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-l][1,3,6,9,11,2,10]pentaoxadiphosphacyclotetradecin-14-yl]-9H-purin-6-yl}amino)-4-oxobutyl]methylcarbamate as the ammonium salt (42 mg, 0.045 mmol) in place of *tert*-butyl [2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate and stirring at rt for 30 min. N-{9-[(2*R*,5*R*,7*R*,8*R*,10*R*,12a*R*,14*R*,15*R*,15a*R*,16*R*)-15-Fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-l][1,3,6,9,11,2,10]pentaoxadiphosphacyclotetradecin-14-yl]-9H-purin-6-yl}-4-(methylamino)butanamide as the 2,2,2-trifluoroacetate salt (Intermediate 17, 41 mg, 100%) was obtained. LCMS (AA): *m*/*z* = 810.2 (M+H).

### Example 13

The compounds listed below in Table 5 were prepared as described in Example 12, substituting the starting material shown in Table 4 for isobutoxycarbonyl 4-[*tert-*butoxycarbonyl(methyl)amino]butanoate (Intermediate 3).

**Table 4**

| Starting material | Product | Analytical data |
|---|---|---|
| 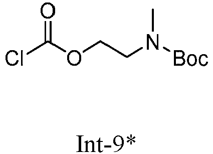 15 equiv. used in Step 1 | Int-18 | LCMS (AA): *m*/*z =* 812.1 (M+H) |
| 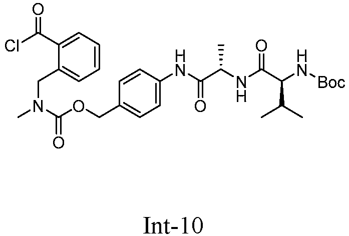 6 equiv. used in Step 1 | Int-31 | LCMS (AA): *m*/*z =* 1177.1 (M+H) |
| 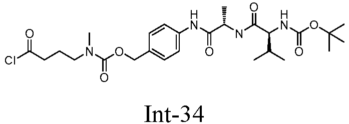 6 equiv. used in Step 1 | Int-35 | LCMS (AA): *m*/*z =* 1129.2 (M+H) |

| | | |
|---|---|---|
| * In step 2, 4M HCl in 1,4-dioxane was used for the deprotection step. | | |

**Table 5**

| Product | Structure |
|---|---|
| Int-18 | 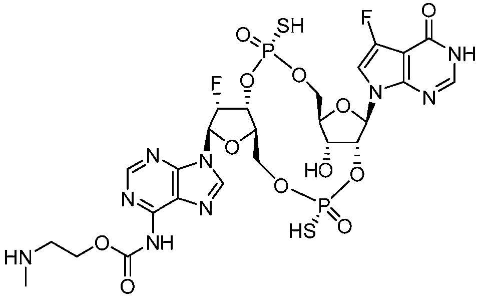 |
| Int-31 | 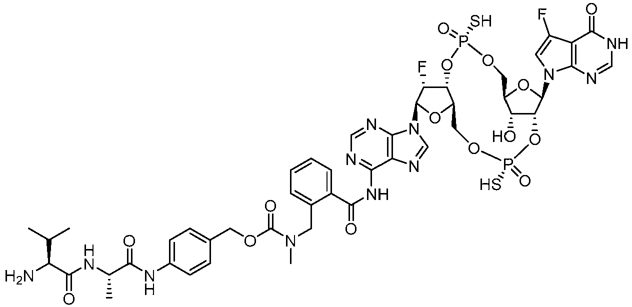 |
| Int-35 | 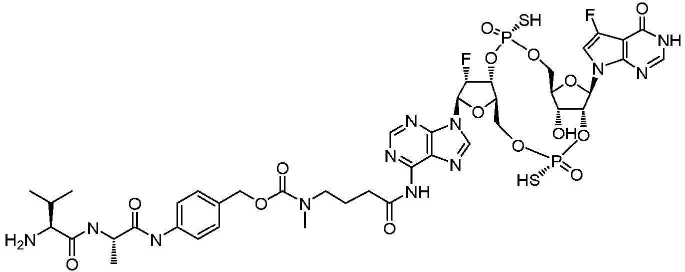 |

### Example 14

The compounds listed in Table 7 were prepared as described in Example 6, substituting the starting material shown in Table 6 for Intermediate 6.

**Table 6**

| Starting material | Product | Analytical data |
|---|---|---|
| 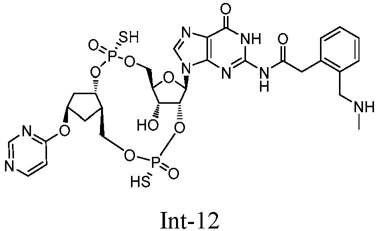 | C-2 | LCMS (AA): *m*/*z =* 1323.4 (M+H); HRMS (*m*/*z*): [M+H]⁺ calcd for C₅₆H₆₈N₁₂O₁₈P₂S₂ 1323.3764; found, 1323.3790. |
| 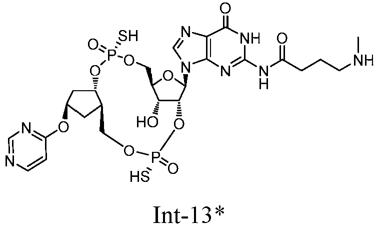 | C-3 | LCMS (AA): *m*/*z =* 1261.4 (M+H); HRMS (*m*/*z*): [M+H]⁺ calcd for C₅₁H₆₆N₁₂O₁₈P₂S₂ 1261.3607; found, 1261.3630. |
| 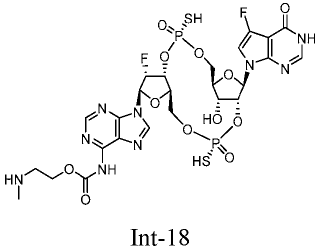 | C-4 | LCMS (AA): *m*/*z =* 1324.1 (M+H); HRMS (*m*/*z*): [M+H]⁺ calcd for C₅₁H₆₁F₂N₁₃O₁₉P₂S₂ 1324.3164; found, 1324.3186. |

| | | |
|---|---|---|
| * In step 2, p-cresol was added to the reaction mixture in a ratio of 2:1 (p-cresol:TFA). | | |

**Table 7**

| Product | Structure |
|---|---|
| C-2 | 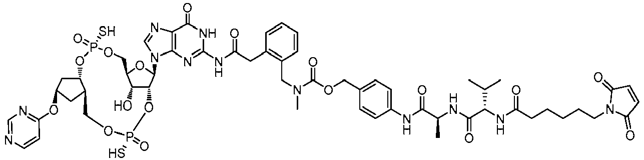 |
| C-3 | 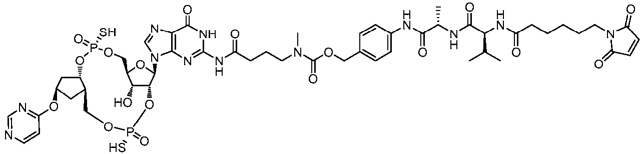 |
| C-4 | 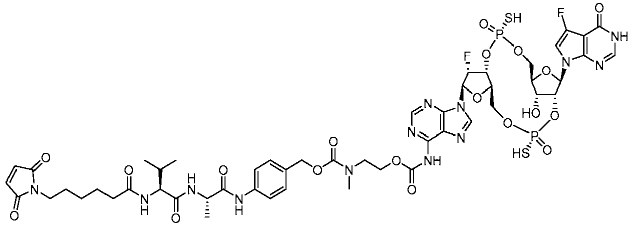 |

### Example 15

The compound listed in Table 8 (Intermediate 19) was prepared as described in Example 6, step 1, substituting the starting material shown in the table for *tert*-butyloxycarbonyl-valyl-alanyl-(4-aminobenzyl)-(4-nitrophenyl)carbonate.

**Table 8**

| Starting material | Product | Analytical data |
|---|---|---|
| 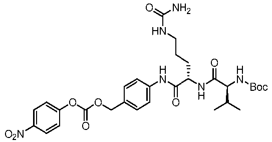 | 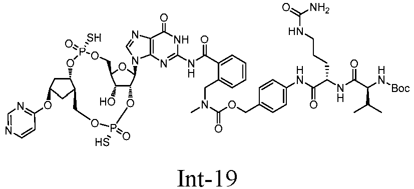 | LCMS (AA): *m*/*z* = 1302.4 (M+H) |

### Example 16

The compound listed in Table 10 was prepared as described in Example 6, steps 2 and 3, substituting the starting material shown in Table 9 for Intermediate 7.

**Table 9**

| Starting material | Product | Analytical data |
|---|---|---|
| 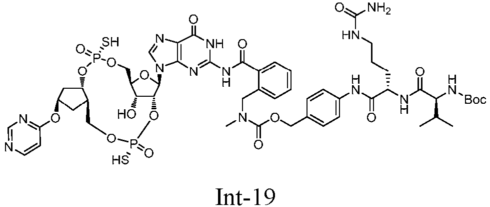 | C-5 | LCMS (AA): *m*/*z* = 1395.2 (M+H); HRMS (*m*/*z*): [M+H]⁺ calcd for C₅₈H₇₂N₁₄O₁₉P₂S₂ 1395.4088; found, 1395.4125. |

**Table 10**

| Product | Structure |
|---|---|
| C-5 | 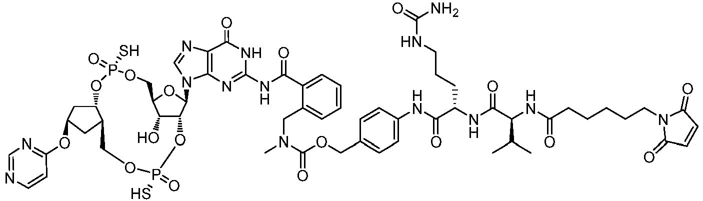 |

### Example 17

The compounds listed in Table 12 were prepared as described in Example 6, step 3, substituting the starting material shown in Table 11 for *N*-succinimidyl 6-maleimidohexanoate.

**Table 11**

| Starting material | Product | Analytical data |
|---|---|---|
| 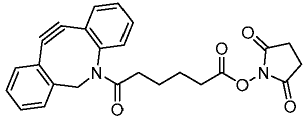 | C-6 | LCMS (AA): *m*/*z* = 1431.2 (M+H); HRMS (*m*/*z*): [M+H]⁺ calcd for C₆₆H₇₂N₁₂O₁₇P₂S₂ 1431.4128; found, 1431.4153. |
| 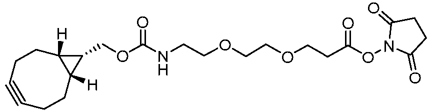 | C-7 | LCMS (AA): *m*/*z* = 726.3 (M/2+H); HRMS (*m*/*z*): [M+H]⁺ calcd for C₆₃H₈₀N₁₂O₂₀P₂S₂ 1451.4601; found, 1451.4608. |
| 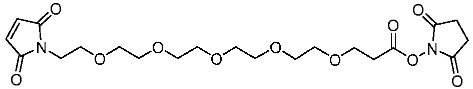 | C-8 | LCMS (AA): *m*/*z* = 1487.2 (M+H); HRMS (*m*/*z*): [M+H]⁺ calcd for C₆₂H₈₀N₁₂O₂₃P₂S₂ 1487.4449; found, 1487.4474. |
| 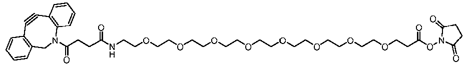 | C-9 | LCMS (AA): *m*/*z* = 913.9 (M/2+H) |
| 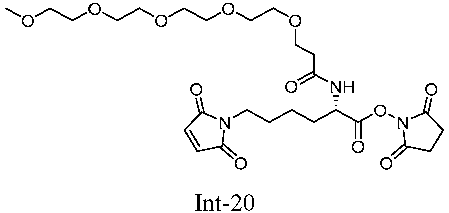 | C-10 | LCMS (AA): *m*/*z* = 1586.0 (M+H); HRMS (*m*/*z*): [M+H]⁺ calcd for C₆₇H₈₉N₁₃O₂₄P₂S₂ 1586.5133; found, 1586.5150. |
| 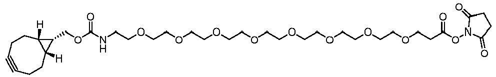 | C-11* | LCMS (AA): *m*/*z* = 1715.8 (M+H) |
| 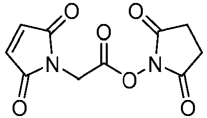 | C-12 | LCMS (AA): *m*/*z* = 1253.3 (M+H) |
| 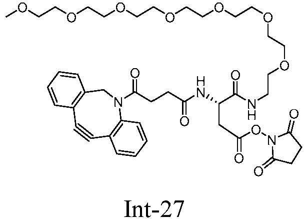 | C-13 | LCMS (AA): *m*/*z* = 920.9 (M/2+H); HRMS (*m*/*z*): [M+H]⁺ calcd for C₈₃H₁₀₄N₁₄O₂₆P₂S ₂ 1839.6236; found, 1839.6307. |
| 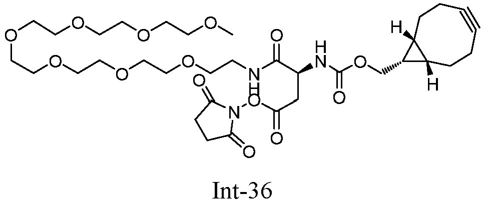 | C-35 | LCMS (AA): *m*/*z* = 1726.3 (M-H); HRMS (*m*/*z*): [M+H]⁺ calcd for C₇₅H₁₀₃N₁₃O₂₆P₂S ₂ 1728.6127; found, 1728.6148. |
| 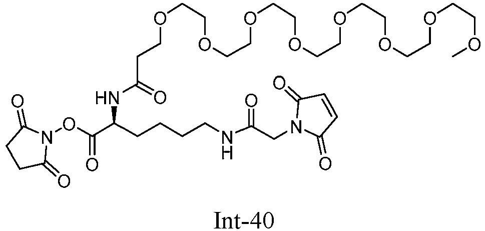 | C-39 | LCMS (AA): *m*/*z* = 1775.8 (M+H); HRMS (*m*/*z*): [M+H]⁺ calcd for C₇₅H₁₀₄N₁₄O₂₈P₂S ₂ 1775.6134; found, 1775.6138. |

| | | |
|---|---|---|
| * Used triethylamine as base instead of *N,N-*diisopropylethylamine. | | |

**Table 12**

| Product | Structure |
|---|---|
| C-6 | 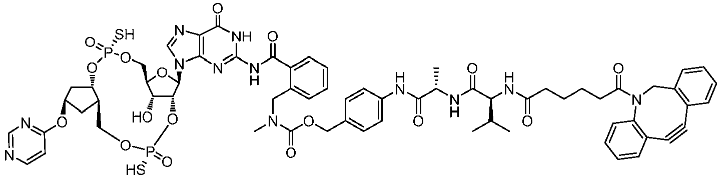 |
| C-7 | 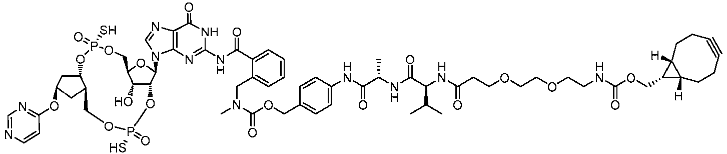 |
| C-8 | 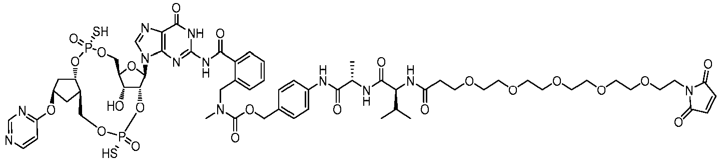 |
| C-9 | 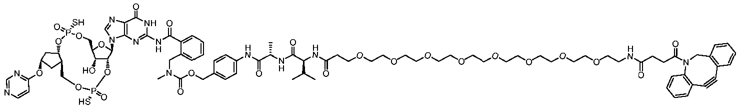 |
| C-10 | 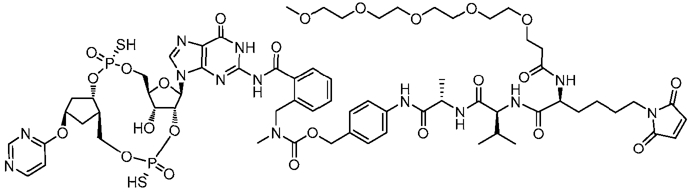 |
| C-11* | 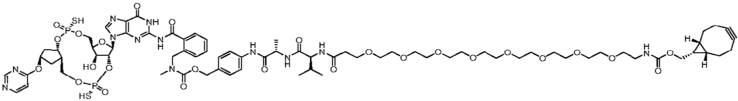 |
| C-12 | 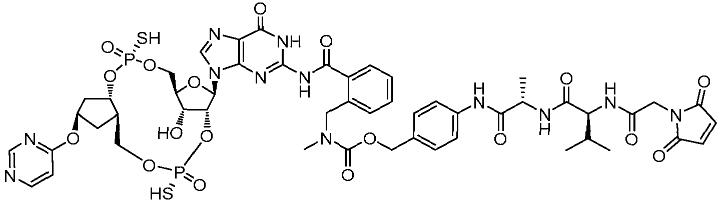 |
| C-13 | 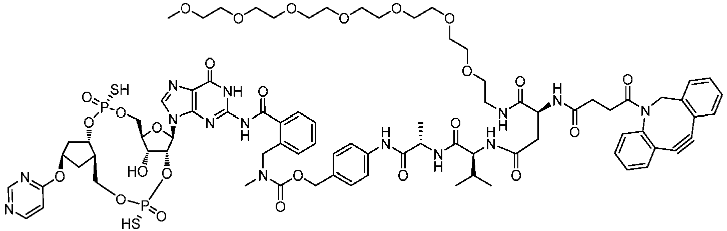 |
| C-35 | 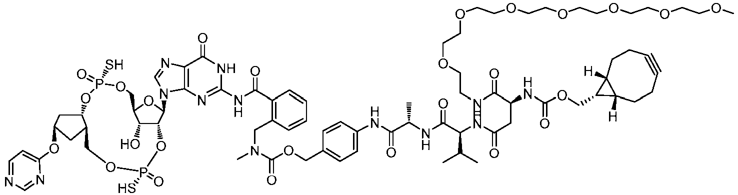 |
| C-39 | 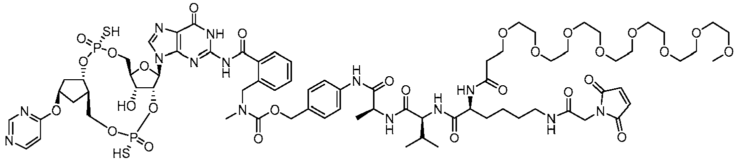 |

### Example 18

The compounds listed in Table 14 were prepared as described in Example 6, steps 2 and 3, substituting the starting materials shown in Table 13 for Intermediate 7 and *N*-succinimidyl 6-maleimidohexanoate respectively.

**Table 13**

| Starting Material in Step 2 | Starting Material in Step 3 | Product | Analytical data |
|---|---|---|---|
| Int-19 | 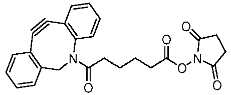 | C-14 | LCMS (AA): *m*/*z =* 759.5 (M/2+H); HRMS (*m*/*z*): [M+H]⁺ calcd for C₆₉H₇₈N₁₄O₁₈P₂S₂ 1517.4608; found, 1517.4635. |
| Int-19 | 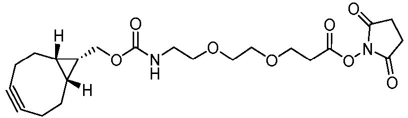 | C-15 | LCMS (AA): *m*/*z =* 769.8 (M/2+H); HRMS (*m*/*z*): [M+H]⁺ calcd for C₆₆H₈₆N₁₄O₂₁P₂S₂ 1537.5081; found, 1537.5120. |
| Int-21 | 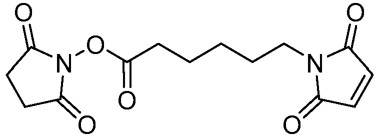 | C-16 | LCMS (AA): *m*/*z =* 1485.3 (M+H); HRMS (*m*/*z*): [M+H]⁺ calcd for C₆₃H₈₂N₁₂O₂₂P₂S₂ 1485.4656; found, 1485.4680. |
| Int-21 | 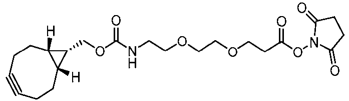 | C-17 | LCMS (AA): *m*/*z =* 1627.2 (M+H) |
| Int-28* | 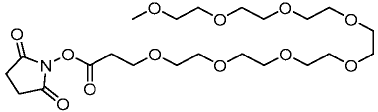 | C-18 | LCMS (AA): *m*/*z =* 1718.3 (M+H); HRMS (*m*/*z*): [M+H]⁺ calcd for C₇₃H₁₀₁N₁₃O₂₇P₂S₂ 1718.5919; found, 1718.5936. |
| Int-29 | 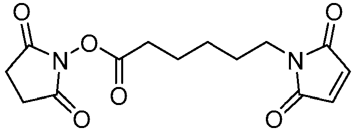 | C-19 | LCMS (AA): *m*/*z =* 1789.0 (M+H); HRMS *(m*/*z):* [M+H]⁺ calcd for C₇₆H₁₀₆N₁₄O₂₈P₂S₂ 1789.6290; found, 1789.6286. |
| Int-31** | 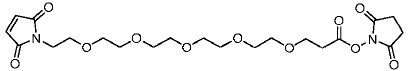 | C-32 | LCMS (AA): *m*/*z =* 1547.9 (M+H); HRMS (*m*/*z*): [M+H]⁺ calcd for C₆₃H₇₇F₂N₁₃O₂₃P₂ S₂ 1548.4213; found, 1548.4236. |
| Int-33 | 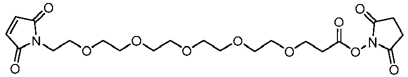 | C-33 | LCMS (AA): *m*/*z =* 1498.3 (M+H); HRMS *(m*/*z):* [M+H]⁺ calcd for C₆₂H₇₇FN₁₅O₂₂PS₂ 1498.4603; found, 1498.4604. |
| Int-38 | 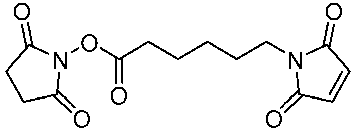 | C-36 | LCMS (AA): *m*/*z =* 1659.1 (M-H); HRMS (*m*/*z*): [M+H]⁺ calcd for C₇₁H₉₈N₁₂O₂₆P₂S₂ 1661.5705; found, 1661.5759. |
| Int-31** | 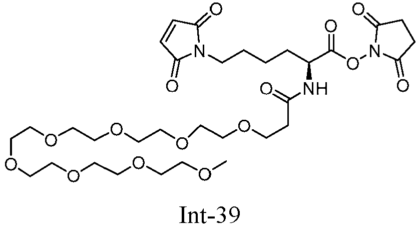 | C-38 | LCMS (AA): *m*/*z =* 1777.6 (M-H); HRMS (*m*/*z*): [M+H]⁺ calcd for C₇₄H₉₈F₂N₁₄O₂₇P₂ S₂ 1779.5683; found, 1779.5716. |
| Int-29 | 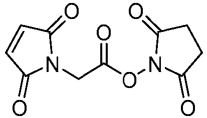 | C-40 | LCMS (AA): *m*/*z =* 1731.4 (M-H); HRMS (*m*/*z*): [M+H]⁺ calcd for;? found,?. |
| Int-38 | 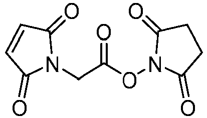 | C-41 | LCMS (AA): *m*/*z =* 1605.4 (M+H); HRMS (*m*/*z*): [M+H]⁺ calcd for C₆₇H₉₀N₁₂O₂₆P₂S₂ 1605.5079; found, 1605.5148. |
| Int-41 | 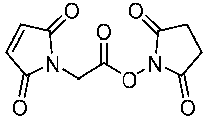 | C-42 | LCMS (AA): *m*/*z =* 1542.1 (M-H); HRMS (*m*/*z*): [M+H]⁺ calcd for C₆₄H₈₃N₁₃O₂₄P₂S₂ 1544.4663; found, 1544.4722. |
| Int-31** | 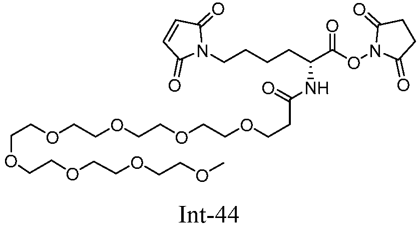 | C-45 | LCMS (AA): *m*/*z =* 1777.5 (M-H). |

| | | | |
|---|---|---|---|
| * Used triethylamine as base instead of *N,N-*diisopropylethylamine. ** Step 2 in Example 6 was skipped. | | | |

**Table 14**

| Product | Structure |
|---|---|
| C-14 | 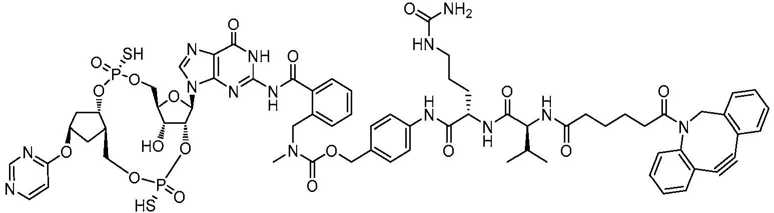 |
| C-15 | 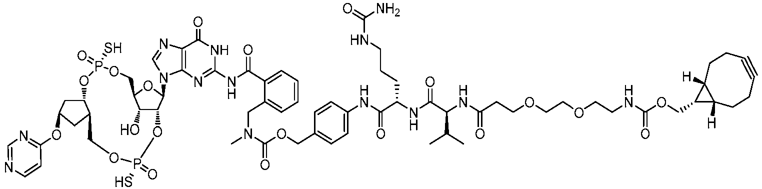 |
| C-16 | 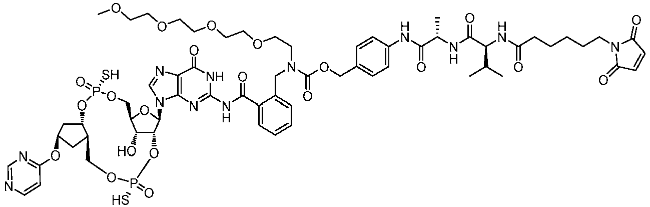 |
| C-17 | 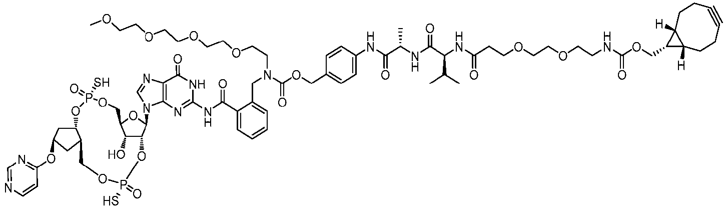 |
| C-18 | 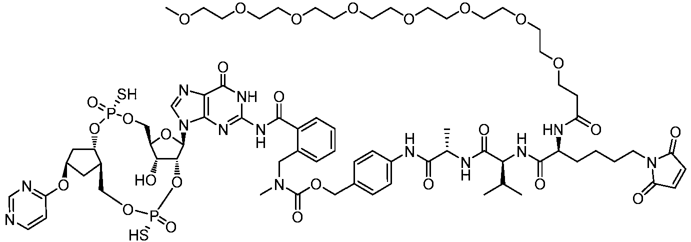 ¹H NMR (400 MHz, METHANOL-*d*₄) δ ppm 0.97 (t, *J*=7.15 Hz, 6 H) 1.24 - 1.40 (m, 2 H) 1.45 (d, *J*=7.09 Hz, 3 H) 1.55 - 1.88 (m, 5 H) 2.13 (m, 1 H) 2.25 - 2.40 (m, 1 H) 2.44 - 2.62 (m, 5 H) 2.89 (s, 3 H) 3.35 (s, 3 H) 3.46 (t, *J*=7.03 Hz, 2 H) 3.50 - 3.57 (m, 2 H) 3.62 (m, 28 H) 3.67 - 3.83 (m, 3 H) 3.98 - 4.08 (m, 1 H) 4.18 (d, *J*=6.97 Hz, 1 H) 4.22 - 4.40 (m, 4 H) 4.48 (d, *J*=7.09 Hz, 1 H) 4.78 (m, 2 H) 5.04 (s, 2 H) 5.45 - 5.64 (m, 2 H) 6.08 - 6.21 (m, 1 H) 6.78 (s, 2 H) 6.83 (d, *J*=6.11 Hz, 1 H) 7.12 - 7.35 (m, 3 H) 7.35 - 7.44 (m, 1 H) 7.45 - 7.61 (m, 3 H) 7.70 (br d, *J*=7.58 Hz, 1 H) 8.40 (d, *J*=5.99 Hz, 2 H) 8.70 (s, 1 H). |
| | ³¹P NMR (162 MHz, METHANOL-*d*₄) δ ppm 52.93 (s, 1 P) 54.97 (s, 1 P). |
| C-19 | 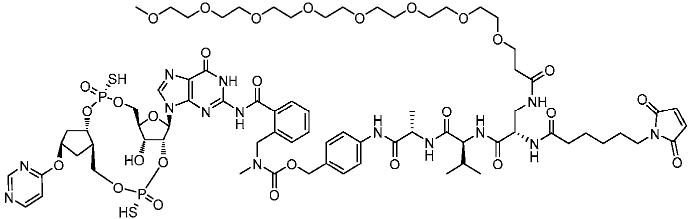 |
| C-32 | 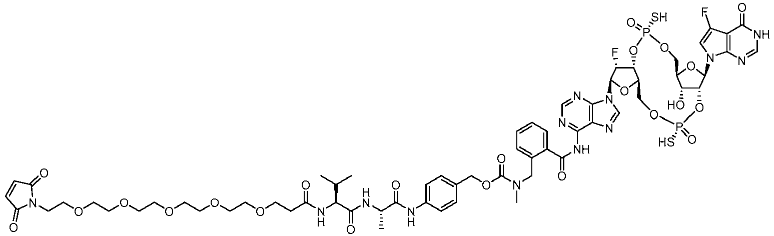 |
| C-33 | 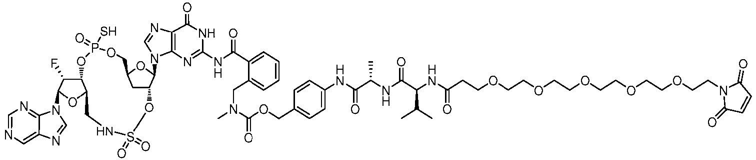 |
| C-36 | 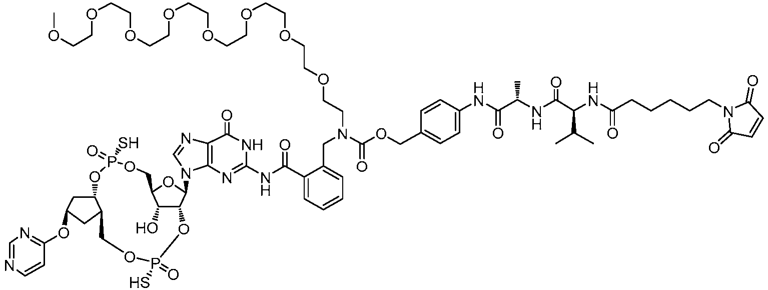 |
| C-38 | 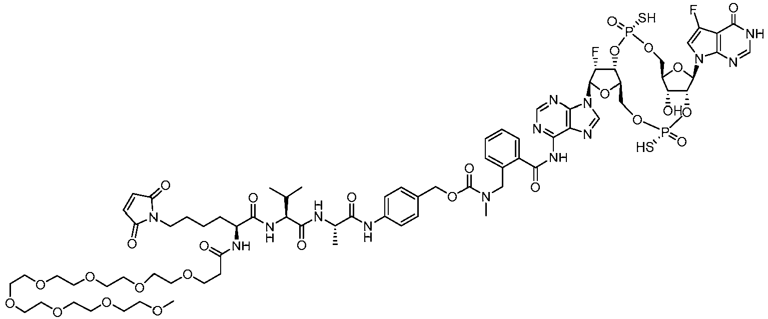 ³¹P NMR (162 MHz, METHANOL-*d*₄) δ ppm 52.26 (s, 1 P) 56.88 (s, 1 P) |
| | ¹H NMR (400 MHz, METHANOL-*d*₄) δ ppm 0.97 (m, 6 H) 1.34 (m, 2 H) 1.47 (d, *J*=7.09 Hz, 3 H) 1.52 - 1.61 (m, 2 H) 1.63 - 1.75 (m, 1 H) 1.77 - 1.88 (m, 1 H) 2.11 - 2.24 (m, 1 H) 2.44 - 2.59 (m, 2 H) 2.94 (br s, 3 H) 3.35 (s, 3 H) 3.45 (m, 2 H) 3.49 - 3.56 (m, 2 H) 3.58 - 3.66 (m, 28 H) 3.73 (t, *J*=6.05 Hz, 2 H) 4.01 (dd, *J*=11.25, 3.42 Hz, 1 H) 4.20 (d, *J*=6.85 Hz, 1 H) 4.29 (s, 1 H) 4.33 - 4.59 (m, 5 H) 4.86 (m, 2 H) 4.96 - 5.08 (m, 3 H) 5.15 - 5.30 (m, 1 H) 5.72 (d, *J*=52.0 Hz, 1 H) 6.35 - 6.53 (m, 2 H) 6.77 (s, 2 H) 7.09 - 7.24 (m, 2 H) 7.26 - 7.35 (m, 1 H) 7.38 (s, 1 H) 7.43 - 7.59 (m, 4 H) 7.76 (s, 1 H) 7.80 (br d, *J*=7.46 Hz, 1 H) 8.42 (s, 1 H) 8.62 - 8.69 (s, 1 H) |
| C-40 | 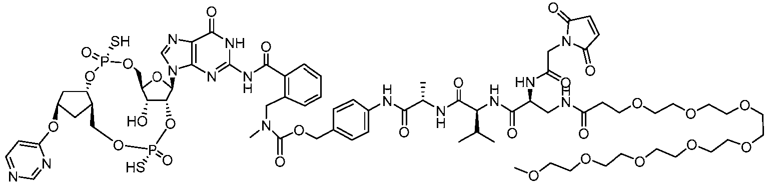 |
| C-41 | 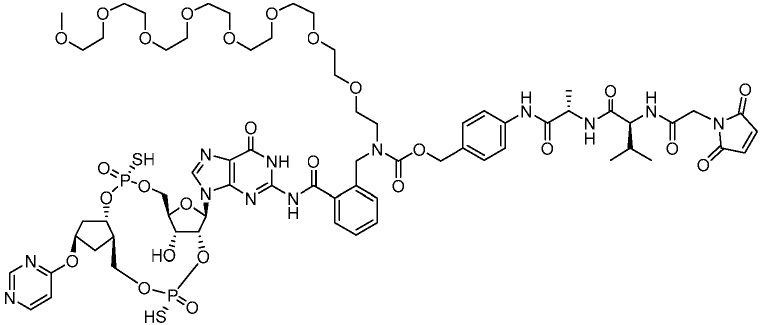 |
| C-42 | 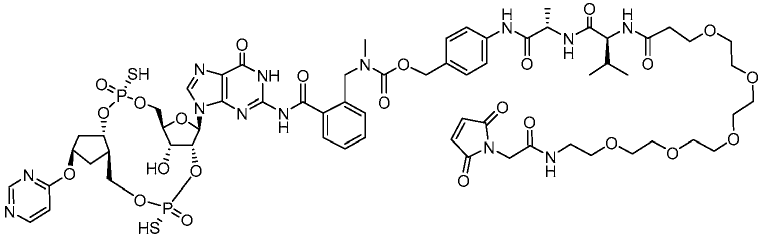 |
| C-45 | 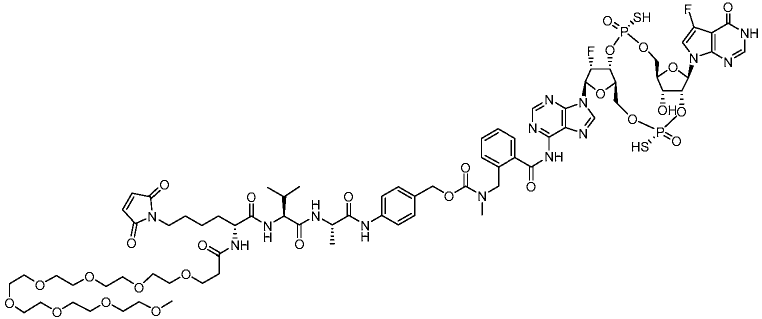 ¹H NMR (400 MHz, METHANOL-*d*₄) δ ppm 0.98 (dd, *J*=12.90, 6.91 Hz, 6 H) 1.23 - 1.40 (m, 2 H) 1.47 (d, *J*=7.21 Hz, 3 H) 1.58 (m, 2 H) 1.73 (m, 2 H) 2.17 - 2.30 (m, 2 H) 2.35 (m, 1 H) 2.91 (s, 3 H) 3.31 (s, 3 H) 3.40 (m, 2 H) 3.46 - 3.60 (m, 30 H) 3.97 (dd, *J*=11.55, 3.36 Hz, 1 H) 4.12 (d, *J*=5.62 Hz, 1 H) 4.19 - 4.28 (m, 2 H) 4.30 - 4.54 (m, 6 H) 4.83 (m, 2 H) 4.93 - 5.07 (m, 3 H) 5.10 - 5.25 (m, 1 H) 5.72 (d, *J*=52.0 Hz, 1 H) 6.36 - 6.48 (m, 2 H) 6.76 (s, 2 H) 7.05 - 7.32 (m, 3 H) 7.35 (s, 1 H) 7.38 - 7.68 (m, 4 H) 7.75 (s, 1 H) 7.76 - 7.83 (m, 1 H) 8.41 (s, 1 H) 8.61 - 8.68 (s, 1 H) |
| | ³¹P NMR (162 MHz, METHANOL-*d*₄) δ ppm 52.35 (s, 1 P) 56.91 (s, 1 P) |

### Example 19

### 4-{[(2S)-2-{[(2S)-2-{[6-(2,5-Dioxo-2,5-dihydro-1H-pyrrol-1-yl)hexanoyl]amino}-3-methylbutanoyl]amino}propanoyl]amino}benzyl [4-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}amino)-2,2-dimethyl-4-oxobutyl]methylcarbamate, Compound 20 (C-20)

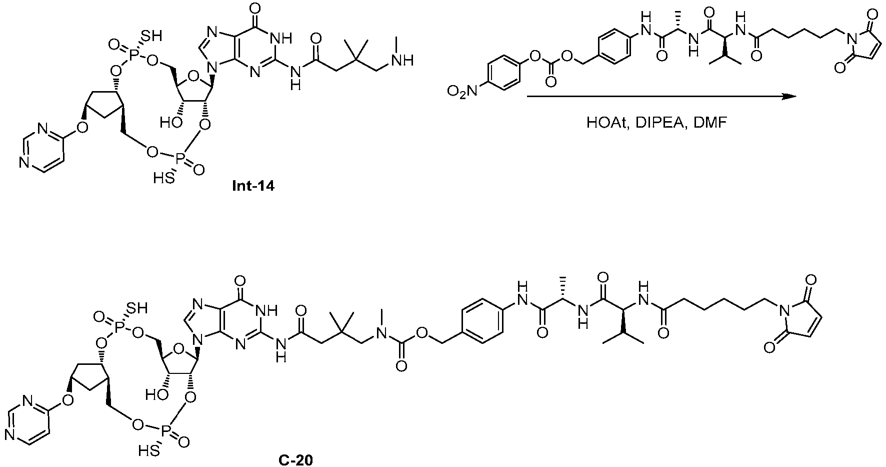

### Step 1: 4-{[(2S)-2-{[(2S)-2-{[6-(2,5-Dioxo-2,5-dihydro-1H-pyrrol-1-yl)hexanoyl]amino}-3-methylbutanoyl]amino}propanoyl]amino}benzyl [4-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}amino)-2,2-dimethyl-4-oxobutyl]methylcarbamate, Compound 20

To a solution of [4-[[(2*S*)-2-[[(2*S*)-2-[6-(2,5-dioxopyrrol-1-yl)hexanoylamino]-3-methyl-butanoyl]amino]propanoyl]amino]phenyl]methyl (4-nitrophenyl) carbonate (47.9 mg, 0.037 mmol) and 1-hydroxy-7-azabenzotriazole (4.08 mg, 0.03 mmol) in DMF (0.60 mL) and *N,N*-diisopropylethylamine (26 uL, 0.15 mmol) was added a solution of N-{9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}-3,3-dimethyl-4-(methylamino)butanamide as the 2,2,2-trifluoroacetate salt (Intermediate 14, 20 mg, 0.02 mmol) in DMF (0.54 mL) at rt. The reaction mixture was allowed to stir at rt for 16 h. Celite was added and the mixture was concentrated to dryness. The crude residue absorbed onto Celite was purified by reverse phase flash column chromatography (0-100% ACN/aqueous ammonium acetate (10 mM)) to provide 4-{[(2*S*)-2-{[(2*S*)-2-{[6-(2,5-dioxo-2,5-dihydro-1H-pyrrol-1-yl)hexanoyl]amino}-3-methylbutanoyl]amino}propanoyl]amino}benzyl [4-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}amino)-2,2-dimethyl-4-oxobutyl]methylcarbamate as the ammonium salt (C-20, 4 mg, 17%). LCMS (AA): *m*/*z* = 1287.4 (M-H).

### Example 20

The compounds listed in Table 16 were prepared as described in Example 19, substituting the starting materials A and B shown in Table 15 for Intermediate 14 and [4-[[(2*S*)-2-[[(2S)-2-[6-(2,5-dioxopyrrol-1-yl)hexanoylamino]-3-methyl-butanoyl]amino]propanoyl]amino]phenyl]methyl (4-nitrophenyl) carbonate respectively.

**Table 15**

| Starting Material A | Starting Material B | Product | Analytical data |
|---|---|---|---|
| Int-17* | 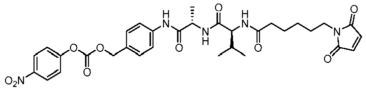 | C-21 | LCMS (AA): *m*/*z =* 1322.3 (M+H); HRMS (*m*/*z*): [M+H]⁺ calcd for C₅₂H₆₃F₂N₁₃O₁₈P₂S₂ 1322.3371; found, 1322.3394. |
| Int-17** | 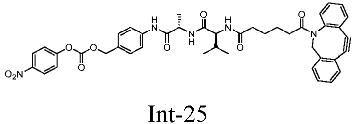 | C-22 | LCMS (AA): *m*/*z =* 1444.8 (M+H) |
| Int-17** | 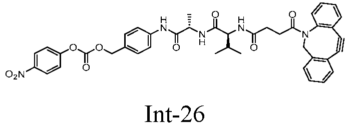 | C-23 | LCMS (AA): *m*/*z =* 1416.8 (M+H) |
| Int-18** | 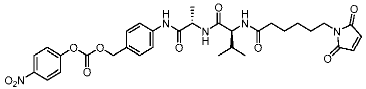 | C-44 | LCMS (AA): *m*/*z =* 1304.4 (M+H); HRMS (*m*/*z*): [M+H]⁺ calcd for C₅₀H₆₃N₁₅O₁₉P₂S₂ 1304.3414; found 1304.3465. |

| | | | |
|---|---|---|---|
| * Used 1-hydroxybenzotriazole and triethylamine in the reaction instead of 1-hydroxy-7-azabenzotriazole and *N,N*-diisopropylethylamine. ** Used 4-dimethylaminopyridine and triethylamine in the reaction instead of 1-hydroxy-7-azabenzotriazole and *N,N*-diisopropylethylamine. | | | |

**Table 16**

| Product | Structure |
|---|---|
| C-21 | 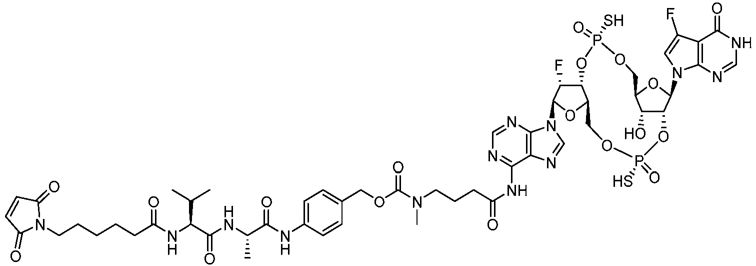 |
| C-22 | 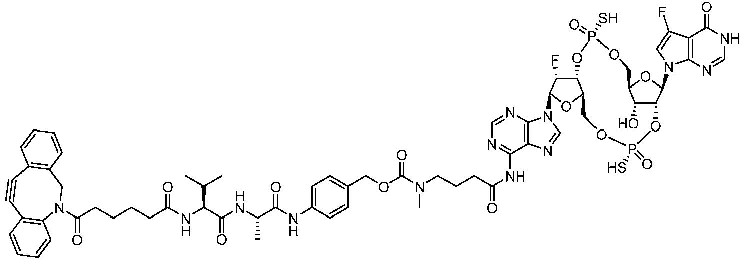 |
| C-23 | 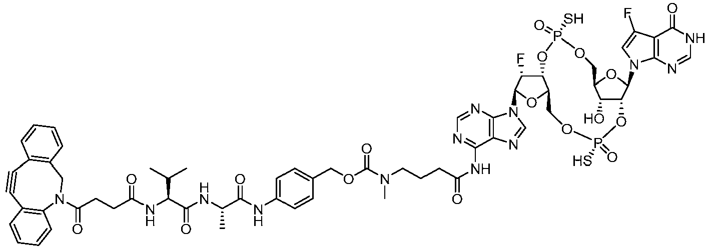 |
| C-44 | 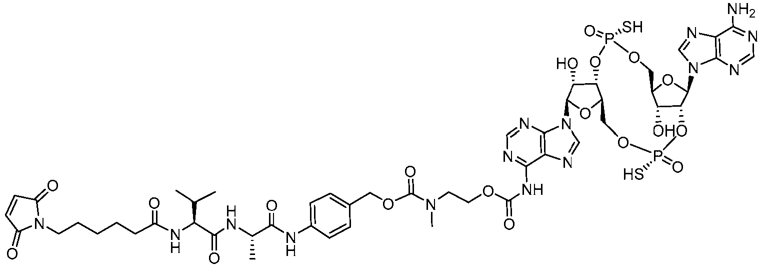 |

### Example 21

### N-{(2S)-6-(2,5-Dioxo-2,5-dihydro-1H-pyrrol-1-yl)-1-[(2,5-dioxopyrrolidin-1-yl)oxy]-1-oxohexan-2-yl}-2,5,8,11,14-pentaoxaheptadecan-17-amide (Intermediate 20)

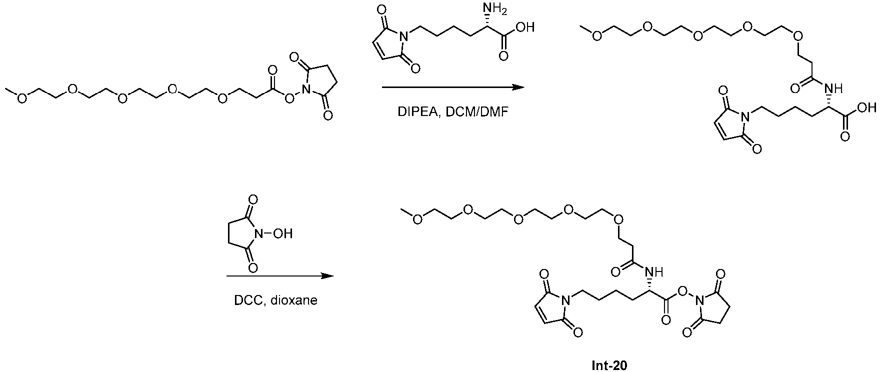

### Step 1: (19S)-19-[4-(2,5-Dioxo-2,5-dihydro-1H-pyrrol-1-yl)butyl]-17-oxo-2,5,8,11,14-pentaoxa-18-azaicosan-20-oic acid

To a solution of 1-[(17-oxo-2,5,8,11,14-pentaoxaheptadecan-17-yl)oxy]pyrrolidine-2,5-dione (4.0 g, 10.5 mmol) in anhydrous DCM (10 mL) was added (*S*)-2-amino-6-(2,5-dioxo-2,5-dihydro-1H-pyrrol-1-yl)hexanoic acid hydrochloride (3.4 g, 12.9 mmol) dissolved in DMF (40 mL) followed by *N,N-*diisopropylethylamine (6.9 mL, 42 mmol). The reaction mixture was allowed to stir at rt for 18 h. The reaction mixture was filtered and concentrated to dryness. The crude residue was purified by preparative HPLC to afford (19*S*)-19-[4-(2,5-dioxo-2,5-dihydro-1H-pyrrol-1-yl)butyl]-17-oxo-2,5,8,11,14-pentaoxa-18-azaicosan-20-oic acid (1.26 g, 24%). LCMS (AA): *m*/*z* = 489.3 (M+H).

### Step 2: N-{(2S)-6-(2,5-Dioxo-2,5-dihydro-1H-pyrrol-1-yl)-1-[(2,5-dioxopyrrolidin-1-yl)oxy]-1-oxohexan-2-yl}-2,5,8,11,14-pentaoxaheptadecan-17-amide, Intermediate 20

To a solution of (19S)-19-[4-(2,5-dioxo-2,5-dihydro-1H-pyrrol-1-yl)butyl]-17-oxo-2,5,8,11,14-pentaoxa-18-azaicosan-20-oic acid (65 mg, 0.133 mmol) and*N,N*'-dicyclohexylcarbodiimide (30 mg, 0.145 mmol) in 1,4-dioxane (0.68 mL) was added *N-*hydroxysuccinimide (15.3 mg, 0.133 mmol) at rt. The reaction mixture was allowed to stir at rt for 16 h. Celite was added and the mixture was filtered, rinsed with 1,4-dioxane and concentrated to dryness. The crude residue absorbed onto Celite was purified by silica gel chromatography (0-10% MeOH/DCM) to provide Intermediate 20 (14.6 mg, 19%). LCMS (AA): *m*/*z* = 586.3 (M+H).

### Example 22

The compounds listed in Table 17 were prepared as described in Example 9, substituting the starting material shown in the table for Intermediate 10.

**Table 17**

| Starting material | Product | Analytic al data |
|---|---|---|
| 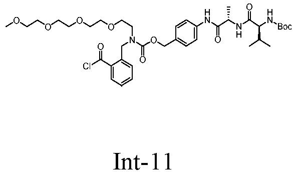 6 equiv. used | 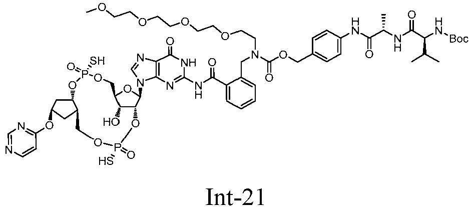 | LCMS (AA): *m*/*z* = 1392.2 (M+H) |
| 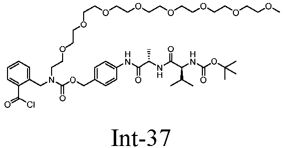 6 equiv. used | 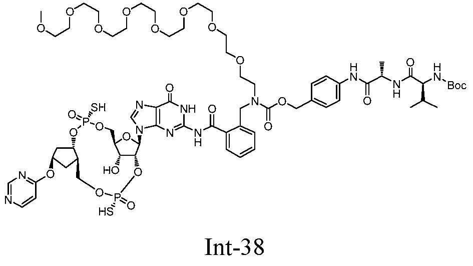 | LCMS (AA): *m*/*z* = 785.3 (M/2+H ) |
| 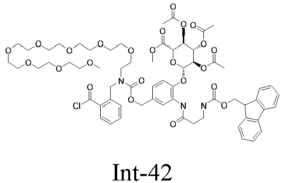 6 equiv. used | 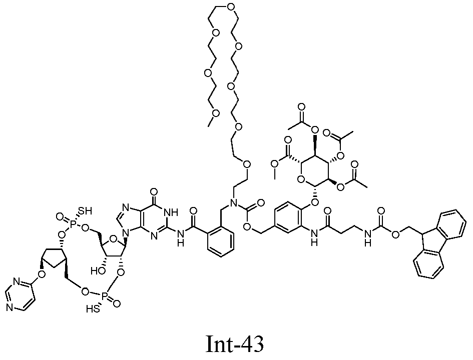 | LCMS (AA): *m*/*z* = 1923.2 (M+H) |

### Example 23

### (2S,3S,4S,5R,6S)-6-{2-[(3-{[6-(2,5-Dioxo-2,5-dihydro-1H-pyrrol-1-yl)hexanoyl]amino}propanoyl)amino]-4-[({[2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl](methyl)carbamoyl}oxy)methyl]phenoxy}-3,4,5-trihydroxytetrahydro-2H-pyran-2-carboxylic acid, Compound 24 (C-24)

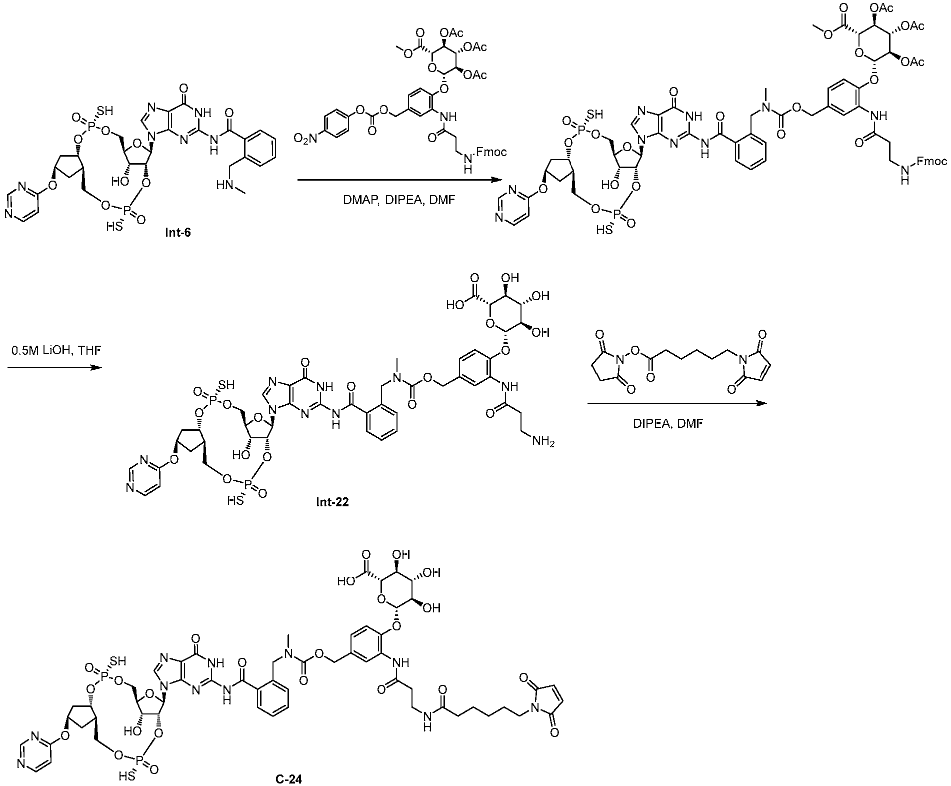

### Step 1: Methyl (2S,3S,4S,5R,6S)-3,4,5-triacetoxy-6-{2-[(3-{[(9H-fluoren-9-ylmethoxy)carbonyl]amino}propanoyl)amino]-4-[({[2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl](methyl)carbamoyl}oxy)methyl]phenoxy}tetrahydro-2H-pyran-2-carboxylate

The title compound was prepared following the procedure described in Example 6, step 1, starting with Intermediate 6 (104 mg, 0.094 mmol) and methyl (2*S*,3*S*,4*S*,5*R*,6*S*)-3,4,5-triacetoxy-6-[2-[3-(9*H*-fluoren-9-ylmethoxycarbonylamino)propanoylamino]-4-[(4-nitrophenoxy)carbonyloxymethyl]phenoxy]tetrahydropyran-2-carboxylate (150 mg, 0.16 mmol) (for synthesis see Jeffrey, S.C. et al. Bioconjugate Chem. 2006, 17, 831-840) using *N,N-*diisopropylethylamine as a base to provide methyl (2*S*,3*S*,4*S*,5*R*,6*S*)-3,4,5-triacetoxy-6-{2-[(3-{[(9H-fluoren-9-ylmethoxy)carbonyl]amino}propanoyl)amino]-4-[({[2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl](methyl)carbamoyl}oxy)methyl]phenoxy}tetrahydro-2H-pyran-2-carboxylate as the ammonium salt (71 mg, 48%). LCMS (AA): *m*/*z* = 1570.9 (M+H).

### Step 2: (2S,3S,4S,5R,6S)-6-{2-[(3-Aminopropanoyl)amino]-4-[({[2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl](methyl)carbamoyl}oxy)methyl]phenoxy}-3,4,5-trihydroxytetrahydro-2H-pyran-2-carboxylic acid, Intermediate 22

To a solution of methyl (2*S*,3*S*,4*S*,5*R*,6*S*)-3,4,5-triacetoxy-6-{2-[(3-{[(9H-fluoren-9-ylmethoxy)carbonyl]amino}propanoyl)amino]-4-[({[2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl](methyl)carbamoyl}oxy)methyl]phenoxy}tetrahydro-2H-pyran-2-carboxylate as the ammonium salt (66 mg, 0.042 mmol) dissolved in THF (13 mL) was added a LiOH (0.5M aqueous solution, 1.25 mL, 0.625 mmol) at rt and the reaction mixture was stirred for 2.5 h. The reaction mixture was neutralized to pH 7 with 1M HCl and the mixture was concentrated to dryness. The crude residue was purified by reverse phase flash column chromatography (0-30% ACN/aqueous ammonium bicarbonate (5 mM)) to provide Intermediate 22 as the ammonium salt (26 mg, 50%). This material was then dissolved in MeOH (5 mL) and triethylamine (1 mL) was added. The mixture was shaken for 1 min. Solvents were removed and the resulting residue was lyophilized overnight to provide (2*S*,3*S*,4*S*,5*R*,6*S*)-6-{2-[(3-aminopropanoyl)amino]-4-[({[2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl](methyl)carbamoyl}oxy)methyl]phenoxy}-3,4,5-trihydroxytetrahydro-2H-pyran-2-carboxylic acid as the *N,N-*diethylethanamine salt (Intermediate 22, 30 mg, 100%). LCMS (AA): *m*/*z* = 1209.0 (M+H).

### Step 3: (2S,3S,4S,5R,6S)-6-{2-[(3-{[6-(2,5-Dioxo-2,5-dihydro-1H-pyrrol-1-yl)hexanoyl]amino}propanoyl)amino]-4-[({[2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl](methyl)carbamoyl}oxy)methyl]phenoxy}-3,4,5-trihydroxytetrahydro-2H-pyran-2-carboxylic acid, Compound 24

The title compound was prepared following the procedure described in Example 6, step 3, using Intermediate 22 (24 mg, 0.02 mmol) in place of Intermediate 8 to provide (2*S*,3*S*,4*S*,5*R*,6*S*)-6-{2-[(3-{[6-(2,5-dioxo-2,5-dihydro-1H-pyrrol-1-yl)hexanoyl]amino}propanoyl)amino]-4-[({[2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl](methyl)carbamoyl}oxy)methyl]phenoxy}-3,4,5-trihydroxytetrahydro-2H-pyran-2-carboxylic acid as the ammonium salt (C-24, 17 mg, 71%). LCMS (AA): *m*/*z =* 1402.4 (M+H). HRMS *(m*/*z):* [M+H]⁺ calcd for C₅₆H₆₅N₁₁O₂₄P₂S₂ 1402.3193; found, 1402.3232.

### Example 24

The compound listed in Table 18 (Compound 25) was prepared as described in Example 23, step 3, substituting the starting material shown in the table for *N*-succinimidyl 6-maleimidohexanoate respectively.

**Table 18**

| Starting material | Product | Analytical data |
|---|---|---|
| 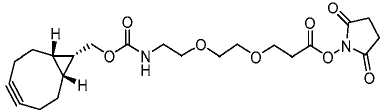 | C-25 | LCMS (AA): *m*/*z* = 1544.8 (M+H); HRMS (*m*/*z*): [M+H]⁺ calcd for C₆₄H₇₉N₁₁O₂₆P₂S₂ 1544.4187; found, 1544.4232. |

### C-25 Structure:

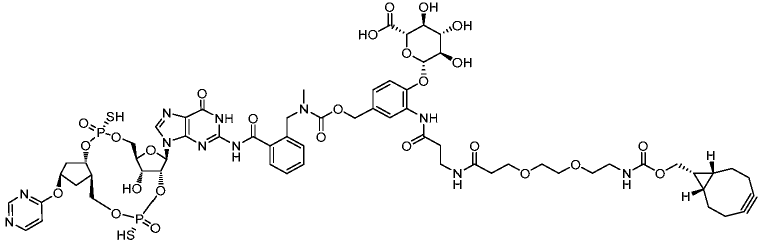

### Example 25

### 4-{[(2S)-2-{[(2S)-2-{[6-(2,5-Dioxo-2,5-dihydro-1H-pyrrol-1-yl)hexanoyl]amino}-3-methylbutanoyl]amino}propanoyl]amino}benzyl {2-[(2R)-2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)pyrrolidin-1-yl]-2-oxoethyl}carbamate, Compound 26 (C-26)

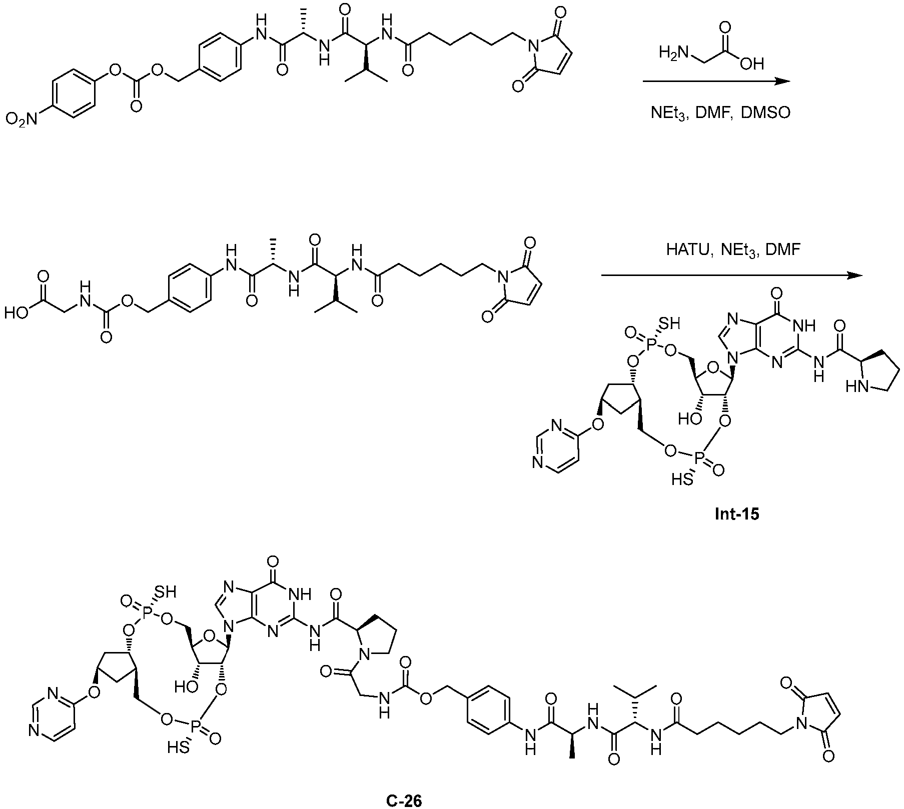

### Step 1: 2-[[4-[[(2S)-2-[[(2S)-2-[6-(2,5-Dioxopyrrol-1-yl)hexanoylamino]-3-methyl-butanoyl]amino]propanoyl]amino]phenyl]methoxycarbonylamino]acetic acid

To a solution of glycine (24 mg, 0.32 mmol) in triethylamine (0.10 mL, 0.72 mmol), DMF (0.50 mL) and DMSO (0.50 mL) was added [4-[[(2*S*)-2-[[(2*S*)-2-[6-(2,5-dioxopyrrol-1-yl)hexanoylamino]-3-methyl-butanoyl]amino]propanoyl]amino]phenyl]methyl (4-nitrophenyl) carbonate (220 mg, 0.34 mmol) at rt. The reaction mixture was stirred at rt for 4 h. The reaction mixture was then cooled to 0 °C. Water (1 mL) was added and the solution was acidified to pH 4 by the addition of formic acid. The mixture was concentrated to dryness and the crude residue was purified by reverse phase flash column chromatography (0-100% ACN/aqueous formic acid (0.1%)) to provide 2-[[4-[[(2*S*)-2-[[(2*S*)-2-[6-(2,5-dioxopyrrol-1-yl)hexanoylamino]-3-methyl-butanoyl]amino]propanoyl]amino]phenyl]methoxycarbonylamino]acetic acid (87 mg, 44%). LCMS (AA): *m*/*z =* 586.3 (M-H).

### Step 2: 4-{[(2S)-2-{[(2S)-2-{[6-(2,5-Dioxo-2,5-dihydro-1H-pyrrol-1-yl)hexanoyl]amino}-3-methylbutanoyl]amino}propanoyl]amino}benzyl {2-[(2R)-2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)pyrrolidin-1-yl]-2-oxoethyl}carbamate, Compound 26

To a solution of 2-[[4-[[(2*S*)-2-[[(2*S*)-2-[6-(2,5-dioxopyrrol-1-yl)hexanoylamino]-3-methyl-butanoyl]amino]propanoyl]amino]phenyl]methoxycarbonylamino]acetic acid (40 mg, 0.068 mmol) and triethylamine (35 uL, 0.25 mmol) in DMF (1 mL) was added 1-[bis(dimethylamino)methylene]-1*H*-1,2,3-triazolo[4,5-*b*]pyridinium 3-oxid hexafluorophosphate (46 mg, 0.12 mmol) at rt and the reaction mixture was stirred for 10 min. To this solution was then added a solution of (2*R*)-N-{9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}pyrrolidine-2-carboxamide as the 2,2,2-trifluoroacetate salt (Intermediate 15, 50 mg, 0.047 mmol) in DMF (0.60 mL) dropwise at rt. The reaction mixture was stirred at rt for 30 min. The mixture was then cooled to 0 °C, water (1 mL) was added, and the solution was acidified to pH 4 by the addition of formic acid. The mixture was concentrated to dryness and the crude residue was purified by reverse phase flash column chromatography (0-100% ACN/aqueous formic acid (0.1%)) to provide 4-{[(2*S*)-2-{[(2*S*)-2-{[6-(2,5-dioxo-2,5-dihydro-1H-pyrrol-1-yl)hexanoyl]amino}-3-methylbutanoyl]amino}propanoyl]amino}benzyl {2-[(2*R*)-2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)pyrrolidin-1-yl]-2-oxoethyl}carbamate (C-26, 32 mg, 50%). LCMS (AA): *m*/*z =* 1316.4 (M+H). HRMS (*m*/*z*): [M+H]⁺ calcd for C₅₃H₆₇N₁₃O₁₉P₂S₂ 1316.3666; found, 1316.3705.

### Example 26

### (2R)-1-({[(2S)-2-{[(2S)-2-{[6-(2,5-Dioxo-2,5-dihydro-1H-pyrrol-1-yl)hexanoyl]amino}-3-methylbutanoyl]amino}propanoyl]amino}acetyl)-N-{9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}pyrrolidine-2-carboxamide, Compound 27 (C-27)

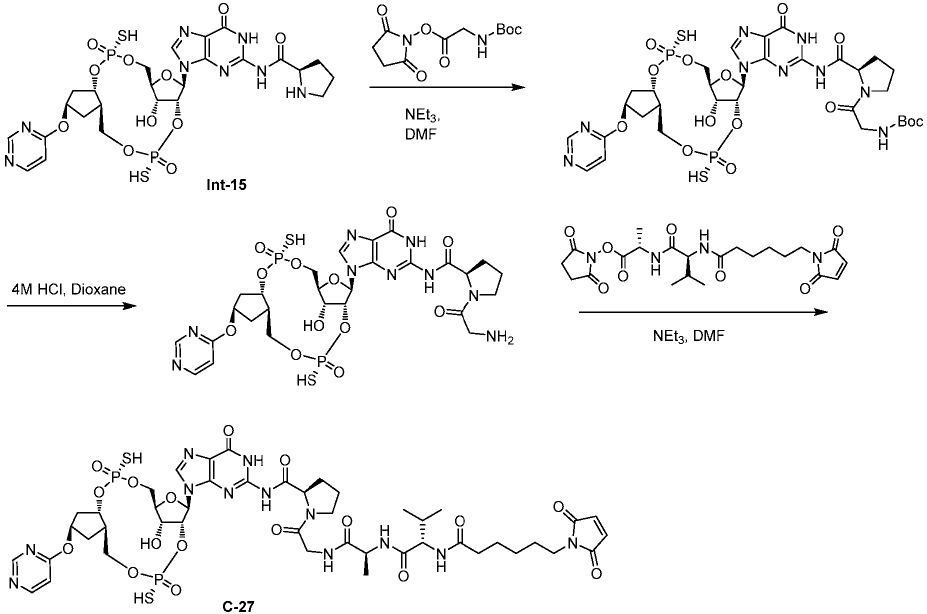

### Step 1: tert-Butyl {2-[(2R)-2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)pyrrolidin-1-yl]-2-oxoethyl}carbamate

To a mixture of Intermediate 15 (60 mg, 0.05 mmol) and 2,5-dioxopyrrolidin-1-yl (tert-butoxycarbonyl)glycinate (19 mg, 0.07 mmol) in DMF (1.6 mL) was added triethylamine (50 uL, 0.355 mmol), and the reaction mixture was stirred at rt for 1 h. The reaction mixture was quenched with water, Celite was added and the reaction mixture was concentrated to dryness. The crude residue absorbed onto Celite was purified by reverse phase column chromatography (0-100% ACN/aqueous ammonium bicarbonate (5 mM)). Triethylamine (14 uL, 0.1 mmol) was added to an aqueous solution of the product. Lyophilization overnight provided *tert*-butyl {2-[(2*R*)-2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)pyrrolidin-1-yl]-2-oxoethyl}carbamate as the *N,N-*diethylethanamine salt (49 mg, 87%). LCMS (AA): *m*/*z* = 904.4 (M+H).

### Step 2: (2R)-1-(Aminoacetyl)-N-{9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}pyrrolidine-2-carboxamide

To a solution of tert-butyl {2-[(2R)-2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl }carbamoyl)pyrrolidin-1-yl]-2-oxoethyl } carbamate as the *N,N-*diethylethanamine salt (37 mg, 0.033 mmol) in MeOH (1 mL) was added hydrochloric acid (4M solution in dioxane, 1 mL, 4 mmol), and the reaction mixture was stirred at rt for 1 h. The reaction mixture was then concentrated to dryness and placed under vacuum for 2 h to provide (2*R*)-1-(aminoacetyl)-N-{9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}pyrrolidine-2-carboxamide as the hydrochloride salt (37 mg, 100%). LCMS (AA): *m*/*z =* 804.2 (M+H).

### Step 3: (2R)-1-({[(2S)-2-{[(2S)-2-{[6-(2,5-Dioxo-2,5-dihydro-1H-pyrrol-1-yl)hexanoyl]amino}-3-methylbutanoyl]amino}propanoyl]amino}acetyl)-N-{9-[(2R,5R,7R,8R,10R,12aR,14R,15aS,16R)-16-hydroxy-2,10-dioxido-14-(pyrimidin-4-yloxy)-2,10-disulfanyldecahydro-5,8-ethanocyclopenta[l][1,3,6,9,11,2,10]pentaoxadiphosphacyclotetradecin-7-yl]-6-oxo-6,9-dihydro-1H-purin-2-yl}pyrrolidine-2-carboxamide, Compound 27

The title compound was prepared following the procedure described in Example 26, step 1, starting with (2*R*)-1-(aminoacetyl)-N-{9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}pyrrolidine-2-carboxamide as the hydrochloride salt (30 mg, 0.027 mmol) and (2,5-dioxopyrrolidin-1-yl) (2*S*)-2-[[(2*S*)-2-[6-(2,5-dioxopyrrol-1-yl)hexanoylamino]-3-methyl-butanoyl]amino]propanoate (24 mg, 0.04 mmol)( for synthesis see US20180015176A1). Purification by reverse phase flash column chromatography (0-100% ACN/aqueous formic acid (0.1%)) gave (2*R*)-1-({[(2*S*)-2-{[(2*S*)-2-{[6-(2,5-dioxo-2,5-dihydro-1H-pyrrol-1-yl)hexanoyl]amino}-3-methylbutanoyl]amino}propanoyl]amino}acetyl)-N-{9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}pyrrolidine-2-carboxamide (C-27, 20 mg, 64%). LCMS (AA): *m*/*z* = 1167.1 (M+H). HRMS *(m*/*z):* [M+H]⁺ calcd for C₄₅H₆₀N₁₂O₁₇P₂S₂ 1167.3189; found, 1167.3206.

### Example 27

### 4-{[(2S)-2-({(2S)-2-[(Aminoacetyl)amino]-3-methylbutanoyl}amino)propanoyl]amino}benzyl [2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate, Compound 28 (C-28)

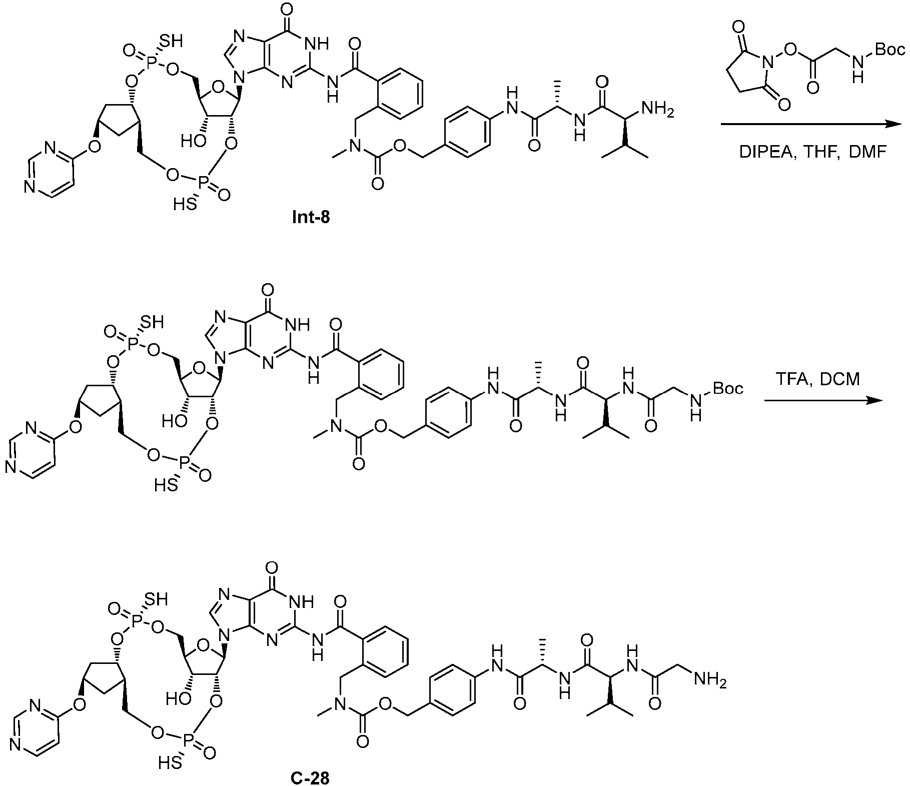

### Step 1: tert-Butyl (2-{[(2S)-1-{[(2S)-1-({4-[({[2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl](methyl)carbamoyl}oxy)methyl]phenyl}amino)-1-oxopropan-2-yl]amino}-3-methyl-1-oxobutan-2-yl]amino}-2-oxoethyl)carbamate

The title compound was prepared following the procedure described in Example 6, step 3, starting with Intermediate 8 (23 mg, 0.016 mmol) and 2,5-dioxopyrrolidin-1-yl (*tert-*butoxycarbonyl)glycinate (11 mg, 0.039 mmol) instead of *N*-succinimidyl 6-maleimidohexanoate. The reaction mixture was stirred at rt for 30 min. Purification by reverse phase flash column chromatography (0-100% ACN/aqueous triethylammonium acetate (10 mM)) gave *tert*-butyl (2-{[(2*S*)-1-{[(2*S*)-1-({4-[({[2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl](methyl)carbamoyl}oxy)methyl]phenyl}amino)-1-oxopropan-2-yl]amino}-3-methyl-1-oxobutan-2-yl]amino}-2-oxoethyl)carbamate as the *N,N-*diethylethanamine salt (13 mg, 57%). LCMS (AA): *m*/*z* = 1273.1 (M+H).

### Step 2: 4-{[(2S)-2-({(2S)-2-[(Aminoacetyl)amino]-3-methylbutanoyl}amino)propanoyl]amino }benzyl [2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate, Compound 28

The title compound was prepared following the procedure described in Example 5, step 2, starting with *tert*-butyl (2-{[(2*S*)-1-{[(2*S*)-1-({4-[({[2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl](methyl)carbamoyl}oxy)methyl]phenyl}amino)-1-oxopropan-2-yl]amino}-3-methyl-1-oxobutan-2-yl]amino}-2-oxoethyl)carbamate as the *N,N-*diethylethanamine salt (6.6 mg, 0.005 mmol) instead of *tert*-butyl [2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate. The reaction mixture was stirred at rt for 5 min to provide 4-{[(2*S*)-2-({(2*S*)-2-[(aminoacetyl)amino]-3-methylbutanoyl}amino)propanoyl]amino }benzyl [2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate as the 2,2,2-trifluoroacetate salt (C-28, 5.6 mg, 91%). LCMS (AA): *m*/*z* = 1173.3 (M+H).

### Example 28

The compounds listed in Table 20 were prepared as described in Example 27, substituting the starting material shown in Table 19 for 2,5-dioxopyrrolidin-1-yl (*tert*-butoxycarbonyl)glycinate.

**Table 19**

| Starting material | Product | Analytical data |
|---|---|---|
| 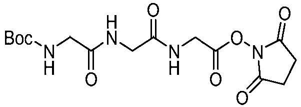 Prepared as described in US Pat. No. 6022966 | C-29 | LCMS (AA): *m*/*z* = 1287.2 (M+H) |
| 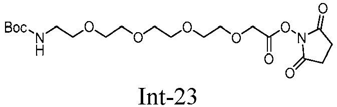 | C-30 | LCMS (AA): *m*/*z* = 1349.3 (M+H) |

**Table 20**

| Product | Structure |
|---|---|
| C-29 | 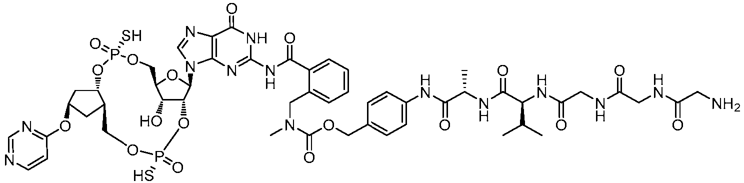 |
| C-30 | 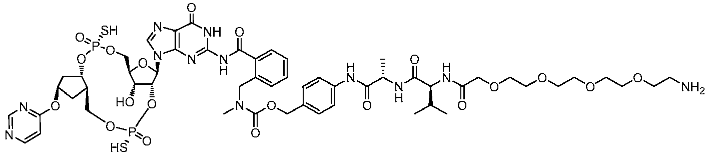 |

### Example 29

### tert-Butyl {14-[(2,5-dioxopyrrolidin-1-yl)oxy]-14-oxo-3,6,9,12-tetraoxatetradec-1-yl}carbamate (Intermediate 23)

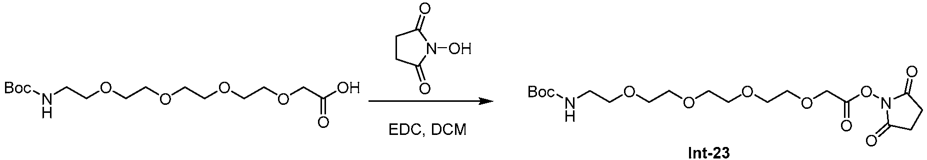

### Step 1: tert-Butyl {14-[(2,5-dioxopyrrolidin-1-yl)oxy]-14-oxo-3,6,9,12-tetraoxatetradec-1-yl}carbamate, Intermediate 23

To a solution of 2,2-dimethyl-4-oxo-3,8,11,14,17-pentaoxa-5-azanonadecan-19-oic acid (120 mg, 0.32 mmol) and 1-(3-dimethylaminopropyl)-3-ethylcarbodiimide (78 mg, 0.49 mmol) dissolved in DCM (6.4 mL) was added *N*-hydroxysuccinimide (46 mg, 0.39 mmol) at rt. The reaction mixture was allowed to stir at rt for 2.5 h. The reaction mixture was quenched with water and extracted with DCM (3x). The combined organic phases were washed with brine, dried (Na₂SO₄), filtered and evaporated to provide crude *tert*-butyl {14-[(2,5-dioxopyrrolidin-1-yl)oxy]-14-oxo-3,6,9,12-tetraoxatetradec-1-yl}carbamate (Intermediate 23, 146 mg, 100%). LCMS (AA): *m*/*z* = 447.2 (M-H).

### Example 30

### tert-Butyl N-[(1S)-2-[(2-chlorocarbonylphenyl)methyl-[(2,4-dimethoxyphenyl)methyl]amino]-1-methyl-2-oxo-ethyl]carbamate (Intermediate 24)

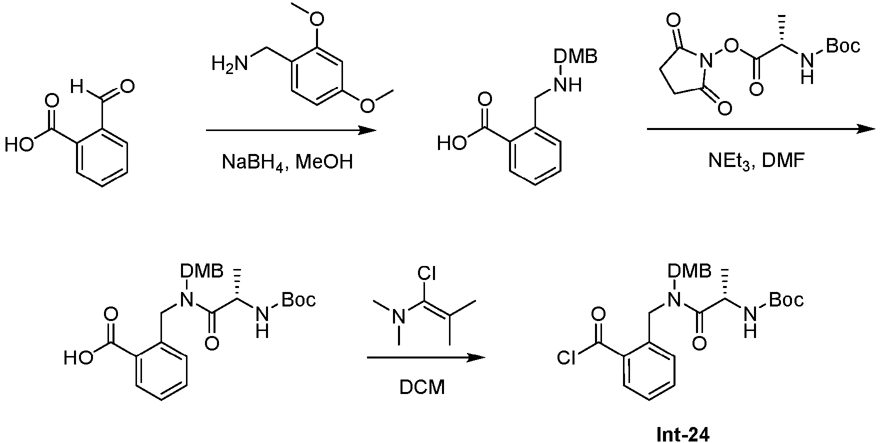

### Step 1: 2-[[(2,4-Dimethoxyphenyl)methylamino]methyl]benzoic acid

The title compound was prepared following the procedure described in Example 10, step 1, starting with 2-carboxybenzaldehyde (6.0 g, 40 mmol) and 2,4-dimethoxybenzylamine (12 mL, 80 mmol) to provide 2-[[(2,4-dimethoxyphenyl)methylamino]methyl]benzoic acid (7.6 g, 61%). LCMS (AA): *m*/*z* = 302.2 (M+H).

### Step 2: 2-[[[(2S)-2-(tert-Butoxycarbonylamino)propanoyl]-[(2,4-dimethoxyphenyl)methyl]amino]methyl]benzoic acid

To a solution of 2-[[(2,4-dimethoxyphenyl)methylamino]methyl]benzoic acid (380 mg, 1.26 mmol) and triethylamine (0.5 mL, 3.55 mmol) in DMF (6 mL) was added 2,5-dioxopyrrolidin-1-yl (*tert*-butoxycarbonyl)-*L*-alaninate (640 mg, 2.2 mmol). Water (2 mL) was added to help solubilize the reaction mixture which was allowed to stir at rt for 1 d. The reaction mixture was then concentrated to dryness and the crude residue was purified by reverse phase flash column chromatography (0-100% ACN/aqueous formic acid (0.1%)) to provide 2-[[[(2*S*)-2-(*tert*-butoxycarbonylamino)propanoyl]-[(2,4-dimethoxyphenyl)methyl]amino]methyl]benzoic acid (322 mg, 51%). LCMS (AA): *m*/*z* = 473.3 (M+H).

### Step 3: tert-Butyl N-[(15)-2-[(2-chlorocarbonylphenyl)methyl-[(2,4-dimethoxyphenyl)methyl]amino]-1-methyl-2-oxo-ethyl]carbamate, Intermediate 24

The title compound was prepared following the procedure described in Example 1, step 2, starting with 2-[[[(2*S*)-2-(*tert*-butoxycarbonylamino)propanoyl]-[(2,4-dimethoxyphenyl)methyl]amino]methyl]benzoic acid (270 mg, 0.57 mmol) instead of 2-(((*tert-*butoxycarbonyl)(methyl)amino)methyl)benzoic acid to provide crude Intermediate 24 (280 mg, 100%).

### Example 31

### 2-({[(2S)-2-{[(2S)-2-{[6-(2,5-Dioxo-2,5-dihydro-1H-pyrrol-1-yl)hexanoyl]amino}-3-methylbutanoyl]amino }propanoyl]amino }methyl)-N-{9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}benzamide, Compound 31 (C-31)

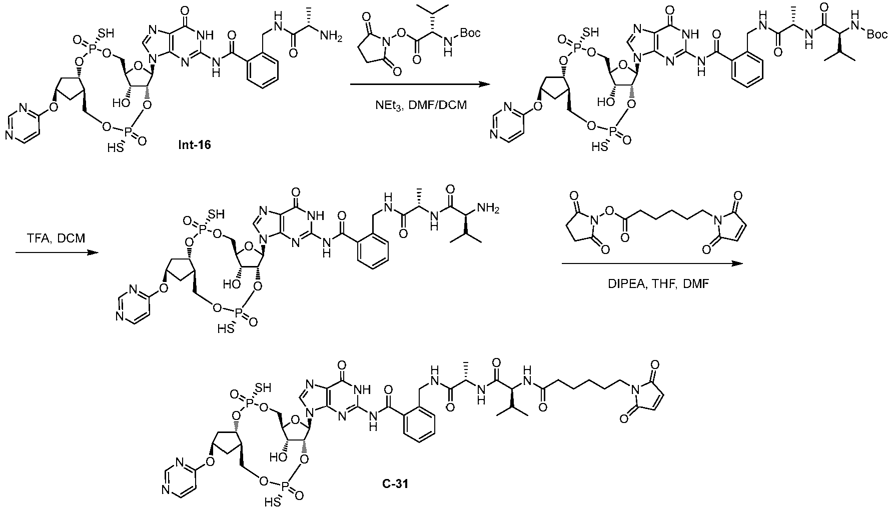

### Step 1: tert-Butyl [(2S)-1-{[(2S)-1-{[2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]amino}-1-oxopropan-2-yl]amino}-3-methyl-1-oxobutan-2-yl]carbamate

The title compound was prepared following the procedure described in Example 26, step 1, starting with Intermediate 16 (45 mg, 0.53 mmol) and 2,5-dioxopyrrolidin-1-yl (*tert-*butoxycarbonyl)-*L*-valinate (100 mg, 0.32 mmol) in place of Intermediate 15 and 2,5-dioxopyrrolidin-1-yl (*tert*-butoxycarbonyl)glycinate respectively. Purification by reverse phase flash column chromatography (0-100% ACN/aqueous ammonium acetate (10 mM)) gave *tert-*butyl [(2*S*)-1-{[(2*S*)-1-{[2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]amino}-1-oxopropan-2-yl]amino}-3-methyl-1-oxobutan-2-yl]carbamate as the ammonium salt (24 mg, 44%). LCMS (AA): *m*/*z* = 1053.0 (M+H).

### Step 2: 2-({[(2S)-2-{[(2S)-2-Amino-3-methylbutanoyl]amino}propanoyl]amino}methyl)-N-{9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}benzamide

The title compound was prepared following the procedure described in Example 5, step 2, starting with *tert*-butyl [(2*S*)-1-{[(2*S*)-1-{[2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]amino}-1-oxopropan-2-yl]amino}-3-methyl-1-oxobutan-2-yl]carbamate as the ammonium salt (24 mg, 0.023 mmol) instead of *tert*-butyl [2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate to provide 2-({[(2*S*)-2-{[(2*S*)-2-amino-3-methylbutanoyl]amino}propanoyl]amino}methyl)-N-{9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}benzamide as the 2,2,2-trifluoroacetate salt (24 mg, 97%). LCMS (AA): *m*/*z* = 953.1 (M+H).

### Step 3: 2-({[(2S)-2-{[(2S)-2-{[6-(2,5-Dioxo-2,5-dihydro-1H-pyrrol-1-yl)hexanoyl]amino}-3-methylbutanoyl]amino }propanoyl]amino }methyl)-N-{9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}benzamide, Compound 31

The title compound was prepared following the procedure described in Example 6, step 3, starting with 2-({[(2*S*)-2-{[(2*S*)-2-amino-3-methylbutanoyl]amino}propanoyl]amino}methyl)-N-{9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}benzamide as the 2,2,2-trifluoroacetate salt (24 mg, 0.022 mmol) instead of Intermediate 8 to provide 2-({[(2*S*)-2-{[(2*S*)-2-{[6-(2,5-dioxo-2,5-dihydro-1H-pyrrol-1-yl)hexanoyl]amino}-3-methylbutanoyl]amino}propanoyl]amino}methyl)-N-{9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}benzamide as the ammonium salt (C-31, 6.2 mg, 23%). LCMS (AA): *m*/*z* = 1146.1 (M+H). HRMS (*m*/*z*): [M+H]⁺ calcd for C₄₆H₅₇N₁₁O₁₆P₂S₂ 1146.2974; found, 1146.2998.

### Example 32

### 4-{[(2S)-2-{[(2S)-2-{[6-(11,12-Didehydrodibenzo[b,f]azocin-5(6H)-yl)-6-oxohexanoyl]amino}-3-methylbutanoyl]amino}propanoyl]amino}benzyl 4-nitrophenyl carbonate (Intermediate 25)

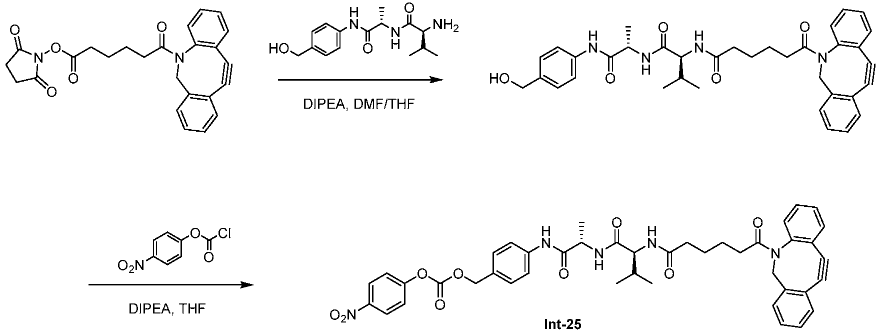

### Step 1: 6-(11,12-Didehydrodibenzo[b,f]azocin-5(6H)-yl)-N-[(2S)-1-{[(2S)-1-{[4-(hydroxymethyl)phenyl]amino}-1-oxopropan-2-yl]amino}-3-methyl-1-oxobutan-2-yl]-6-oxohexanamide

To a solution of (2*S*)-2-amino-N-[(1*S*)-2-[4-(hydroxymethyl)anilino]-1-methyl-2-oxoethyl]-3-methyl-butanamide (240 mg, 0.82 mmol) dissolved in DMF (2 mL) and THF (5.5 mL) was added *N,N-*diisopropylethylamine (0.39 mL, 2.24 mmol). The reaction mixture was cooled to 0 °C and 1-{[6-(11,12-didehydrodibenzo[b,f]azocin-5(6H)-yl)-6-oxohexanoyl]oxy}pyrrolidine-2,5-dione (320 mg, 0.75 mmol) was added. The reaction mixture was warmed to rt and allowed to stir for 30 min. The reaction mixture was diluted with water and extracted with EtOAc (3x). The combined organic phases were washed with an aqueous saturated solution of sodium bicarbonate and brine, dried (Na₂SO₄), filtered and evaporated to dryness. The crude residue was purified by silica gel chromatography (50-100% EtOAc/DCM) to provide 6-(11,12-didehydrodibenzo[b,f]azocin-5(6H)-yl)-N-[(2*S*)-1-{[(2*S*)-1-{[4-(hydroxymethyl)phenyl]amino}-1-oxopropan-2-yl]amino}-3-methyl-1-oxobutan-2-yl]-6-oxohexanamide (347 mg, 76%). LCMS (AA): *m*/*z* = 609.4 (M+H).

### Step 2: 4-{[(2S)-2-{[(2S)-2-{[6-(11,12-Didehydrodibenzo[b,f]azocin-5(6H)-yl)-6-oxohexanoyl]amino}-3-methylbutanoyl]amino}propanoyl]amino}benzyl 4-nitrophenyl carbonate, Intermediate 25

To a solution of 6-(11,12-didehydrodibenzo[b,f]azocin-5(6H)-yl)-N-[(2*S*)-1-{[(2*S*)-1-{[4-(hydroxymethyl)phenyl]amino}-1-oxopropan-2-yl]amino}-3-methyl-1-oxobutan-2-yl]-6-oxohexanamide (257 mg, 0.42 mmol) in THF (4.2 mL) and *N,N*-diisopropylethylamine (0.37 mL, 2.1 mmol) cooled to 0 °C was added 4-nitrophenyl chloroformate (362 mg, 1.69 mmol) in one portion. The reaction mixture was warmed to rt and stirred for 3 h. The reaction mixture was diluted with water and extracted with DCM (3x). The combined organic phases were washed with an aqueous saturated solution of sodium bicarbonate and brine, dried (Na₂SO₄), filtered and evaporated to dryness. The crude residue was purified by silica gel chromatography (0-100% EtOAc/DCM) to provide 4-{[(2*S*)-2-{[(2*S*)-2-{[6-(11,12-didehydrodibenzo[b,f]azocin-5(6H)-yl)-6-oxohexanoyl]amino}-3-methylbutanoyl]amino}propanoyl]amino}benzyl 4-nitrophenyl carbonate (Intermediate 25, 137 mg, 42%). LCMS (AA): *m*/*z* = 774.4 (M+H).

### Example 33

The compound listed in Table 21 (Intermediate 26) was prepared as described in Example 32, substituting the starting material shown in the table for 1-{[6-(11,12-didehydrodibenzo[b,f]azocin-5(6H)-yl)-6-oxohexanoyl]oxy}pyrrolidine-2,5-dione.

**Table 21**

| Starting material | Product | Analytical data |
|---|---|---|
| 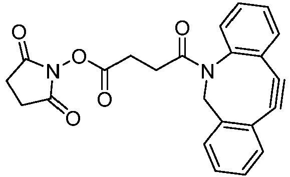 | 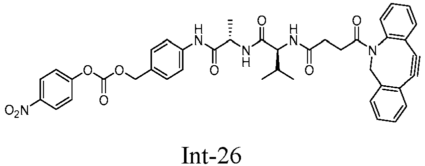 | LCMS (AA): *m*/*z* = 746.3 (M+H) |

### Example 34

### (2S)-2-{[4-(11,12-Didehydrodibenzo[b,f]azocin-5(6H)-yl)-4-oxobutanoyl]amino}-4-[(2,5-dioxopyrrolidin-1-yl)oxy]-N-(2,5,8,11,14,17,20-heptaoxadocosan-22-yl)-4-oxobutanamide (Intermediate 27) and (25S)-25-{[4-(11,12-didehydrodibenzo[b,f]azocin-5(6H)-yl)-4-oxobutanoyl]amino}-24-oxo-2,5,8,11,14,17,20-heptaoxa-23-azaheptacosan-27-oic acid (Intermediate 27A)

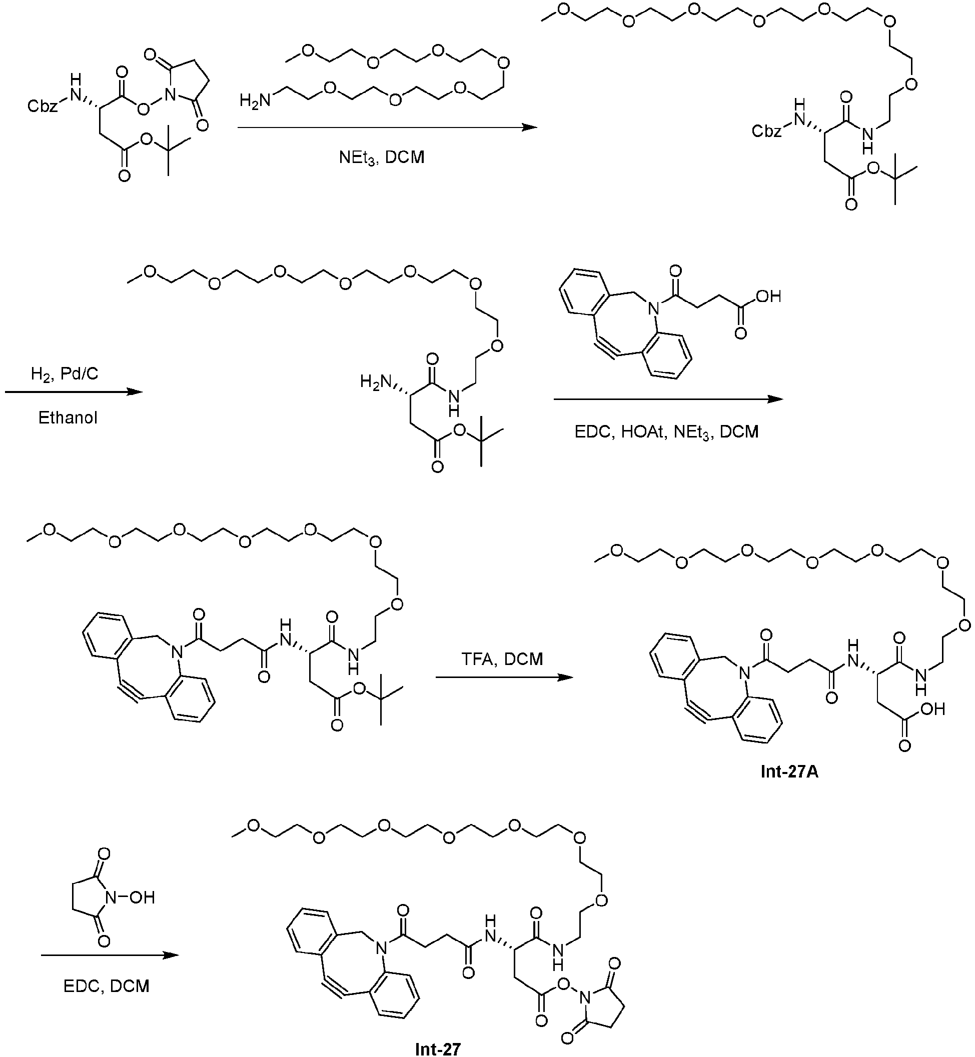

### Step 1: tert-Butyl (25S)-25-{[(benzyloxy)carbonyl]amino}-24-oxo-2,5,8,11,14,17,20-heptaoxa-23-azaheptacosan-27-oate

The title compound was prepared following the procedure described in Example 26, step 1, starting with *tert*-butyl (3*S*)-3-{[(benzyloxy)carbonyl]amino}-4-[(2,5-dioxopyrrolidin-1-yl)oxy]-4-oxobutanoate (2.2 g, 5.3 mmol) and 2,5,8,11,14,17,20-heptaoxadocosan-22-amine (2.0 g, 5.9 mmol) in place of Intermediate 15 and 2,5-dioxopyrrolidin-1-yl (*tert-*butoxycarbonyl)glycinate respectively. Purification by silica gel chromatography (0-10% MeOH/DCM) gave *tert*-butyl (25*S*)-25-{[(benzyloxy)carbonyl]amino}-24-oxo-2,5,8,11,14,17,20-heptaoxa-23-azaheptacosan-27-oate (3.8 g, 100%).

### Step 2: tert-Butyl (25S)-25-amino-24-oxo-2,5,8,11,14,17,20-heptaoxa-23-azaheptacosan-27-oate

*tert-*Butyl (25*S*)-25-{[(benzyloxy)carbonyl]amino}-24-oxo-2,5,8,11,14,17,20-heptaoxa-23-azaheptacosan-27-oate (3.8 g, 5.9 mmol) was dissolved in ethanol (100 mL). Palladium (10% on carbon, 1.38 g, 1.17 mmol) was added and the mixture was stirred under hydrogen (15 psi) for 3 h. The reaction mixture was filtered, washed with methanol and the filtrate was evaporated to dryness to provide *tert*-butyl (25*S*)-25-amino-24-oxo-2,5,8,11,14,17,20-heptaoxa-23-azaheptacosan-27-oate (2.56 g, 85%).

### Step 3: tert-Butyl (25S)-25-{[4-(11,12-didehydrodibenzo[b,f]azocin-5(6H)-yl)-4-oxobutanoyl]amino}-24-oxo-2,5,8,11,14,17,20-heptaoxa-23-azaheptacosan-27-oate

To a solution of *tert*-butyl (25*S*)-25-amino-24-oxo-2,5,8,11,14,17,20-heptaoxa-23-azaheptacosan-27-oate (3.32 g, 6.5 mmol), 4-(11,12-didehydrodibenzo[b,f]azocin-5(6H)-yl)-4-oxobutanoic acid (1.5 g, 4.91 mmol) and 1-hydroxy-7-azabenzotriazole (884 mg, 6.57 mmol) in DCM (50 mL) was added 1-(3-dimethylaminopropyl)-3-ethylcarbodiimide (1.25 g, 6.56 mmol) and triethylamine (1.26 mL, 9.1 mmol) at 0 °C. The reaction mixture was warmed to rt and stirred for 16 h. The reaction was diluted with EtOAc and potassium carbonate (20 mL, 4.0 M in water) was added. The mixture was stirred for 15 min and the organic phase was isolated, washed with an aqueous saturated solution of sodium bicarbonate ,brine, dried (Na₂SO₄), filtered and evaporated to dryness. The crude residue was purified by silica gel chromatography (0-10% MeOH/DCM) to provide *tert*-butyl (25*S*)-25-{[4-(11,12-didehydrodibenzo[b,f]azocin-5(6H)-yl)-4-oxobutanoyl]amino}-24-oxo-2,5,8,11,14,17,20-heptaoxa-23-azaheptacosan-27-oate (2.27 g, 44%).

### Step 4: (25S)-25-{[4-(11,12-Didehydrodibenzo[b,f]azocin-5(6H)-yl)-4-oxobutanoyl]amino}-24-oxo-2,5,8,11,14,17,20-heptaoxa-23-azaheptacosan-27-oic acid

The title compound was prepared following the procedure described in Example 5, step 2, starting with *tert*-butyl (25*S*)-25-{[4-(11,12-didehydrodibenzo[b,f]azocin-5(6H)-yl)-4-oxobutanoyl]amino}-24-oxo-2,5,8,11,14,17,20-heptaoxa-23-azaheptacosan-27-oate (278 mg, 0.35 mmol) instead of *tert*-butyl [2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate. Purification by silica gel chromatography (0-20% MeOH/DCM) gave (25S)-25-{[4-(11,12-didehydrodibenzo[b,f]azocin-5(6H)-yl)-4-oxobutanoyl]amino}-24-oxo-2,5,8,11,14,17,20-heptaoxa-23-azaheptacosan-27-oic acid (Intermediate 27A, 180 mg, 70%). LCMS (AA): *m*/*z* = 742.4 (M+H).

### Step 5: (2S)-2-{[4-(11,12-Didehydrodibenzo[b,f]azocin-5(6H)-yl)-4-oxobutanoyl]amino}-4-[(2,5-dioxopyrrolidin-1-yl)oxy]-N-(2,5,8,11,14,17,20-heptaoxadocosan-22-yl)-4-oxobutanamide, Intermediate 27

The title compound was prepared following the procedure described in Example 29, starting with (25*S*)-25-{[4-(11,12-didehydrodibenzo[b,f]azocin-5(6H)-yl)-4-oxobutanoyl]amino}-24-oxo-2,5,8,11,14,17,20-heptaoxa-23-azaheptacosan-27-oic acid (60 mg, 0.08 mmol) in place of 2,2-dimethyl-4-oxo-3,8,11,14,17-pentaoxa-5-azanonadecan-19-oic acid. The reaction mixture was stirred for 30 min at rt to provide (2*S*)-2-{[4-(11,12-Didehydrodibenzo[b,f]azocin-5(6H)-yl)-4-oxobutanoyl]amino}-4-[(2,5-dioxopyrrolidin-1-yl)oxy]-N-(2,5,8,11,14,17,20-heptaoxadocosan-22-yl)-4-oxobutanamide (Intermediate 27, 68 mg, 100%). LCMS (AA): *m*/*z* = 839.4 (M+H).

### Example 35

The compounds listed in Table 23 were prepared as described in Example 6, step 3, substituting the starting material shown in Table 22 for *N*-succinimidyl 6-maleimidohexanoate.

**Table 22**

| Starting material | Product | Analytical data |
|---|---|---|
| 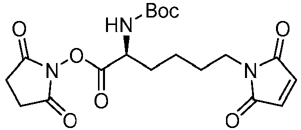 | Int-28 * | LCMS (AA): *m*/*z* = 1424.0 (M+H) |
| 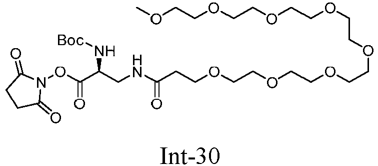 | Int-29 | LCMS (AA): *m*/*z* = 1696.0 (M+H) |
| 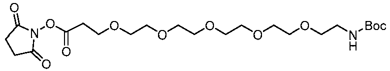 | Int-41 | LCMS (AA): *m*/*z* = 1504.7 (M-H) |

| | | |
|---|---|---|
| * Used triethylamine as base instead of *N,N-*diisopropylethylamine. | | |

**Table 23**

| Product | Structure |
|---|---|
| Int-28* | 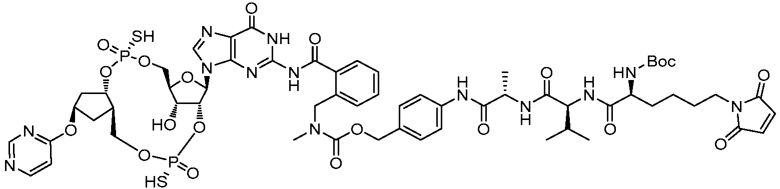 |
| Int-29 | 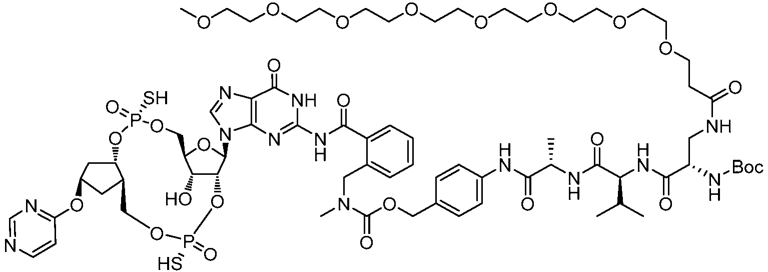 |
| Int-41 | 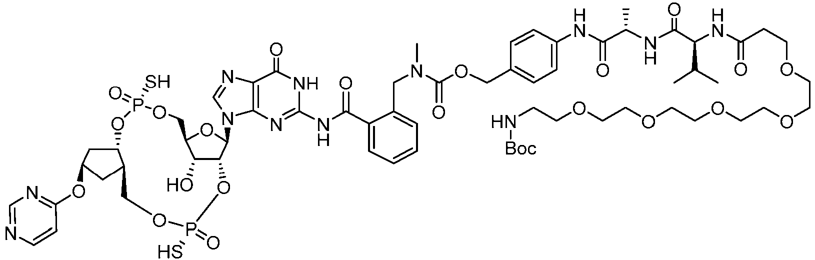 |

### Example 36

### tert-Butyl {(29S)-30-[(2,5-dioxopyrrolidin-1-yl)oxy]-26,30-dioxo-2,5,8,11,14,17,20,23-octaoxa-27-azatriacontan-29-yl}carbamate (Intermediate 30)

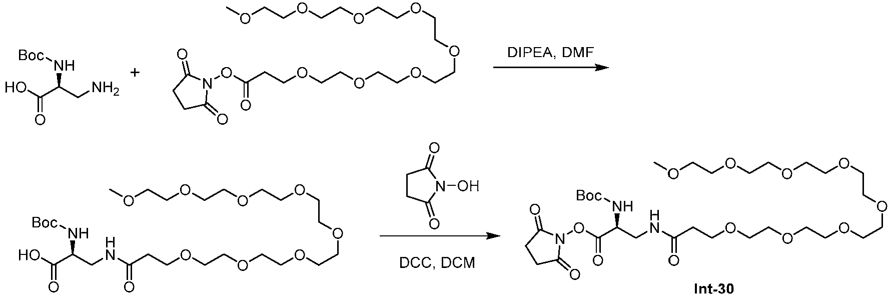

### Step 1: (29S)-29-[(tert-Butoxycarbonyl)amino]-26-oxo-2,5,8,11,14,17,20,23-octaoxa-27-azatriacontan-30-oic acid

The title compound was prepared following the procedure described in Example 21, step 1, starting with (*S*)-3-amino-2-((tert-butoxycarbonyl)amino)propanoic acid (238 mg, 1.17 mmol) and 1-[(26-oxo-2,5,8,11,14,17,20,23-octaoxahexacosan-26-yl)oxy]pyrrolidine-2,5-dione (300 mg, 0.58 mmol) in place of (S)-2-amino-6-(2,5-dioxo-2,5-dihydro-1H-pyrrol-1-yl)hexanoic acid hydrochloride and 1-[(17-oxo-2,5,8,11,14-pentaoxaheptadecan-17-yl)oxy]pyrrolidine-2,5-dione using DMF as the solvent and stirring at rt for 2 h. Purification by reverse phase flash column chromatography (0-100% ACN/aqueous triethylammonium acetate (10 mM)) gave (29*S*)-29-[(*tert*-butoxycarbonyl)amino]-26-oxo-2,5,8,11,14,17,20,23-octaoxa-27-azatriacontan-30-oic acid (344 mg, 99%). LCMS (AA): *m*/*z* = 599.4 (M+H).

### Step 2: tert-Butyl {(29S)-30-[(2,5-dioxopyrrolidin-1-yl)oxy]-26,30-dioxo-2,5,8,11,14,17,20,23-octaoxa-27-azatriacontan-29-yl}carbamate, Intermediate 30

The title compound was prepared following the procedure described in Example 21, step 2, starting with (29*S*)-29-[(*tert*-butoxycarbonyl)amino]-26-oxo-2,5,8,11,14,17,20,23-octaoxa-27-azatriacontan-30-oic acid (100 mg, 0.166 mmol) instead of (19*S*)-19-[4-(2,5-dioxo-2,5-dihydro-1H-pyrrol-1-yl)butyl]-17-oxo-2,5,8,11,14-pentaoxa-18-azaicosan-20-oic acid using DCM as the solvent and stirring at rt for 1 h. The reaction mixture was filtered and concentrated to provide crude *tert*-butyl {(29*S*)-30-[(2,5-dioxopyrrolidin-1-yl)oxy]-26,30-dioxo-2,5,8,11,14,17,20,23-octaoxa-27-azatriacontan-29-yl}carbamate (Intermediate 30, 116 mg, 100%).

### Example 37

### [4-[[(2S)-2-[[(2S)-2-(tert-Butoxycarbonylamino)-3-methyl-butanoyl]amino]propanoyl]amino]phenylmethyl N-[[2-[[9-[(2R,3R,5S)-5-[[bis(4-methoxyphenyl)-phenyl-methoxy]methyl]-3-hydroxy-tetrahydrofuran-2-yl]-6-oxo-1H-purin-2-yl]carbamoyl]phenyl]methyl]-N-methyl-carbamate (Intermediate 32)

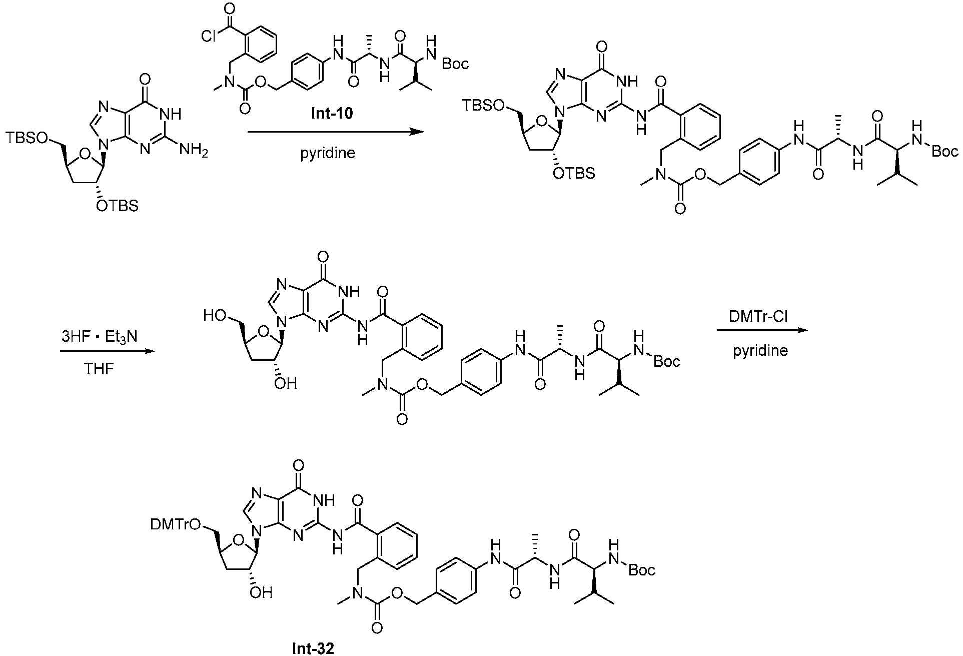

### Step 1: [4-[[(2S)-2-[[(2S)-2-(tert-Butoxycarbonylamino)-3-methyl-butanoyl]amino]propanoyl]amino]phenylmethyl N-[[2-[[9-[(2R,3R,5S)-3-[tert-butyl(dimethyl)silyl]oxy-5-[[tert-butyl(dimethyl)silyl]oxymethyl]tetrahydrofuran-2-yl]-6-oxo-1H-purin-2-yl]carbamoyl]phenyl]methyl]-N-methyl-carbamate

2-Amino-9-[(2*R*,3*R*,5*S*)-3-[*tert*-butyl(dimethyl)silyl]oxy-5-[[*tert-*butyl(dimethyl)silyl]oxymethyl]tetrahydrofuran-2-yl]-1H-purin-6-one (1.62 g, 3.27 mmol) (for synthesis see Prakash, T.P. J. Med. Chem. 2005, 48, 1199-1210) was dissolved in dry pyridine and concentrated to dryness (3 x 3mL) and then placed under vacuum for 15 min. The residue was dissolved in pyridine (43 mL) under an argon atmosphere and a solution of Intermediate 10 (5.91 g, 9.80 mmol) in pyridine (32 mL) was added. The reaction mixture was allowed to stir at rt for 16 h. MeOH (20 mL) was added and the reaction mixture was concentrated to dryness. MeOH (100 mL) and ammonium hydroxide (28-30% solution in water, 50 mL) were added and the resulting mixture was allowed to stir for 60 min. The reaction mixture was concentrated to dryness and the crude residue was adsorbed onto Celite and purified by silica gel chromatography (0-100% EtOAc/hexanes) to provide [4-[[(2*S*)-2-[[(2*S*)-2-(*tert-*butoxycarbonylamino)-3-methyl-butanoyl]amino]propanoyl]amino]phenyl]methyl N-[[2-[[9-[(2*R*,3*R*,5*S*)-3-[*tert*-butyl(dimethyl)silyl]oxy-5-[[*tert-*butyl(dimethyl)silyl]oxymethyl]tetrahydrofuran-2-yl]-6-oxo-1H-purin-2-yl]carbamoyl]phenyl]methyl]-N-methyl-carbamate (2.17 g, 63%). LCMS (AA): *m*/*z* = 1062.0 (M+H).

### Step 2: [4-[[(2S)-2-[[(2S)-2-(tert-Butoxycarbonylamino)-3-methyl-butanoyl]amino]propanoyl]amino]phenyl]methyl N-[[2-[[9-[(2R,3R,5S)-3-hydroxy-5-(hydroxymethyl)tetrahydrofuran-2-yl]-6-oxo-1H-purin-2-yl]carbamoyl]phenyl]methyl]-N-methyl-carbamate

To a polypropylene tube was added [4-[[(2*S*)-2-[[(2*S*)-2-(*tert*-butoxycarbonylamino)-3-methyl-butanoyl]amino]propanoyl]amino]phenyl]methyl N-[[2-[[9-[(2*R*,3*R*,5*S*)-3-[*tert-*butyl(dimethyl)silyl]oxy-5-[[*tert*-butyl(dimethyl)silyl]oxymethyl]tetrahydrofuran-2-yl]-6-oxo-1H-purin-2-yl]carbamoyl]phenyl]methyl]-N-methyl-carbamate (2.17 g, 2.04 mmol) dissolved in THF (27 mL). Triethylamine (2.26 mL, 16.1 mmol) was added followed by triethylamine trihydrofluoride (1.19 mL, 7.33 mmol) and the reaction mixture was stirred at rt for 16 h. The reaction mixture was quenched slowly by the addition of an aqueous saturated solution of sodium bicarbonate and then diluted with EtOAc. The organic phase was separated and washed with brine, dried (Na₂SO₄), filtered and evaporated to dryness. The crude residue was purified by silica gel chromatography (0-20% MeOH/DCM) to provide [4-[[(2*S*)-2-[[(2*S*)-2-(*tert-*butoxycarbonylamino)-3-methyl-butanoyl]amino]propanoyl]amino]phenyl]methyl N-[[2-[[9-[(2*R*,3*R*,5*S*)-3-hydroxy-5-(hydroxymethyl)tetrahydrofuran-2-yl]-6-oxo-1H-purin-2-yl]carbamoyl]phenyl]methyl]-N-methyl-carbamate (1.18 g, 69%). LCMS (AA): *m*/*z* = 834.4 (M+H).

### Step 3: [4-[[(2S)-2-[[(2S)-2-(tert-Butoxycarbonylamino)-3-methyl-butanoyl]amino]propanoyl]amino]phenylmethyl N-[[2-[[9-[(2R,3R,5S)-5-[[bis(4-methoxyphenyl)-phenyl-methoxy]methyl]-3-hydroxy-tetrahydrofuran-2-yl]-6-oxo-1H-purin-2-yl]carbamoyl]phenyl]methyl]-N-methyl-carbamate, Intermediate 32

[4-[[(2*S*)-2-[[(2*S*)-2-(*tert*-butoxycarbonylamino)-3-methyl-butanoyl]amino]propanoyl]amino]phenyl]methyl N-[[2-[[9-[(2*R*,3*R*,5*S*)-3-hydroxy-5-(hydroxymethyl)tetrahydrofuran-2-yl]-6-oxo-1H-purin-2-yl]carbamoyl]phenyl]methyl]-N-methyl-carbamate (1.13 g, 1.36 mmol) was dissolved in dry pyridine and concentrated to dryness (3 x 15 mL). The residue was dissolved in pyridine (13.3 mL) and 4,4'-dimethoxytrityl chloride (650 mg, 1.9 mmol) was added followed by 4-dimethylaminopyridine (8 mg, 0.068 mmol). The reaction mixture was allowed to stir at rt for 2 h. The reaction mixture was concentrated to dryness and adsorbed onto Celite and purified by silica gel chromatography (0-5% MeOH/DCM, with 0.5% NEt₃) to provide [4-[[(2*S*)-2-[[(2*S*)-2-(*tert*-butoxycarbonylamino)-3-methyl-butanoyl]amino]propanoyl]amino]phenyl]methyl N-[[2-[[9-[(2*R*,3*R*,5*S*)-5-[[bis(4-methoxyphenyl)-phenyl-methoxy]methyl]-3-hydroxy-tetrahydrofuran-2-yl]-6-oxo-1H-purin-2-yl]carbamoyl]phenyl]methyl]-N-methyl-carbamate (Intermediate 32, 1.12 g, 73%). LCMS (AA): *m*/*z* = 1136.5 (M+H).

### Example 38

### 4-{[(2S)-2-({(2S)-2-[(tert-Butoxycarbonyl)amino]-3-methylbutanoyl }amino)propanoyl]amino }benzyl [2-({9-[(2S,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-6-oxo-6,9-dihydro-1H-purin-2-yl}carbamoyl)benzyl]methylcarbamate or 4-{[(2S)-2-({(2S)-2-[(tert-butoxycarbonyl)amino]-3-methylbutanoyl }amino)propanoyl]amino }benzyl [2-({9-[(2R,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-6-oxo-6,9-dihydro-1H-purin-2-yl}carbamoyl)benzyl]methylcarbamate (Intermediate 33)

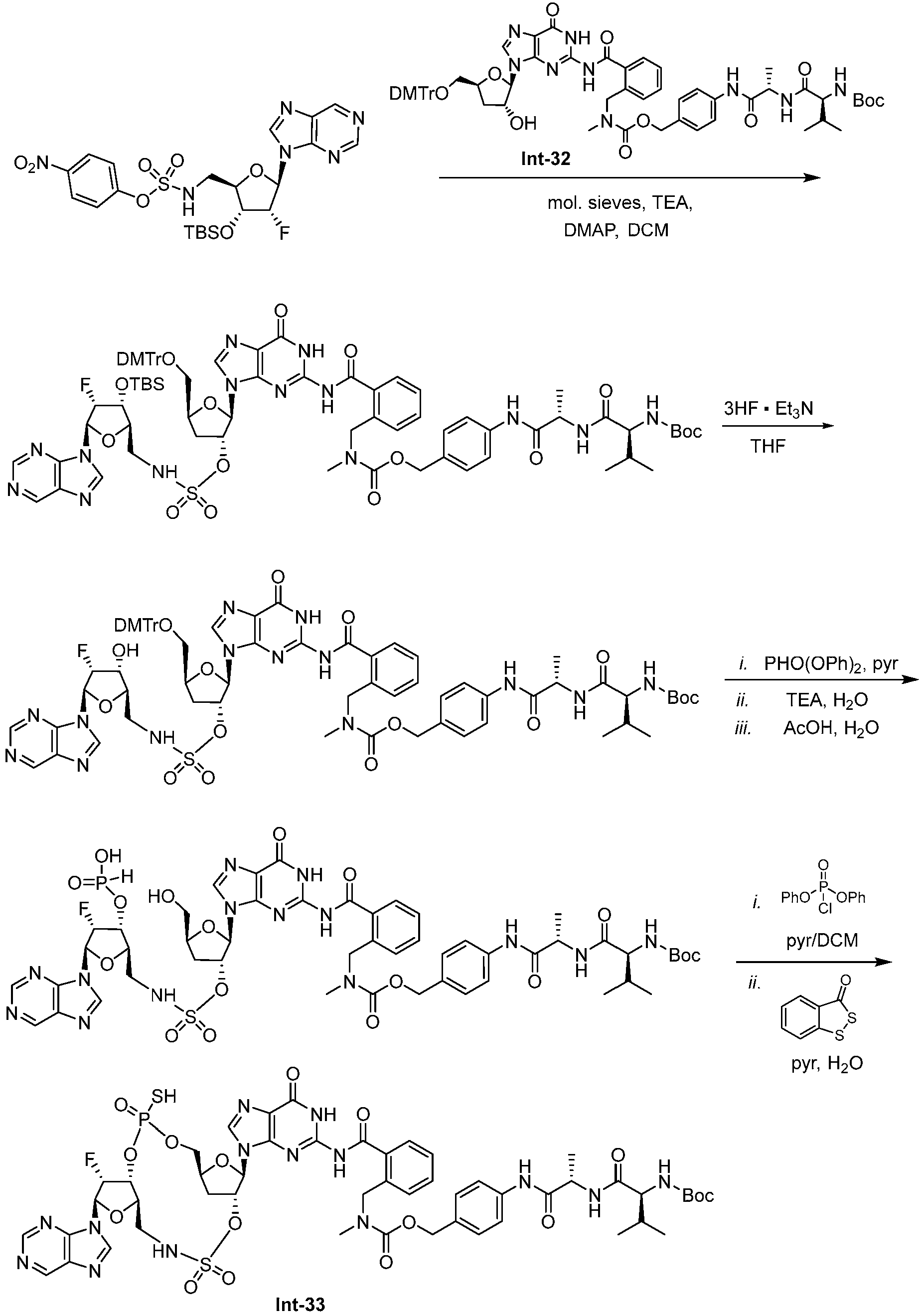

### Step 1: [4-[[(2S)-2-[[(2S)-2-(tert-Butoxycarbonylamino)-3-methyl-butanoyl]amino]propanoyl]amino]phenyl]methyl N-[[2-[[9-[(2R,3R,5S)-5-[[bis(4-methoxyphenyl)-phenyl-methoxy]methyl]-3-[[(2R,3R,4R,5R)-3-[tert-butyl(dimethyl)silyl]oxy-4-fluoro-5-purin-9-yl-tetrahydrofuran-2-yl]methylsulfamoyloxy]tetrahydrofuran-2-yl]-6-oxo-1H-purin-2-yl]carbamoyl]phenyl]methyl]-N-methyl-carbamate

A mixture of [4-[[(2*S*)-2-[[(2*S*)-2-(*tert*-butoxycarbonylamino)-3-methyl-butanoyl]amino]propanoyl]amino]phenyl]methyl N-[[2-[[9-[(2*R*,3*R*,5*S*)-5-[[bis(4-methoxyphenyl)-phenyl-methoxy]methyl]-3-hydroxy-tetrahydrofuran-2-yl]-6-oxo-1H-purin-2-yl]carbamoyl]phenyl]methyl]-N-methyl-carbamate (Intermediate 32, 1.1 g, 0.97 mmol), (4-nitrophenyl) N-[[(2*R*,3*R*,4*R*,5*R*)-3-[*tert*-butyl(dimethyl)silyl]oxy-4-fluoro-5-purin-9-yl-tetrahydrofuran-2-yl]methyl]sulfamate (667 mg, 1.17 mmol)(for synthesis see PCT Application No. PCT/IB2019/052364), 4-dimethylaminopyridine (122 mg, 0.97 mmol) and 4Å molecular sieves (1.74 g) in dry DCM (13.6 mL) was stirred under argon at rt for 10 min. Triethylamine (0.60 mL, 4.3 mmol) was then added and the reaction mixture was allowed to stir at 40 °C for 16 h. The reaction mixture was filtered and concentrated, and the resulting residue was subjected to purification by reverse phase column chromatography (10-100% ACN/aqueous ammonium bicarbonate (5 mM)) to provide [4-[[(2*S*)-2-[[(2*S*)-2-(*tert*-butoxycarbonylamino)-3-methyl-butanoyl]amino]propanoyl]amino]phenyl]methyl N-[[2-[[9-[(2*R*,3*R*,5*S*)-5-[[bis(4-methoxyphenyl)-phenyl-methoxy]methyl]-3-[[(2*R*,3*R*,4*R*,5*R*)-3-[*tert*-butyl(dimethyl)silyl]oxy-4-fluoro-5-purin-9-yl-tetrahydrofuran-2-yl]methylsulfamoyloxy]tetrahydrofuran-2-yl]-6-oxo-1H-purin-2-yl]carbamoyl]phenyl]methyl]-N-methyl-carbamate (0.94 g, 62%). LCMS (AA): *m*/*z* = 1565.0 (M+H).

### Step 2: [4-[[(2S)-2-[[(2S)-2-(tert-Butoxycarbonylamino)-3-methyl-butanoyl]amino]propanoyl]amino]phenyl]methyl N-[[2-[[9-[(2R,3R,5S)-5-[[bis(4-methoxyphenyl)-phenyl-methoxy]methyl]-3-[[(2R,3R,4R,5R)-4-fluoro-3-hydroxy-5-purin-9-yl-tetrahydrofuran-2-yl]methylsulfamoyloxy]tetrahydrofuran-2-yl]-6-oxo-1H-purin-2-yl]carbamoyl]phenyl]methyl]-N-methyl-carbamate

To a solution of [4-[[(2*S*)-2-[[(2*S*)-2-(*tert*-butoxycarbonylamino)-3-methyl-butanoyl]amino]propanoyl]amino]phenyl]methyl N-[[2-[[9-[(2*R*,3*R*,5*S*)-5-[[bis(4-methoxyphenyl)-phenyl-methoxy]methyl]-3-[[(2*R*,3*R*,4*R*,5*R*)-3-[*tert*-butyl(dimethyl)silyl]oxy-4-fluoro-5-purin-9-yl-tetrahydrofuran-2-yl]methylsulfamoyloxy]tetrahydrofuran-2-yl]-6-oxo-1H-purin-2-yl]carbamoyl]phenyl]methyl]-N-methyl-carbamate (939 mg, 0.60 mmol) in THF (12 mL) in a polypropylene tube was added triethylamine trihydrofluoride (0.52 mL, 3.17 mmol) and triethylamine (0.98 mL, 6.95 mmol). The reaction solution was stirred at room temperature overnight and then diluted with dichloromethane and washed successively with saturated aqueous NaHCO₃ and brine. The organic phase was dried over anhydrous MgSO₄, filtered and concentrated. The residue obtained was purified by reverse phase column chromatography (10-100% ACN/aqueous ammonium bicarbonate (5 mM)) to provide [4-[[(2*S*)-2-[[(2*S*)-2-(*tert-*butoxycarbonylamino)-3-methyl-butanoyl]amino]propanoyl]amino]phenyl]methyl N-[[2-[[9-[(2*R*,3*R*,5*S*)-5-[[bis(4-methoxyphenyl)-phenyl-methoxy]methyl]-3-[[(2*R*,3*R*,4*R*,5*R*)-4-fluoro-3-hydroxy-5-purin-9-yl-tetrahydrofuran-2-yl]methylsulfamoyloxy]tetrahydrofuran-2-yl]-6-oxo-1H-purin-2-yl]carbamoyl]phenyl]methyl]-N-methyl-carbamate (795 mg, 89%). LCMS (AA): *m*/*z* = 1452.2 (M+H).

### Step 3: [(2R,3R,4R,5R)-2-[[[(2R,3R,5S)-2-[2-[[2-[[[4-[[(2S)-2-[[(2S)-2-(tert-Butoxycarbonylamino)-3-methyl-butanoyl]amino]propanoyl]amino]phenyl]methoxycarbonylmethyl-amino]methyl]benzoyl]amino]-6-oxo-1H-purin-9-yl]-5-(hydroxymethyl)tetrahydrofuran-3-yl]oxysulfonylamino]methyl]-4-fluoro-5-purin-9-yl-tetrahydrofuran-3-yl]oxyphosphinic acid

Diphenyl phosphite (0.225 mL, 1.17 mmol) was added to a 0 °C solution of [4-[[(2*S*)-2-[[(2*S*)-2-(*tert*-butoxycarbonylamino)-3-methyl-butanoyl]amino]propanoyl]amino]phenyl]methyl N-[[2-[[9-[(2*R*,3*R*,5*S*)-5-[[bis(4-methoxyphenyl)-phenyl-methoxy]methyl]-3-[[(2*R*,3*R*,4*R*,5*R*)-4-fluoro-3-hydroxy-5-purin-9-yl-tetrahydrofuran-2-yl]methylsulfamoyloxy]tetrahydrofuran-2-yl]-6-oxo-1H-purin-2-yl]carbamoyl]phenyl]methyl]-N-methyl-carbamate (792 mg, 0.55 mmol) in pyridine (2 mL). Upon complete addition, the reaction mixture was allowed to stir at rt for 1 h. Triethylamine (0.62 mL, 4.37 mmol) and water (0.51 mL, 28.3 mmol) were then added and the reaction mixture was stirred an additional 60 minutes at rt and then concentrated to dryness. The residue was then dissolved in acetic acid (1.25 mL, 21.8 mmol) and water (0.33 mL, 18.6 mmol) and allowed to stir at rt for 1 h. The reaction mixture was quenched by the addition of triethylsilane (4.3 mL, 26.2 mmol) and stirred at rt for 1 h. The reaction mixture was then concentrated to dryness and the residue purified by reverse phase column chromatography (10-100% ACN/aqueous ammonium bicarbonate (5 mM)) to provide [(2*R*,3*R*,4*R*,5*R*)-2-[[[(2*R*,3*R*,5*S*)-2-[2-[[2-[[[4-[[(2*S*)-2-[[(2*S*)-2-(*tert*-butoxycarbonylamino)-3-methyl-butanoyl]amino]propanoyl]amino]phenyl]methoxycarbonyl-methyl-amino]methyl]benzoyl]amino]-6-oxo-1H-purin-9-yl]-5-(hydroxymethyl)tetrahydrofuran-3-yl]oxysulfonylamino]methyl]-4-fluoro-5-purin-9-yl-tetrahydrofuran-3-yl]oxyphosphinic acid as the ammonium salt (396 mg, 59%). LCMS (AA): *m*/*z =* 1213.4 (M+H).

### Step 4: 4-{[(2S)-2-({(2S)-2-[(tert-Butoxycarbonyl)amino]-3-methylbutanoyl }amino)propanoyl]amino }benzyl [2-({9-[(2S,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-6-oxo-6,9-dihydro-1H-purin-2-yl}carbamoyl)benzyl]methylcarbamate or 4-{[(2S)-2-({(2S)-2-[(tert-butoxycarbonyl)amino]-3-methylbutanoyl }amino)propanoyl]amino }benzyl [2-({9-[(2R,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-6-oxo-6,9-dihydro-1H-purin-2-yl}carbamoyl)benzyl]methylcarbamate, Intermediate 33

Diphenyl phosphorochloridate (0.055 mL, 0.26 mmol) was added to pyridine (1.6 mL) and this solution was cooled to -35 °C. A solution of [(2*R*,3*R*,4*R*,5*R*)-2-[[[(2*R*,3*R*,5*S*)-2-[2-[[2-[[[4-[[(2*S*)-2-[[(2*S*)-2-(*tert-*butoxycarbonylamino)-3-methyl-butanoyl]amino]propanoyl]amino]phenyl]methoxycarbonyl-methyl-amino]methyl]benzoyl]amino]-6-oxo-1H-purin-9-yl]-5-(hydroxymethyl)tetrahydrofuran-3-yl]oxysulfonylamino]methyl]-4-fluoro-5-purin-9-yl-tetrahydrofuran-3-yl]oxyphosphinic acid as the ammonium salt (80 mg, 0.065 mmol) in DCM (2.5 mL) and pyridine (3.2 mL) was added dropwise to the cooled reaction mixture over 15 min. The resulting solution was stirred at -35 °C for 30 min. Solid 3H-1,2-benzodithiol-3-one (25 mg, 0.15 mmol) and water (0.056 mL) were added respectively to the cooled reaction mixture which was further stirred at rt for 1 h. The reaction solution was then cooled in an ice bath to 0 °C and was quenched by the addition of a solution of aqueous sodium thiosulfate (0.2M in water, 0.14 mL, 0.70 mmol). The mixture was stirred at rt for 5 min and concentrated under reduced pressure. The resulting residue was purified by reverse phase column chromatography (0-50% ACN/aqueous triethylammonium acetate (10 mM)) to afford 4-{[(2*S*)-2-({(2*S*)-2-[(*tert-*butoxycarbonyl)amino]-3-methylbutanoyl }amino)propanoyl]amino }benzyl [2-({9-[(2*S*,5*S*,7*R*,8*R*,12a*R*,14*R*,15*R*,15a*R*)-15-fluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-6-oxo-6,9-dihydro-1H-purin-2-yl}carbamoyl)benzyl]methylcarbamate or 4-{[(2*S*)-2-({(2*S*)-2-[(*tert-*butoxycarbonyl)amino]-3-methylbutanoyl }amino)propanoyl]amino }benzyl [2-({9-[(2*R*,5*S*,7*R*,8*R*,12a*R*,14*R*,15*R*,15a*R*)-15-fluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-6-oxo-6,9-dihydro-1H-purin-2-yl}carbamoyl)benzyl]methylcarbamate as the *N*,*N-*diethylethanamine salt (Intermediate 33, 27 mg, 31%). LCMS (AA): *m*/*z* = 1227.9 (M+H). ¹H NMR (400 MHz, CD₃OD) δ 9.14 (s, 1H), 8.75 (s, 1H), 8.63 (s, 1H), 8.30 (s, 1H), 7.53 - 7.36 (m, 5H), 7.34 - 7.17 (m, 3H), 6.47 (d, *J =* 19.3 Hz, 1H), 6.16 (d, *J =* 5.1 Hz, 1H), 5.87 - 5.82 (m, 1H), 5.76 - 5.55 (m, 1H), 5.50 - 5.40 (m, 1H), 5.00 (s, 2H), 4.79 - 4.75 (m, 2H), 4.68 - 4.61 (m, 1H), 4.58 - 4.47 (m, 2H), 4.44 - 4.39 (m, 1H), 4.09 - 4.02 (m, 1H), 3.92 - 3.89 (m, 1H), 3.67 - 3.62 (m, 1H), 3.57 - 3.53 (m, 1H), 3.01 - 2.94 (m, 1H), 2.87 (s, 3H), 2.74 - 2.68 (m, 1H), 2.11 - 2.03 (m, 1H), 1.45 - 1.43 (m, 3H), 1.44 (s, 9H), 0.98 (d, *J =* 6.7 Hz, 3H), 0.93 (d, *J =* 6.7 Hz, 3H). ³¹P NMR (162 MHz, CD₃OD) δ 57.35 (s, 1P).

### Example 38A

### 4-{[(25S,29S,32S)-25-{[4-(11,12-didehydrodibenzo[b,f]azocin-5(6H)-yl)-4-oxobutanoyl]amino}-29-isopropyl-32-methyl-24,27,30,33-tetraoxo-2,5,8,11,14,17,20-heptaoxa-23,28,31-triazatritriacontan-33-yl]amino }benzyl [4-({9-[(2R,5R,7R,8R,10R,12aR,14R,15R,15aR,16R)-15-fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-l][1,3,6,9,11,2,10]pentaoxadiphosphacyclotetradecin-14-yl]-9H-purin-6-yl}amino)-4-oxobutyl]methylcarbamate (C-34)

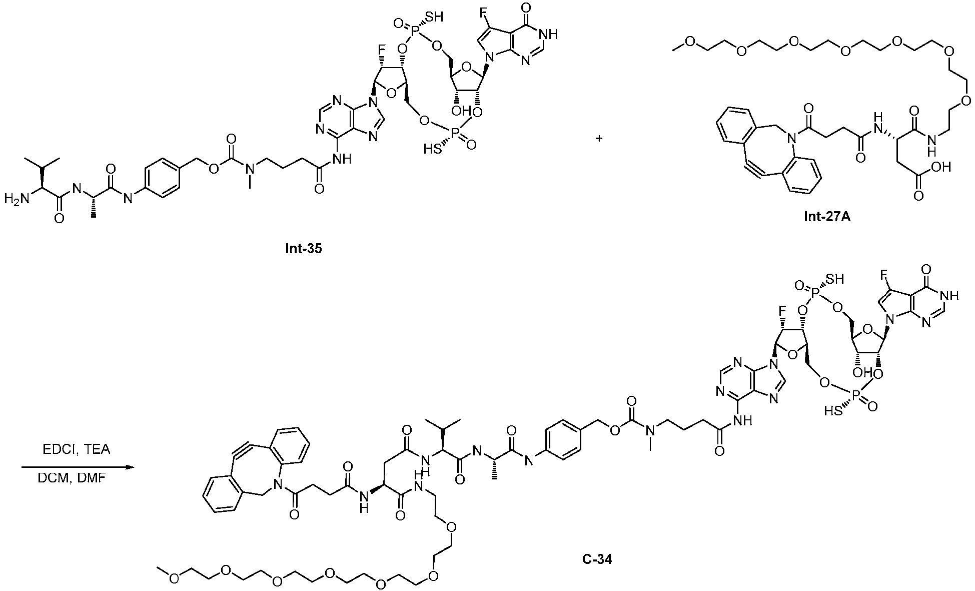

To a suspension of 4-{[(2*S*)-2-{[(2*S*)-2-amino-3-methylbutanoyl]amino}propanoyl]amino} benzyl [4-({9-[(2*R*,5*R*,7*R*,8*R*,10*R*,12a*R*,14*R*,15*R*,15a*R*,16*R*)-15-fluoro-7-(5-fluoro-4-oxo-3,4-dihydro-7*H-*pyrrolo[2,3-d]pyrimidin-7-yl)-16-hydroxy-2,10-dioxido-2,10-disulfanyloctahydro-12*H*-5,8-methanofuro[3,2-1][1,3,6,9,11,2,10] pentaoxadiphosphacyclotetradecin-14-yl]-9*H*-purin-6-yl}amino)-4-oxobutyl]methylcarbamate (Int-35, 17.4 mg, 0.0154 mmol), 1-ethyl-3-(3-dimethylaminopropyl)carbodiimide hydrochloride (3.49 mg, 0.0182 mmol) and (25*S*)-25-{[4-(11,12-didehydrodibenzo[b,f]azocin-5(6*H*)-yl)-4-oxobutanoyl]amino}-24-oxo-2,5,8,11,14,17,20-heptaoxa-23-azaheptacosan-27-oic acid (Int-27A) (10.4 mg, 0.0140 mmol) was added triethylamine (0.004 mL, 0.0266 mmol) in DCM (0.37 mL). The mixture was allowed to stir at rt for 20 mins, then DMF (0.15 mL, 2.23 mmol) was added. After this mixture was stirred at rt for 2 h, solvent was concentrated. The crude residue was purified by reverse phase flash column chromatography (0-50% ACN/ aqueous triethylammonium acetate (10 mM)) to provide 4-{[(25*S*,29*S*,32*S*)-25-{[4-(11,12-didehydrodibenzo[b,f]azocin-5(6*H*)-yl)-4-oxobutanoyl]amino}-29-isopropyl-32-methyl-24,27,30,33-tetraoxo-2,5,8,11,14,17,20-heptaoxa-23,28,31-triazatritriacontan-33-yl]amino }benzyl [4-({9-[(2*R*,5*R*,7*R*,8*R*,10*R*,12a*R*,14*R*,15*R*,15a*R*,16*R*)-15-fluoro-7-(5-fluoro-4-oxo-3,4-dihydro-7*H*-pyrrolo[2,3-d]pyrimidin-7-yl)-16-hydroxy-2,10-dioxido-2,10-disulfanyloctahydro-12*H*-5,8-methanofuro[3,2-l][1,3,6,9,11,2,10]pentaoxadiphosphacyclotetradecin-14-yl]-9*H*-purin-6-yl}amino)-4-oxobutyl]methylcarbamate (C-34) (2.0 mg, 6.9%) as the *N*,*N-*diethylethanamine salt. LCMS (AA): *m*/*z =* 926.9 (M/2+H). HRMS (*m*/*z*): [M+H]⁺ calcd for C₈₀H₁₀₁F₂N₁₅O₂₆P₂S₂ 1852.6000; found, 1852.6006.

### Example 38B

### (1R,8S,9s)-bicyclo[6.1.0]non-4-yn-9-ylmethyl {(25S)-27-[(2,5-dioxopyrrolidin-1-yl)oxy]-24,27-dioxo-2,5,8,11,14,17,20-heptaoxa-23-azaheptacosan-25-yl}carbamate

### (Intermediate 36)

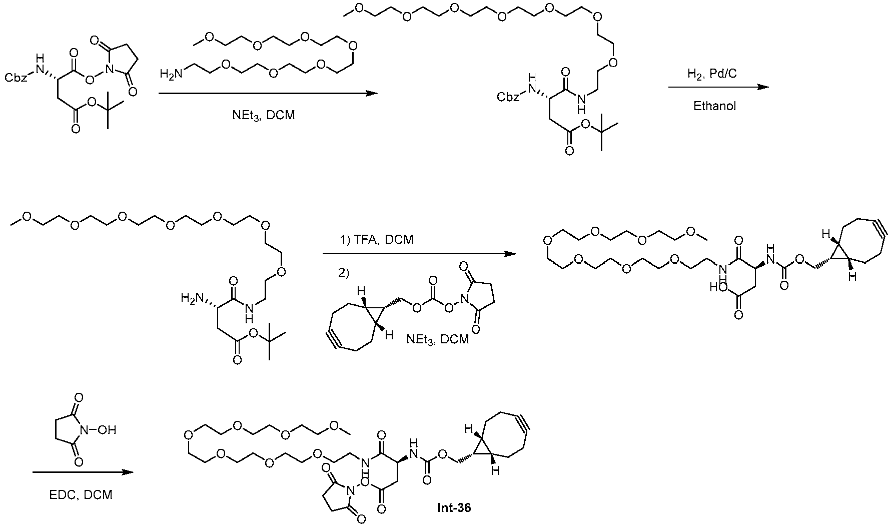

### Step 1: tert-butyl (25S)-25-{[(benzyloxy)carbonyl]amino}-24-oxo-2,5,8,11,14,17,20-heptaoxa-23-azaheptacosan-27-oate

The title compound was prepared following the procedure described in Example 26, step 1, starting with *tert*-Butyl (3*S*)-3-1[(benzyloxy)carbonyl]amino1-4-[(2,5-dioxopyrrolidin-1-yl)oxy]-4-oxobutanoate (2.2 g, 5.3 mmol) and 2,5,8,11,14,17,20-heptaoxadocosan-22-amine (2.0 g, 5.9 mmol) in place of Intermediate 15 and 2,5-dioxopyrrolidin-1-yl (*tert-*butoxycarbonyl)glycinate respectively. Purification by silica gel chromatography (0-10% MeOH/DCM) gave *tert*-butyl (25*S*)-25-{[(benzyloxy)carbonyl]amino}-24-oxo-2,5,8,11,14,17,20-heptaoxa-23-azaheptacosan-27-oate (3.8 g, 100%).

### Step 2: tert-Butyl (25S)-25-amino-24-oxo-2,5,8,11,14,17,20-heptaoxa-23-azaheptacosan-27-oate

*tert*-Butyl (25*S*)-25-{[(benzyloxy)carbonyl]amino}-24-oxo-2,5,8,11,14,17,20-heptaoxa-23-azaheptacosan-27-oate (3.8 g, 5.9 mmol) was dissolved in ethanol (100 mL). Palladium (10% on carbon, 1.38 g, 1.17 mmol) was added and the mixture was stirred under hydrogen (15 psi) for 3 h. The reaction mixture was filtered, washed with methanol and the filtrate was evaporated to dryness to provide *tert*-butyl (25*S*)-25-amino-24-oxo-2,5,8,11,14,17,20-heptaoxa-23-azaheptacosan-27-oate (2.56 g, 85%).

### Step 3: (25S)-25-({[(1R,8S,9s)-bicyclo[6.1.0]non-4-yn-9-ylmethoxy]carbonyl}amino)-24-oxo-2,5,8,11,14,17,20-heptaoxa-23-azaheptacosan-27-oic acid

To a solution of (25*S*)-25-amino-24-oxo-2,5,8,11,14,17,20-heptaoxa-23-azaheptacosan-27-oate (587 mg, 1.15 mmol) in DCM (11.3 mL) was added TFA (11.3 mL). The mixture was stirred at rt for 1h. The reaction was concentrated and azeotroped with toluene, and further dried on high vac to give crude intermediate as an oil. To the crude oil intermediate was added 1-({[(1*R*,8*S*,9s)-bicyclo[6.1.0]non-4-yn-9-ylmethoxy]carbonyl}oxy)pyrrolidine-2,5-dione (279 mg, 0.959 mmol) and DCM (9.8 mL), triethylamine (1.34 mL, 9.59 mmol) at rt. The mixture was stirred at rt for 2 h. The reaction mixture was diluted with EtOAc (50 mL), and potassium carbonate solution (10 mL, 4.0 M in water,). Separated organic layer was washed with brine, dried over Na2SO4, filtered, and concentrated. The residue was purified by reverse phase column chromatography (0-100% ACN/ aqueous ammonium bicarbonate (5 mM)). Desired fractions were concentrated in vacuo to give (25*S*)-25-({[(1*R*,8*S*,9s)-bicyclo[6.1.0]non-4-yn-9-ylmethoxy]carbonyl}amino)-24-oxo-2,5,8,11,14,17,20-heptaoxa-23-azaheptacosan-27-oic acid (19 mg, 3.1% yield). LCMS (AA): *m*/*z* = 629.2 (M-H); ¹H NMR (400 MHz, METHANOL-d4) δ = 4.46 (dd, *J* = 5.7, 7.4 Hz, 1 H), 4.25 - 4.12 (m, 2 H), 3.68 - 3.51 (m, 28 H), 3.41 - 3.34 (m, 5 H), 2.80 - 2.60 (m, 2 H), 2.33 - 2.12 (m, 4 H), 1.69 - 1.54 (m, 2 H), 1.40 (quin, *J* = 8.7 Hz, 1 H), 1.02 - 0.89 (m, 2 H).

### Step 4: (1R,8S,9s)-bicyclo[6.1.0]non-4-yn-9-ylmethyl {(25S)-27-[(2,5-dioxopyrrolidin-1-yl)oxy]-24,27-dioxo-2,5,8,11,14,17,20-heptaoxa-23-azaheptacosan-25-yl}carbamate (Intermediate 36)

The title compound was prepared following the procedure described in Example 29, starting with (25*S*)-25-({[(1*R*,8*S*,9s)-bicyclo[6.1.0]non-4-yn-9-ylmethoxy]carbonyl}amino)-24-oxo-2,5,8,11,14,17,20-heptaoxa-23-azaheptacosan-27-oic acid in place of 2,2-dimethyl-4-oxo-3,8,11,14,17-pentaoxa-5-azanonadecan-19-oic acid. The reaction mixture was stirred for 30 min at rt to provide (1*R*,8*S*,9s)-bicyclo[6.1.0]non-4-yn-9-ylmethyl {(25*S*)-27-[(2,5-dioxopyrrolidin-1-yl)oxy]-24,27-dioxo-2,5,8,11,14,17,20-heptaoxa-23-azaheptacosan-25-yl}carbamate (Intermediate 36, 22 mg, 100%). LCMS (AA): *m*/*z* = 728.4 (M+H).

### Example 38C

The compounds listed in Table 22A (Intermediate 37 and Intermediate 42) were prepared as described in Example 10, substituting the starting materials shown in step 1 and step 2 for 2,5,8,11,14,17,20-heptaoxadocosan-22-amine (Intermediate 37 and Intermediate 42) and methyl (2*S*,3*S*,4*S*,5*R*,6*S*)-3,4,5-triacetoxy-6-{2-[(3-{[(9H-fluoren-9-ylmethoxy)carbonyl]amino}propanoyl)amino]-4-({[(4-nitrophenoxy)carbonyl]oxy}methyl)phenoxy}tetrahydro-2*H*-pyran-2-carboxylate (Intermediate 42).

**Table 22A**

| Starting materials (step 1) | Starting materials (step 2) | Product | Analytical data of step 2 benzoic acid intermediate |
|---|---|---|---|
| 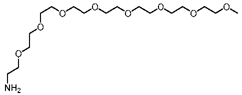 | 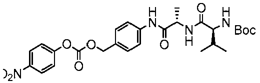 | 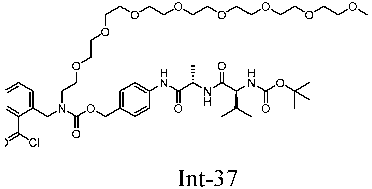 | LCMS (AA): *m*/*z =* 935.6 (M-H); |
| 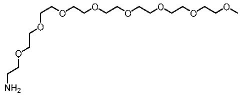 | 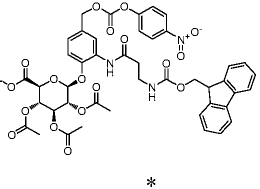 | 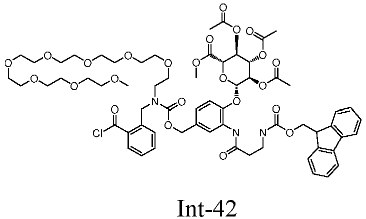 | LCMS (AA): *m*/*z =* 1290.2 (M-H); |

| | | | |
|---|---|---|---|
| * DMAP was not used in step 2 | | | |

### Example 38D

### 4-{[(2S,5S)-57-amino-5-isopropyl-2-methyl-4,7-dioxo-10,13,16,19,22,25,28,31,34,37,40,43,46,49,52,55-hexadecaoxa-3,6-diazaheptaspentacontan-1-oyl]amino}benzyl [2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate (C-37)

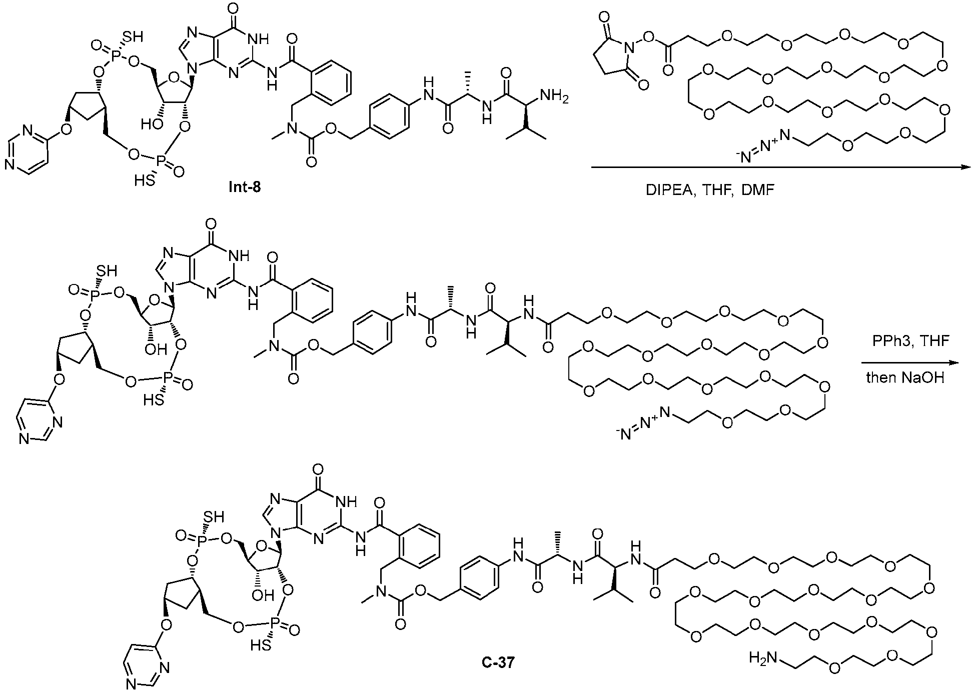

### Step 1: 4-{[(53S,56S)-1-azido-53-isopropyl-56-methyl-51,54,57-trioxo-3,6,9,12,15,18,21,24,27,30,33,36,39,42,45,48-hexadecaoxa-52,55-diazaheptapentacontan-57-yl]amino}benzyl [2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate

To a solution of intermediate 8 (18.68 mg, 0.01674 mmol), 1-[(1-azido-51-oxo-3,6,9,12,15,18,21,24,27,30,33,36,39,42,45,48-hexadecaoxahenpentacontan-51-yl)oxy]pyrrolidine-2,5-dione (32.3 mg, 0.03348 mmol), THF (0.6 mL) and DMF (0.2 mL) was added DIEA (0.044 mL, 0.2511 mmol). The mixture was stirred at rt for 3 h, then quenched with MeOH (5 mL). After most solvent was evaporated, the residue was purified by reverse phase column chromatography (0-50% ACN/ aqueous ammonium bicarbonate (5 mM)). Desired fractions were concentrated and lyophilized to give title compound (17.8 mg, 55%) as a white powder. LCMS (AA): *m*/*z* = 1915 (M-H).

### Step 2: 4-{[(2S,5S)-57-amino-5-isopropyl-2-methyl-4,7-dioxo-10,13,16,19,22,25,28,31,34,37,40,43,46,49,52,55-hexadecaoxa-3,6-diazaheptapentacontan-1-oyl]amino}benzyl [2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate (C-37)

To a 50-mL round-bottom flask was added 4-{[(53*S*,56*S*)-1-azido-53-isopropyl-56-methyl-51,54,57-trioxo-3,6,9,12,15,18,21,24,27,30,33,36,39,42,45,48-hexadecaoxa-52,55-diazaheptapentacontan-57-yl]amino}benzyl [2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1*H*-purin-2-yl}carbamoyl)benzyl]methylcarbamate (17.7 mg, 0.00923 mmol), THF (1.0 mL), and triphenylphosphine (24.2 mg, 0.0923 mmol). This mixture was stirred at rt overnight. To the reaction mixture was added sodium hydroxide (0.0554 mL, 1.0 mol/L in water). After the mixture was stirred at rt for 1 h, it was concentrated. The residue was purified by reverse phase column chromatography (0-50% ACN/ aqueous ammonium bicarbonate (5 mM)). Desired fractions were collected and lyophilized to give 4-{[(2*S*,5*S*)-57-amino-5-isopropyl-2-methyl-4,7-dioxo-10,13,16,19,22,25,28,31,34,37,40,43,46,49,52,55-hexadecaoxa-3,6-diazaheptapentacontan-1-oyl]amino}benzyl [2-({9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1*H*-purin-2-yl}carbamoyl)benzyl]methylcarbamate (C-37) (5.7 mg, 32%) as a white fluffy powder. LCMS (AA): *m*/*z =* 1889 (M-H). HRMS (*m*/*z*): [M+H]⁺ calcd for C₈₀H₁₂₄N₁₂O₃₂P₂S₂ 1891.7434; found 1891.7433.

### Example 38E

### N-{(2S)-6-(2,5-dioxo-2,5-dihydro-1H-pyrrol-1-yl)-1-[(2,5-dioxopyrrolidin-1-yl)oxy]-1-oxohexan-2-yl}-2,5,8,11,14,17,20,23-octaoxahexacosan-26-amide (Intermediate 39)

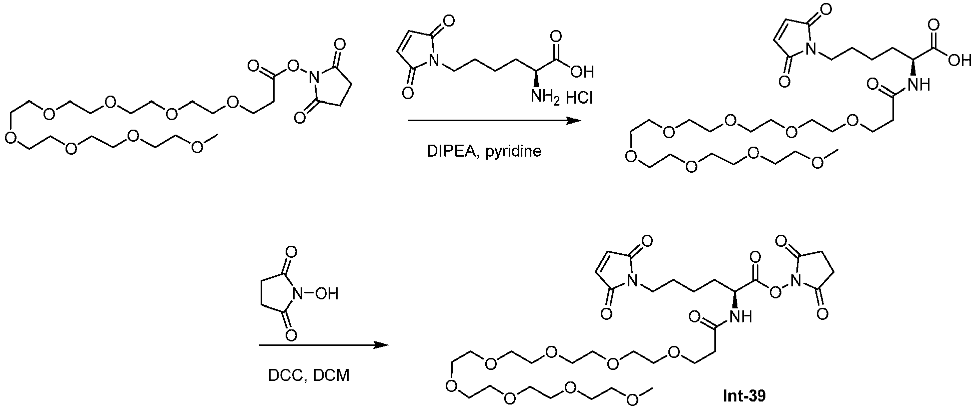

### Step 1: (28S)-28-[4-(2,5-dioxo-2,5-dihydro-1H-pyrrol-1-yl)butyl]-26-oxo-2,5,8,11,14,17,20,23-octaoxa-27-azanonacosan-29-oic acid

To a mixture of (2*S*)-2-amino-6-(2,5-dioxopyrrol-1-yl)hexanoic acid hydrochloride (1.05 g, 4.00 mmol) and
1-[(26-oxo-2,5,8,11,14,17,20,23-octaoxahexacosan-26-yl)oxy]pyrrolidine-2,5-dione (2.08 g, 4.04 mmol) in pyridine (21.0 mL) was added DIEA (1.43 mL, 8.20 mmol) and the mixture was allowed to stir at rt for 30 mins then cooled to 0 °C and stirred overnight. The volatile solvent was removed and the residue was azeotroped with toluene (3×20 mL) and acetonitrile (10 mL) to give (28*S*)-28-[4-(2,5-dioxo-2,5-dihydro-1*H*-pyrrol-1-yl)butyl]-26-oxo-2,5,8,11,14,17,20,23-octaoxa-27-azanonacosan-29-oic acid (4.00 g, 100% yield) as a colorless oil. LCMS (AA): *m*/*z* = 619.3 (M-H).

### Step 2: N-{(2S)-6-(2,5-dioxo-2,5-dihydro-1H-pyrrol-1-yl)-1-[(2,5-dioxopyrrolidin-1-yl)oxy]-1-oxohexan-2-yl}-2,5,8,11,14,17,20,23-octaoxahexacosan-26-amide (Intermediate 39)

A mixture of *N*-hydroxysuccinimide (469 mg, 4.08 mmol), *N,N'-*dicyclohexylcarbodiimide (824 mg, 4.00 mmol), and the crude (28S)-28-[4-(2,5-dioxo-2,5-dihydro-1*H*-pyrrol-1-yl)butyl]-26-oxo-2,5,8,11,14,17,20,23-octaoxa-27-azanonacosan-29-oic acid from step 1 (4.00 g, 4.00mmol) in DCM (76.7 mL, 1200 mmol) was allowed to stir at rt for 16 h. Solid crashed out from this reaction was filtered and the filtrate was concentrated to give a crude oil. Purification by silica gel chromatography (0-20% IPA/DCM) gave *N*-{(2*S*)-6-(2,5-dioxo-2,5-dihydro-1*H*-pyrrol-1-yl)-1-[(2,5-dioxopyrrolidin-1-yl)oxy]-1-oxohexan-2-yl}-2,5,8,11,14,17,20,23-octaoxahexacosan-26-amide (Intermediate 39) (1.70g, 52%) as a colorless oil. ¹H NMR (400 MHz, METHANOL-*d*₄) δ ppm 1.42 - 1.55 (m, 2 H) 1.64 (m, 2 H) 1.81 - 1.93 (m, 1 H) 1.94 - 2.01 (m, 1 H) 2.52 (m, 2 H) 2.81 - 2.86 (m, 4 H) 3.36 (s, 3 H) 3.51 - 3.57 (m, 4 H) 3.60 - 3.66 (m, 26 H) 3.68 - 3.76 (m, 2 H) 4.03 - 4.17 (m, 2 H) 4.74 - 4.80 (m, 1 H) 6.74 - 6.86 (s, 2 H).

### Example 38F

### N-{(2S)-6-{[(2,5-dioxo-2,5-dihydro-1H-pyrrol-1-yl)acetyl]amino}-1-[(2,5-dioxopyrrolidin-1-yl)oxy]-1-oxohexan-2-yl}-2,5,8,11,14,17,20,23-octaoxahexacosan-26-amide (Intermediate 40)

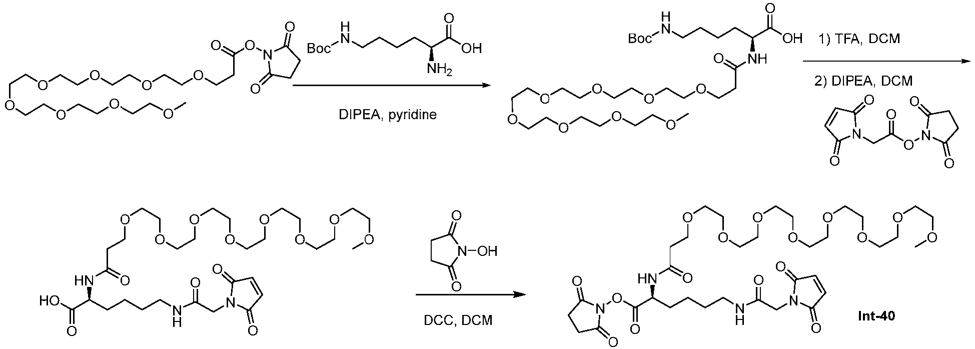

### Step 1: (28S)-28-{4-[(tert-butoxycarbonyl)amino]butyl }-26-oxo-2,5,8, 11, 14, 17,20,23-octaoxa-27-azanonacosan-29-oic acid

The title compound was prepared following the procedure described in Example 38E, step 1, starting with *N*6-(*tert*-butoxycarbonyl)-L-lysine (0.49 g, 1.99 mmol) in place of (2*S*)-2-amino-6-(2,5-dioxopyrrol-1-yl)hexanoic acid hydrochloride. Purification by reverse phase column chromatography (0-60% ACN/ aqueous formic acid (0.1%)) afforded (28*S*)-28-{4-[(*tert-*butoxycarbonyl)amino]butyl}-26-oxo-2,5,8,11,14,17,20,23-octaoxa-27-azanonacosan-29-oic acid (1.14 g, 90%) as a colorless oil. LCMS (AA): *m*/*z* = 639.4 (M-H).

### Step 2: (28S)-28-(4-{[(2,5-dioxo-2,5-dihydro-1H-pyrrol-1-yl)acetyl]amino}butyl)-26-oxo-2,5,8,11,14,17,20,23-octaoxa-27-azanonacosan-29-oic acid

To a solution of (28*S*)-28-{4-[(*tert*-butoxycarbonyl)amino]butyl}-26-oxo-2,5,8,11,14,17,20,23-octaoxa-27-azanonacosan-29-oic acid (148 mg, 0.2310 mmol) in DCM (2.0 mL) was added TFA (2.0 mL) at rt. The mixture was stirred at rt for 15 min, evaporated and then co-evaporated with toluene (x3) to give a crude TFA salt. After this crude product was further dried under high vac for 2 h, maleimidoacetic acid NHS ester (69.89 mg, 0.2772 mmol) in DCM (2650 mg, 2.00 mL, 31.2 mmol), and DIEA (0.121 mL, 0.6929 mmol) were added, and this reaction was stirred at rt for 16 h. The reaction mixture was evaporated. Purification by reverse phase column chromatography (10-30% ACN/ aqueous ammonium acetate (10 mM)) afforded (28*S*)-28-(4-{[(2,5-dioxo-2,5-dihydro-1*H*-pyrrol-1-yl)acetyl]amino}butyl)-26-oxo-2,5,8,11,14,17,20,23-octaoxa-27-azanonacosan-29-oic acid (120 mg, 38%) as an oil. LCMS (AA): *m*/*z* = 676.3 (M-H).

### Step 3: N-{(2S)-6-{[(2,5-dioxo-2,5-dihydro-1H-pyrrol-1-yl)acetyl]amino}-1-[(2,5-dioxopyrrolidin-1-yl)oxy]-1-oxohexan-2-yl}-2,5,8,11,14,17,20,23-octaoxahexacosan-26-amide (Intermediate 40)

To a flask containing (28*S*)-28-(4-{[(2,5-dioxo-2,5-dihydro-1*H*-pyrrol-1-yl)acetyl]amino}butyl)-26-oxo-2,5,8,11,14,17,20,23-octaoxa-27-azanonacosan-29-oic acid (311 mg, 0.459mmol) and DCM (2.94 mL) were added *N,N*'-dicyclohexylcarbodiimide (94.7 mg, 0.459 mmol) and *N*-hydroxysuccinimide (53.9 mg, 0.459 mmol). The suspension was stirred under Argon atmosphere at rt for 1 hr. The reaction mixture was then cooled to 0 °C, filtered through Celite and rinsed with MeCN. The filtrate was evaporated. The residue was re-dissolved in DCM, washed with water (x2), dried with MgSO4, and concentrated to give *N-*{(2*S*)-6-{[(2,5-dioxo-2,5-dihydro-1*H*-pyrrol-1-yl)acetyl]amino}-1-[(2,5-dioxopyrrolidin-1-yl)oxy]-1-oxohexan-2-yl}-2,5,8,11,14,17,20,23-octaoxahexacosan-26-amide (Intermediate 40) (300 mg, 84.4%) as a sticky oil. LCMS (AA): *m*/*z =* 773.2 (M-H).

### Example 38G

The compound listed in Table 22C (C-43) was prepared as described in Example 23 step 2 and step 3, substituting the starting material shown in the table for methyl (2S,3S,4S,5R,6S)-3,4,5-triacetoxy-6-{2-[(3-{[(9H-fluoren-9-ylmethoxy)carbonyl]amino}propanoyl)amino]-4-[({[2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10] pentaoxadiphosphacyclotetradecin-7-yl]-6-oxo-6,9-dihydro-1*H*-purin-2-yl}carbamoyl) benzyl](methyl)carbamoyl}oxy)methyl]phenoxy}tetrahydro-2*H*-pyran-2-carboxylate.

**Table 22B**

| Starting material | Product | Analytical data |
|---|---|---|
| 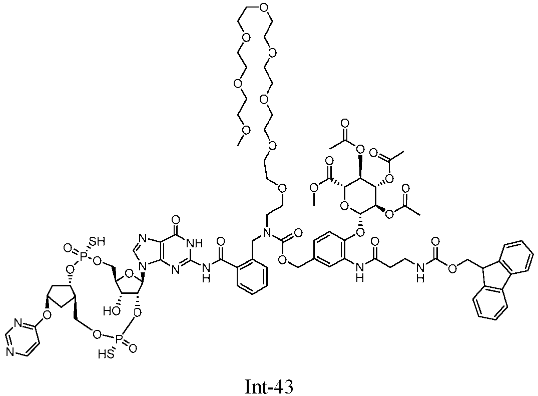 | C-43 | LCMS (AA): *m*/*z* = 1755 (M+H); HRMS (*m*/*z*): [M+H]⁺ calcd for C₇₂H₉₇N₁₁O₃₂P₂S₂ 1754.5291; found, 1754.5321. |

**Table 22C**

| Product | Structure |
|---|---|
| C-43 | 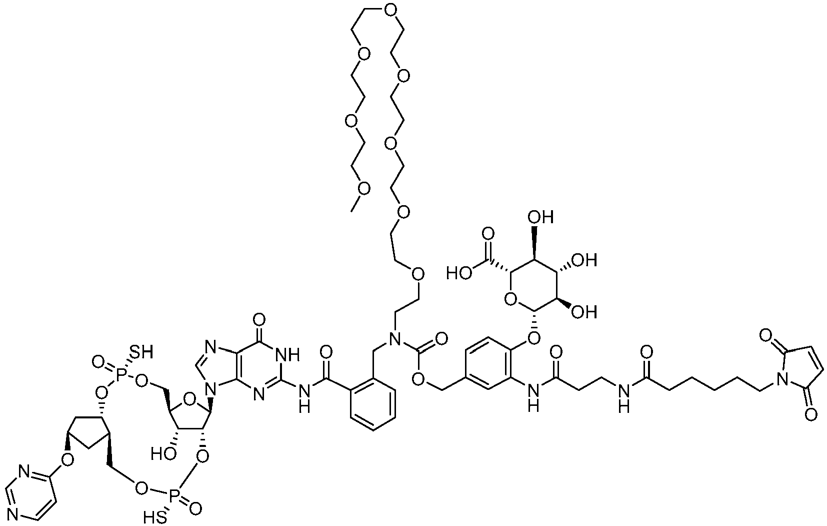 |

### Example 38H

The compound Intermediate-44 listed in Table 22D was prepared as described in Example 38E, substituting the starting material (2*R*)-2-amino-6-(2,5-dioxopyrrol-1-yl)hexanoic acid hydrochloride shown in Table 22D for (2*S*)-2-amino-6-(2,5-dioxopyrrol-1-yl)hexanoic acid hydrochloride in step 1.

**Table 22D**

| Starting material | Product | Analytical data |
|---|---|---|
| 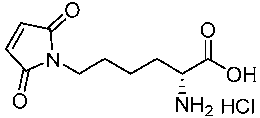 | 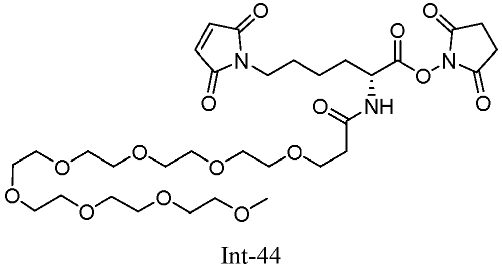 | LCMS (AA): *m*/*z* = 718.3 (M+H) |

### Example 39

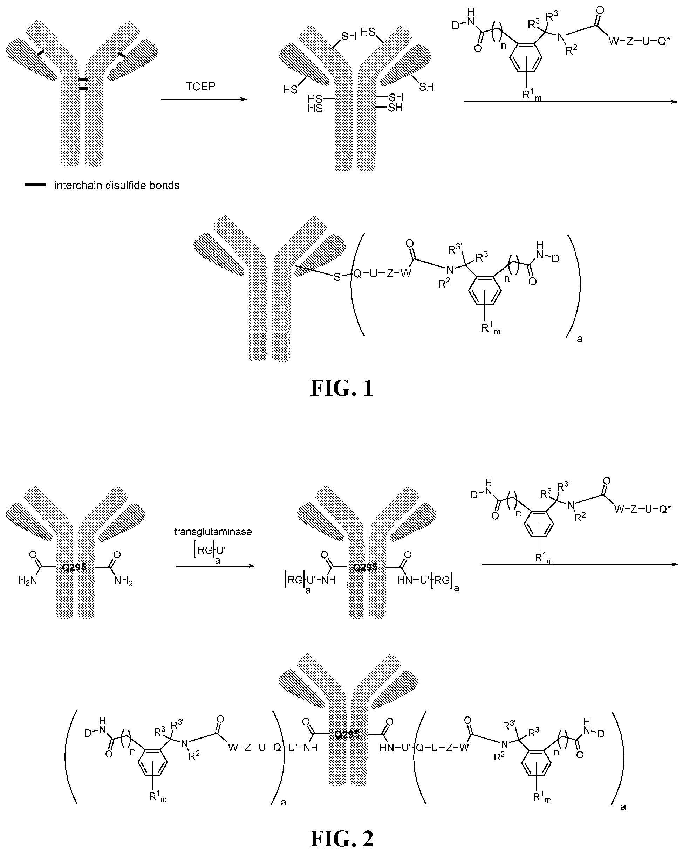

### Procedure for Preparation of Ab-STING Agonist Conjugates via Stochastic Cysteine Conjugation (Depicted in Figure 1)

To a solution of an anti-GCC antibody (clone 5F9, see PCT Publication No. WO 2011/050242, 60 mg/mL) in 50 mM histidine, 100 mM arginine, pH 6.0 buffer was added 0.5M Tris/25 mM EDTA pH 8.0 buffer until the pH of the solution was 7.5-8.0. To the above solution was added TCEP (10 mM solution in H₂O, 3-5 equiv.). The reaction mixture was purged with argon and incubated at rt or 37 °C for 2 h with gentle shaking. The desired linker-payload construct (10 mM solution in DMA, 5-9 equiv.) was then added slowly into the above mixture. The reaction was purged with argon and incubated at 37 °C for another 1.5-2 h with gentle shaking. The reaction mixture was purified following the preparative SEC method described herein to give the ADC. The ADC concentration, percentage aggregation, and DAR were determined by UV absorbance, analytical SEC, and LC-QTOF respectively, as described in analytical methods.

The above procedure can be used to prepare other antibody conjugates.

### Example 40

The antibody drug conjugates listed in Table 24 were prepared as described in Example 39, using the starting linker-payload constructs shown as the starting material.

**Table 24**

| Starting material | ADC product | Payload | DAR | Aggregation % | Yield % |
|---|---|---|---|---|---|
| C-21 | ADC-1 | Compound No. 14 | 4.5 | 3.09 | 100 |
| C-27 | ADC-2 | Compound I-5c | 3.7 | 2.08 | 56 |
| C-3 | ADC-3 | Compound I-5c | 2.9 | BLQ | 57 |
| C-16 | ADC-4 | Compound I-5c | 3.5 | 2.13 | 69 |
| C-8 | ADC-5 | Compound I-5c | 3.6 | 1.54 | 50 |
| C-24 | ADC-6 | Compound I-5c | 2.5 | 2.58 | 56 |
| C-26 | ADC-7 | Compound I-5c | 4.1 | BLQ | 42 |
| C-18 | ADC-8 | Compound I-5c | 3.9 | 0.59 | 83 |
| | | | | | |
| C-1 | ADC-9 | Compound I-5c | 4.3 | BLQ | 29 |
| C-4 | ADC-15 | Compound No. 14 | 3.1 | BLQ | 63 |
| C-38 | ADC-16 | Compound No. 14 | 3.7 | BLQ | 60 |

### Example 41

### Procedure for Preparation of Ab-STING Agonist Conjugates via Transglutaminase Conjugation (Depicted in Figure 2)

Deglycosylation: A solution of anti-GCC antibody (clone 5F9, see PCT Publication No. WO 2011/050242, 60 mg/mL) in 50 mM histidine, 100 mM arginine, pH 6.0 buffer was diluted with an equal volume of pH 7.2 PBS. To the solution was added N-Glycosydase F (New England Biolabs, P0704S, 500,000 units/mL, 300 units per 1mg of antibody) and the reaction mixture was heated to 37 °C with gentle mixing overnight. The resulting deglycosylated 5F9 was buffer-exchanged with PBS pH 7.2.

Transglutaminase conjugation: To the deglycosylated 5F9 solution (10~20 mg/mL) in PBS prepared above was added a 0.1M DMSO solution of 29-azido-3,6,9,12,15,18,21,24,27-nonaoxanonacosan-1-amine (azido-PEG9-amine, BroadPharm, BP-23556, 40 equivalents) followed by transglutaminase (ACTIVA^{™}, Ajinomoto, 5~10 mg per 1 mg of antibody). The reaction mixture was heated to 37 °C with gentle mixing overnight. The product was purified following the preparative SEC method described herein to give 5F9-NH-PEG-azide.

Strain-promoted azide-alkyne cycloaddition: To a solution of SF9-NH-PEG-azide conjugate prepared above (2~15mg/mL in PBS) was added a 4~10 mM DMSO solution of the strained-alkyne containing linker-payload constructs (3-5 equivalents in which DMSO <10% of the total solvent volume). The resulting solution was gently stirred at rt overnight. The product was purified following the preparative SEC method described herein to give the ADC. The ADC concentration, percentage aggregation, and DAR were determined by UV absorbance, analytical SEC, and LC-QTOF respectively, as described in analytical methods.

The above procedure can be used to prepare other antibody conjugates.

### Example 42

The antibody drug conjugates listed in Table 25 were prepared as described in Example 41, using the starting linker-payload constructs shown as the starting material in the table.

**Table 25**

| Starting material | ADC product | Payload | DAR | Aggregation % | Yield % |
|---|---|---|---|---|---|
| C-15 | ADC-10 | Compound I-5c | 1.8 | BLQ | 40 |
| C-13 | ADC-11 | Compound I-5c | 2 | BLQ | 81 |

### Example 43

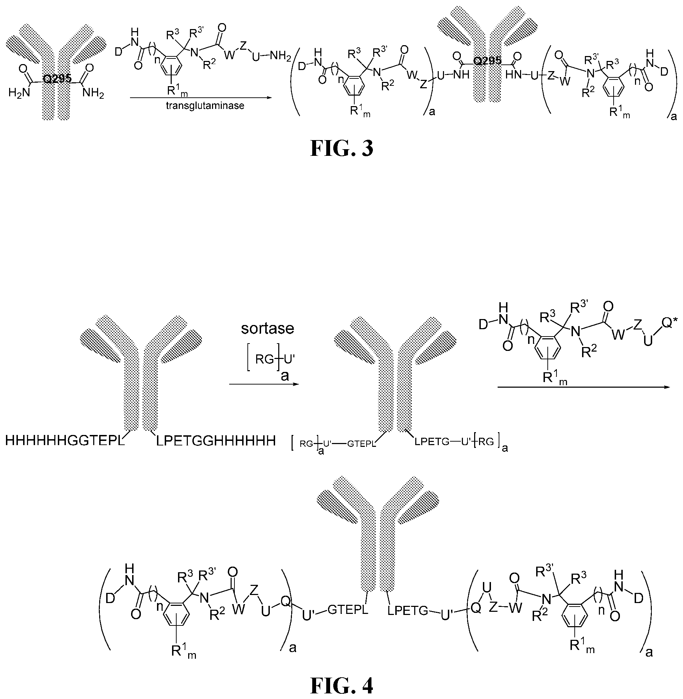

### Preparation of Ab-STING Agonist Conjugates viaTransglutaminase Conjugation (depicted in Figure 3)

To the deglycosylated 5F9 solution (10~20 mg/mL, prepared following the deglycosylation procedure described in Example 41) in PBS was added 1M Tris, 5M NaCl, pH 8.0 buffer (10~20% of the total volume) to adjust pH to 8.0. To the solution was added 10mM DMSO solution of primary amine-containing linker-payload constructs (20 equiv) followed by transglutaminase (ACTIVA^{™}, Ajinomoto, 100-150 mg per 1 mg of antibody). The reaction mixture was heated to 37 °C with gentle mixing overnight. The product was purified using HiTrap Protein A HP column (GE Healthcare, 17-0402-01), by first washing with 20mM phosphate pH 7.0 and then eluting ADCs with 0.1M citric acid pH 4.0.

The above procedure can be used to prepare other antibody conjugates.

### Example 44

The antibody drug conjugates listed in Table 26 were prepared as described in Example 43, using the starting linker-payload constructs shown as the starting material in the table.

**Table 26**

| Starting material | ADC product | Payload | DAR | Aggregation % | Yield % |
|---|---|---|---|---|---|
| C-30 | ADC-17 | Compound I-5c | 1.6 | BLQ | 72 |
| C-37 | ADC-18 | Compound I-5c | 1.2 | BLQ | 72 |

### Example 45

### 2-amino-N-(2-((2-(2-azidoacetamido)ethyl)amino)-2-oxoethyl)acetamide

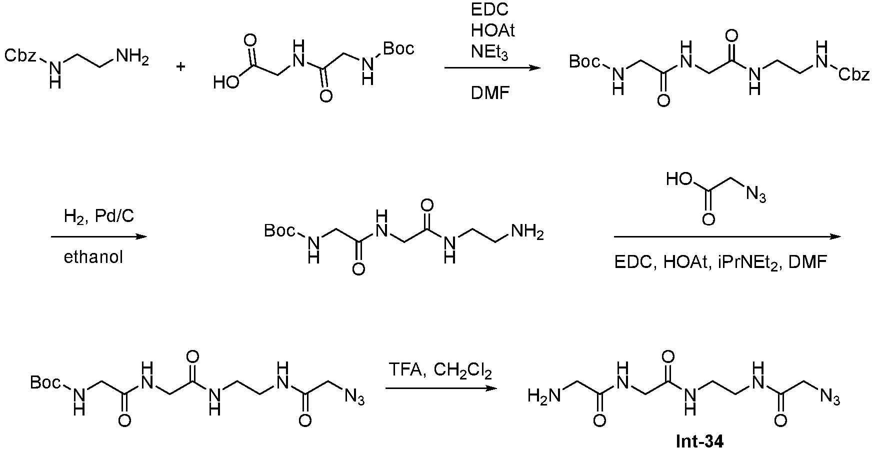

### Step 1: benzyl (2,2-dimethyl-4,7,10-trioxo-3-oxa-5,8,11-triazatridecan-13-yl)carbamate

To a solution of Boc-Gly-Gly-OH (550 mg, 2.32 mmol), EDC·HCl (437 mg, 2.28 mmol) and HOAt (317 mg, 2.33 mmol) in DMF (8.0 mL) was added triethylamine (0.340 mL, 2.44 mmol) at 0 °C . The resulting yellow heterogeneous mixture was allowed to stir for 15min. A solution of benzyl (2-aminoethyl)carbamate (500 mg, 2.44 mmol) in DMF (6.0 mL) was added slowly. In 10min, the mixture was allowed to warm to room temperature and stirred over 3 days. The reaction mixture was partitioned into water(100mL) and EtOAc (50mL). The separated aqueous layer was extracted with EtOAc (50mL). Combined organic layer was washed with 50mL of water, then brine, dried over Na₂SO₄ and concentrated. The crude product was purified by silica gel chromatography (0-6% MeOH/CH₂Cl₂) to provide tert-butyl N-[2-[[2-[2-(benzyloxycarbonylamino)ethylamino]-2-oxo-ethyl]amino]-2-oxo-ethyl]carbamate (617 mg, 1.51 mmol, 65% yield) as a white solid. LCMS (FA): m/z = 431.2 (M+Na). ¹H NMR (400 MHz, DMSO-d₆) δ 7.97-7.97 (m, 1H), 7.86-7.84 (m, 1H), 7.39-7.29 (m, 5H), 7.27-7.24 (m, 1H), 7.01-6.98 (m, 1H), 5.02 (s, 2H), 3.67 (d, *J*=5.62 Hz, 2H), 3.58 (d, *J*=5.87 Hz, 2H), 3.03-3.18 (m, 4H), 1.39 (s, 9H).

### Step 2: tert-butyl (2-((2-((2-aminoethyl)amino)-2-oxoethyl)amino)-2-oxoethyl)carbamate

To a solution of *tert*-butyl *N*-[2-[[2-[2-(benzyloxycarbonylamino)ethylamino]-2-oxoethyl]amino]-2-oxo-ethyl]carbamate (615 mg, 1.51 mmol) in ethanol (20.0 mL) was added 10% Pd/C (62.0 mg, 0.0583 mmol). The mixture was purged with H₂ (1 atm) and stirred at room temperature for 3 h. Celite was added to the reaction mixture and the slurry was filtered over a short bed of Celite. The filtrate was concentrated and the residue was dried by co-evaporation with toluene followed by high vacuum to give *tert*-butyl *N*-[2-[[2-(2-aminoethylamino)-2-oxoethyl]amino]-2-oxo-ethyl]carbamate (407 mg, 1.48 mmol, 98.5% yield) as an off white solid. LCMS (FA): m/z = 275.2 (M+H). ¹H NMR (400 MHz, DMSO-d₆) δ 8.05-7.89 (m, 1H), 7.80-7.63 (m, 1H), 7.08-6.96 (m, 1H), 3.67 (d, *J*=5.75 Hz, 2H), 3.56 (d, *J*=5.87 Hz, 2H), 3.06 (q, *J*=6.24 Hz, 2H), 2.59-2.53 (m, 2H), 1.87-1.61 (m, 2H), 1.39 (s, 9H).

### Step 3: tert-butyl N-[2-[[2-[2-[(2-azidoacetyl)amino]ethylamino]-2-oxo-ethyl]amino]-2-oxoethyl]carbamate

A mixture of EDC·HCl (57.6 mg, 0.300 mmol), HOAt (43.6 mg, 0.320 mmol), and 2-azidoacetic acid (27.0 µL, 0.315 mmol) in dichloromethane (2.70 mL) was stirred for 10 minutes. The mixture was cooled in ice bath, and then *tert*-butyl *N*-[2-[[2-(2-aminoethylamino)-2-oxoethyl]amino]-2-oxo-ethyl]carbamate (75.0 mg, 0.273 mmol) was added, followed by *N,N-*diisopropylethylamine (55.8 µL, 0.320 mmol) in a few minutes. The mixture was allowed to stir for 5min at 0 °C and then at rt overnight. Water (2mL) and EtOAc (5mL) were added and the mixture was vigorously stirred for 20min. The mixture was diluted with EtOAc (25mL) and then washed with 50% brine/water(20mL) and brine. Separated organic layer was dried over Na₂SO₄ and concentrated. Purification by silica gel chromatography (0-8% MeOH/CH₂Cl₂) provided *tert-*butyl *N*-[2-[[2-[2-[(2-azidoacetyl)amino]ethylamino]-2-oxo-ethyl]amino]-2-oxo-ethyl]carbamate (88.0 mg, 0.246 mmol, 90.1% yield) as a glassy solid. LCMS (FA): m/z = 356.2 (M-H). ¹H NMR (400 MHz, DMSO-d₆) δ 8.16-8.08 (m, 1H), 8.04-7.92 (m, 1H), 7.92-7.81 (m, 1H), 7.05-6.94 (m, 1H), 3.82 (s, 2H), 3.67 (d, *J*=5.62 Hz, 2H), 3.58 (d, *J*=5.87 Hz, 2H), 3.10-3.19 (m, 4H), 1.39 (s, 9H).

### Step 4: 2-amino-N-[2-[2-[(2-azidoacetyl)amino]ethylamino]-2-oxo-ethyl]acetamide (Intermediate 34)

To a solution of *tert*-butyl *N*-[2-[[2-[2-[(2-azidoacetyl)amino]ethylamino]-2-oxoethyl]amino]-2-oxo-ethyl]carbamate (86.0 mg, 0.241 mmol) in dichloromethane (4.0 mL) was added trifluoroacetic acid (1.00 mL, 8.77 mmol). The mixture was allowed to stir at room temperature for 30 min. The mixture was diluted with 5mL of toluene and concentrated. The residue was taken up in 5 mL of water and lyophilized to provide 2-amino-*N*-[2-[2-[(2-azidoacetyl)amino]ethylamino]-2-oxo-ethyl]acetamide 2,2,2-trifluoroacetic acid salt (102 mg, 0.275 mmol, quantitative) a colorless syrup. LCMS (FA): m/z = 258.1 (M+H). ¹H NMR (400 MHz, DMSO-d₆) δ 8.55-8.62 (m, 1H), 8.12-8.20 (m, 1H), 8.02-8.09 (m, 1H), 7.90-8.02 (m, 3H), 3.74-3.82 (s, 2H), 3.80 (m, 2H), 3.55-3.68 (m, 2H), 3.11-3.19 (m, 4H).

### Example 46

### Production and purification of 5F9-LPETGG-His6

5F9-LPETGG-His6 protein was produced using the transient Expi293F mammalian protein expression system kit from Thermo Fisher, cat. no. A14635, which includes the Expi293FTM cells, Expi293TM Expression growth medium, and Expifectamine 293TM transfection kit. 1.8 liters of Expi293FTM cells at a density of 2.8x10⁶ cells/mL in a 5L vented shake flask were transfected with 0.666mgs of light chain DNA plus 0.334mgs of heavy chain DNA mixed with 5.4mL Expifectamine 293TM transfection reagent and incubated overnight in a 37°C, 8% CO₂ incubator and shaking at 100rpm. The next day 10mLs enhancer 1 and 100mLs enhancer 2 from the kit were added to the flask. The flask was incubated at above conditions and harvested on day 5 post transfection. The cells were spun down at 9000rpm for 10 minutes at 4°C then filter sterilized through a 0.22µM filter unit and stored at 4°C until being purified over a 30mL MabSelect SuRe LX Protein A column (GE Healthcare, catalog#17-5474-03) that was freshly packed. After the supernant was loaded over the column using a peristalic pump, the resin was washed with 60 mL [2 column volumes (CV]) of low salt buffer (25 mM Trisodium Citrate pH 7.6, 125 mM NaCl) then washed with 60 mL (2CV) of high salt buffer (25 mM Trisodium Citrate pH 7.6, 2M NaCl), followed by another 60 mL (2CV) low salt buffer wash. The protein was eluted by pH gradient elution with 25mM Citric acid, 125mM NaCl pH8 to pH2.2 and peak fractions collected. Each fraction was neutralized with 1M Trisodium citrate pH8.2, to 70mM for approximate pH 6. The fractions in the main peak (2A5-2C5) were pooled. The pooled fractions were concentrated and buffer exchanged into 25 mM Sodium citrate pH 5.5, 125 mM NaCl buffer using a Sartorius VIVAFLOW200 cartridge with molecular weight cutoff of 50kDa. The sample was then filtered through a 0.22 µM syringe filter unit, aliquoted and stored at -80°C. The final yield was 623 mgs of purified 5F9-LPETGG-His6 antibody with >95% purity and endotoxin of 0.64 EU/mL.
Heavy chain sequence: 
MGWSCIILFLVATATGVHSQVQLQQWGAGLLKPSETLSLTCAVFGGSFSGYYWSWIRQP
PGKGLEWIGEINHRGNTNDNPSLKSRVTISVDTSKNQFALKLSSVTAADTAVYYCARER
GYTYGNFDHWGQGTLVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLSLSPGKAGGGS
LPETGGHHHHHH (signal sequence underlined) (SEQ ID NO. 1)
Light chain sequence:

MGWSCIILFLVATATGVHSEIVMTQSPATLSVSPGERATLSCRASQSVSRNLAWYQQKP
GQAPRLLIYGASTRATGIPARFSGSGSGTEFTLTIGSLQSEDFAVYYCQQYKTWPRTFGQ
GTNVEIKRTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGEC (signal
sequence underlined) (SEQ ID NO. 2)

### Example 47

### Procedure for Preparation of Ab-STING Agonist Conjugates via Sortase Conjugation (Depicted in Figure 4)

Sortase conjugation: The 5F9 antibody containing LPETGG-His6 at its C terminus of heavy chains was generated in accordance with the procedures described in Example 46. A solution of 5F9 containing LPETG-His6 tags at C-terminus (5F9-LPETGG-His6) of heavy chains in 25 mM citrate, 125 mM NaCl, pH 5.5 buffer was diluted with 50 mM HEPES, 150 mM NaCl, pH 7.45 buffer so that the concentration of the protein was 48 µM. To the solution was added CaCl₂ (4 mM aqueous solution, 80~100 equivalents) and 2-amino-*N*-[2-[2-[(2-azidoacetyl)amino]ethylamino]-2-oxo-ethyl]acetamide (Intermediate 34, 800 µM solution in 50 mM HEPES, 150 mM NaCl, pH 7.45 buffer with 0.1% DMSO, 20 equivalents) followed by Sortase A Q60-K206-P94S-D160N-D165A-K196 His6 (expressed as described in Antos, J. M., Ingram, J., Fang, T., Pishesha, N., Truttmann, M. C., Ploegh, H. L. (2017). Site-specific protein labeling via sortase-mediated transpeptidation. Current Protocols in Protein Science, 89, 15.3.1-15.3.19, 2.0 µM solution in 50 mM HEPES, 150 mM NaCl, pH 7.45 buffer, 0.03 equivalents). The reaction mixture was stirred at rt for 4 h. The product was purified by Capturem nickel column (from Takara, washed with PBS) followed by dialysis, or following preparative SEC method described herein to give 5F9-LPETG-azide.

Strain-promoted azide-alkyne cycloaddition: To a solution of 5F9- LPETGG-NH(CH₂)₂NHCOCH₂-azide conjugate prepared above (2~15mg/mL in PBS) was added a 4~10 mM DMSO solution of the strained-alkyne containing linker-payload construct (3~5 equivalents in which DMSO <10% of the total solvent volume). The resulting solution was gently stirred at rt overnight. The product was purified following the preparative SEC method described herein to give the ADC. The ADC concentration, percentage aggregation, and DAR were determined by UV absorbance, analytical SEC, and LC-QTOF respectively, as described in analytical methods.

The above procedure can be used to prepare other antibody conjugates.

### Example 48

The antibody drug conjugates listed in Table 27 were prepared as described in Example 47, using the starting linker-payload constructs shown as the starting material in the table.

**Table 27**

| Starting material | ADC product | Payload | DAR | Aggregation % | Yield % |
|---|---|---|---|---|---|
| C-15 | ADC-12 | Compound I-5c | 1.4 | 3.67 | 60 |
| C-9 | ADC-13 | Compound I-5c | 1.8 | BLQ | 66 |
| | | | | | |
| C-13 | ADC-14 | Compound I-5c | 1.4 | 3.7 | 84 |

### Example 49

### BIOLOGICAL PROTOCOLS AND STING CELLULAR DATA

### Tritosomal Assay Conditions

A test compound (121 uM linker-payload construct solution in DMSO or an ADC solution in PBS, 4.65 uL) was spiked into a mixture of 125 uL rat liver tritosomes (purchased from XenoTech) and 433 uL of an aqueous buffer solution (54.7 mg/mL phosphate, 1.7 mg/mL EDTA, pH 6.0) and the solution was incubated at 37 °C. 40 uL of sample was removed at 10 and 30 min, 1, 3, 5, and 24 h and treated with 160 uL of 0.1% formic acid in methanol solution on a 96-well plate and then stored at -80 °C. After collecting the last time point, each sample was thawed and treated with 200 uL of 0.1% formic acid in methanol solution containing 150 nM carbutamide (internal standard). The samples were centrifuged at 4000g for 10 min, and analyzed following the LC/MS/MS method described herein. Excel-Fit program was used to calculate t_{1/2}. The calculated t_{1/2} is reported in Table 28. As shown in Table 28, no free payload release from ADC-3 in this tritosomal assay was observed within 24 h while ADC-9 having the same payload successfully released the payload with a t_{1/2} of 7 hours, indicating that the linker of ADC-9 led to markedly improved rate of payload liberation under surrogate cellular release conditions as compared to ADC-3.

**Table 28**

| ADC | Payload | t_{1/2} (hour) |
|---|---|---|
| ADC-1 | Compound No. 14 | 2.4 |
| ADC-3 | Compound I-5c | -* |
| ADC-9 | Compound I-5c | 7.0 |
| ADC-15 | Compound No. 14 | 2.9 |
| ADC-16 | Compound No. 14 | 3.7 |

| | | |
|---|---|---|
| * No free payload release was observed within 24 h | | |

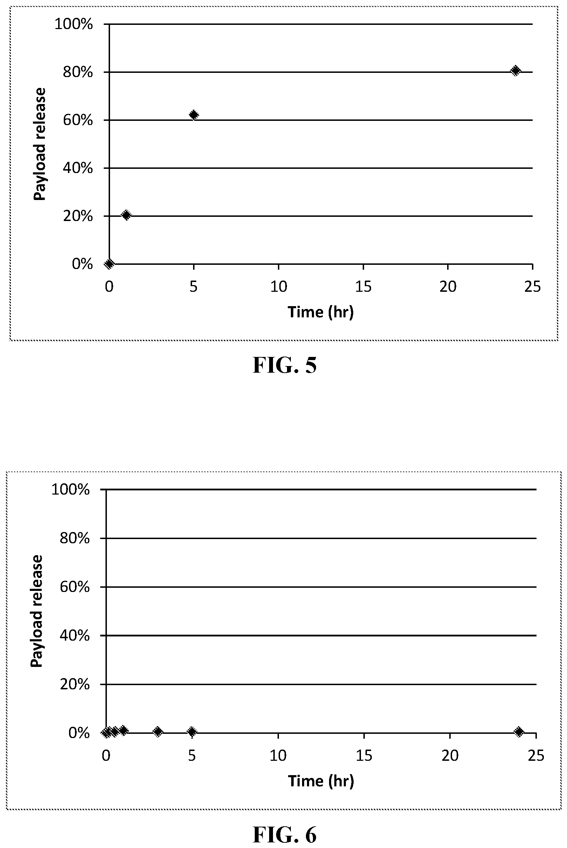

Graphical representation of the payload release over time for ADC-1, ADC-3, and ADC-9 in Table 28 is shown in Figures 5, 6, and 7.

### Plasma Stability Assay Conditions

Test compounds were spiked into 1 mL of plasma at a concentration of 10 µg/mL and then 5 equal volume aliquots were dispensed into 2 mL Eppendorf microfuge tubes (labeled 0, 24, 48, 72, and 96 hours). The time 0 sample tubes were immediately stored at -80 °C and the remaining tubes were incubated at 37 °C with moderate shaking. Aliquots were removed from the incubator at their corresponding time point and stored at -80°C. After all samples have been collected, they were thawed at rt and placed on wet ice. 50 µL of each sample was dispensed in triplicate into 96-well microtiter plate. Samples were quenched with 200 µL of ice cold methanol containing 50 nM of internal standard. Samples were vortexed for 2 min then centrifuged at 3000 rpm for 10 min. 185 µL of supernatant was transferred to a clean injection plate then dried down under N₂ gas at 40 °C. Dried sample extracts were reconstituted with 100 µL of LCMS grade water then vortexed for 1 min in preparation for LC-MS/MS analysis.

Each sample was separated by reverse phase HPLC using a Synergi 2.5µ Polar-RP 100A C18 column (2.0 mm X 30 mm), (Phenomenex^{®}) at 40 °C using a gradient consisting of 0.1% formic acid in water (Solvent A) and 0.1% formic acid in acetonitrile (Solvent B). Analytes were detected by positive ion spray in multiple-reaction monitoring (MRM) mode using a SCIEX API 4500 QTRAP instrument. Percentage payload loss in human, primate and mouse plasma at various time points is reported in Table 29.

**Table 29**

| ADC | Payload loss (%) in human plasma | | | | Payload loss (%) in cyno plasma | | | | Payload loss (%) in mouse plasma | | | |
|---|---|---|---|---|---|---|---|---|---|---|---|---|
| | 1d | 2d | 3d | 4d | 1d | 2d | 3d | 4d | 1d | 2d | 3d | 4d |
| ADC-9 | 0.1 | 0.7 | 1.1 | 1.7 | 0.3 | 0.3 | 1.0 | 1.2 | 1.4 | 2.1 | 3.0 | 3.4 |
| ADC-2 | 5.7 | 11.1 | 13.2 | 20.8 | 7.3 | 14.2 | 20.9 | 25.8 | 5.8 | 9.4 | 14.1 | 18.8 |
| ADC-4 | 0.1 | 0.8 | 1.6 | 1.8 | 0 | 0.7 | 1.3 | 1.6 | 0.4 | 0.8 | 1.0 | 5.6 |
| ADC-3 | 1.3 | 3.7 | 5.4 | 7.3 | 1.4 | 3.2 | 5.5 | 7.6 | 0.7 | 1.5 | 1.0 | 1.3 |
| ADC-1 | 11.5 | 21.6 | 32.1 | 40.9 | NA | NA | NA | NA | 9.2 | 16.0 | 23.3 | 30.4 |
| ADC-10 | 0 | 0 | 1.2 | 1.7 | 0 | 0 | 1.1 | 1.5 | 2 | 1.8 | 1.9 | 2.6 |
| ADC-5 | 0 | 0.9 | 1.6 | 2.1 | 0.5 | 0.7 | 1.3 | 1.8 | 2.2 | 1.5 | 2.2 | 6.3 |
| ADC-6 | 0 | 0 | 1.7 | 2.4 | 0.6 | 1.1 | 1.5 | 2.3 | 0.3 | 0.7 | 1.0 | 0.9 |
| ADC-7 | 6.3 | 12.0 | 15.5 | 23.4 | 6.0 | 11.6 | 16.3 | 20.5 | 4.0 | 6.0 | 3.6 | 19.9 |
| ADC-8 | 0.2 | 0.6 | 0.9 | 1.2 | 0.3 | 0.5 | 0.7 | 1.0 | 0.5 | 0.7 | 1.3 | 1.3 |
| ADC-12 | 0 | 0 | 0.3 | 1.7 | 0 | 0.4 | 1.1 | 1.3 | 21.2 | 25.6 | 27.2 | 28.8 |
| ADC-14 | 0 | 0.7 | 2.2 | 2.6 | 0.5 | 1.0 | 1.6 | 2.4 | 16.5 | 15.0 | 17.6 | 17.8 |
| ADC-15 | 0.9 | 2.0 | 3.0 | 4.1 | 0 | 1.6 | 2.2 | 3.1 | 3.6 | 3.7 | 6.6 | 8.0 |
| ADC-16 | 1.0 | 2.0 | 2.5 | 3.6 | 0.9 | 1.1 | 1.7 | 2.6 | 2.5 | 8.6 | 8.4 | 11.7 |

### THP1 Dual Lucia Reporter Gene Assay Conditions

THP1-Dual^{™} KI-hSTING-R232 cells (InvivoGen #thpd-r232) were derived from the human THP-1 monocyte cell line by stable biallelic knockout of the endogenous human HAQ STING gene and knockin of the R232 variant of human STING. These cells also stably express inducible secreted Lucia luciferase reporter gene under the control of an ISG54 (interferon-stimulated gene) minimal promoter in conjunction with five IFN-stimulated response elements (ISRE). The expression of reporter gene allows the study of the IFN regulatory factor (IRF) pathway by assessing the activity of Lucia luciferase. In addition to human STING and luciferase, these cells were engineered to stably express human Guanylyl cyclase C (GCC) to allow the study of target-mediated activation of IRF pathway. Non-GCC expressing vector cells were used as a control.

On the day of experiment, the cells were plated to a white, 384-well plate (Corning 356661) at 15,000 cells/25 µL per well density in growth media (RPMI 1640, 2 mM L-glutamine, 25 mM HEPES, 10% heat-inactivated fetal bovine serum, 100 µg/mL Normocin^{™}, 100 U/mL-100 µg/mL Pen-Strep, 10 µg/mL of blasticidin, 100 µg/mL of Zeocin, and 1 µg/mL of Puromycin). The cell plates were dosed with 5 µL of the GCC-targeting-ADC samples, and then incubated at 37 °C for 20 hours. At the end of the incubation, 10 µL/well of the QUANTI-Luc^{™} (InvivoGen # rep-qlc1) were added, and luminescence was measured immediately using the LeadSeeker.

For the assay method described above, percent luminescence signal induction for each test ADC, at various concentrations, was calculated relative to untreated and control treated samples. Compound concentration versus percent signal induction curves were fitted to generate EC₅₀ values. One skilled in the art will appreciate that the values generated as EC₅₀ values are subject to experimental variation. The observed EC₅₀ and Emax are reported in Table 30. As demonstrated in Table 30 below, ADC-16 was about 21 fold more potent than ADC-15 in the vector THP1 cells, likely due to more rapid intracellular payload release from ADC-16 as compared to ADC-15.

**Table 30**

| ADC | hGCC-expressing THP1 | | Vector THP 1 | |
|---|---|---|---|---|
| | EC₅₀ nM | Emax | EC₅₀ nM | Emax |
| ADC-1 | 0.068 | 123 | 3.9 | 82.3 |
| ADC-9 | 0.10 | 91.6 | >280 | 39.6 |
| ADC-2 | 0.28 | 80.7 | >370 | 17.0 |
| ADC-10 | 0.10 | 81.3 | >180 | 3.8 |
| ADC-3 | >290 | 0.9 | >290 | 0.4 |
| ADC-4 | 0.047 | 96.6 | 3.92 | 67.6 |
| ADC-5 | 0.089 | 82.5 | 11.0 | 49.8 |
| ADC-11 | 0.12 | 86.9 | >200 | 12.6 |
| ADC-6 | 0.11 | 90.8 | >250 | 28.3 |
| ADC-7 | 0.57 | 90.1 | >380 | 15.1 |
| ADC-8 | 0.17 | 102.2 | >430 | 40.2 |
| ADC-12 | 0.10 | 94.8 | >140 | 22.4 |
| ADC-13 | 0.060 | 95 | NA | NA |
| ADC-14 | 0.074 | 86.9 | >140 | 15.4 |
| ADC-15 | 0.067 | 108 | 242 | 54.9 |
| ADC-16 | 0.040 | 97.7 | 11.2 | 83.0 |
| ADC-17 | 0.032 | 105 | >500 | 3.46 |
| ADC-18 | <0.025 | 100 | >500 | 1.18 |

### Example 50

### Pharmacokinetics evaluation in mouse

For *in vivo* evaluation of the ADCs in Balb/C mouse bearing GCC-expressing CT26 colon carcinoma mouse tumors, female Balb/C mice at 6-8 weeks of age (purchased from Jackson Laboratory) were used. CT26.WT parental cells were obtained from ATCC, Catalog# CRL-2638. GCC-expressing CT26 cells were constructed at Takeda by transducing CT26.WT with lentiviral vector pLenti6.3 containing human GCC gene to obtain CT26 pLenti6.3 GCC clone A5. CT26 pLenti6.3 GCC clone A5 cells were grown in sterile conditions in a 37°C incubator with 5% CO₂ for 12 -14 days. Cells were grown in RPMI-1640 medium with 10% fetal bovine serum. Cells were passaged every 3-4 days using 0.05% Trypsin/EDTA to detach cells. On the day of implantation the cells were lifted and re-suspended in RPM-1640 serum-free media at a concentration of 0.5 x 106 cells /100 µL. CT26 pLenti6.3 GCC clone A5 cells were implanted by subcutaneous injection into the mouse lower flank using 27 gauge needle (0.5 x 10⁶ cells /100 µl injection volume per mouse). After cell implantation, tumors were measured by caliper and mice were weighed two times per week once tumors were palpable. Tumors were measured in two dimensions. Caliper measurements were calculated using (L x W²)/2. Mice were fed with normal diet and housed in a SPF animal facility in accordance with the Guide for Care and Use of Laboratory Animals and regulations of the Institutional Animal Care and Use Committee. Animals were kept at a temperature of 18-26 °C, a relative humidity of 50 ± 20% and intermittent light and dark cycles of 12 hours with food and water available ad libitum.

Pharmacokinetics of the ADCs were studied following injection of ADCs into Balb/C mice bearing CT26-GCC tumors (n=3/treatment group) when the mean tumor volume (MTV) reached 500-800 mm³. Serum samples were taken at various time points and stored frozen for analysis.

The mouse plasma levels of total antibodies and conjugated payloads were measured by a 2-in-1 immunocapture based LC/MS assay on a Shimadzu UHPLC system interfaced to a Sciex 6500 QTRAP mass spectrometer. Briefly, mouse plasma samples were incubated with antihuman IgG coated magnetic beads for 45 min at room temperature, then non-specifically bound proteins were removed by washing the magnetic beads with PBST (PBS buffer at pH 7.4, containing 0.05% tween 20) and PBS buffer consecutively. After that, both naked antibodies (DAR=0) and ADCs (DAR≥1) were eluted from the magnetic beads into 0.1% trifluoroacetic acid. After neutralizing the eluents and spiking in stable isotope labeled internal standards, one aliquot of sample was pipetted out and digested with papain for 1 hour at 37°C then used for the LC/MS analysis of conjugated payloads. The remaining samples were subjected to trypsin/lys-C digestion for 1 hour at 70°C then used for the LC/MS analysis of total antibodies.

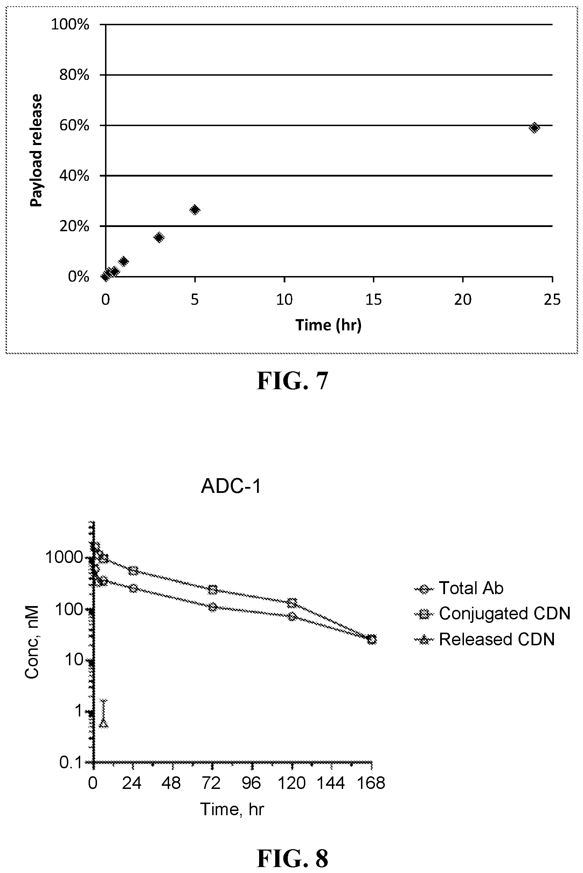

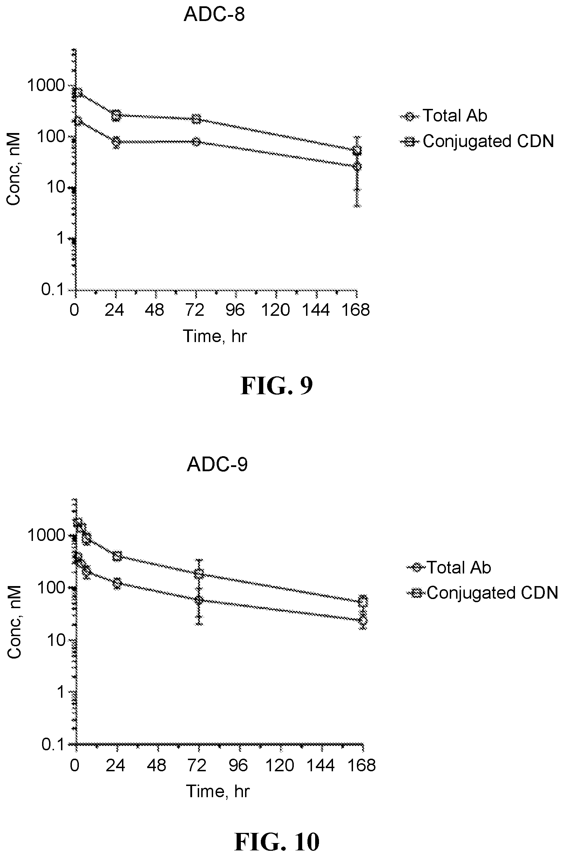

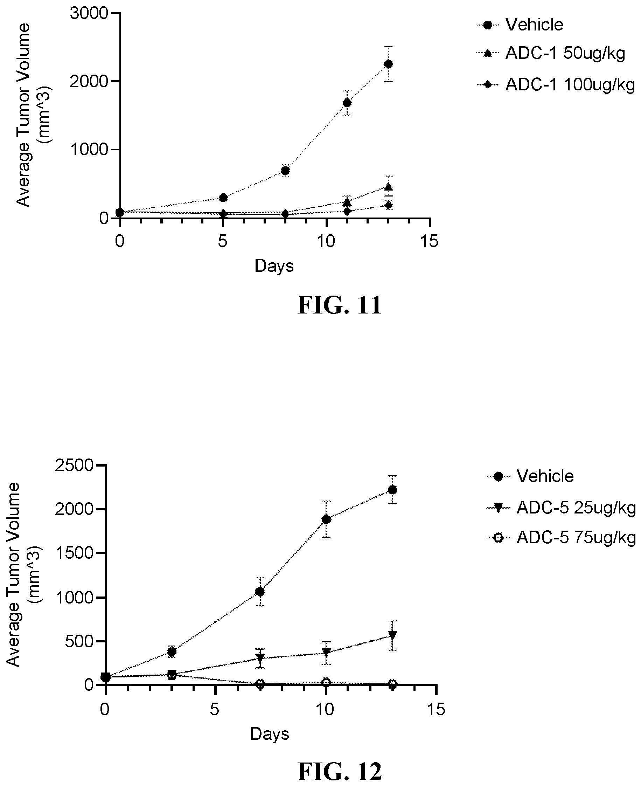

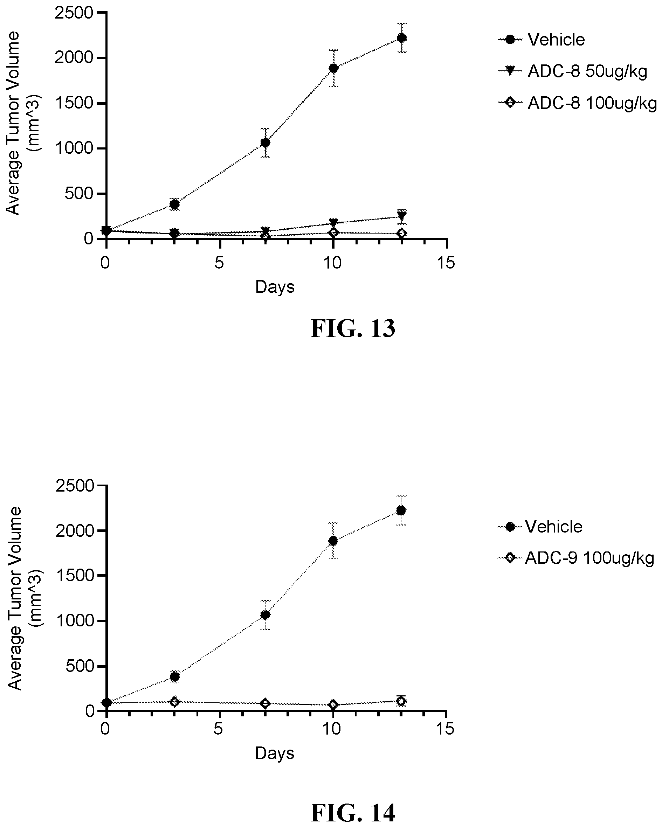

The free payload in the circulation was also measured by LC/MS after performing plasma protein precipitation. In short, mouse plasma was mixed with 8 volumes of methanol containing stable isotope labeled internal standard, then the supernatants were evaporated to dryness at 40°C under a gentle nitrogen stream. Finally, the residues were reconstituted in LC/MS grade water prior to LC/MS analysis. The PK profile of ADC-1, ADC-8, and ADC-9 is summarized in Table 31. Graphical representation of the plasma PK is shown in Figures 12, 13, and 14. As shown in Table 24, the DAR values of ADC-1, ADC-8 and ADC-9 are 4.5, 3.9, and 4.3, respectively, which leads to the higher molar concentration of the conjugated CDN obtained at time 0 than that of total Ab shown in each of Figures 8-10. However, as shown in Fig. 8, the molar concentration of the conjugated CDN from ADC-1 dropped faster than that of the total Ab and the two concentrations merged on day 7 (leading to DAR of about 1 on that day), indicating that the DAR of ADC-1 decreased over time due to the lower stability of its linker. In contrast, as shown in Figures 9 and 10, the molar concentration of the conjugated CDN from both ADC-8 and ADC-9 continued to be higher than that of their respective total Ab throughout the course of the experiment. In addition, the longer half life of conjugated payload/CDN from ADC-8 and ADC-9 as compared to ADC-1, as demonstrated in Table 31, is likely a result of the enhanced linker stability in ADC-8 and ADC-9. In summary, this experiment indicates that the linkers of ADC-8 and ADC-9 have improved stability in mouse plasma relative to that of ADC-1.

**Table 31**

| ADC (payload dose) | | Half life (h) | AUC (last) (h*nM) |
|---|---|---|---|
| ADC-1 (0.1 mg/kg) | Conjugated payload | 33 | 51432 |
| | Antibody | 45 | 23280 |
| ADC-9 (0.05 mg/kg) | Conjugated payload | 50 | 46262 |
| | Antibody | 62 | 13313 |
| ADC-8 (0.055 mg/kg) | Conjugated payload | 60 | 37315 |
| | Antibody | 65 | 12540 |

### Example 51

### Antitumor activity evaluation in mouse

Balb/C mouse bearing the GCC-expressing CT26 colon carcinoma mouse tumors were generated as described in Example 50.

When the mean tumor volume (MTV) reached approximately 75 mm³, animals were randomized into several treatment groups (n=8/group) and dosed once by intravenous route with various doses of ADC. Tumors continued to be measured two times a week. Effects of ADC treatment on tumor growth were evaluated by measuring tumor volumes and comparing to vehicle treated tumors.

An endpoint for efficacy studies was achieved when tumor size reached a volume of 10% of the animals body weight or 2 cm in either direction. Following injection, mice were also closely monitored for signs of clinical deterioration. If for any reason mice showed any signs of morbidity, including respiratory distress, hunched posture, decreased activity, hind leg paralysis, tachypnea as a sign for pleural effusions, weight loss approaching 20% or 15% plus other signs, or if their ability to carry on normal activities (feeding, mobility), was impaired, mice were euthanized.

Graphical representation of the observed antitumor activity for ADC-1, ADC-5, ADC-8, and ADC-9 is shown in Figures 7, 8, 9, and 10, respectively.

### LIPID CONJUGATES:

### Abbreviations:

- AA: LCMS method using ammonium acetate
- Ac: acetate
- ACN: acetonitrile
- atm: atmosphere
- aq: aqueous
- BBN: borabicyclo(3.3.1)nonane
- Bn: benzyl
- Boc: *tert*-butoxycarbonyl
- (Bpin)₂: bis(pinacolato)diboron
- *t*Bu: *tert*-butyl
- Bz: benzoyl
- C: Celsius
- CAD: Charged Aerosol Detection
- cGAMP: Cyclic guanosine monophosphate-adenosine monophosphate
- DBAD: di-*tert*-butyl azodicarboxylate
- DBU: 1,8-diazabicyclo[5.4.0]undec-7-enedichloroacetic acid
- DCA: dichloroacetic acid
- DCE: dichloroethane
- DCM: dichloromethane
- DEAD: diethyl azodicarboxylate
- DIAD: diisopropyl azodicarboxylate
- DIPEA: *N,N*-diisopropylethylamine
- DMAP: 4-dimethylaminopyridine
- DMF: *N,N*-dimethylformamide
- DMSO: dimethylsulfoxide
- DMTr: 4,4'-dimethoxytrityl
- DOTMA: 1,2-di-O-octadecenyl-3-trimethylammonium propane
- DPPC: 1,2-dipalmitoyl-sn-glycero-3-phosphocholine
- DSPC: 1,2-distearoyl-sn-glycero-3-phosphocholine
- Et: ethyl
- EtOH: ethanol
- EtOAc: ethyl acetate
- FA: LCMS method using formic acid
- h: hours
- Int: Intermediate
- HPLC: high pressure liquid chromatography
- HRMS: high resolution mass spectrometry
- IC₅₀: inhibitory concentration 50%
- IPA: isopropyl alcohol
- IPC: diisopinocampheyl
- LCMS: liquid chromatography mass spectrometry
- LC/MS/MS: liquid chromatography/tandem mass spectrometry
- LDA: lithium diisopropylamide
- LHMDS: lithium bis(trimethylsilyl)amide
- *m*/*z*: mass to charge
- MHz: mega hertz
- Me: methyl
- MeOH: methanol
- min: minutes
- mL: milliliters
- MMP: methylmorpholine
- MS: mass spectrum
- nBu: n-butane
- NMP: 1-methyl-2-pyrrolidinone
- NMR: nuclear magnetic resonance
- PE: petroleum ether
- Ph: phenyl
- psi: pounds per square inch
- pyr: pyridine
- rt: room temperature
- SFC: supercritical fluid chromatography
- T3P: 2,4,6-tripropyl-1,3,5,2,4,6-trioxatriphosphorinane-2,4,6-trioxide
- TDA-1: *tris*[2-(2-methoxyethoxy)ethyl]amine
- TBAF: tetrabutylammonium fluoride
- TBS: *tert*-butyldimethylsilyl
- TBTU: *O*-(benzotriazol-1-yl)-*N*,*N*,*N'*,*N'*-tetramethyluronium tetrafluoroborate
- TEA: triethylamine
- TFA: trifluoroacetic acid
- TIDPSi: 1,1,3,3-tetraisopropyldisiloxane
- TIPS: triisopropylsilyl
- THF: tetrahydrofuran
- UPLC: ultra performance liquid chromatography
- Xantphos: 4,5-bis(diphenylphosphino)-9,9-dimethylxanthene

### Analytical Methods:

### NMR conditions:

NMR spectra were recorded on a Bruker Avance III 400 spectrometer 400 MHz ¹H frequency unless otherwise stated. ³¹P NMR and ¹⁹F NMR spectra were run on the same spectrometer at 162 MHz and 376 MHz respectively. All heteronuclear experiments were acquired with ¹H decoupling unless otherwise stated.

### LCMS conditions:

LCMS spectra were recorded on a Hewlett-Packard HP1100 or Agilent 1100 Series LC system connected to a Micromass mass spectrometer using reverse phase C18 columns. Various gradients and run times were selected in order to best characterize the compounds. Mobile phases were based on ACN/water or MeOH/water gradients and contained either 0.1% formic acid (methods indicated as FA) or 10 mM ammonium acetate (methods indicated as AA). One example of a solvent gradient that was used was 100% mobile phase A (mobile phase A = 99% water + 1% ACN + 0.1% formic acid) to 100% mobile phase B (mobile phase B = 95% ACN + 5% water + 0.1% formic acid) at a flow rate of 1 mL/min for a 16.5 min run.

In some cases, LCMS spectra were recorded on an Agilent 1290 Infinity UPLC system connected to an Agilent 6130 mass spectrometer, a Waters Acquity UPLC system connected to a Waters Acquity SQ mass spectrometer, or an Agilent 1100 Series HPLC system connected to a Waters Micromass ZQ mass spectrometer using reverse phase C18 columns. Various gradients and run times were selected in order to best characterize the compounds. Mobile phases were based on ACN/water or MeOH/water gradients and contained either 0.1% formic acid (methods indicated as FA) or 10 mM ammonium acetate (methods indicated as AA). One example of a solvent gradient that was used was 95% mobile phase A (mobile phase A = 99% water + 1% ACN + 0.1% formic acid) to 100% mobile phase B (mobile phase B = 95% ACN + 5% water + 0.1% formic acid) at a flow rate of 0.5 mL/min for a 5 min run.

### Preparative HPLC:

Preparative HPLC are conducted using Phenomenex 5 micron C5 21.2x250 mm eluting with solvent A (99% 10mM ammonium acetate /1% acetonitrile) / solvent B (5% 10mM ammonium acetate / 95% Acetonitrile) gradients using a Gilson instrument operated by 322 pumps with the UV/visible 155 detector triggered fraction collection set to from 200 nm to 400 nm. Mass gated fraction collection is conducted on an Agilent 1100 LC/MSD instrument.

One of ordinary skill in the art will recognize that modifications of the gradient, column length, and flow rate are possible and that some conditions may be more suitable for compound characterization than others, depending on the chemical species being analyzed.

### Preparative SFC:

Preparative SFC is conducted using 10, 20 or 30mm x 250mm ChiralPak columns (typically IA, IB, IC, ID, IE and IF), 10 or 20 mm x 250 mm Phenomenex Lux Cellulose-4, or 2-ethylpyridine columns eluting with appropriate percentages of supercritical carbon dioxide and alcohol containing 0.3% diethyl amine or 0.3% TEA or 0.3% formic acid or without any acid or base additives. Isocratic conditions with flow rates in the range of 10-100 mL/min and a column temperature of 40 °C are typical. Preparative SFC is conducted on A Jasco SFC prep purification system with UV/visible triggered fraction collection set to from 200 nm to 400 nm and back pressure regulation set to 10 MPa.

One of ordinary skill in the art will recognize that modifications of the gradient, column length, and flow rate are possible and that some conditions may be more suitable for compound characterization than others, depending on the chemical species being analyzed.

### HPLC conditions for Example B:

HPLC analysis was conducted using SHIMADZU LC-2010C with SHISEIDO 3 micron C18 4.6x75 mm eluting with solvent A (80% water/ 10% 50mM ammonium acetate/ 10% acetonitrile) / solvent B (10% 50mM ammonium acetate / 90% acetonitrile) gradients, flow rate at 1 mL/min, column temperature at 40 °C and UV detection at 254 nm.

### LC/MS/MS conditions for Example B:

LC/MS/MS analysis was conducted using Shimadzu UltraFast liquid chromatography and autosampler connected with AB SCIEX 4500 or AB SCIEX 5500 Triple Quad Mass spectrometry, or equivalent system.

For the analysis of Compound No.14 and Compound I-5c, 10 mM ammonium acetate in 99% water and 1% acetonitrile or 0.1% formic acid in water was prepared as mobile phase A solvent, and 10 mM ammonium acetate in 5% water and 95% acetonitrile or 0.1% formic acid in 5% water and 95% acetonitrile was used as mobile phase B solvent. The liquid chromatography was run at 1.5 mL/min for 3 minutes in gradient. The HPLC column was Waters Xselect C18 CSH 3.5 micron column with 2.1 mm ID x 30 mm length, and the column temperature was room temperature.

For the analysis of Compound CP-1, 10 mM ammonium acetate in 99% water and 1% acetonitrile was prepared as mobile phase A solvent, and 10 mM ammonium acetate in 5% water and 95% acetonitrile was made for mobile phase B solvent. The liquid chromatography was run at 1.5 ml/min for 3.5 minutes in gradient. The HPCL column was Osaka Soda Capcell PAK C1 UG120 5 micron, 2.0 mm I.D. x 35 mm length, and the column temperature was room temperature.

Analyst Software 1.7 was used to analyze MS/MS peak area and calculate the concentration of the samples.

### UPLC-CAD conditions:

UPLC-CAD analysis was conducted using Thermo Scientific^{™} Dionex^{™} Corona^{™} Veo^{™} RS (rapid separation) Charged Aerosol Detector and Waters ACQUITY UPLC system with ACE Excel C4 2 micron 2.1x100 mm or similar column eluting with gradient method using solvent A (10mM ammonium acetate in water, 2.6mL ammonium hydroxide in 4L) / solvent B (10mM ammonium acetate in methanol, 2.6mL ammonium hydroxide 95% in 4L) using flow rate at 0.8 mL/min and column temperature at 35 °C. Up to ±20% variation may result from the UPLC-CAD analysis.

### Example A1

### tert-butyl [4-({9-[(2R,5R,7R,8R,10R,12aR,14R,15R,15aR,16R)-15-fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-l][1,3,6,9,11,2,10]pentaoxadiphosphacyclotetradecin-14-yl]-9H-purin-6-yl}amino)-4-oxobutyl]methylcarbamate TEA salt, intermediate-1

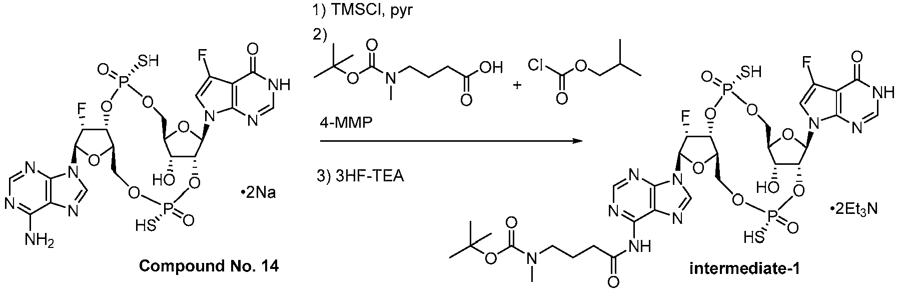

Compound No. 14 (300 mg, 0.40 mmol) was dissolved in dry pyridine and concentrated to dryness (5 mL x 3 times). The residue was dissolved in pyridine (6.41 mL) under an atmosphere of argon and treated with chlorotrimethylsilane (0.30 mL, 2.38 mmol) cooled with an ice-water bath. The reaction was allowed to warm up to rt and stirred for 2h to produce TMS protected Compound No. 14. Separately (after ~30 min of above reaction being stirred at rt), to an ice-water bath cooled solution of 4-((*tert*-butoxycarbonyl)(methyl)amino)butanoic acid (520.0 mg, 2.27 mmol) in tetrahydrofuran (10.7 mL) was added 4-methylmorpholine (0.28 mL, 2.50 mmol), followed by isobutyl chloroformate (0.29 mL, 2.27 mmol) in THF slowly. The cloudy suspension was allowed to warm up to rt and stirred for 1h and then added slowly to the above TMS protected intermediate via a syringe. The reaction mixture was stirred at rt overnight. MeOH (10 mL) was added into the above reaction mixture and the reaction mixture was concentrated. The residue was re-dissolved in MeOH (3.8 mL). Triethylamine trihydrofluoride (0.26 mL, 1.59 mmol) was added into the ice-water bath cooled reaction mixture and was allowed to warm up to rt and stirred for 30min. The reaction mixture was absorbed on diatomaceous earth (Celite^{®}) (1 g) and was purified by reverse phase flash column chromatography (0-50% ACN in aqueous triethylammonium acetate (10 mM)) to provide intermediate-1 as a TEA salt (214.0 mg, 48.5 %). LCMS (AA): *m*/*z* = 910.2 (M+H). ¹H NMR (D₂O) δ 8.55 (s, 1H), 8.20 (s, 1H), 7.87 (s, 1H), 7.14 (d, *, J* = 4.0 Hz, 1H), 6.42 (d, *J* = 16.0 Hz, 1H), 6.29 (d, *J =* 8.0 Hz, 1H), 5.5d (dd, *J =* 4.0, 52.0 Hz, 1H), 5.04-4.93 (m, 1H), 4.91-4.86 (m, 1H), 4.66 (d, *J =* 4.0 Hz, 1H), 4.46 (d, *J =* 8.0 Hz, 1H), 4.33-4.25 (m, 3H), 4.14 (dd, *J* = 4.0, 12.0 Hz, 1H), 3.94 (dd, *J =* 4.0, 8.0 Hz, 1H), 3.25 (br, m, 2H), 3.05 (q, *J =* 8.0 Hz, 15H), 2.73 (s, 3H), 2.59-2.54 (m, 1H), 1.90-1.82 (m, 1H), 1.20 (s, 9H), 1.13 (t, *J =* 8.0 Hz, 22H). ³¹P NMR (D₂O) δ 55.46 (s, 1P), 52.06 (s, 1P). ¹⁹F NMR (D₂O) δ -164.19 (s, br, 1F), -200.75 to -200.99 (m, 1F).

### Example A2

### [(2R)-3-[Hydroxy-[2-[[5-[[(1S)-1-[[(1S)-2-[4-(hydroxymethyl)anilino]-1-methyl-2-oxoethyl]carbamoyl]-2-methyl-propyl]amino]-5-oxo-pentanoyl]amino]ethoxy]phosphoryl]oxy-2-octadecanoyloxy-propyl] octadecanoate, intermediate-2

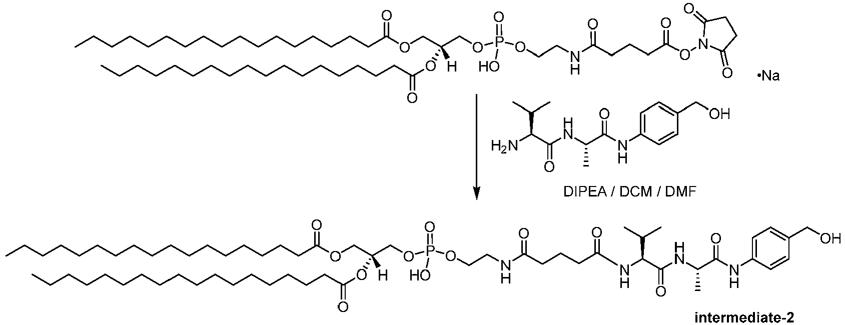

(2*S*)-2-Amino-N-[(1*S*)-2-[4-(hydroxymethyl)anilino]-1-methyl-2-oxo-ethyl]-3-methylbutanamide (112.1 mg, 0.38 mmol) was suspended in toluene in a vial and evaporated to dry (1mL x 3). The residue was dissolved in DMF (1.5 mL) and treated with DIPEA (88.9 uL, 0.51 mmol). The DMF solution was then added into sodium [(2R)-2,3-di(octadecanoyloxy)propyl] 2-[[5-(2,5-dioxopyrrolidin-1-yl)oxy-5-oxo-pentanoyl]amino]ethyl phosphate (250.0 mg, 0.25 mmol) in DCM (15 mL). The reaction mixture was stirred at rt for 4h. Most of the solvent (DCM) was evaporated and the residual solution (DMF) was poured into ice-cold HCl aqueous solution (0.1 N, 5mL)). The solid was collected by filtration and washed with water (3 mL x 3) and DCM (3 mL x 3) and dried under vacuum to give intermediate-2 (247.0 mg, 85.2 %). LCMS (AA): *m*/*z* = 1137.8 (M+H). ¹H NMR (MeOD/CDCl₃) δ 7.55 (buried inside CDCl₃ peak, 2H), 7.29 (d, , *J* = 8.0 Hz, 1H), 5.25-5.22 (m, 1H), 4.53-4.49 (buried inside MeOD residue water peak, 3H), 4.40 (dd, *J =* 4.0, 12.0 Hz, 1H), 4.19-4.00 (m, 6 H), 3.49-3.39 (m, 2H), 2.35-2.30 (m, 6H), 2.22 (t, *J=* 8.0 Hz, 2H), 2.17-2.09 (m, 1H), 1.97-1.89 (m, 2H), 1.65-1.58 (m, 4H), 1.45 (d, *J=* 8.0 Hz, 3H), 1.35-1.28 (br, m, 56H), 0.99-0.97 (m, 6H), 0.89-0.86 (6H). ³¹P NMR (MeOD/CDCl₃) δ 0.77 (s, 1P).

### Example A3

### [(2R)-3-[hydroxy-[2-[[5-[[(1S)-2-methyl-1-[[(1S)-1-methyl-2-[4-[(4-nitrophenoxy)carbonyloxymethyl]anilino]-2-oxo-ethyl]carbamoyl]propyl]amino]-5-oxo-pentanoyl]amino]ethoxy]phosphoryl]oxy-2-octadecanoyloxy-propyl] octadecanoate, intermediate-3

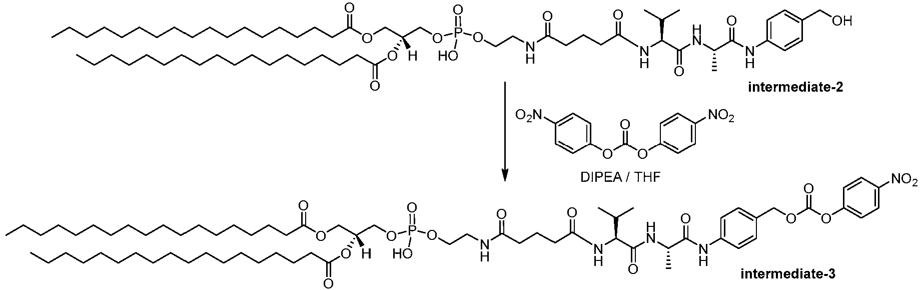

To a vial was added intermediate-2 (247.0 mg, 0.22 mmol), THF (4.0 mL) and DIPEA (83.3 µL, 0.48 mmol). The solution was cooled with ice-water bath under N₂ atmosphere and then bis(4-nitrophenyl)carbonate (97%, 74.9 mg, 0.24 mmol) was added in one portion. The pale yellow solution was stirred at 50 °C for 1h. Additional bis(4-nitrophenyl)carbonate (97%, 145.3 mg, 0.48 mmol) was added and stirred at 50 °C for 1h and then rt overnight. The reaction mixture was poured into ice-cold HCl aqueous solution (0.05 N, 30 mL). Collected the solid by filtration and washed with water (10 mL x 3) and EtOAc (8 mL x 6) to give a solid intermediate-3 (282.8 mg, 79.5 %). LCMS (FA): *m*/*z* = 1302.8 (M+H). ¹H NMR (MeOD/CDCl₃) δ 8.28 (d, *J=* 8.0 Hz, 1H), 7.65 (d, *J =* 8.0 Hz, 1H), 7.42-7.38 (m, 4H), 5.25-5.22 (m, 3H), 4.53-4.49 (buried inside MeOD residue water peak, 1H), 4.38 (dd, *J* = 4.0, 12.0 Hz, 1H), 4.19-4.02 (m, 6 H), 3.61-3.387 (m, 2H), 2.35-2.29 (m, 6H), 2.22-2.20 (m, 2H), 2.17-2.08 (m, 1H), 1.98-1.90 (m, 2H), 1.64-1.56 (m, 4H), 1.46 (d, *J* = 8.0 Hz, 3H), 1.35-1.28 (br, m, 56H), 0.99-0.97 (m, 6H), 0.89-0.85 (6H). ³¹P NMR (MeOD/CDCl₃) δ 0.74 (s, 1P).

### Example A4

### (2S,5S,19R)-2-({4-[({[4-({9-[(2R,5R,7R,8R,10R,12aR,14R,15R,15aR,16R)-15-fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-l][1,3,6,9,11,2,10]pentaoxadiphosphacyclotetradecin-14-yl]-9H-purin-6-yl}amino)-4-oxobutyl](methyl)carbamoyl}oxy)methyl]phenyl}carbamoyl)-16-hydroxy-5-isopropyl-16-oxido-4,7,11-trioxo-19-(stearoyloxy)-15,17-dioxa-3,6,12-triaza-16lambda~5~-phosphaicosan-20-yl octadecanoate, CP-1

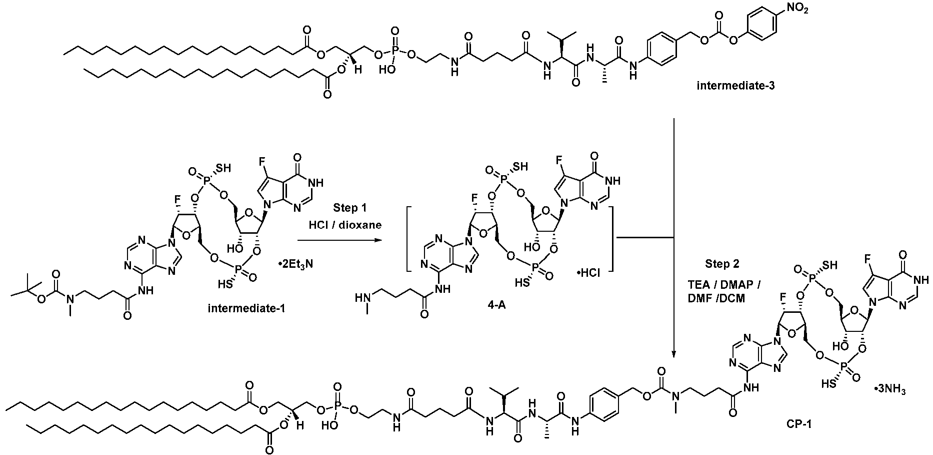

Intermediate-1 (15.0 mg, 0.013 mmol) was dissolved in 1,4-dioxane (0.29 mL) and treated with 4M HCl in 1,4-dioxane (0.15 ml, 0.6 mml) at rt for 1h. The volatile was removed and dried under vacuum for 1h to give 4-A as a crude product directly used for the next step. Separately, a vial was charged with intermediate-3 (17.6 mg, 0.013 mmol), DMAP (3.3 mg, 0.027 mmol), TEA (0.015 ml, 0.11 mmol) and DCM (0.35 mL). To this mixture was added a solution of 4-A in DMF (0.42 mL) slowly. The reaction was stirred at rt for 15min and concentrated to a small volume (~0.5 mL). The crude compound was purified by preparative HPLC (Phenomenex 5 micron C5 21.2x250 mm eluting with solvent A [99% 10mM ammonium acetate /1% Acetonitrile] / solvent B [5% 10mM ammonium acetate / 95% Acetonitrile] from 60 % B to 100 % B in 16 min at a flow rate of 20 mL / min ) to provide CP-1 as the ammonium salt (11.0 mg, 40.3 %). LCMS (AA): *m*/*z* = 1972.8 (M+H). ¹H NMR (MeOD) δ 8.55 (br, s, 1H), 8.28 (br, s, 1H), 7.75 (br, s, 1H) 7.52-7.42 (m, 2H), 7.24-7.17 (m, 3H), 6.43-6.36 (m, 2H), 5.60 (dd, *J* = 4.0 and 52.0 Hz, 1H), 5.17-5.12 (m, 1H), 5.10-5.00 (m, 1H), 4.99 (s, 2H), 4.95-4.89 (m, 1H), 4.78-4.76 (buried inside MeOD residue water peak, 1H), 4.48-4.28 (m, 6H), 4.23 (m, 1H), 4.13-4.09 (m, 2H), 3.96-3.82 (m, 5H), 3.42-3.33 (m, 4H), 2.91 (s, 3H), 2.73-2.64 (m, 2H), 2.31-2.16 (m, 8H), 2.11-2.03 (m, 1H), 1.99-1.92 (m, 2H), 1.89-1.82 (m, 2H), 1.57-1.47 (m,4H), 1.49 (d, *J =* 8.0 Hz, 3H), 1.27-1.28 (br, 56H), 0.93-0.91 (m, 6H), 0.85-0.81 (m, 6H). ³¹P NMR (MeOD) δ 57.00 (s, 1P), 52.26 (s, 1P), -0.20 (s, 1P). ¹⁹F NMR (MeOD) δ -165.24 to -165.46 (s, br, 1F), - 202.98 to -203.24 (m, 1F). HRMS (C₈₈H₁₃₈F₂N₁₃O₂₅P₃S₂) Expected: 1972.8622; Observed: 1972.8674

### Example A5

### 2-[(tert-butoxycarbonyl)(methyl)amino]ethyl {9-[(1R,3R,6R,8R,9R,10R,12R,15R,17R,18R)-9-fluoro-17-(5-fluoro-4-oxo-3,4-dihydro-7H-pyrrolo[2,3-d]pyrimidin-7-yl)-18-hydroxy-3,12-dioxido-3,12-disulfanyl-2,4,7,11,13,16-hexaoxa-3,12-diphosphatricyclo[13.2.1.0~6,10~]octadec-8-yl]-9H-purin-6-yl} carbamate, Intermediate-4

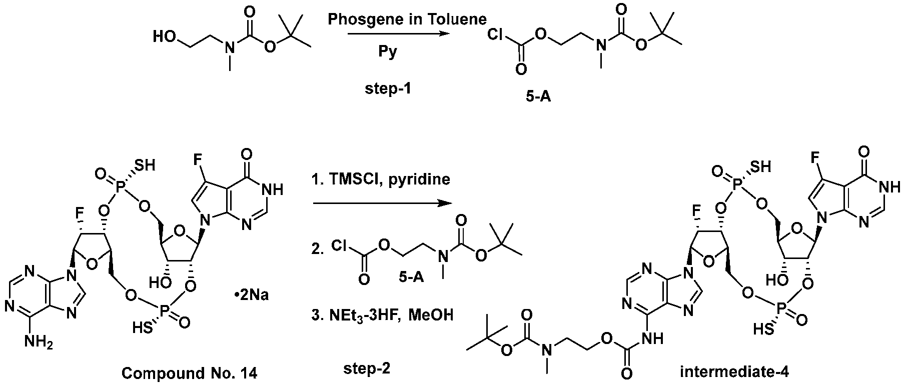

### Step 1: 2-[tert-butoxycarbonyl(methyl)amino]ethyl carbonochloridate (5-A)

*tert*-butyl (2-hydroxyethyl)(methyl)carbamate (1.40 g, 7.99 mmol) in DCM (100 ml) with Pyridine (1.26 mL) was added slowly to a 15% phosgene solution in toluene (16 ml, 22.4 mmol) at -78°C. The reaction mixture was then warmed to rt and stirred for 1.5 h. The solvent was then concentrated to dryness to provide crude 5-A (1.90 g, 100%).

### Step 2: 2-[(tert-butoxycarbonyl)(methyl)amino]ethyl {9-[(1R,3R,6R,8R,9R,10R,12R,15R,17R,18R)-9-fluoro-17-(5-fluoro-4-oxo-3,4-dihydro-7H-pyrrolo[2,3-d]pyrimidin-7-yl)-18-hydroxy-3,12-dioxido-3,12-disulfanyl-2,4,7,11,13,16-hexaoxa-3,12-diphosphatricyclo[13.2.1.0~6,10~]octadec-8-yl]-9H-purin-6-yl}carbamate, intermediate-4

Compound No. 14 (313 mg, 0.40 mmol) was dissolved in dry pyridine and concentrated to dryness (3 x 5 mL). The residue was dissolved in pyridine (6.70 mL) under an atmosphere of argon and treated with chlorotrimethylsilane (0.378 mL, 2.97 mmol) cooled with an ice-water bath. The reaction was allowed to warm up to rt and stirred for 20 mins to produce TMS protected Compound No. 14 in solution. **5-A** (1.90 g, 7.52 mmol) was dissolved in DCM (12.5mL) and was then added to the above mentioned solution via syringe. The reaction mixture was stirred at rt under an argon atmosphere for 16 h. The reaction mixture was then concentrated to dryness. MeOH (10 mL) and ammonium hydroxide (28-30% solution in water, 10 mL) were added to the residue and allowed to stir for 30 min. The reaction mixture was concentrated to dryness and the residue was dissolved in MeOH (15 mL). Triethylamine trihydrofluoride (0.12 mL, 0.75 mmol) was added and the reaction mixture was stirred at rt for 30 min. The reaction mixture was concentrated to dryness and the crude residue was adsorbed onto Celite and purified by reverse phase flash column chromatography (0-50% ACN in aqueous triethylammonium acetate (10 mM)) to provide intermediate-4 as the TEA salt (181 mg, 39.3 %). LCMS (AA): *m*/*z* = 912.3 (M+H). ¹H NMR (400 MHz, DMSO-d6) δ 12.06(br, s, 1H), 10.56 (br, s, 1H), 9.16 (br, 2H), 8.62 (s, 1H), 8.52 (s, 1H), 7.89(d, *J =* 4.0 Hz, 1H), 7.63 (s, 1H), 6.35 (dd, *J =* 4.0 and 12.0 Hz, 1H), 6.26 (d, *J* = 8.0 Hz, 1H), 5.80 (d, *J =* 52.0 Hz, 1H), 5.19-5.14 (m, 1H), 4.95-4.93 (m, 1H), 4.79-4.78 (m, 1H), 4.56 (br, s, 1H), 4.33 (br, s, 1H), 4.21-4.12 (m, 5H), 4.00-3.90 (m, 1H), 3.72-3.69 (m, 1H), 3.45-3.43 (m, 2H), 3.05 (br, 12H), 2.86-2.83 (m, 3H), 1.34 (br, 9H), .115-1.11(m, 18H). ³¹P NMR (DMSO-d6) δ 54.04 (s, 1P), 47.46 (s, 1P). ¹⁹F NMR (DMSO-d6) δ - 165.06 (s, br, 1F), -207.23 to -207.54 (m, 1F)

### Example A6

### (2S,5S,19R)-2-{[4-({[{2-[({9-[(2R,5R,7R,8R,10R,12aR,14R,15R,15aR,16R)-15-fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-l][1,3,6,9,11,2,10]pentaoxadiphosphacyclotetradecin-14-yl]-9H-purin-6-yl}carbamoyl)oxy]ethyl}(methyl)carbamoyl]oxy}methyl)phenyl]carbamoyl}-16-hydroxy-5-isopropyl-16-oxido-4,7,11-trioxo-19-(stearoyloxy)-15,17-dioxa-3,6,12-triaza-16lambda~5~-phosphaicosan-20-yl octadecanoate, CP-2

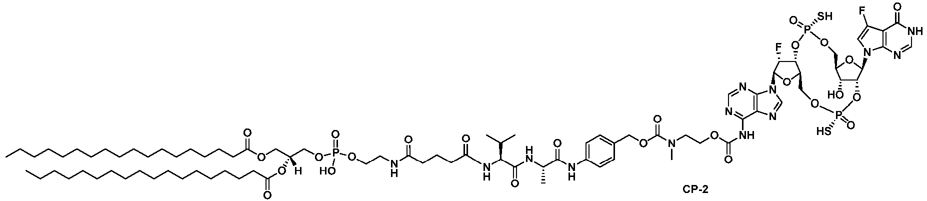

The title compound was prepared using similar procedure as shown in Example A4 using intermediate-4 (77.0 mg, 0.069 mmol) in place of intermediate-1 to obtain CP-2 as the ammonium salt (42.0 mg, 30.0 %). LCMS (AA): m/z = 1974.6 (M+H). 1H NMR (MeOD) δ 8.52 (br, d, J = 12.0 Hz, 1H), 8.33 (br, d, J = 8.0 Hz, 1H), 7.74 (s, 1H), 7.50 (br, d, *J* = 8.0 Hz 1H), 7.40 (br, d, *J =* 8.0 Hz, 1H), 7.28 (br, 1H), 7.24 (br, d, *J =* 8.0 Hz, 1H), 7.14 (br, d, *J* = 8.0 Hz, 1H), 6.43-6.34 (m, 2H), 5.56 (d, *J =* 52.0 Hz, 1H), 5.17-5.06 (m, 2H), 5.01-4.93 (m, 3H), 4.78-4.76 (buried inside MeOD residue water peak, 1H), 4.47-4.29 (m, 8H), 4.21 (m, 1H), 4.13-4.09 (dd, *J =* 8.0 and 12.0 Hz, 2H), 3.95-3.82 (m, 5H), 3.63-3.58 (m, 2H), 3.33 (br, d, *J=* 8.0 Hz, 2H), 2.99 (s, 3H), 2.30-2.22 (m, 6H), 2.17 (t, d, *J =* 8.0 Hz, 2H), 2.10-2.04 (m, 1H), 1.88-1.81 (m, 2H), 1.56-1.48 (m,4H), 1.49 (br, d, *J* = 8.0 Hz, 3H), 1.27-1.21 (br, m, 56H), 0.93-0.90 (m, 6H), 0.84-0.81 (m, 6H). ³¹P NMR (DMSO-d6) δ 53.64 (s, 1P), 46.87 (s, 1P), -0.45 (s, 1P). ¹⁹F NMR (MeOD) δ -165.49 to -165.66 (m, 1F), -203.23 to -203.70 (m, 1F). HR-MS (C₈₇H₁₃₆F₂N₁₃O₂₆P₃S₂) Expected : 1974.8415; Observed: 1974.8455.

### Example A7

### [4-(hydroxymethyl)phenyl] 4-(aminomethyl)benzoate, intermediate-5

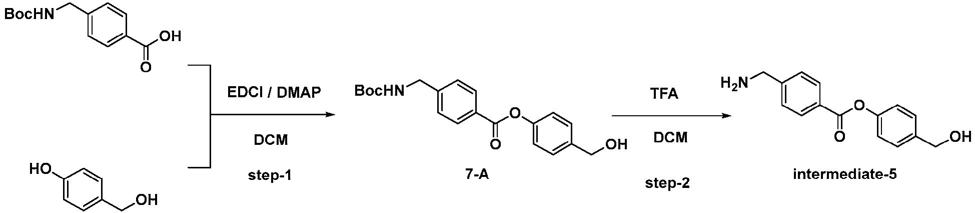

### Step 1: [4-(hydroxymethyl)phenyl] 4-[(tert-butoxycarbonylamino)methyl]benzoate

A 100 mL round bottom flask was charged with 4-(Boc-aminomethyl)benzoic acid (503.0 mg, 1.98 mmol), 4-hydroxybenzyl alcohol (97%, 304.3 mg, 2.38 mmol). The mixture was then suspended in DCM (15 mL) and treated with 1-(3-dimethylaminopropyl)-3-ethylcarbodiimide (97%, 634.3 mg, 3.96 mmol). The reaction was maintained at rt for 2h and was washed with ice-cold HCl (0.1N, 10mL), followed by water (10 mL) and sat. NaHCO₃ (10 mL) and dried and purified by silica gel chromatography (0-100% EtOAc/Hexanes) to give **7-A** (385.0 mg, 54.4 %). LCMS (AA): *m*/*z* = 356.00 (M-H). ¹H NMR (DMSO-d6) δ 8.09 (d, *J* = 8.0 Hz, 1H), 7.53 (t, *J* = 4.0 Hz, 1H), 7.47-7.39 (m, 4H), 7.22 (d, *J* = 8.0 Hz, 1H), 5.25 (t, *J* = 8.0 Hz, 1H), 4.53 (d, *J* = 8.0 Hz, 2H), 4.24 (br, d, *J* = 4.0 Hz, 2H), 1.41 (s, 9 H).

### Step 2: [4-(hydroxymethyl)phenyl] 4-(aminomethyl)benzoate, intermediate-5

[4-(hydroxymethyl)phenyl] 4-[(tert-butoxycarbonylamino)methyl]benzoate 7-A (100.0 mg, 0.28 mmol) was dissolved in DCM (2 mL) and treated with TFA (0.8 mL, 10 mmol) at rt for 30min. The volatile was removed by rotary evaporation and the residue was re-dissolved in MeOH (2 mL) and kept at 37 °C for 90 min. After completely removed the volatile, the residue was co-evaporated with MeOH (2 mL x 2) and DCM (4 mL x 2) to give intermediate-5 as the TFA salt (110.0 mg, quant. yield). LCMS (AA): *m*/*z* = 258.20 (M+H). ¹H NMR (DMSO-d6) δ 8.37 (br, 3H), 8.19 (d, *J* = 8.0 Hz, 2H), 7.68 (d, *J=* 8.0 Hz, 2H), 7.42 (d, *J* = 8.0 Hz, 2H), 7.24 (d, *J* = 8.0 Hz, 2H), 4.54 (s, 2H), 4.20 (br, s, 2H).

### Example A8

### (2R)-3-((Hydroxy(2-(5-((4-((4-(hydroxymethyl)phenoxy)carbonyl)benzyl)amino)-5-oxopentanamido)ethoxy)phosphoryl)oxy)propane-1,2-diyl distearate, intermediate-6

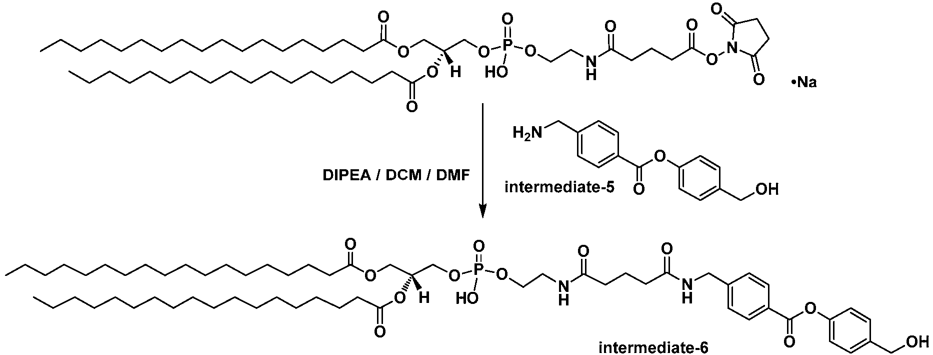

[4-(hydroxymethyl)phenyl] 4-(aminomethyl)benzoate trifluoroacetic acid salt (64.1 mg, 0.17 mmol) was dissolved in DMF (0.8 mL). The solution was added into the other solution of sodium [(2*R*)-2,3-di(octadecanoyloxy)propyl] 2-[[5-(2,5-dioxopyrrolidin-1-yl)oxy-5-oxo-pentanoyl]amino]ethyl phosphate (113.0 mg, 0.115 mmol) in DCM (3.2 mL). Then DIPEA (0.04 mL, 0.23 mmol) was added into above mixture and the stirred for 3.5 h at rt. Most of the solvent (DCM) was evaporated and the residual solution (DMF) was poured into ice cold HCl solution (0.1 N, 5mL). The solid was collected by filtration and washed with ice cold HCl solution (0.1 N, 4mL x 6) and water (3 mL x 6) and dried under vacuum to give intermediate-6 (106.0 mg, 83.6 %). LCMS (AA): weak signal. ¹H NMR (MeOD/CDCl₃) δ 8.03 (d, *J* = 8.0 Hz, 2H), 7.31 (d, *J* = 8.0 Hz, 4H), 7.06 (d, *J =* 8.0 Hz, 2H), 5.13-5.10 (m, 1H), 4.55 (s, 2H), 4.37 (s, 2H), 4.07-4.05 (m, 1H), 4.07-3.93 (m, 5H), 3.37-3.34 (br, 2H), 2.24-2.13 (m, 8H), 1.86-1.82 (m, 2H), 1.49 (br, 4H), 1.14 (br, 56H), 0.78 (m, 6H). ³¹P NMR (MeOD/CDCl₃) δ 3.06 (s, 1P).

### Example A9

### (2R)-3-((Hydroxy(2-(5-((4-((4-((((4-nitrophenoxy)carbonyl)oxy)methyl)phenoxy)carbonyl)benzyl)amino)-5-oxopentanamido)ethoxy)phosphoryl)oxy)propane-1,2-diyl distearate, intermediate-7

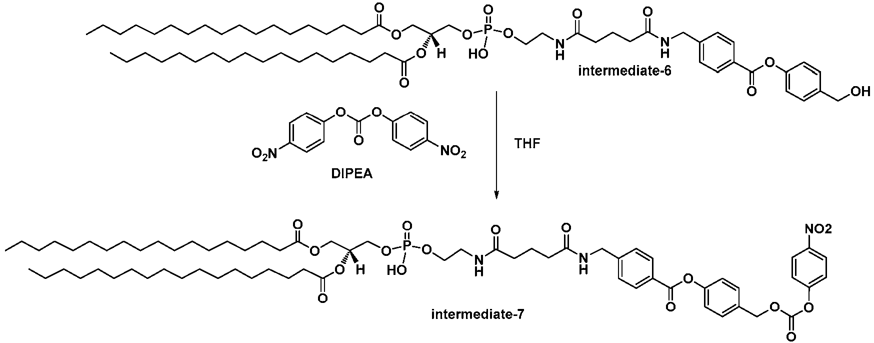

Intermediate-6 (106 mg, 0.096 mmol) was dissolved in THF (2 mL) and treated with DIPEA (0.037 mL, 0.21 mmol). To this solution was added bis(4-nitrophenyl)carbonate (64.4 mg, 0.21 mmol). The reaction mixture was kept at rt for 1 h and then additional bis(4-nitrophenyl)carbonate (64.4 mg, 0.21 mmol) and DIPEA (0.037 mL, 0.21 mmol) were added. The reaction was heated at 50°C for another 1h. The reaction mixture was added into ice-cold HCl solution (0.05 N, 100 mL). The precipitate was collected by filtration and washed with water (2 mL x4). The solid was suspended in EtOAc (3 mL) and sonicated for 5 sec. and filtrated. Washed the solid with EtOAc (2 mL x 3) to obtain solid intermediate-7 (121.9 mg, 57.4%). LCMS (AA): weak signal. ¹H NMR (MeOD/CDCl₃) δ 8.26 (d, *J =* 8.0 Hz, 2H), 8.12 (d, *J* = 8.0 Hz, 2H), 7.50 (d, *J =* 8.0 Hz, 4H), 7.40 (d, *J =* 8.0 Hz, 2H), 7.37 (d, *J =* 8.0 Hz, 2H), 7.23 (d, *J* = 8.0 Hz, 2H), 5.29 (s, 2H), 5.21-5.18 (m, 1H), 4.48 (s, 2H), 4.31 (dd, *J* = 4.0 and 12.0 Hz,1H), 4.14-4.01 (m, 5H), 3.45 (br, t, *J* = 4.0 Hz, 2H), 2.32-2.23 (m, 8H), 1.96-1.92 (m, 2H), 1.57 (br, 4H), 1.22 (br, 56H), 0.84 (m, 6H).

### Example A10

### 4-({[{2-[({9-[(2R,5R,7R,8R,10R,12aR,14R,15R,15aR,16R)-15-fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-l][1,3,6,9,11,2,10]pentaoxadiphosphacyclotetradecin-14-yl]-9H-purin-6-yl}carbamoyl)oxy]ethyl}(methyl)carbamoyl]oxy}methyl)phenyl 4-[(15R)-12-hydroxy-12-oxido-3,7,18-trioxo-15-(stearoyloxy)-11,13,17-trioxa-2,8-diaza-12lambda~5~-phosphapentatriacont-1-yl]benzoate, CP-3

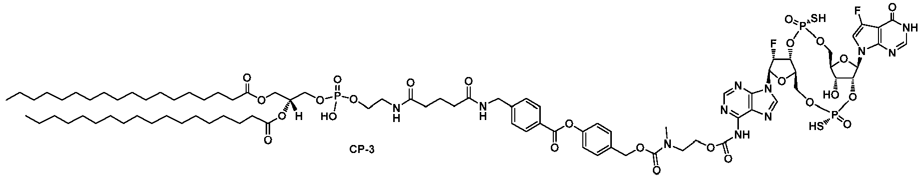

The title compound was prepared using similar procedure as shown in Example A4 using intermediate-7 (8.0 mg, 0.0072 mmol) in place of intermediate-3 and intermediate-4 (9.1 mg, 0.0072 mmol) in place of intermediate-1 to obtain CP-3 as the ammonium salt (4.2 mg, 29.0 %). LCMS (AA): *m*/*z* = 1939.1 (M+H). ¹H NMR (MeOD) δ 8.65-8.59 (m, 1H), 8.42 (br, d, *J* = 16.0 Hz, 1H), 8.14 (d, *J* = 8.0 Hz, 2H), 7.85 (s, 1H), 7.74 (s, 1H), 7.51 (d, *J* = 8.0 Hz, 2H), 7.48-7.39 (m, 3H), 7.20 (br, d, *J* = 8.0 Hz, 1H), 7.11 br, d, *J* = 8.0 Hz, 1H), 6.50 (dd, *J* = 1.2 and 8.0 Hz, 1H), 6.42 (br, d, *J =* 8.0 Hz, 1H), 5.80-5.61 (m, 1H), 5.27-5.10 (m, 4H), 5.08-5.0 (m, 1H), 4.82 (m, 1H), 4.55-4.32 (m, 9H), 4.30 (br, 1H), 4.04-3.98 (m, 3H), 3.96-3.91 (m, 2H), 3.83-3.65 (m, 2H), 3.44 (t, *J* = 4.0 Hz, 2H), 3.10 (br, d, *J* = 12.0 Hz, 3H), 2.38-2.28 (m, 8H), 2.02-1.94 (m, 2H), 1.65-1.57 (m, 4H), 1.30 (br, 56H), 0.93-0.90 (m, 6H). ³¹P NMR (MeOD) δ 57.08 (s, 1P), 52.51 (s, 1P), 0.36 (s, 1P). ¹⁹F NMR (MeOD) δ -165.58 to -165.67 (m, 1F), -203.66 to -203.99 (m, 1F) HR-MS (C₈₇H₁₂₈F₂N₁₁O₂₆P₃S₂) Expected : 1938.7727; Observed: 1938.7713

### Example A11

### 4-{[(2S)-2-({(2S)-2-[(tert-butoxycarbonyl)amino]-3-methylbutanoyl}amino)propanoyl]amino}benzyl [2-({9-[(2R,5R,7R,8R,10R,12aR,14R,15R,15aR,16R)-15-fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-l][1,3,6,9,11,2,10]pentaoxadiphosphacyclotetradecin-14-yl]-9H-purin-6-yl}carbamoyl)benzyl]methylcarbamate, intermediate-8

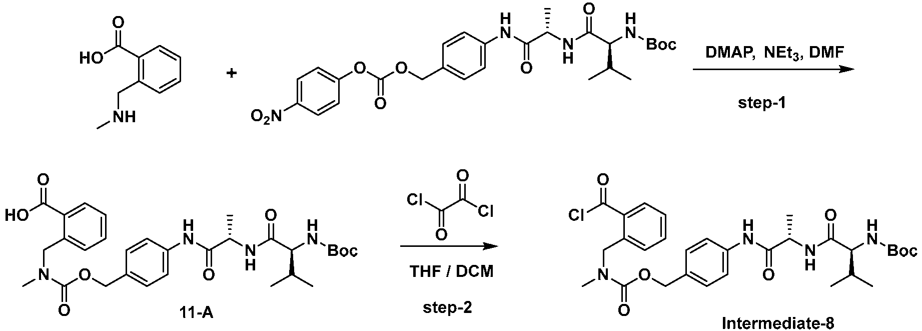

### Step 1: 2-[[[4-[[(2S)-2-[[(2S)-2-(tert-Butoxycarbonylamino)-3-methyl-butanoyl]amino]propanoyl]amino]phenyl]methoxycarbonyl-methyl-amino]methyl]benzoic acid, 11-A

The title compound was prepared following the procedure described in Example A4, step 2, starting with 2-[(methylamino)methyl]benzoic acid HCl (150 mg, 0.72 mmol) in place of de-Boc intermediate-1. Following purification by silica gel chromatography (0-25% MeOH/DCM) gave 11-A (306 mg, 67%). LCMS (AA): *m*/*z* = 583.4 (M-H).

### Step 2: [4-[[(2S)-2-[[(2S)-2-(tert-Butoxycarbonylamino)-3-methyl-butanoyl]amino]propanoyl]amino]phenyl]methyl N-[(2-chlorocarbonylphenyl)methyl]-N-methyl-carbamate, intermediate-8

To a solution of 11-A (295 mg, 0.50 mmol) in THF (1.5 mL) cooled to 0 °C was added oxalyl chloride (2.0 M solution in DCM, 0.25 mL, 0.50 mmol) followed by 3 drops of DMF. The reaction mixture was allowed to stir at 0 °C for 45 min. The mixture was then concentrated to dryness to provide crude intermediate-8 directly used for the next step (304 mg, 100%).

### Example A12

### 4-{[(2S)-2-({(2S)-2-[(tert-butoxycarbonyl)amino]-3-methylbutanoyl}amino)propanoyl]amino}benzyl [2-({9-[(2R,5R,7R,8R,10R,12aR,14R,15R,15aR,16R)-15-fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-l][1,3,6,9,11,2,10]pentaoxadiphosphacyclotetradecin-14-yl]-9H-purin-6-yl}carbamoyl)benzyl]methylcarbamate, intermediate-9

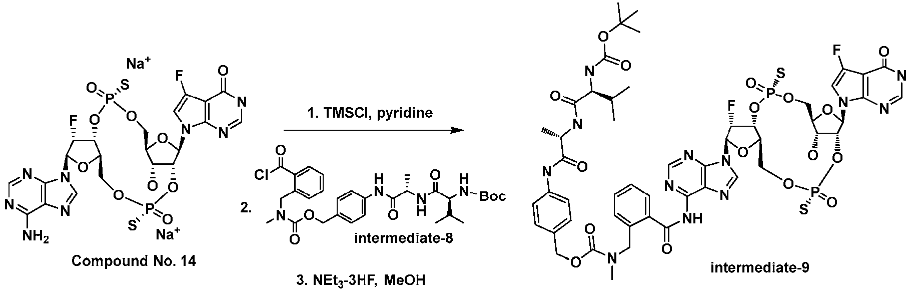

Compound No. 14 (107 mg, 0.117 mmol) was added into a round bottom flask (50mL) and co-evaporated with dry pyridine (2 mL x 3 times). The residue was then re-dissolved in dry pyridine (4.26 mL, 52.7 mmol) and treated with chlorotrimethylsilane (0.121 mL, 0.934 mmol) and stirred at rt for 30min. To the above solution was added intermediate-8 (423 mg, 0.70 mmol) in pyridine (1.89 mL, 23.4 mmol) and stirred overnight at rt. The reaction mixture was quenched with 10 ml MeOH and stirred for 30 mins, remove all solvent and ammonium hydroxide (28-30% solution in water, 10 mL) were added and allowed to stir for 30 min. The reaction mixture was concentrated to give a crude dark solid. The solid was re-dissolved in MeOH (1.42 mL) and treated with triethylamine trihydrofluoride (0.12 mL, 0.75 mmol) at 0 °C and allowed to warm up to rt for 30 min. The mixture was evaporated and purified by reverse phase flash column chromatography (0-50% ACN in aqueous ammonium bicarbonate (5 mM)) and further purified by preparative HPLC (Phenomenex 5 micron C5 21.2x250 mm eluting with solvent A (99% 10mM ammonium acetate /1% Acetonitrile) / solvent B (5% 10mM ammonium acetate / 95% Acetonitrile) from 60 % B to 100 % B in 16 min at a flow rate of 20 mL / min) to provide intermediate-9 as the ammonium salt. LCMS (AA): *m*/*z* = 1277.8 (M+H). ¹H NMR (MeOD) δ 8.68 (s, 1H), 8.44 (s, 1H), 7.82 (br, d, *J* = 8.0 Hz, 1H), 7.77 (s, 1H), 7.52-7.39 (m, 4H), 7.39 (s, 1H), 7.23-7.26 (m, 3H), 6.50-6.43 (m, 2H), 5.74 (dd, *J* = 4.0 and 52.0 Hz, 1H), 5.25-5.15 (m, 1H), 5.07-5.04 (m, 3H), 4.93-4.89 (m, 2H), 4.56-4.49 (m, 2H), 4.46-4.39(m, 3H), 4.30 (br, 1H), 4.03 (dd, *J* = 4.0 and12.0 Hz, 1H), 3.95 (d, *J* = 4.0 Hz, 1H), 2.95 (br, 3H), 2.13-2.11 (m, 1H), 1.48 (d, *J* = 8.0 Hz, 1H), 1.45 (s, 9H), 1.00 (d, *J* = 4.0 Hz, 3H), 0.94 (*J* = 4.0 Hz, 3H), ³¹P NMR (MeOD) δ 56.91 (s, 1P), 52.36 (s, 1P). ¹⁹F NMR (MeOD) δ -165.65 (s, 1F), -203.16 to -203.35 (m, 1F).

### Example A13

### (2S,5S,19S)-2-({4-[({[2-({9-[(2R,5R,7R,8R,10R,12aR,14R,15R,15aR,16R)-15-fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-l][1,3,6,9,11,2,10]pentaoxadiphosphacyclotetradecin-14-yl]-9H-purin-6-yl}carbamoyl)benzyl](methyl)carbamoyl}oxy)methyl]phenyl}carbamoyl)-16-hydroxy-5-isopropyl-16-oxido-4,7,11-trioxo-19-(stearoyloxy)-15,17-dioxa-3,6,12-triaza-16lambda~5~-phosphaicosan-20-yl octadecanoate, (CP-4)

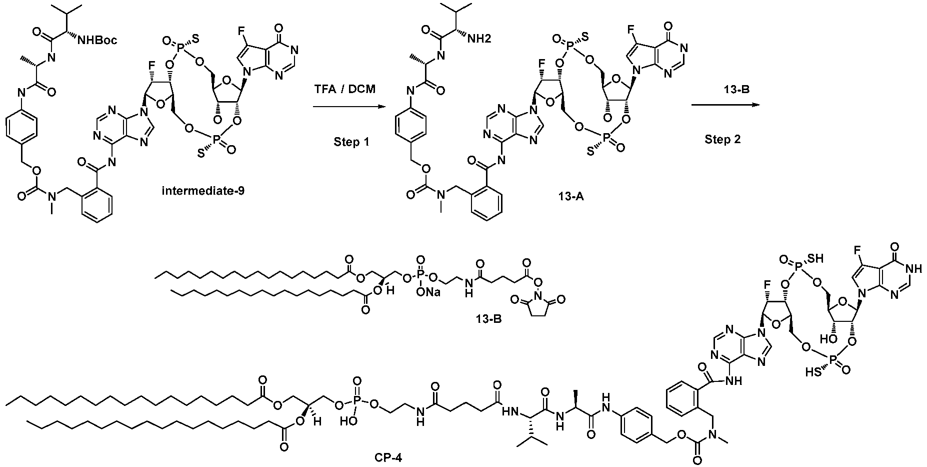

### Step 1: 4-{[(2S)-2-{[(2S)-2-amino-3-methylbutanoyl]amino}propanoyl]amino}benzyl [2-({9-[(2R,5R,7R,8R,10R,12aR,14R,15R,15aR,16R)-15-fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-1][1,3,6,9,11,2,10]pentaoxadiphosphacyclotetradecin-14-yl]-9H-purin-6-yl}carbamoyl)benzyl]methylcarbamate (13-A)

Intermediate-9 (56 mg, 0.045 mmol) was added to a round bottom flask and cooled to 0°C. A solution of TFA (0.24 mL, 3.2 mmol) and DCM (0.58 mL) was then added via syringe and the reaction was stirred at 0 °C for 30 min. The reaction mixture was then concentrated to dryness and placed under vacuum for 2 h to provide **13-A** as the TFA salt (56 mg, 100%). LCMS (AA): *m*/*z* = 1177.0 (M+H).

### Step 2: (2S,5S,19S)-2-({4-[({[2-({9-[(2R,5R,7R,8R,10R,12aR,14R,15R,15aR,16R)-15-fluoro-7-(5-fluoro-4-oxo-3,4-dihydro-7H-pyrrolo[2,3-d]pyrimidin-7-yl)-16-hydroxy-2,10-dioxido-2,10-disulfanyloctahydro-12H-5,8-methanofuro[3,2-l][1,3,6,9,11,2,10]pentaoxadiphosphacyclotetradecin-14-yl]-9H-purin-6-yl}carbamoyl)benzyl](methyl)carbamoyl}oxy)methyl]phenyl}carbamoyl)-16-hydroxy-5-isopropyl-16-oxido-4,7,11-trioxo-19-(stearoyloxy)-15,17-dioxa-3,6,12-triaza-16lambda~5~-phosphaicosan-20-yl octadecanoate, CP-4

To a solution of 13-A (56 mg, 0.037 mmol) and 13-B (40.0 mg, 0.041 mmol) in THF (1.4 mL) and DMF (0.7 mL) was added *N,N-*diisopropylethylamine (65 uL, 0.37 mmol) dropwise. The reaction mixture was allowed to stir at rt for 16 h. The reaction mixture was then concentrated to dryness and the crude residue was purified by preparative HPLC (Phenomenex 5 micron C5 21.2x250 mm eluting with solvent A (99% 10mM ammonium acetate /1% Acetonitrile) / solvent B (5% 10mM ammonium acetate / 95% Acetonitrile) from 60 % B to 100 % B in 16 min at a flow rate of 20 mL / min) to provide CP-4 as the ammonium salt (32.8 mg, 42%). LCMS (AA): ½ *m*/*z* = 1011.1 (M+H). ¹H NMR (MeOD) δ 8.78 (s, 1H), 8.74 (s, 1H), 7.90-7.88 (m, 1H), 7.78 (s, 1H), 7.64-7.46 (m, 4H), 7.36 (s, 1H), 7.33-7.13 (m, 3H), 6.54-6.48 (m, 2H), 5.79 (dd, *J* = 4.0 and 52.0 Hz, 1H), 5.26-5.13 (m, 2H), 5.05-4.99 (m, 3H), 4.84 (m, 2H buried in water peak), 4.78-4.77 (m, 1H), 4.59-4.36 (m, 6H), 4.30-4.29 (m, 1H), 4.22-4.17 (m, 2H), 4.03-3.92 (m, 5H), 3.43 (t, *J* = 4.0 Hz, 2H), 2.96 (s, 3H), 2.89-2.24 (m, 8H), 2.20-2.12 (m, 1H), 1.98-1.90 (m, 2H), 1.67-1.56 (m, br, 4H), 1.48 (d, *J* = 8.0 Hz, 3H), 1.30 (br, 56H), 1.02-0.99 (m, 6H), 0.93-0.90 (m, 6H). ³¹P NMR (MeOD) δ 57.47 (s, 1P), 52.87 (s, 1P), 0.12 (s, 1P). ¹⁹F NMR (MeOD) δ -165.91to -166.08 (m, 1F), -203.70 to -203.94 (m, 1F). HR-MS (C₉₂H₁₃₈F₂N₁₃O₂₅P₃S₂) Expected: 2020.8622, Observed: 2020.8601.

### Example A14

### 4-{[(2S)-2-({(2S)-2-[(tert-Butoxycarbonyl)amino]-3-methylbutanoyl}amino)propanoyl]amino}benzyl [2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl]methylcarbamate, intermediate-10

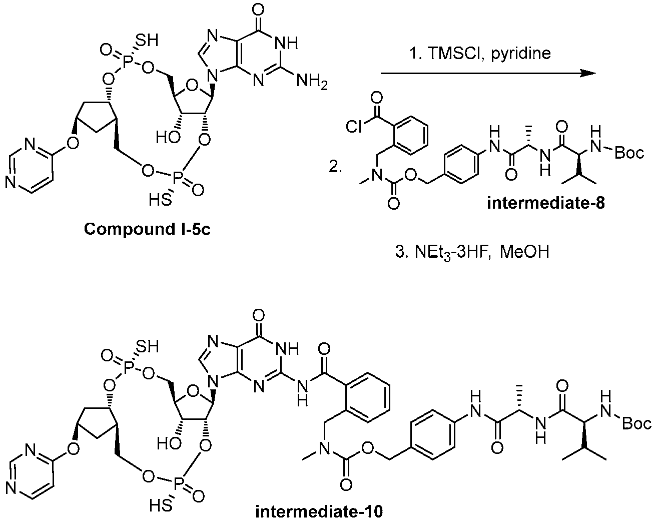

The title compound was prepared following the procedure described in Example A12, substituting 2-Amino-9-[(2*R*,5*R*,7*R*,8*R*,10*R*,12a*R*,14*R*,15a*S*,16*R*)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-1,9-dihydro-6H-purin-6-one as the TEA salt (Compound I-5c) (for synthesis see PCT/IB2018/058846) for Compound I-5c . The crude product was purified by reverse phase flash column chromatography (0-50% ACN in aqueous ammonium bicarbonate (5 mM)) to provide intermediate-10 as the ammonium salt. To convert salts, this material was then dissolved in MeOH (2 mL) and triethylamine (5 mL) was added. The mixture was shaken for 1 min. solvents removed and lyophilized overnight to provide intermediate-10 as TEA salt (147 mg, 48%). LCMS (AA): *m*/*z* = 1216.3 (M+H). ¹H NMR (400 MHz, MeOD) δ 8.70 (s, 1H), 8.43 (brs, 1H), 8.40 (d, *J* = 5.9 Hz, 1H), 7.70 (d, *J* = 7.5 Hz, 1H), 7.56 - 7.46 (m, 3H), 7.42 - 7.38 (m, 1H), 7.32 - 7.19 (m, 3H), 6.84 (d, *J* = 5.5 Hz, 1H), 6.15 (d, J = 7.3 Hz, 1H), 5.62 - 5.56 (m, 1H), 5.54 - 5.48 (m, 1H), 5.08 - 5.02 (m, 1H), 5.04 (s, 2H), 4.88 - 4.83 (m, 1H), 4.81 - 4.76 (m, 2H), 4.53 - 4.47 (m, 1H), 4.37 - 4.22 (m, 3H), 4.08 - 4.03 (m, 1H), 3.92 - 3.89 (m, 1H), 3.82 - 3.74 (m, 1H), 2.88 (s, 3H), 2.58 - 2.30 (m, 4H), 2.10 - 2.03 (m, 1H), 1.54 - 1.47 (m, 1H), 1.46 - 1.44 (m, 3H), 1.44 (s, 9H), 0.98 (d, *J* = 6.8 Hz, 3H), 0.93 (d, *J* = 6.8 Hz, 3H). ³¹P NMR (162 MHz, MeOD) δ 55.01 (s, 1P), 53.03 (s, 1P).

### Example A15

### (2S,5S,19R)-16-hydroxy-2-({4-[({[2-({9-[(2R,5R,7R,8R,10R,12aR,14R,15aS,16R)-16-hydroxy-2,10-dioxido-14-(pyrimidin-4-yloxy)-2,10-disulfanyldecahydro-5,8-methanocyclopenta[l][1,3,6,9,11,2,10]pentaoxadiphosphacyclotetradecin-7-yl]-6-oxo-6,9-dihydro-1H-purin-2-yl}carbamoyl)benzyl](methyl)carbamoyl}oxy)methyl]phenyl}carbamoyl)-5-isopropyl-16-oxido-4,7,11-trioxo-19-(stearoyloxy)-15,17-dioxa-3,6,12-triaza-161ambda~5~-phosphaicosan-20-yl octadecanoate, CP-5

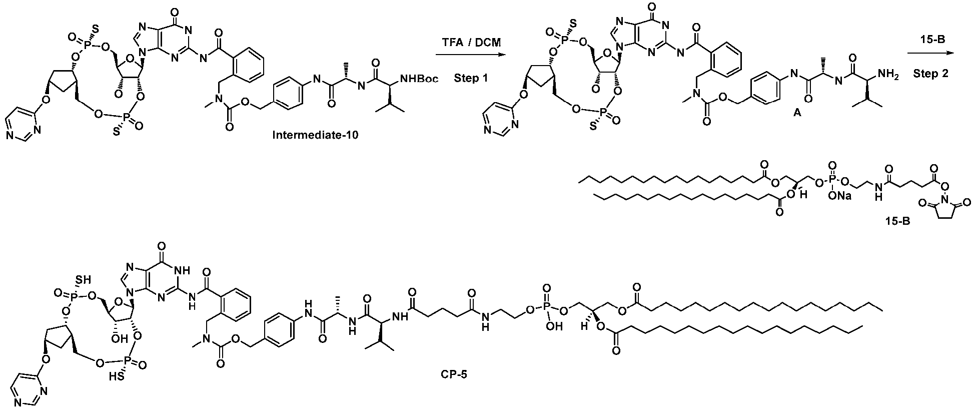

The title compound was prepared using similar procedure as shown for Example A13, starting with intermediate-10 instead of intermediate-9 to obtain CP-5 (96.0 mg, 48.8 % for 2 steps). LCMS (AA): *m*/*z* = 1959.8 (M+H). ¹H NMR (MeOD) δ 8.58 (s, 1H), 8.33 (s, 1H), 8.28 (d, *J* = 8.0 Hz, 1H), 7.58 (d, *J* = 8.0 Hz, 1H), 7.45-7.36 (m, 3H), 7.29 (t, *J* = 8.0 Hz, 1H), 7.14 (br, 3H), 6.72 (d, *J* = 8.0 Hz, 1H), 6.04 (d, *J* = 8.0 Hz, 1H), 5.48 (br, 1H), 5.38-5.33 (m, 1H), 5.13-5.08 (m, 1H), 4.94-4.89 (m, 3H), 4.71 (buried inside MeOD residue water peak, 1H ), 4.67 (m, 2H), 4.42-4.38 (m, 1H), 4.34-4.30 (m, 1H), 4.25-4.04 (m, 5H), 3.95-3.91 (m, 1H), 3.87 (t, *J* = 8.0 Hz, 2H), 3.82-3.78 (m, 2H), 3.69-3.62 (m, 1H), 3.29 (t, *J* = 4.0 Hz, 2H), 2.77 (s, 3H), 2.47-2.17 (m, 10H), 2.13 (t, *J* = 8.0 Hz, 2H), 2.05-1.97 (m, 1H), 1.82-1.77 (m, 2H), 1.53-1.43 (br, m, 4H), 1.41-1.38 (m, 1H), 1.34 (d, *J* = 8.0 Hz, 3H), 1.17 (br, 56H), 0.89-0.86 (m, 6H), 0.80-0.76 (m, 6H). ³¹P NMR (DMSO-d6) δ 55.29 (s, 1P), 53.05 (s, 1P), 0.29 (s, 1P). HR-MS (C₉₁H₁₄₁N₁₂O₂₅P₃S₂) Expected: 1959.8858, Observed: 1959.8886.

It is to be appreciated that the Detailed Description section, and not the Summary and Abstract sections, is intended to be used to interpret the claims. The Summary and Abstract sections may set forth one or more but not all exemplary embodiments of the present disclosure as contemplated by the inventor(s), and thus, are not intended to limit the present disclosure and the appended claims in any way.

The present disclosure has been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments will so fully reveal the general nature of the disclosure that others can, by applying knowledge within the skill of the art, readily modify and/or adapt for various applications such specific embodiments, without undue experimentation, without departing from the general concept of the present disclosure. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the present disclosure should not be limited by any of the above-described exemplary embodiments, but should be defined only in accordance with the following claims and their equivalents.

Aspects and features of the present disclosure are set out in the following numbered clauses which contain the subject matter of the claims of the parent application as filed.
1. A compound of formula (I): 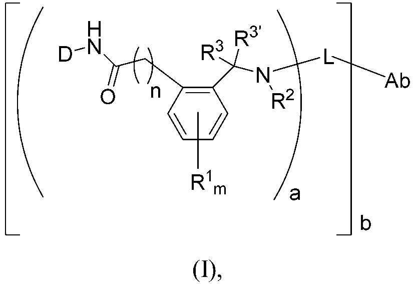 or a pharmaceutically acceptable salt thereof, wherein:
   a is an integer from 1 to 20;
   b is an integer from 1 to 20;
   m is 0, 1, 2, 3, or 4;
   n is 0 or 1;
   D-NH- is a portion of an amino-substituted compound, wherein the amino-substituted compound has the formula D-NH₂;
   each R¹ is independently selected from C₁-C₄alkyl, O-C₁-C₄alkyl, and halogen;
   R² is selected from C₁-C₄alkyl and -(CH₂CH₂O)ₛ-CH_{3;} wherein s is an integer from 1 to 10;
   R³ and R^{3'} are each independently selected from hydrogen and C₁-C₃alkyl;
   L is a cleavable linker; and
   Ab is an antibody, antibody fragment or an antigen-binding fragment.
2. A compound of clause 1, or a pharmaceutically acceptable salt thereof, wherein:
   a is an integer from 1 to 4;
   b is an integer from 1 to 10; and
   m is 0.
3. A compound of clause 1 or 2, or a pharmaceutically acceptable salt thereof, wherein:
   m is 0;
   n is 0; and
   R³ and R^{3'} are each hydrogen.
4. A compound of any one of clauses 1 to 3, or a pharmaceutically acceptable salt thereof, wherein L is 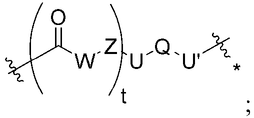 wherein:
   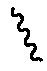 is the point of attachment to the nitrogen atom;
   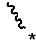 is the point of attachment to Ab;
   t is an integer from 1 and 10;
   W is absent or a self-immolative group;
   Z is absent or a peptide of 2 to 5 amino acids;
   U and U' are independently absent or a spacer; and
   Q is a heterobifunctional group;
   provided that W and Z are not both absent.
5. A compound of clause 4, or a pharmaceutically acceptable salt thereof, wherein W is a self-immolative group selected from 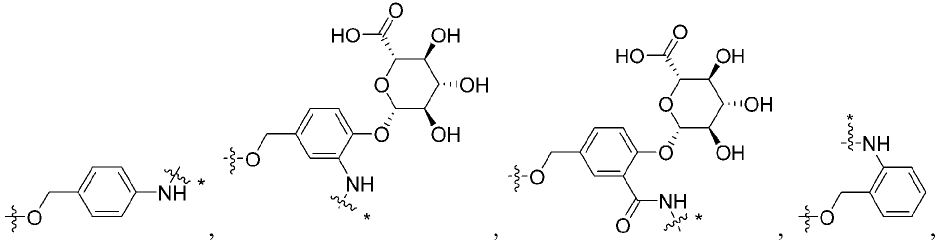 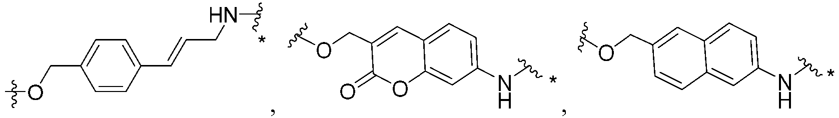 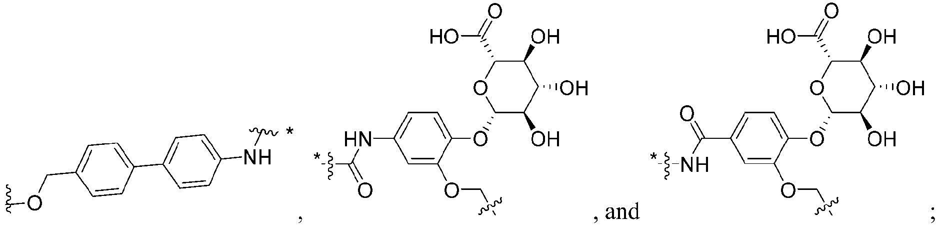 wherein:
   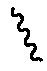 is the point of attachment to the carbonyl group; and
   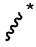 is the point of attachment to Z.
6. A compound of clause 5, or a pharmaceutically acceptable salt thereof, wherein W is selected from 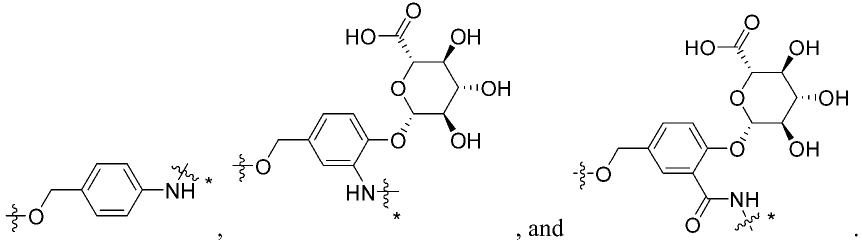
7. A compound of any one of clauses 4 to 6, or a pharmaceutically acceptable salt thereof, wherein Z is a peptide capable of being enzymatically cleaved.
8. A compound of clause 7, or a pharmaceutically acceptable salt thereof, wherein Z is cathepsin cleavable.
9. A compound of any one of clauses 4 to 8, or a pharmaceutically acceptable salt thereof, wherein Z is a two-amino acid peptide selected from Val-Cit, Cit-Val, Val-Ala, Ala-Val, Phe-Lys, and Lys-Phe.
10. A compound of any one of clauses 4 to 9, or a pharmaceutically acceptable salt thereof, wherein U and U' are independently selected from 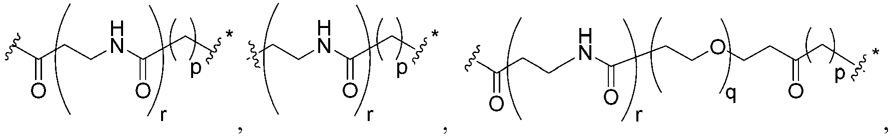 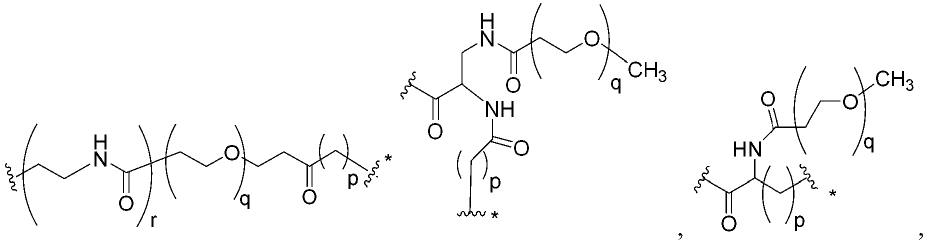 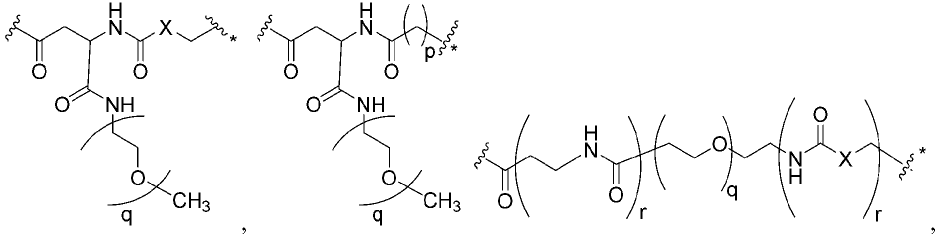 and 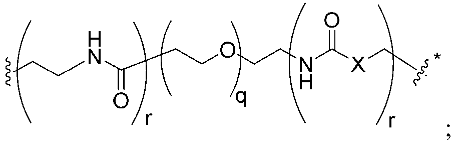 wherein:
   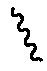 is the point of attachment to Z;
   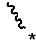 is the point of attachment to Q;
   p is an integer from 1 to 6;
   q is an integer from 1 to 20;
   X is O or -CH₂-; and
   each r is independently 0 or 1.
11. A compound of any one of clauses 4 to 10, or a pharmaceutically acceptable salt thereof, wherein Q is a heterobifunctional group which is attached to Ab through chemical or enzyme-mediated conjugation.
12. A compound of clause 11, or a pharmaceutically acceptable salt thereof, wherein Q is selected from 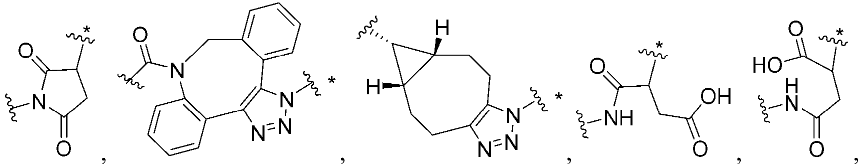 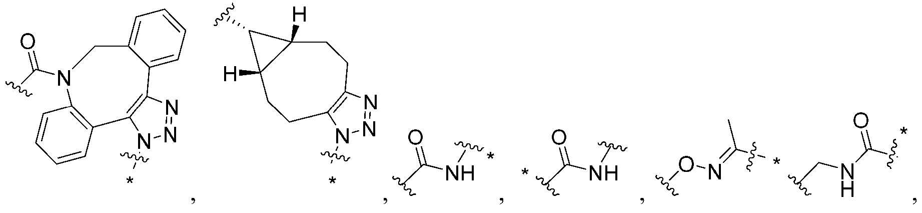 and 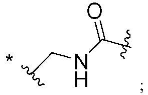 wherein
   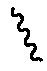 is the point of attachment to U or, when U is absent, the point of attachment to Z; and
   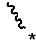 is the point of attachment to U', or, when U' is absent, the point of attachment to Ab.
13. A compound of clause 4, wherein
   t is 1;
   W is absent or a self-immolative group; and
   Z is absent or a peptide of 2 amino acids.
14. A compound of any one of clauses 1 to 13, wherein R² is -CH₃, or -(CH₂CH₂O)ₛ-CH₃ and s is an integer from 1 to 10.
15. A compound of any one of clauses 1 to 14, wherein D-NH₂ is an amino-substituted compound that modulates STING activity.
16. A compound of clause 15, wherein the amino-substituted compound that modulates STING activity comprises a guanine or an adenine derivative.
17. A compound of clause 15, wherein the amino-substituted compound that modulates STING activity is a compound of formula (II): 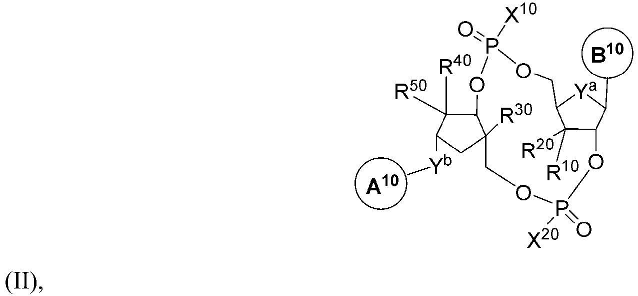 or a pharmaceutically acceptable salt thereof, wherein:
   X¹⁰ is SH or OH;
   X²⁰ is SH or OH;
   Y^{a} is O, S, or CH₂;
   Y^{b} is O, S, NH, or NR^{a}, wherein R^{a} is C₁-C₄alkyl;
   R¹⁰ is hydrogen, fluoro, OH, NH₂, OR^{b}, or NHR^{b};
   R²⁰ is hydrogen or fluoro;
   R³⁰ is hydrogen; R⁴⁰ is hydrogen, fluoro, OH, NH₂, OR^{b}, or NHR^{b}; or R³⁰ and R⁴⁰ are taken together to form CH₂O;
   R⁵⁰ is hydrogen or fluoro;
   R^{d} is C₁-C₆alkyl, halo(C₁-C₆)alkyl, or C₃-C₆cycloalkyl;
   Ring A¹⁰ is an optionally substituted 5- or 6-membered monocyclic heteroaryl ring containing 1-4 heteroatoms selected from N, O, or S, or an optionally substituted 9 or 10 membered bicyclic heteroaryl ring containing 1-5 heteroatoms selected from N, O, or S; wherein ring A¹⁰ comprises at least one N atom in the ring, and wherein Y^{b} is attached to a carbon atom of ring A¹⁰; and
   Ring B¹⁰ is an optionally substituted 9 or 10-membered bicyclic heteroaryl ring containing from 2 to 5 heteroatoms selected from N, O, or S; wherein ring B¹⁰ comprises at least two N atoms in the ring;
   provided that either ring A¹⁰ or ring B¹⁰ is attached to the carbonyl group of formula (I) through an -NH- group.
18. A compound of any one of clauses 15 to 17, wherein the amino-substituted compound that modulates STING activity is 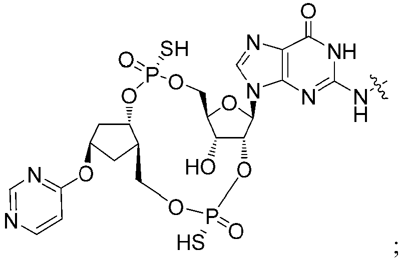 or a pharmaceutically acceptable salt thereof, wherein 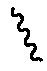 is the point of attachment to the carbonyl group of formula (I).
19. A compound of clause 15, wherein the amino-substituted compound that modulates STING activity is a compound of formula (III): 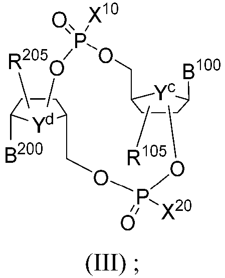 or a pharmaceutically acceptable salt thereof; wherein
   X¹⁰ is SH or OH;
   X²⁰ is SH or OH;
   Y^{c} is O, S, or CH₂;
   Y^{d} is O, S, or CH₂;
   B¹⁰⁰ is a group represented by formula (**B¹-A**) or formula (**B¹-B**): 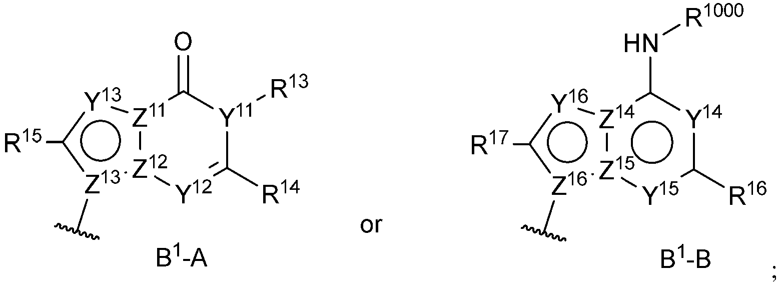
   R¹³, R¹⁴, R¹⁵, R¹⁶ and R¹⁷ are each independently a hydrogen atom or a substituent;
   R¹⁰⁰⁰ is hydrogen or a bond to the carbonyl group of formula (I);
   Y¹¹, Y¹², Y¹³, Y¹⁴, Y¹⁵ and Y¹⁶ are each independently N or CR^{1a}, wherein R^{1a} is hydrogen or a substituent;
   Z¹¹, Z¹², Z¹³, Z¹⁴, Z¹⁵ and Z¹⁶ are each independently N or C;
   R¹⁰⁵ is a hydrogen atom or a substituent;
   B²⁰⁰ is a group represented by formula (**B²-A**) or formula (**B²-B**): 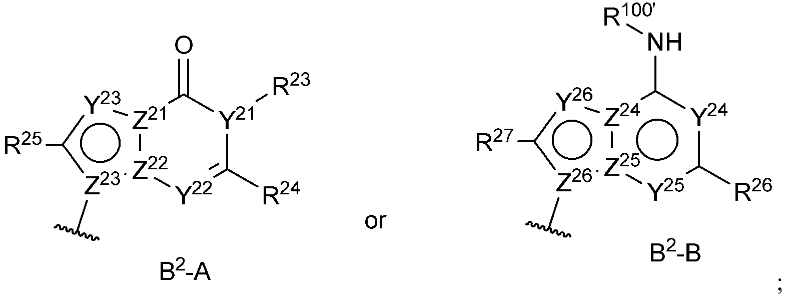
   R²³, R²⁴, R²⁵, R²⁶ and R²⁷ are each independently a hydrogen atom or a substituent;
   R^{100'} is hydrogen or a bond to the carbonyl group of formula (I);
   Y²¹, Y²², Y²³, Y²⁴, Y²⁵ and Y²⁶ are each independently N or CR^{2a}, wherein R^{2a} is hydrogen or a substituent;
   Z²¹, Z²², Z²³, Z²⁴, Z²⁵ and Z²⁶ are each independently N or C; and
   R²⁰⁵ is a hydrogen atom or a substituent; wherein R¹⁰⁵ and R²⁰⁵ are each independently attached to 2- or 3-position of the 5-membered ring they are attached to respectively;
   provided that:
      one of B¹⁰⁰ or B²⁰⁰ is attached to the carbonyl group of formula (I) through an -NH- group.
20. A compound of clause 19, or a pharmaceutically acceptable salt thereof, of formula (IIIa): 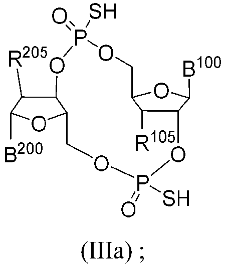 or a pharmaceutically acceptable salt thereof; wherein
   B¹⁰⁰ is a group represented by formula (**B¹-A**) or formula (**B¹-B**): 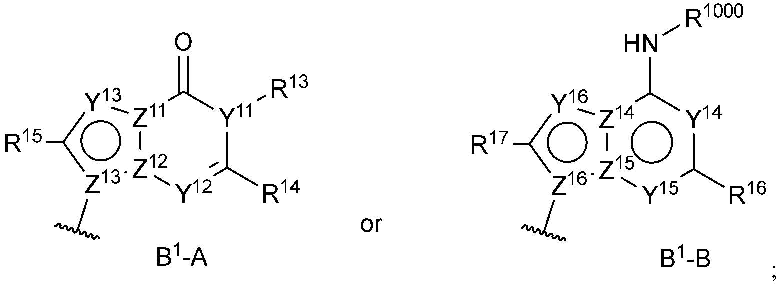
   R¹³, R¹⁴, R¹⁵, R¹⁶ and R¹⁷ are each independently a hydrogen atom or a substituent;
   R¹⁰⁰⁰ is hydrogen or a bond to the carbonyl group of formula (I);
   Y¹¹, Y¹², Y¹³, Y¹⁴, Y¹⁵ and Y¹⁶ are each independently N or CR^{1a}, wherein R^{1a} is hydrogen or a substituent;
   Z¹¹, Z¹², Z¹³, Z¹⁴, Z¹⁵ and Z¹⁶ are each independently N or C;
   R¹⁰⁵ is a hydrogen atom or a substituent;
   B²⁰⁰ is a group represented by formula (**B²-A**) or formula (**B²-B**): 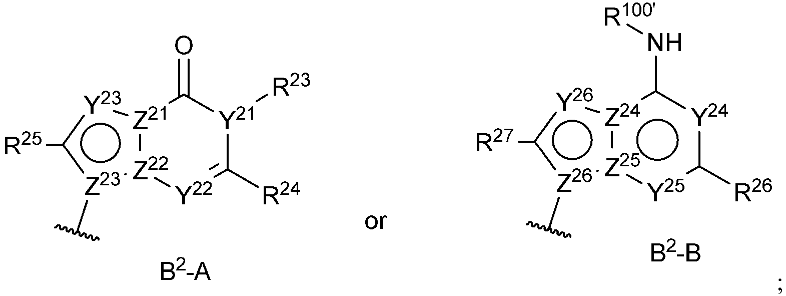
   R²³, R²⁴, R²⁵, R²⁶ and R²⁷ are each independently a hydrogen atom or a substituent;
   R^{100'} is hydrogen or a bond to the carbonyl group of formula (I);
   Y²¹, Y²², Y²³, Y²⁴, Y²⁵ and Y²⁶ are each independently N or CR^{2a}, wherein R^{2a} is hydrogen or a substituent;
   Z²¹, Z²², Z²³, Z²⁴, Z²⁵ and Z²⁶ are each independently N or C; and
   R²⁰⁵ is a hydrogen atom or a substituent; wherein R¹⁰⁵ and R²⁰⁵ are each independently attached to 2- or 3-position of the 5-membered ring they are attached to respectively;
   provided that:
      one of B¹⁰⁰ or B²⁰⁰ is: 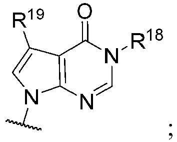
   wherein:
      R¹⁸ is hydrogen or C₁₋₆ alkyl; and
      R¹⁹ is a halogen atom;
      and the other is attached to the carbonyl group of formula (I) through an -NH- group.
21. A compound of clause 19 or 20 wherein the amino-substituted compound that modulates STING activity is: 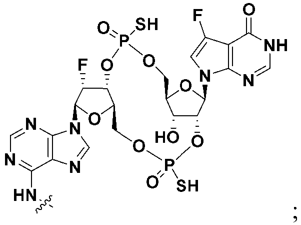 or a pharmaceutically acceptable salt thereof, wherein 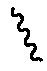 is the point of attachment to the carbonyl group of formula (I).
22. A compound of formula (IV): 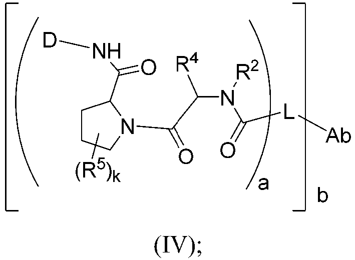 or a pharmaceutically acceptable salt thereof, wherein:
   a is an integer from 1 to 20;
   b is an integer from 1 to 20;
   k is 0, 1, 2, or 3;
   m is 0, 1, 2, 3, or 4;
   D-NH- is a portion of an amino-substituted compound, where the amino-substituted compound has the formula D-NH₂;
   R² is selected from H, C₁-C₄alkyl and -(CH₂CH₂O)ₛ-CH_{3;} wherein s is an integer from 1 to 10;
   R⁴ is selected from hydrogen and any naturally occurring amino acid side chain;
   R⁵ is selected from C₁-C₄alkyl, and O-C₁-C₄alkyl;
   L is a cleavable linker; and
   Ab is an antibody, antibody fragment or an antigen-binding fragment.
23. A compound of clause 22, or a pharmaceutically acceptable salt thereof, wherein L is 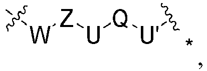 wherein:
   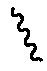 is the point of attachment to the carbonyl group;
   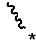 is the point of attachment to Ab;
   W is a self-immolative group;
   Z is absent or a peptide of 2 to 5 amino acids; and
   U and U' are independently absent or a spacer; and
   Q is a heterobifunctional group.
24. A compound of formula (V): 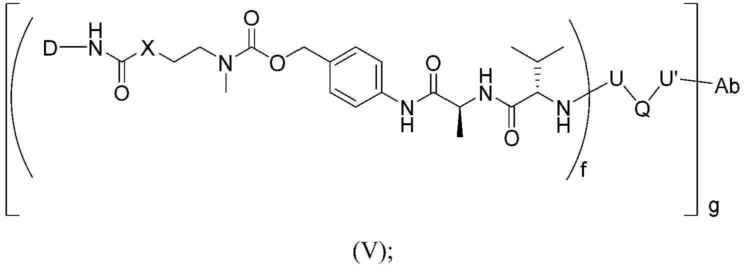 or a pharmaceutically acceptable salt thereof, wherein:
   D-NH- is 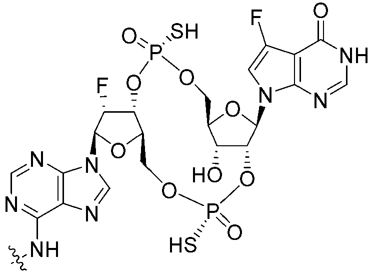 or 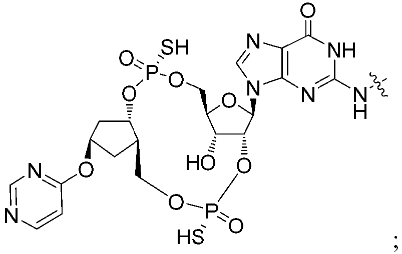
   X is O or CH₂;
   f is an integer from 1 to 10;
   g is an integer from 1 to 20;
   U and U' are independently absent or a spacer;
   Q is a heterobifunctional group; and
   Ab is an antibody, antibody fragment or an antigen-binding fragment.
25. A compound of clause 24, wherein X is O.
26. A compound of formula (VI): 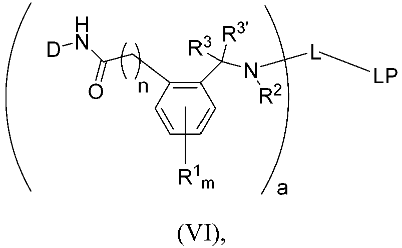 or a pharmaceutically acceptable salt thereof, wherein:
   a is an integer from 1 to 20;
   m is 0, 1, 2, 3, or 4;
   n is 0 or 1;
   D-NH- is a portion of an amino-substituted compound, wherein the amino-substituted compound has the formula D-NH₂;
   each R¹ is independently selected from C₁-C₄alkyl, O-C₁-C₄alkyl, and halogen;
   R² is selected from C₁-C₄alkyl and -(CH₂CH₂O)ₛ-CH_{3;} wherein s is an integer from 1 to 10;
   R³ and R^{3'} are each independently selected from hydrogen and C₁-C₃alkyl;
   L is a cleavable linker; and
   LP is a lipid.
27. The compound of clause 26, wherein the lipid is cholesterol or a phospholipid.
28. The compound of clause 27, wherein the lipid is a phospholipid.
29. The compound of clause 28, wherein the phospholipid is selected from a phosphatidylcholine, a phosphatidylgycerol, a phosphatidic acid, a phosphatidylethanolamine, a phosphatidylserine, a sphingomyelin, a soybean phospholipid, and an egg yolk phospholipid.
30. The compound of clause 28, wherein the phospholipid is a phosphatidylethanolamine, wherein the phosphadtidylethanolamine is 1,2-distearoyl-sn-glycero-3-phosphorylethanolamine.
31. A lipid complex comprising a compound of any one of clauses 26 to 30.
32. The lipid complex of clause 31, wherein the lipid complex is in the form of a liposome.
33. The lipid complex of clause 31 or 32, further comprising one or more phospholipids.
34. The lipid complex of clause 33, wherein the one or more phospholipids are selected from a phosphatidylcholine, a phosphatidylgycerol, a phosphatidic acid, a phosphatidylethanolamine, a phosphatidylserine, a sphingomyelin, a soybean phospholipid, and an egg yolk phospholipid.
35. The lipid complex of clause 34, wherein the phospholipid is a phosphatidylcholine, wherein the phosphatidylcholine is 1,2-distearoyl-sn-glycero-3-phosphocholine.
36. The lipid complex of any one of clauses 31 to 35, which further comprises a fatty alcohol.
37. The lipid complex of any one of clauses 31 to 36, which further comprises cholesterol.
38. The lipid complex of any one of clauses 31 to 37, which further comprises at least one PEGylated lipid.
39. The lipid complex of clause 38, wherein the PEGylated lipid is selected from a PEGylated phospholipid and PEGylated diacylglycerol.
40. The liposome of clause 39, wherein the PEGylated lipid is N-(carbonyl-methoxypolyethyleneglycol 2000)-1,2-distearoyl-sn-glycero-3-phosphoethanolamine.
41. A pharmaceutical composition comprising a compound of any one of clauses 1 to 40, or a pharmaceutically acceptable salt thereof, and one or more pharmaceutically acceptable carriers.
42. A method of treating cancer in a subject in need thereof, the method comprising administering to the subject a pharmaceutically acceptable amount of a compound of any of clauses 1 to 40.
43. A method for stimulating an immune response in a subject in need thereof, the method comprising administering to the subject a pharmaceutically acceptable amount of a compound, lipid complex, or composition of any one of clauses 1 to 41.

## Claims

1. A compound of formula (XX): 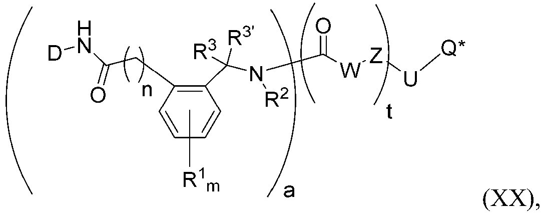 or a pharmaceutically acceptable salt thereof, wherein:
a is an integer from 1 to 20;
m is 0, 1, 2, 3, or 4;
n is 0 or 1;
t is an integer from 1 to 10;
D-NH- is a portion of an amino-substituted compound, wherein the amino-substituted compound has the formula D-NH₂;
each R¹ is independently selected from C₁-C₄alkyl, O-C₁-C₄alkyl, and halogen;
R² is selected from C₁-C₄alkyl and -(CH₂CH₂O)ₛ-CH_{3;} wherein s is an integer from 1 to 10;
R³ and R^{3'} are each independently selected from hydrogen and C₁-C₃alkyl;
W is selected from: 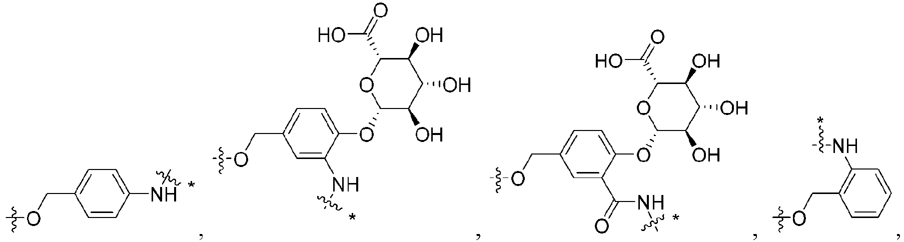 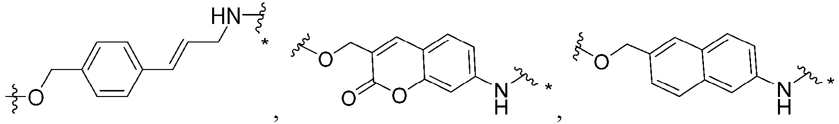 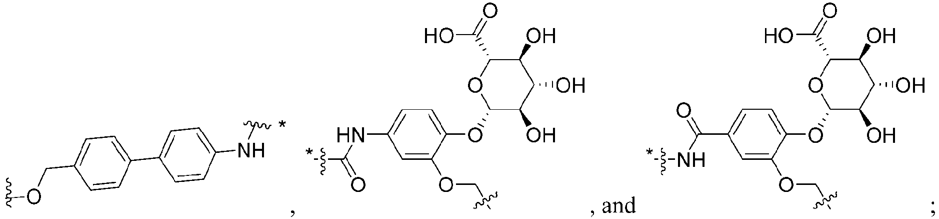
wherein:
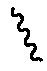 is the point of attachment to the carbonyl group; and
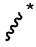 is the point of attachment to Z;
Z is absent or a peptide of 2 to 5 amino acids;
U is selected from: 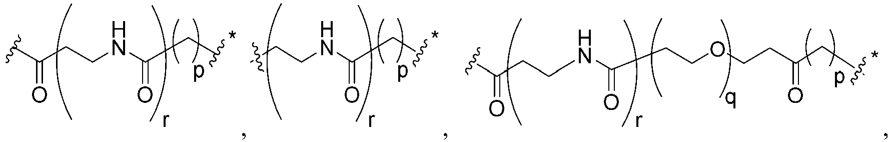 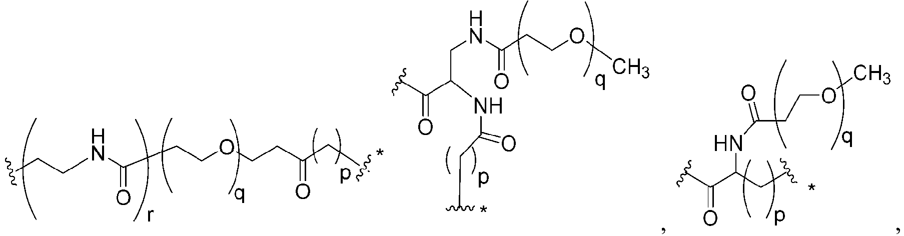 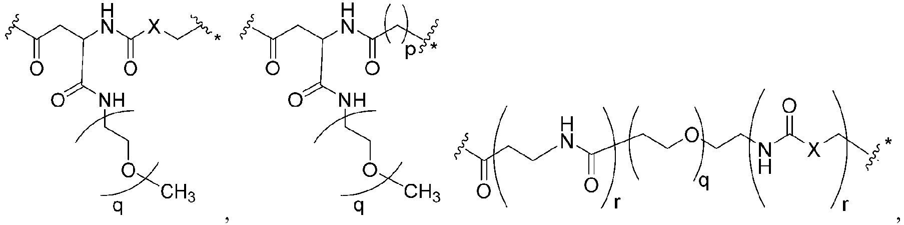 and 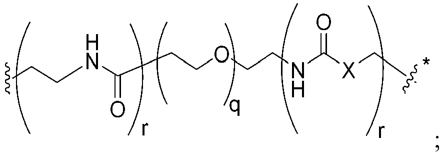 wherein:
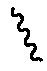 is the point of attachment to Z;
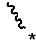 is the point of attachment to Q*;
p is an integer from 1 to 6;
q is an integer from 1 to 20;
X is O or -CH₂-;
each r is independently 0 or 1; and
Q* is reactive functional group capable of conjugating to an antibody, antibody fragment, or antigen-binding fragment.

2. A compound of claim 1, wherein Q* is maleimide or a cyclooctyne.

3. A compound of claim 1, wherein Q* is selected from: 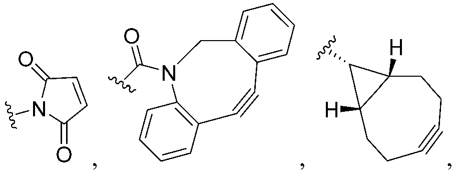 and -NH₂.

4. A compound of any one of claims 1-3, wherein Z is absent or a peptide of 2 amino acids.

5. A compound of claim 1, wherein D-NH is selected from:

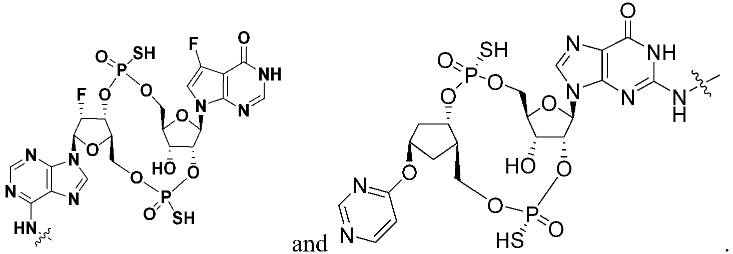

6. A compound of claim 5 selected from: 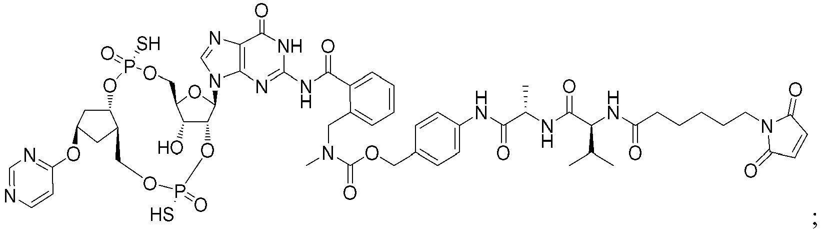 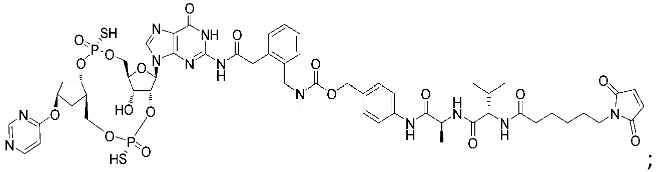 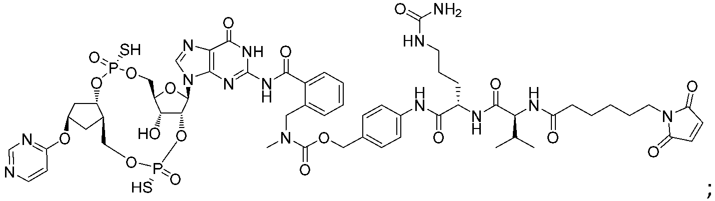 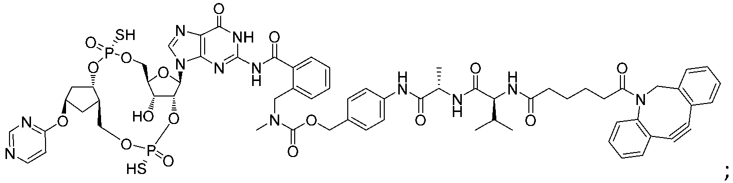 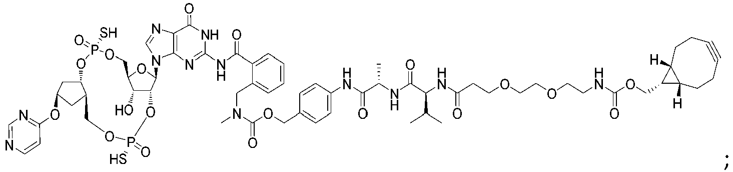 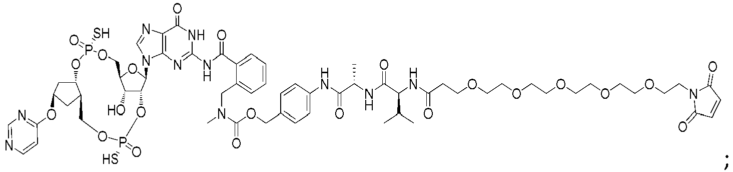 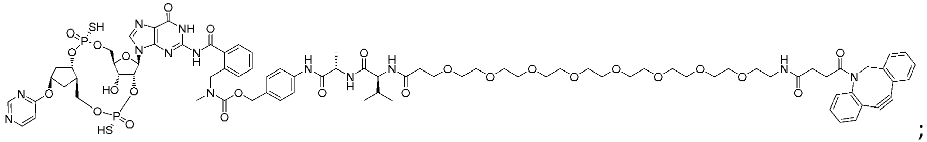 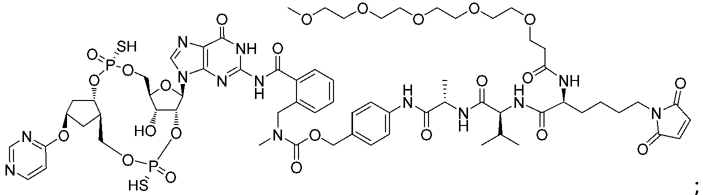 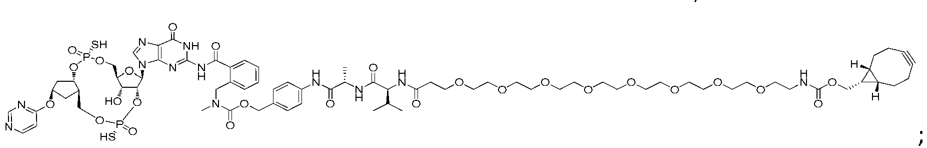 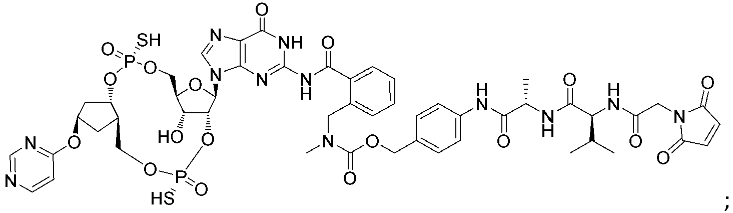 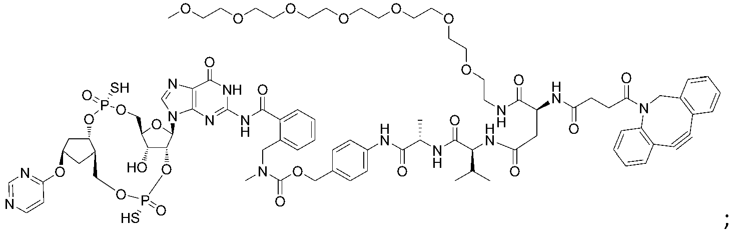 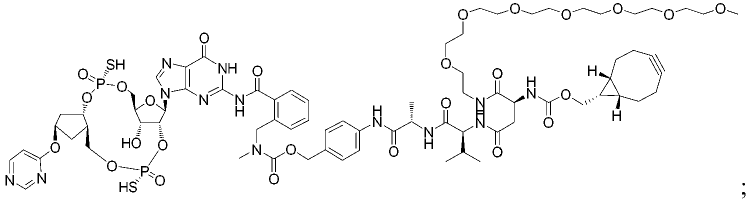 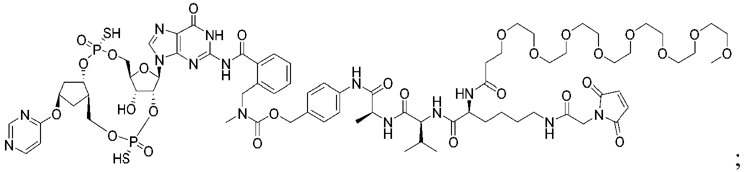 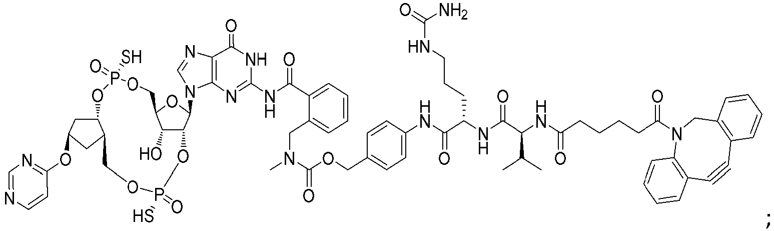 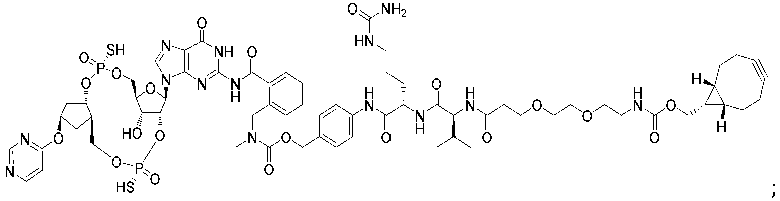 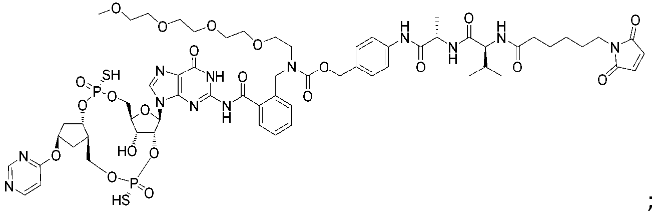 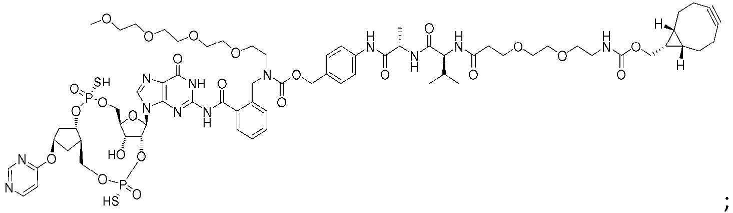 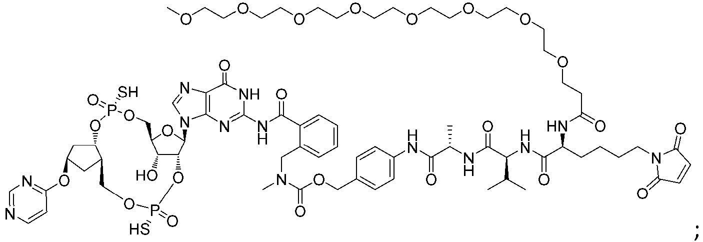 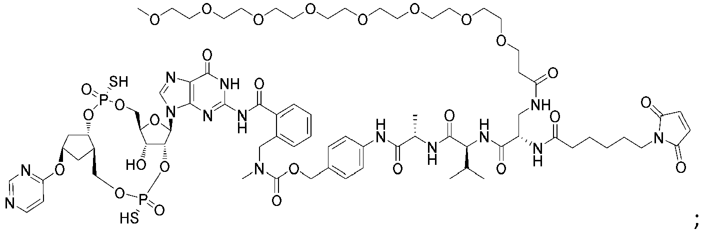 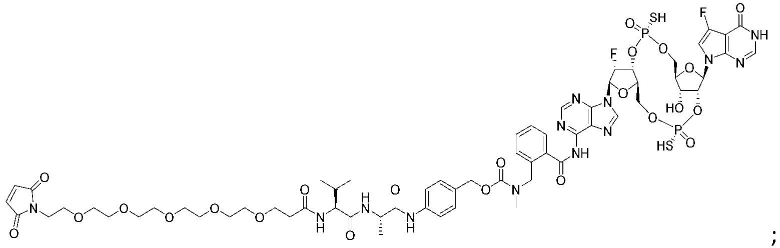 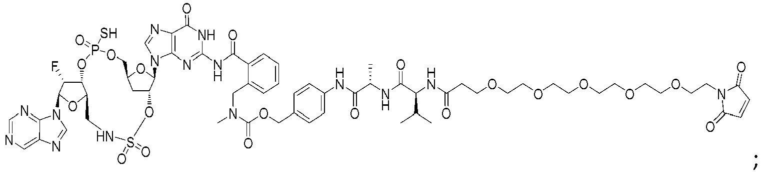 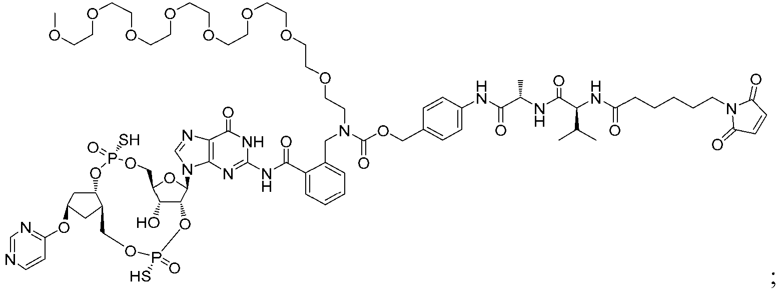 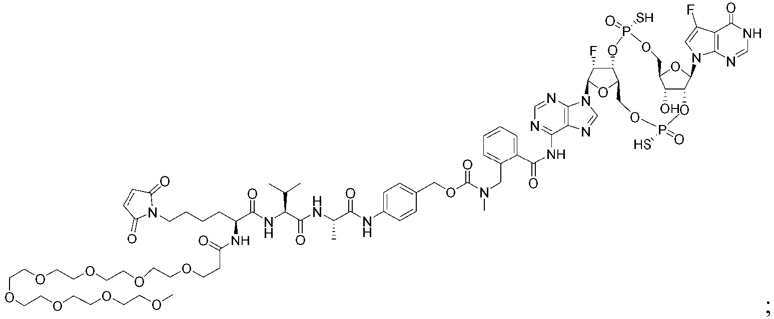 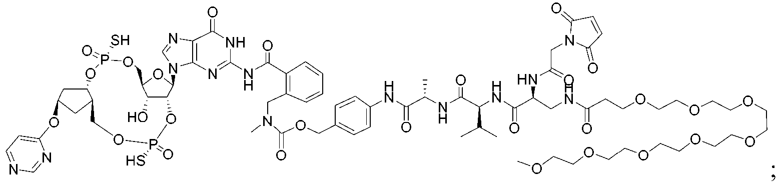 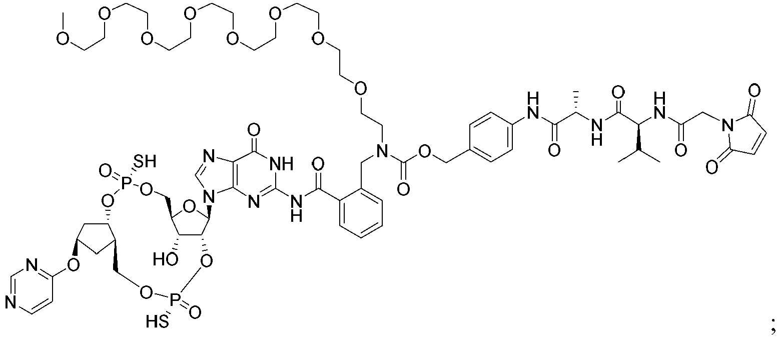 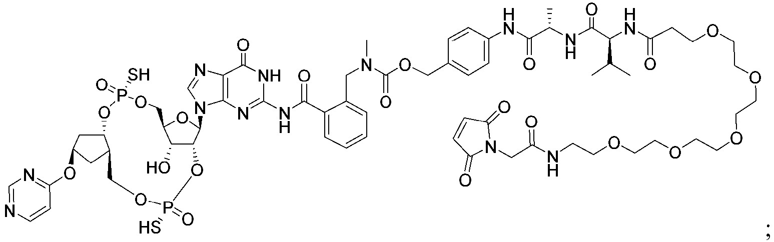 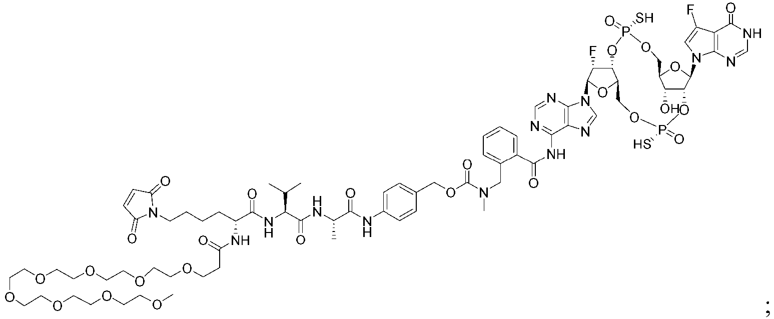 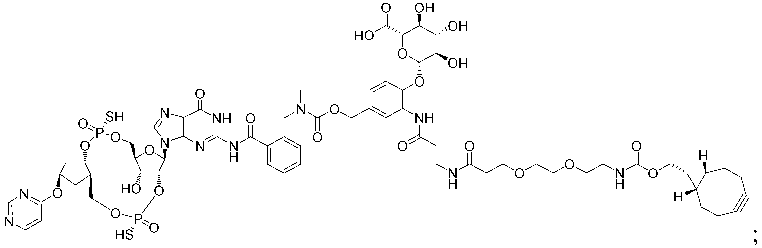 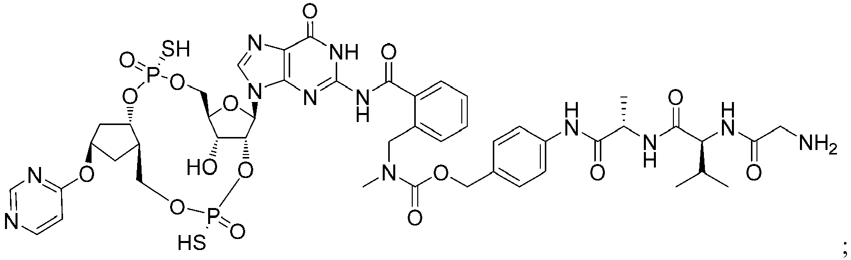 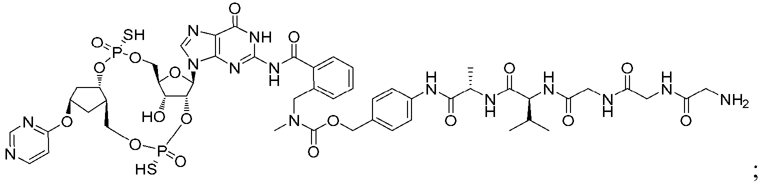 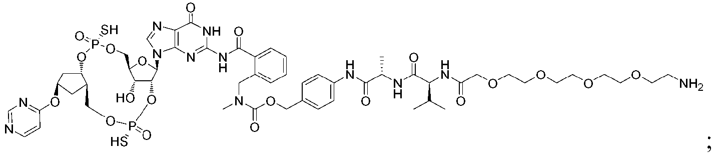 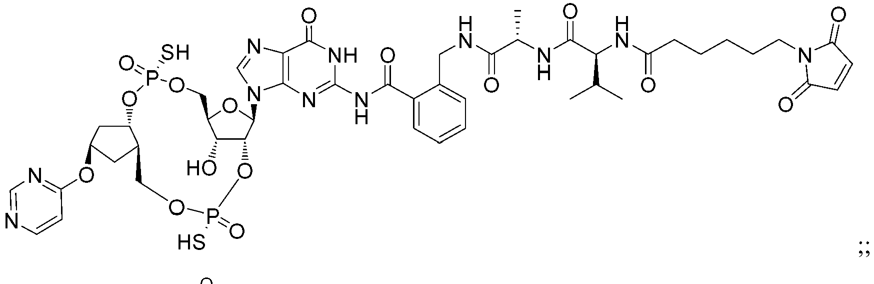 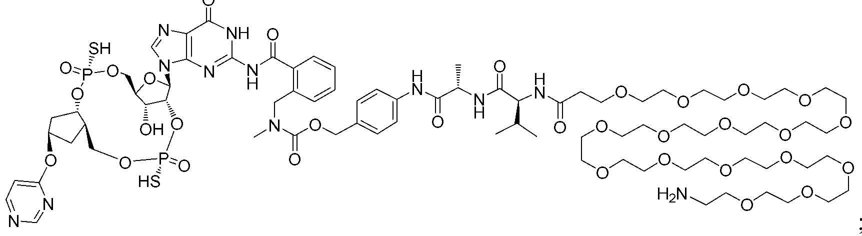 and

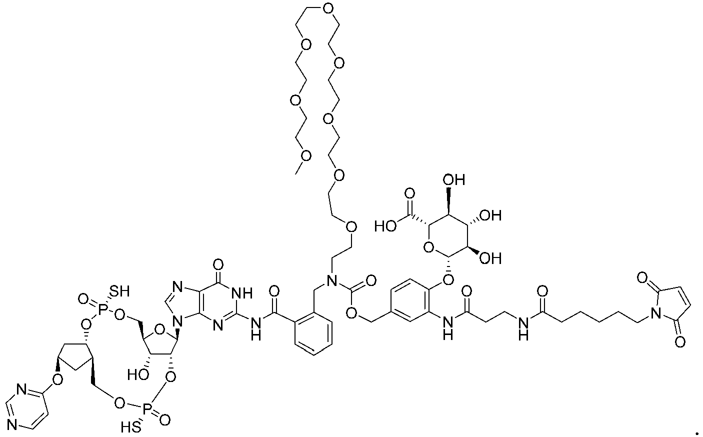

7. A compound of claim 6 selected from: 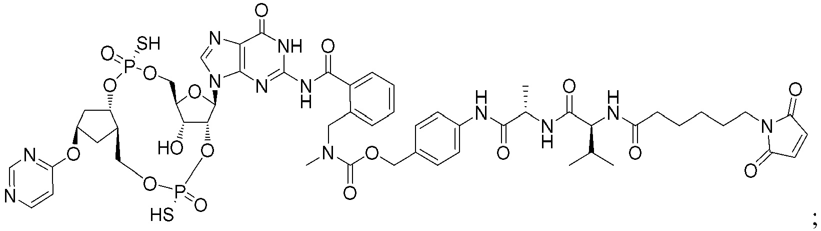 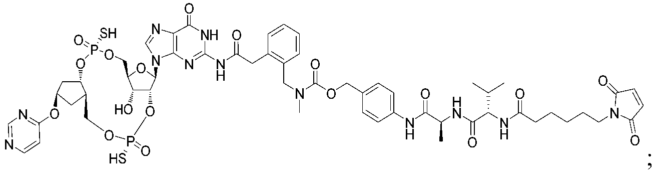 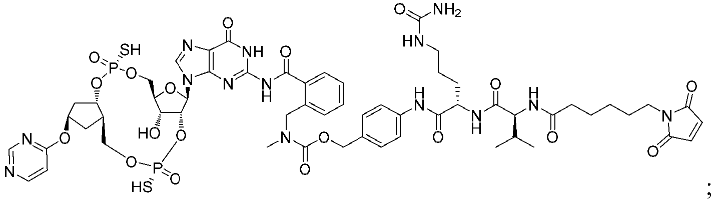 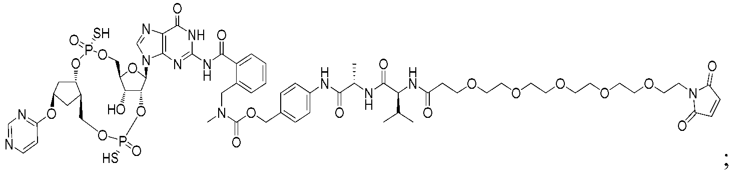 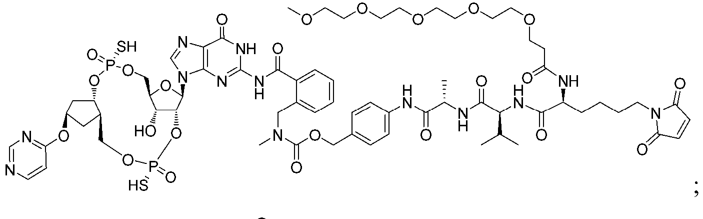 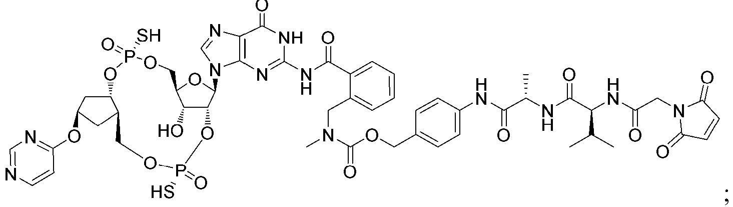 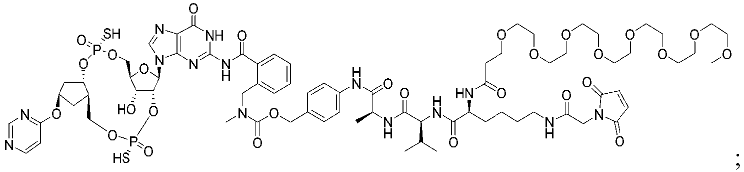 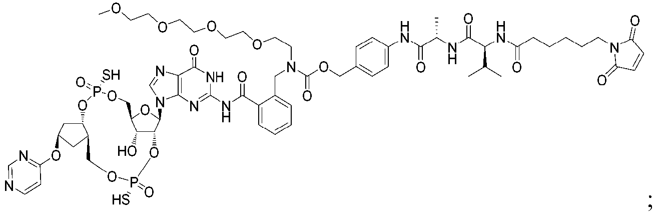 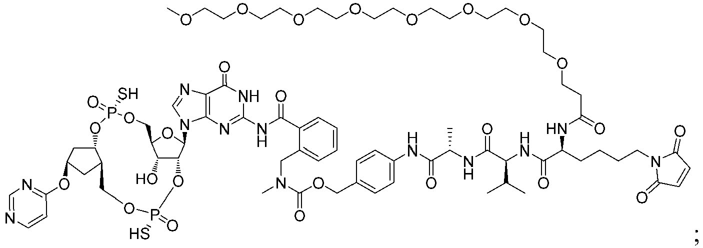 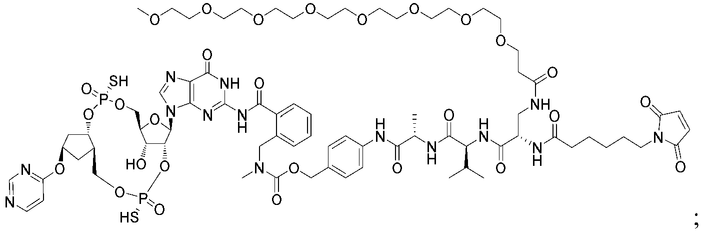 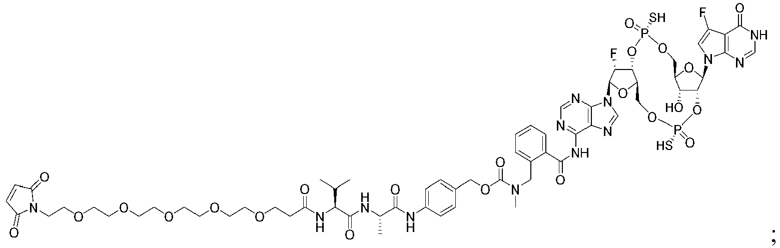 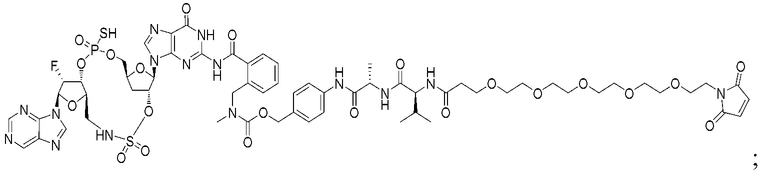 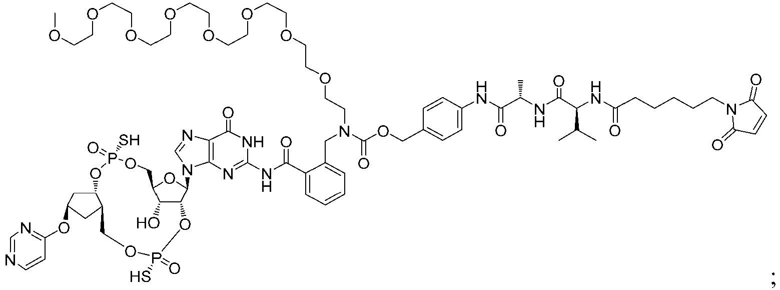 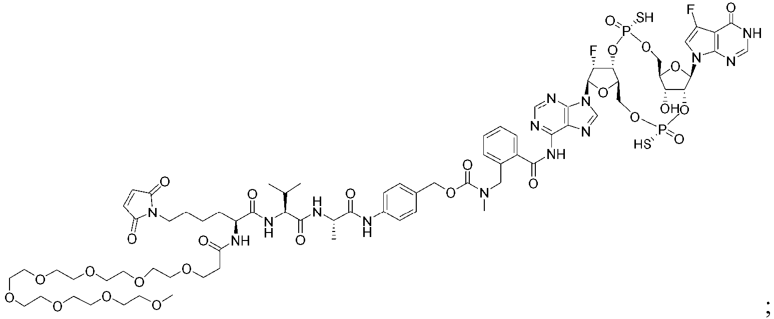 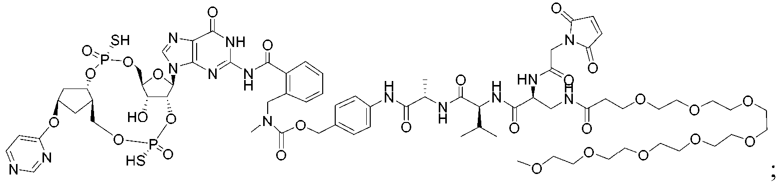 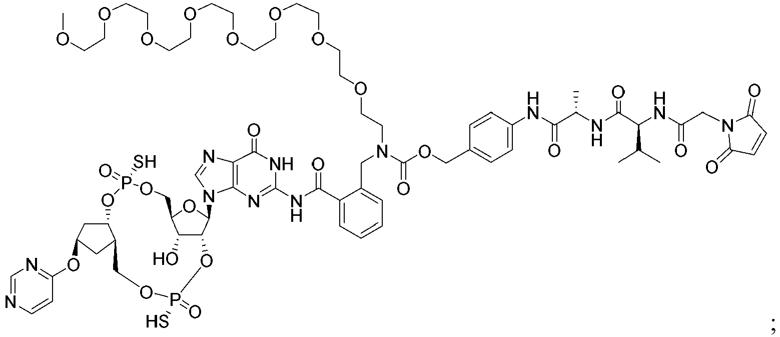 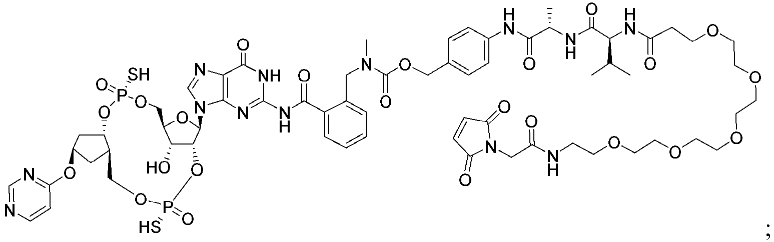 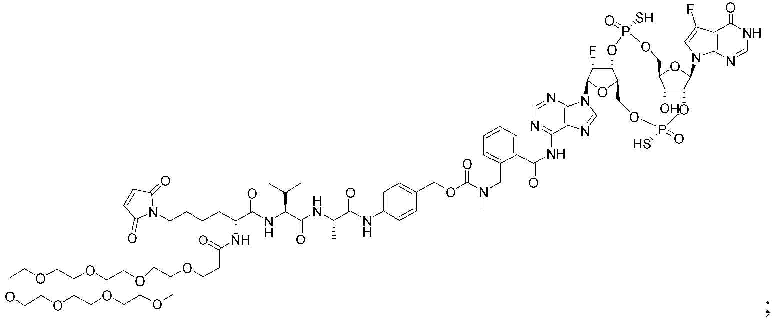 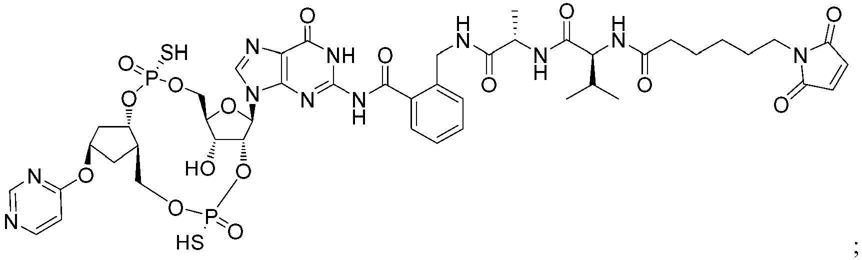 and

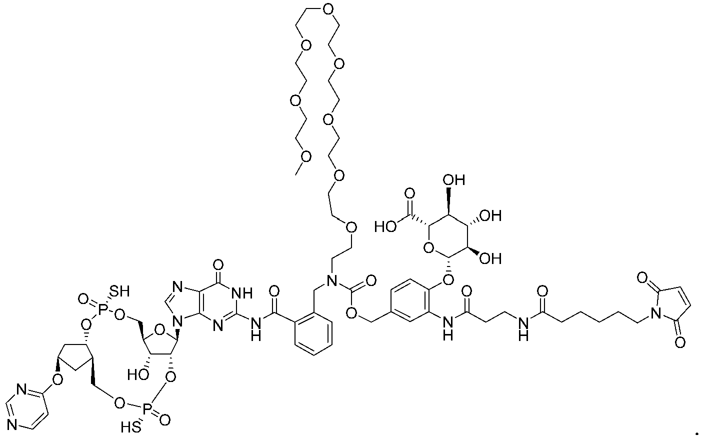

8. A compound of claim 6 selected from: 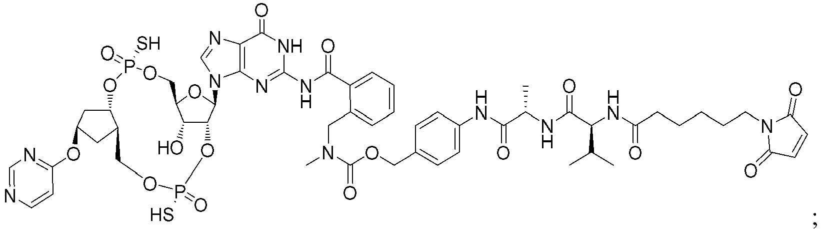 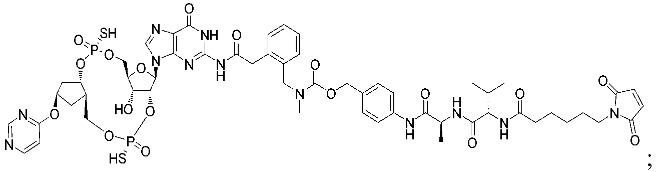 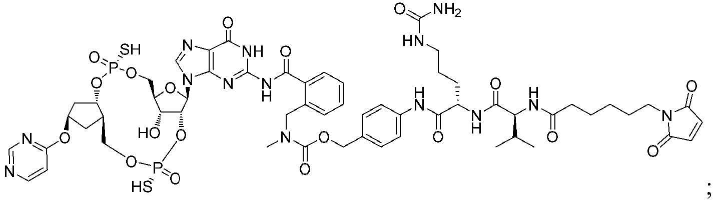 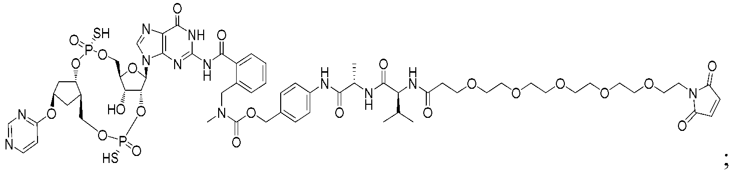 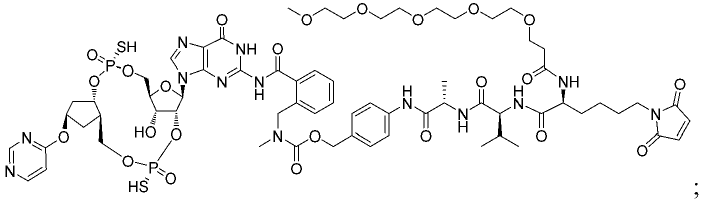 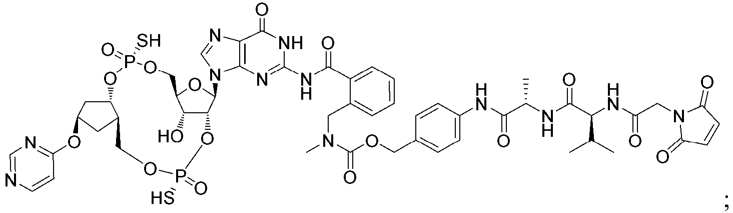 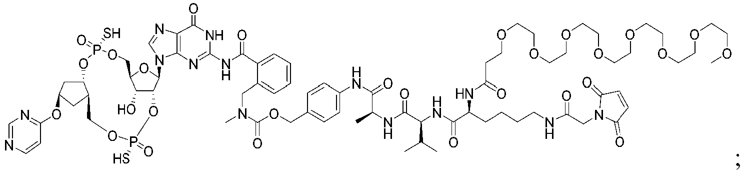 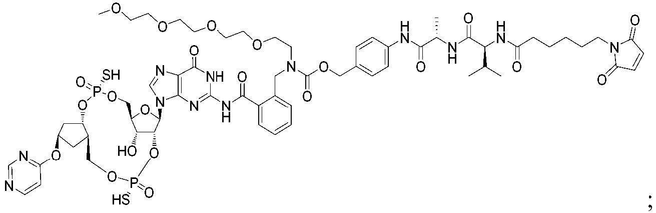 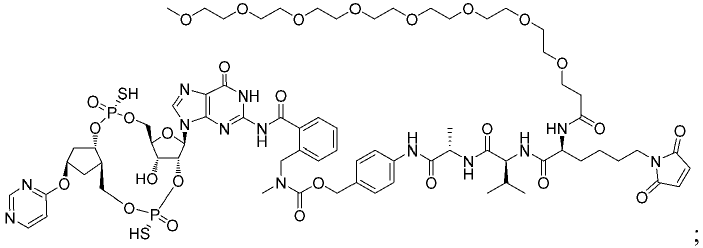 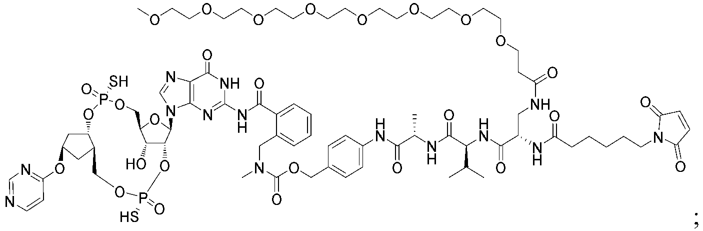 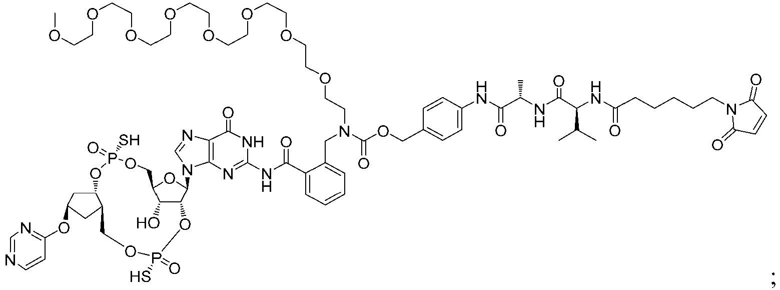 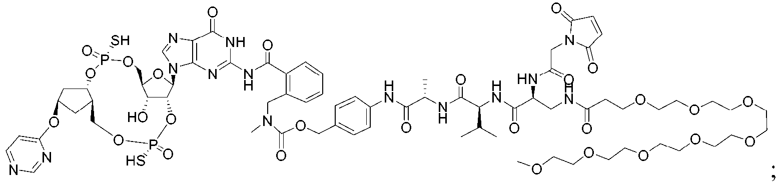 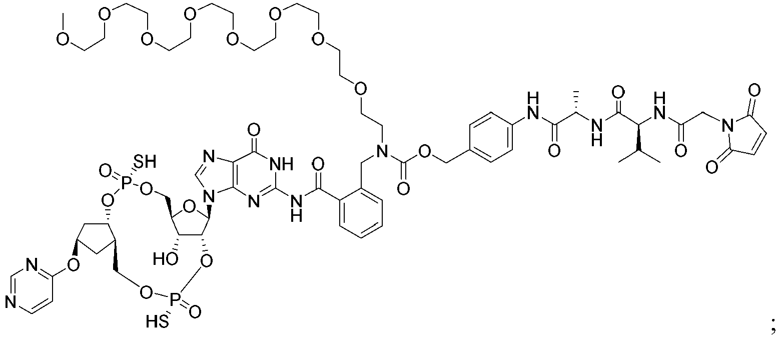 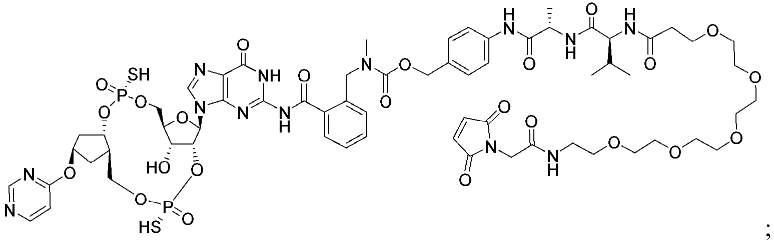 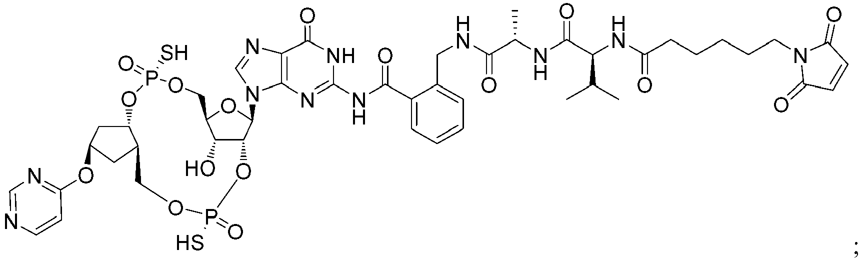 and

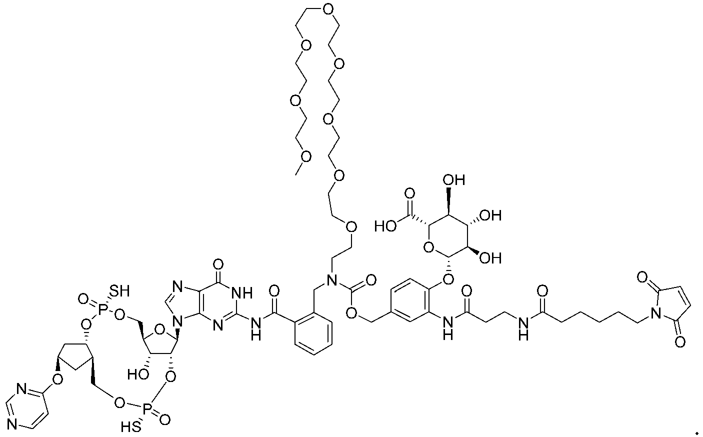

9. A compound of claim 6 selected from: 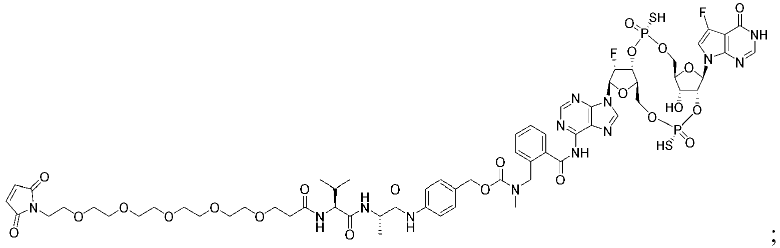 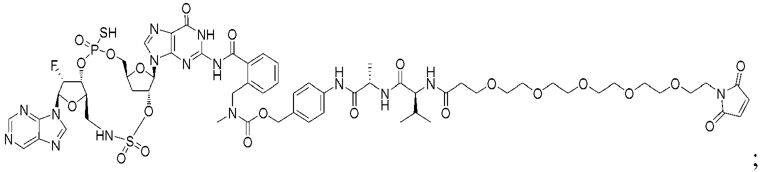 and

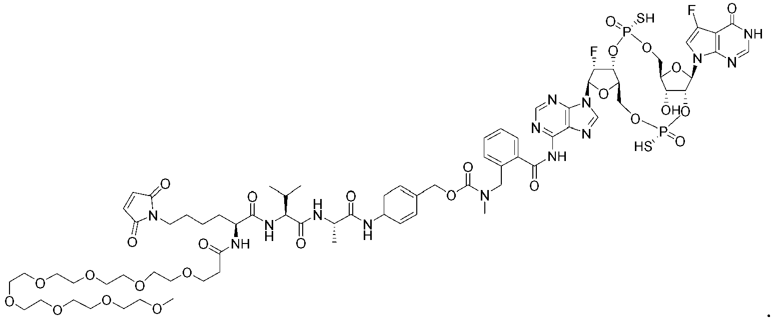

10. A compound of claim 6 having the formula:

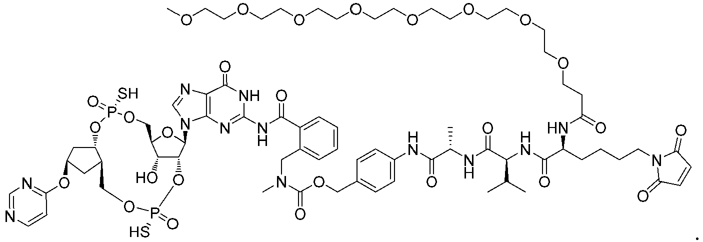

11. A compound of claim 6 having the formula:

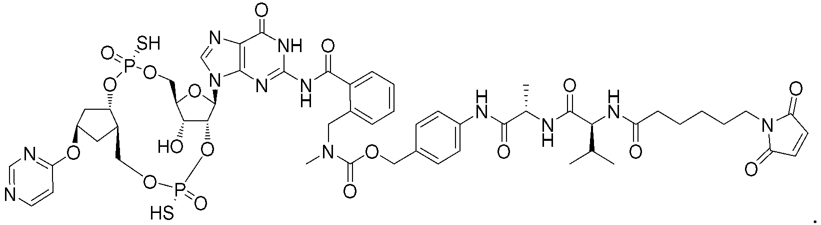

12. A compound of claim 6 having the formula:

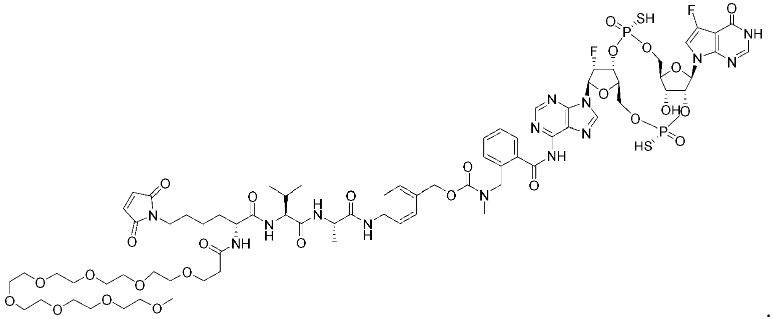

13. A compound of claim 6 having the formula:

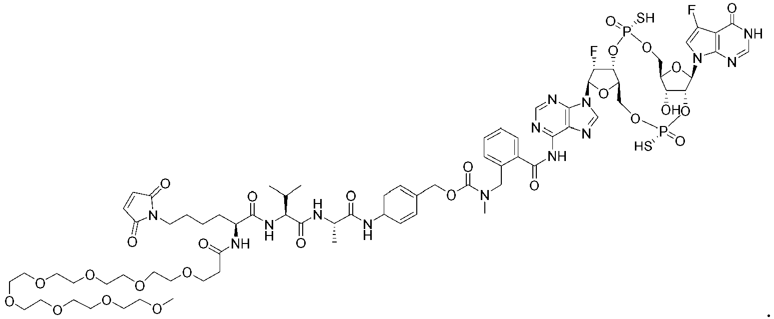

14. A compound of claim 6 having the formula:
